# EUROPEAN PATENT APPLICATION

(11) **EP 2 857 416 A1**
(43) Date of publication of application: **08.04.2015**
(21) Application number: 13187133.7
(22) Date of filing: 02.10.2013
(51) Int. Cl.: C07K 14/435

(54) **Non-ribosomal protein synthesis pigment fusion peptides**

(71) Applicant: Deutsches Krebsforschungszentrum, 69120 Heidelberg (DE); Ruprecht-Karls-Universität Heidelberg, 69117 Heidelberg (DE)
(72) Inventor: The designation of the inventor has not yet been filed
(74) Representative: Dick, Alexander

(57) **Abstract**

The present invention relates to a polypeptide or polypeptide complex, comprising at least one non-ribosomal peptide synthesis (NRPS) amino acid module functionally connected to at least one pigment module. The present invention further relates to a labeled oligopeptide comprising a non-naturally attached NRPS pigment or/and polyketide pigment, to a polynucleotide encoding a fusion polypeptide, a vector, preferably an expression vector, comprising the polynucleotide of the present invention and to a host cell comprising the polypeptide or polypeptide complex and/or the polynucleotide, and/or the vector according to the present invention. Moreover, the present invention relates to in vitro and in vivo method of producing a labeled oligopeptide, as well as to methods of optimizing the same.

SEQUENCE LISTING

<110>  Deutsches Krebsforschungszentrum

<120>  non-ribosomal protein synthesis pigment fusion peptides

<130>  DK12217EP

<160>  37

<170>  PatentIn version 3.5

<210>  1
<211>  3855
<212>  DNA
<213>  Photorhabdus luminescens

<400>  1
atgttagaaa ataatattac acaatgtgac tcaatcaatg atgtttatct taaagaagaa      60

gcaataacat tgatggatat gcttgagagt caacttaagc accaggcaga tggatatgtt     120

gttattgatc aagaagaatc tctcagttac gctgatttct atttgagggt gaaagagata     180

gggtattgtc tgtcagaaat tagctcaaag aattcggtgg gtattgggct tttttgtgat     240

ccttctatag atttaatttg tggtgcatgg ggtattttgt cagcggataa agcttatttg     300

ccgttatcgc ctgactatcc aactgaacgc ctcaaatata tgatagaaga ttctggtatt     360

gatgtgattt ttacgcaatc gcacttaaaa gcacagctac aggacattgc accaaaatca     420

gtattaatta tgacaccaga agatgtcgct ctgacgataa aaacacgaac aatagaagat     480

attctgggca cagttcaagt tcctaaaccc actagtctgg cttatattat ttatacctct     540

ggtagcacgg gtaagccaaa gggagtgatg attgaacatc acagtattgt aaatcaaatg     600

agatttcttg caaaagcgtt caaattagga tgtcattccc ggattttaca gaaaacacca     660

atgagttttg atgcggctca atgggaaatt ctagcgcctg caattggtgg tcaagtgatt     720

atgggtcctt taggttgcta tcgcgatccg gatgcaatta ttaaaaccat tcttcagcat     780

caagtaacga ctttgcaatg tgttcctact ttgctacaag cgttactgga taatcctaat     840

tttttggatt gcttatcatt gactcaagta ttcagtgggg gagaagcgct gacaaccaaa     900

ttagccacgc aatttttgaa tagttttact cactgtgaat taatcaattt atatggcccg     960

acagaatgta cgattaattc atcatttttc cgggtgacaa atgagacttt gccgaattat    1020

caaacctcta tttcgattgg tgcacctgta gataataccg aatactacgt tcttgatgat    1080

gatagattac ctgtggcggt tggcgaaatt ggcgagcttt atatttcggg tgctcaatta    1140

gcacgtggtt atttgcataa accagaaatg acaaaagata aatttatttg taatcacctt    1200

gtatcaggaa ctcaacatca atggttatat cgaacgggag atctggtaac cagaggggct    1260

gatggtaata cttattttgt tggtcgggtt gatagccagg tcaaattacg aggttaccgt    1320

attgagcttg atgaaatacg ccatgcgatt gaagaacata gctggataaa gacggcggca    1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaagtgg attatcaatc tttatcagaa tctaaagccg tggagaatgt ttcaacacag 2820

cgtctattgg tgccattaca tacagatact gaaataaggc ttggaaaaat ttggatggaa 2880

gtactgaaat gggattcagt atctgccctc gatgattttt tcgaaagtgg gggtaattct 2940

ttgatggccg ttgcaatggt taataagatc aatgcggcct ttaatattcg ttttccgtta 3000

cagatacttt ttcaatctcc taatatagca gaattggcta agtggattga acagacagac 3060

tctaaaacaa tatcaagatt aattttattg aatcaggcaa gcaaagaccc catttactgt 3120

tggccgggtt tgggcggata tcctatgagt ttgagattgc ttgctaataa agtcgttcct 3180

gatcgggcat tttatggaat acaggcatat gggataaacg agagtgaaat accgttttct 3240

tctatccaga gaatggcaga agaggatatt aaagagataa agaaaataca gccagaaggg 3300

ccatatatat tgtggggata ttcatttggt gcccgagtag catttgaagt tgcataccag 3360

cttgaacaag cgggagaaga agttaacgca ttgaatttat tggctccggg atctcctcat 3420

cttgatatga agcaagcgga atatatggat aaaggcgctg aatttactaa tccggctttt 3480

gttaaaatac ttttttctgt attttctcgt tcaatcaaca gcccaatggt taaaacttgc 3540

ttagaacaag taaatagtga aacgacattt attaacttta tatgtagtcg ttttaaaaac 3600

ttggaaccat cattagtaaa acgtatcgtt aggattgtga ctttgactta tgatttcaag 3660

tacagtattg atgagcttta tcacagacac ctaaaggcac ctataactat tttcaaggcg 3720

aatagagata atgattcatt tatcgaggaa tcggatgtga tttcatcaat gtcgcctaaa 3780

ataattgaat taatatcgga tcactatcaa ctgttggaaa gtgaaggtgt tgctgagatt 3840

gagaaaataa tctaa 3855

<210> 2
<211> 4402
<212> DNA
<213> Artificial Sequence

<220>
<223> engineered and functional indC from P. luminescens where we
      replaced the native T-domain with a ccdB gene which is toxic to
      normal E. coli cells. We used this variant to easily exchange
      T-domains without any background cells.

<400> 2
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540

ggtagcacgg gtaagccaaa gggagtgatg attgaacatc acagtattgt aaatcaaatg 600

agatttcttg caaaagcgtt caaattagga tgtcattccc ggattttaca gaaaacacca 660

atgagttttg atgcggctca atgggaaatt ctagcgcctg caattggtgg tcaagtgatt 720

atgggtcctt taggttgcta tcgcgatccg gatgcaatta ttaaaaccat tcttcagcat 780

caagtaacga ctttgcaatg tgttcctact ttgctacaag cgttactgga taatcctaat 840

tttttggatt gcttatcatt gactcaagta ttcagtgggg gagaagcgct gacaaccaaa 900

ttagccacgc aatttttgaa tagttttact cactgtgaat taatcaattt atatggcccg 960

acagaatgta cgattaattc atcatttttc cgggtgacaa atgagacttt gccgaattat 1020

caaacctcta tttcgattgg tgcacctgta gataataccg aatactacgt tcttgatgat 1080

gatagattac ctgtggcggt tggcgaaatt ggcgagcttt atatttcggg tgctcaatta 1140

gcacgtggtt atttgcataa accagaaatg acaaaagata aatttatttg taatcacctt 1200

gtatcaggaa ctcaacatca atggttatat cgaacgggag atctggtaac cagaggggct 1260

gatggtaata cttattttgt tggtcgggtt gatagccagg tcaaattacg aggttaccgt 1320

attgagcttg atgaaatacg ccatgcgatt gaagaacata gctggataaa gacggcggca 1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaagtgg attatcaatc tttatcagaa tctaaagccg tggagaatgt ttcaacacag 2820

cgtctattgg tgccattaca tacagatact actggctgtg tataagggag cctgacattt 2880

atattcccca gaacatcagg ttaatggcgt ttttgatgtc attttcgcgg tggctgagat 2940

cagccacttc ttccccgata acggagaccg gcacactggc catatcggtg gtcatcatgc 3000

gccagctttc atccccgata tgcaccaccg ggtaaagttc acgggagact ttatctgaca 3060

gcagacgtgc actggccagg gggatcacca tccgtcgccc gggcgtgtca ataatatcac 3120

tctgtacatc cacaaacaga cgataacggc tctctctttt ataggtgtaa accttaaact 3180

gcatttcacc agcccctgtt ctcgtcagca aaagagccgt tcatttcaat aaaccgggcg 3240

acctcagcca tcccttcctg attttccgct ttccagcgtt cggcacgcag acgacgggct 3300

tcattctgca tggttgtgct taccagaccg gagatattga catcatatat gccttgagca 3360

actgatagct gtcgctgtca actgtcactg taatacgctg cttcatagca tacctctttt 3420

tgacatactt cgggtataca tatcagtata tattcttata ccgcaaaaat cagcgcgcaa 3480

atacgcatac tgttatctgg cttttagtaa gccggatcca cgcgccttta atattcgttt 3540

tccgttacag atactttttc aatctcctaa tatagcagaa ttggctaagt ggattgaaca 3600

gacagactct aaaacaatat caagattaat tttattgaat caggcaagca aagaccccat 3660

ttactgttgg ccgggtttgg gcggatatcc tatgagtttg agattgcttg ctaataaagt 3720

cgttcctgat cgggcatttt atggaataca ggcatatggg ataaacgaga gtgaaatacc 3780

gttttcttct atccagagaa tggcagaaga ggatattaaa gagataaaga aaatacagcc 3840

agaaggccca tatatattgt ggggatattc atttggtgcc cgagtagcat ttgaagttgc 3900

ataccagctt gaacaagcgg gagaagaagt taacgcattg aatttattgg ctccgggatc 3960

tcctcatctt gatatgaagc aagcggaata tatggataaa ggcgctgaat ttactaatcc 4020

ggcttttgtt aaaatacttt tttctgtatt ttctcgttca atcaacagcc caatggttaa 4080

aacttgctta gaacaagtaa atagtgaaac gacatttatt aactttatat gtagtcgttt 4140

taaaaacttg gaaccatcat tagtaaaacg tatcgttagg attgtgactt tgacttatga 4200

tttcaagtac agtattgatg agctttatca cagacaccta aaggcaccta taactatttt 4260

caaggcgaat agagataatg attcatttat cgaggaatcg gatgtgattt catcaatgtc 4320

gcctaaaata attgaattaa tatcggatca ctatcaactg ttggaaagtg aaggtgttgc 4380

tgagattgag aaaataatct aa 4402

<210> 3
<211> 4004
<212> DNA
<213> Artificial Sequence

<220>
<223>  engineered and functional indC from P. luminescens where we
      replaced the native T-domain with the T-domain of the bpsA
      indigoidine synthetase from Streptomyces lavendulae lavendulae
      ATCC11924

<400>  3
atgttagaaa ataatattac acaatgtgac tcaatcaatg atgtttatct taaagaagaa      60

gcaataacat tgatggatat gcttgagagt caacttaagc accaggcaga tggatatgtt     120

gttattgatc aagaagaatc tctcagttac gctgatttct atttgagggt gaaagagata     180

gggtattgtc tgtcagaaat tagctcaaag aattcggtgg gtattgggct tttttgtgat     240

ccttctatag atttaatttg tggtgcatgg ggtattttgt cagcggataa agcttatttg     300

ccgttatcgc ctgactatcc aactgaacgc ctcaaatata tgatagaaga ttctggtatt     360

gatgtgattt ttacgcaatc gcacttaaaa gcacagctac aggacattgc accaaaatca     420

gtattaatta tgacaccaga agatgtcgct ctgacgataa aaacacgaac aatagaagat     480

attctgggca cagttcaagt tcctaaaccc actagtctgg cttatattat ttatacctct     540

ggtagcacgg gtaagccaaa gggagtgatg attgaacatc acagtattgt aaatcaaatg     600

agatttcttg caaaagcgtt caaattagga tgtcattccc ggattttaca gaaaacacca     660

atgagttttg atgcggctca atgggaaatt ctagcgcctg caattggtgg tcaagtgatt     720

atgggtcctt taggttgcta tcgcgatccg gatgcaatta ttaaaaccat tcttcagcat     780

caagtaacga ctttgcaatg tgttcctact ttgctacaag cgttactgga taatcctaat     840

tttttggatt gcttatcatt gactcaagta ttcagtgggg gagaagcgct gacaaccaaa     900

ttagccacgc aatttttgaa tagttttact cactgtgaat taatcaattt atatggcccg     960

acagaatgta cgattaattc atcatttttc cgggtgacaa atgagacttt gccgaattat    1020

caaacctcta tttcgattgg tgcacctgta gataataccg aatactacgt tcttgatgat    1080

gatagattac ctgtggcggt tggcgaaatt ggcgagcttt atatttcggg tgctcaatta    1140

gcacgtggtt atttgcataa accagaaatg acaaaagata aatttatttg taatcacctt    1200

gtatcaggaa ctcaacatca atggttatat cgaacgggag atctggtaac cagaggggct    1260

gatggtaata cttattttgt tggtcgggtt gatagccagg tcaaattacg aggttaccgt    1320

attgagcttg atgaaatacg ccatgcgatt gaagaacata gctggataaa gacggcggca    1380

atgttaatta agaaggatgc cagaacgggt ttccaaaatc tcatcgcgtg tgtggaatta    1440

gatgagaaag aagctgcatt gatggatcaa ggtaatagta gctcacatca caaatcaaaa    1500

gccgataaac tacaggtgaa agcccaactt tctaattctg gttgtcgaag tgaagagtta    1560

tgtgaaaatc gccctacatt cttacttcct tatcaagaag gggagataaa acagagagaa    1620

tatgcatttg gacgcaagac atatcgctat tttgagggaa cagaaataac ggtagagaaa    1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaagatcg atgtgaaagc actggccgct tctgaccagg tcaacgctga gctggtggaa 2820

cggcccttcg tcgcacctag gaccgaaaca gagaaggaaa tcgcagccgt gtgggagaaa 2880

gccctgagac gcgaaaatgc tagtgtccag gacgatttct ttgagtccgg cggaaactct 2940

ctgatcgccg tcggcctggt gagggaactg aatgctagac tgggagtgtc cctgcctctg 3000

cagagtgtcc tggagtcacc aacaattgaa aagctggccg ggattcagta tctgccctcg 3060

atgatttttt cgaaagtggg ggtaattctt tgatggccgt tgcaatggtt aataagatca 3120

atgcggcctt taatattcgt tttccgttac agatactttt tcaatctcct aatatagcag 3180

aattggctaa gtggattgaa cagacagact ctaaaacaat atcaagatta attttattga 3240

atcaggcaag caaagacccc atttactgtt ggccgggttt gggcggatat cctatgagtt 3300

tgagattgct tgctaataaa gtcgttcctg atcgggcatt ttatggaata caggcatatg 3360

ggataaacga gagtgaaata ccgttttctt ctatccagag aatggcagaa gaggatatta 3420

aagagataaa gaaaatacag ccagaagggc catatatatt gtggggatat tcatttggtg 3480

cccgagtagc atttgaagtt gcataccagc ttgaacaagc gggagaagaa gttaacgcat 3540

tgaatttatt ggctccggga tctcctcatc ttgatatgaa gcaagcggaa tatatggata 3600

aaggcgctga atttactaat ccggcttttg ttaaaatact tttttctgta ttttctcgtt 3660

caatcaacag cccaatggtt aaaacttgct tagaacaagt aaatagtgaa acgacattta 3720

ttaactttat atgtagtcgt tttaaaaact tggaaccatc attagtaaaa cgtatcgtta 3780

ggattgtgac tttgacttat gatttcaagt acagtattga tgagctttat cacagacacc 3840

taaaggcacc tataactatt ttcaaggcga atagagataa tgattcattt atcgaggaat 3900

cggatgtgat ttcatcaatg tcgcctaaaa taattgaatt aatatcggat cactatcaac 3960

tgttggaaag tgaaggtgtt gctgagattg agaaaataat ctaa 4004

<210> 4
<211> 3995
<212> DNA
<213> Artificial Sequence

<220>
<223> engineered and functional indC from P. luminescens where we
      replaced the native T-domain with the T-domain of the plu2642
      protein from P. luminescens

<400> 4
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540

ggtagcacgg gtaagccaaa gggagtgatg attgaacatc acagtattgt aaatcaaatg 600

agatttcttg caaaagcgtt caaattagga tgtcattccc ggattttaca gaaaacacca 660

atgagttttg atgcggctca atgggaaatt ctagcgcctg caattggtgg tcaagtgatt 720

atgggtcctt taggttgcta tcgcgatccg gatgcaatta ttaaaaccat tcttcagcat 780

caagtaacga ctttgcaatg tgttcctact ttgctacaag cgttactgga taatcctaat 840

tttttggatt gcttatcatt gactcaagta ttcagtgggg gagaagcgct gacaaccaaa 900

ttagccacgc aatttttgaa tagttttact cactgtgaat taatcaattt atatggcccg 960

acagaatgta cgattaattc atcatttttc cgggtgacaa atgagacttt gccgaattat 1020

caaacctcta tttcgattgg tgcacctgta gataataccg aatactacgt tcttgatgat 1080

gatagattac ctgtggcggt tggcgaaatt ggcgagcttt atatttcggg tgctcaatta 1140

gcacgtggtt atttgcataa accagaaatg acaaaagata aatttatttg taatcacctt 1200

gtatcaggaa ctcaacatca atggttatat cgaacgggag atctggtaac cagaggggct 1260

gatggtaata cttattttgt tggtcgggtt gatagccagg tcaaattacg aggttaccgt 1320

attgagcttg atgaaatacg ccatgcgatt gaagaacata gctggataaa gacggcggca 1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaaatcg atttcgacac attacaagta ctggtcagca cagtatcaca cagtccacag 2820

gtactcccaa gcacctcgac agaaacacag atcgtaaaga tatgggaaga agtgctaacg 2880

cgagaaagca tatctaccga agatgacttc tttgctttag gtggcaattc tctgatagcc 2940

gtccatctga tacaacgttt aaatgaagaa tttgcgttat cgctacctct ccatactcta 3000

tttgaggccg caacggttaa acaattggca gggattcagt atctgccctc gatgattttt 3060

tcgaaagtgg gggtaattct ttgatggccg ttgcaatggt taataagatc aatgcggcct 3120

ttaatattcg ttttccgtta cagatacttt ttcaatctcc taatatagca gaattggcta 3180

agtggattga acagacagac tctaaaacaa tatcaagatt aattttattg aatcaggcaa 3240

gcaaagaccc catttactgt tggccgggtt tgggcggata tcctatgagt ttgagattgc 3300

ttgctaataa agtcgttcct gatcgggcat tttatggaat acaggcatat gggataaacg 3360

agagtgaaat accgttttct tctatccaga gaatggcaga agaggatatt aaagagataa 3420

agaaaataca gccagaaggg ccatatatat tgtggggata ttcatttggt gcccgagtag 3480

catttgaagt tgcataccag cttgaacaag cgggagaaga agttaacgca ttgaatttat 3540

tggctccggg atctcctcat cttgatatga agcaagcgga atatatggat aaaggcgctg 3600

aatttactaa tccggctttt gttaaaatac ttttttctgt attttctcgt tcaatcaaca 3660

gcccaatggt taaaacttgc ttagaacaag taaatagtga aacgacattt attaacttta 3720

tatgtagtcg ttttaaaaac ttggaaccat cattagtaaa acgtatcgtt aggattgtga 3780

ctttgactta tgatttcaag tacagtattg atgagcttta tcacagacac ctaaaggcac 3840

ctataactat tttcaaggcg aatagagata atgattcatt tatcgaggaa tcggatgtga 3900

tttcatcaat gtcgcctaaa ataattgaat taatatcgga tcactatcaa ctgttggaaa 3960

gtgaaggtgt tgctgagatt gagaaaataa tctaa 3995

<210> 5
<211> 3983
<212> DNA
<213> Artificial Sequence

<220>
<223> engineered and functional indC from P. luminescens where we
      replaced the native T-domain with the T-domain of the delH4
      protein from Delftia acidovorans SPH-1

<400> 5
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540
ggtagcacgg gtaagccaaa gggagtgatg attgaacatc acagtattgt aaatcaaatg 600
agatttcttg caaaagcgtt caaattagga tgtcattccc ggattttaca gaaaacacca 660
atgagttttg atgcggctca atgggaaatt ctagcgcctg caattggtgg tcaagtgatt 720
atgggtcctt taggttgcta tcgcgatccg gatgcaatta ttaaaaccat tcttcagcat 780
caagtaacga ctttgcaatg tgttcctact ttgctacaag cgttactgga taatcctaat 840
tttttggatt gcttatcatt gactcaagta ttcagtgggg gagaagcgct gacaaccaaa 900
ttagccacgc aatttttgaa tagttttact cactgtgaat taatcaattt atatggcccg 960
acagaatgta cgattaattc atcatttttc cgggtgacaa atgagacttt gccgaattat 1020
caaacctcta tttcgattgg tgcacctgta gataataccg aatactacgt tcttgatgat 1080
gatagattac ctgtggcggt tggcgaaatt ggcgagcttt atatttcggg tgctcaatta 1140
gcacgtggtt atttgcataa accagaaatg acaaaagata aatttatttg taatcacctt 1200
gtatcaggaa ctcaacatca atggttatat cgaacgggag atctggtaac cagaggggct 1260
gatggtaata cttattttgt tggtcgggtt gatagccagg tcaaattacg aggttaccgt 1320
attgagcttg atgaaatacg ccatgcgatt gaagaacata gctggataaa gacggcggca 1380
atgttaatta agaaggatgc cagaacgggt ttccaaaatc tcatcgcgtg tgtggaatta 1440
gatgagaaag aagctgcatt gatggatcaa ggtaatagta gctcacatca caaatcaaaa 1500
gccgataaac tacaggtgaa agcccaactt tctaattctg gttgtcgaag tgaagagtta 1560
tgtgaaaatc gccctacatt cttacttcct tatcaagaag ggagataaaa acagagagaa 1620
tatgcatttg gacgcaagac atatcgctat tttgagggaa cagaaataac ggtagagaaa 1680
ttaaaaaaat tgctgacagc cactcaatcg aatgaaatta gctctttgcc actgagtcat 1740
ctaaccctga atgatttcgg ttatgcattg cgttattttg gtcagtttac cagccatcaa 1800
cgtttattgc ccaaatatgc ctatgcttca ccgggtgctc tctatgcgac acaaatgtat 1860
tttgaattgc ataatgttct cggtttggat gcggggattt actattatca tccagtgaca 1920
cataagttaa taaaaatttc aacattgagt cgtcggcaaa tgccaacgat aaaagtgcat 1980
tttattggca agcatgaagc cattgagccc gtttataaga acaatataca agaagttctg 2040
gaaatggaag cgggccatat gatgggtctt tttgatgacg tattaccgga aattggcttg 2100
agtattggta aaagtgaata tcaagatgaa tgtccagatt ggtatgatgg tgatattcag 2160
gattattatc ttggtgcatt tgaaatatgt agctatgaac atggattgcc gccatttgag 2220
actgatattt atttacaaac acatgcccat aaaatacctg agatgccgtg tggtttatat 2280
cacttttcta acggggaatt tgtacgaata agtgatgata ttgtccgaaa aaaggatgtt 2340
attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaagctgg accggcaggc cctgcccgcg ttcggcatgc cagccgccag ccaggctccc 2820

gagggcgaac tggagacgct gctggcccgt atctgggccg aggtgctggg cctggagcgg 2880

gtggggcgca gcgacaactt cttcgcgctg ggcggtgatt ccatcctggg cctgcagatc 2940

gtctcgcgcc tgcgccgctt cggctggaag ctgtcgccac ggcagctgtt cgagcggcaa 3000

agcattgccg agctggcggg gattcagtat ctgccctcga tgattttttc gaaagtgggg 3060

gtaattcttt gatggccgtt gcaatggtta ataagatcaa tgcggccttt aatattcgtt 3120

ttccgttaca gatacttttt caatctccta atatagcaga attggctaag tggattgaac 3180

agacagactc taaaacaata tcaagattaa ttttattgaa tcaggcaagc aaagacccca 3240

tttactgttg gccgggtttg ggcggatatc ctatgagttt gagattgctt gctaataaag 3300

tcgttcctga tcgggcattt tatggaatac aggcatatgg gataaacgag agtgaaatac 3360

cgttttcttc tatccagaga atggcagaag aggatattaa agagataaag aaaatacagc 3420

cagaagggcc atatatattg tggggatatt catttggtgc ccgagtagca tttgaagttg 3480

cataccagct tgaacaagcg ggagaagaag ttaacgcatt gaatttattg gctccgggat 3540

ctcctcatct tgatatgaag caagcggaat atatggataa aggcgctgaa tttactaatc 3600

cggcttttgt taaaatactt ttttctgtat tttctcgttc aatcaacagc ccaatggtta 3660

aaacttgctt agaacaagta aatagtgaaa cgacatttat taactttata tgtagtcgtt 3720

ttaaaaactt ggaaccatca ttagtaaaac gtatcgttag gattgtgact ttgacttatg 3780

atttcaagta cagtattgat gagctttatc acagacacct aaaggcacct ataactattt 3840

tcaaggcgaa tagagataat gattcattta tcgaggaatc ggatgtgatt tcatcaatgt 3900

cgcctaaaat aattgaatta atatcggatc actatcaact gttggaaagt gaaggtgttg 3960

ctgagattga gaaaataatc taa 3983

<210> 6
<211> 3917
<212> DNA
<213> Artificial Sequence

<220>
<223> engineered and functional indC from P. luminescens where we

replaced the native T-domain with a synthetic T-domain of our own
design (variant 1)

<400> 6
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540

ggtagcacgg gtaagccaaa gggagtgatg attgaacatc acagtattgt aaatcaaatg 600

agatttcttg caaaagcgtt caaattagga tgtcattccc ggattttaca gaaaacacca 660

atgagttttg atgcggctca atgggaaatt ctagcgcctg caattggtgg tcaagtgatt 720

atgggtcctt taggttgcta tcgcgatccg gatgcaatta ttaaaaccat tcttcagcat 780

caagtaacga ctttgcaatg tgttcctact ttgctacaag cgttactgga taatcctaat 840

tttttggatt gcttatcatt gactcaagta ttcagtgggg gagaagcgct gacaaccaaa 900

ttagccacgc aatttttgaa tagttttact cactgtgaat taatcaattt atatggcccg 960

acagaatgta cgattaattc atcatttttc cgggtgacaa atgagacttt gccgaattat 1020

caaacctcta tttcgattgg tgcacctgta gataataccg aatactacgt tcttgatgat 1080

gatagattac ctgtggcggt tggcgaaatt ggcgagcttt atatttcggg tgctcaatta 1140

gcacgtggtt atttgcataa accagaaatg acaaaagata aatttatttg taatcacctt 1200

gtatcaggaa ctcaacatca atggttatat cgaacgggag atctggtaac cagaggggct 1260

gatggtaata cttattttgt tggtcgggtt gatagccagg tcaaattacg aggttaccgt 1320

attgagcttg atgaaatacg ccatgcgatt gaagaacata gctggataaa gacggcggca 1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaagtgg attatcaatc tttatcagaa tctaaagccg tggagaatgt ttcaacacag 2820

cgtctattgg tgccattaca tacagatact gaaatccgtc tggcgaaaat ctggatggaa 2880

gttctgaaat gggactctgt ttctgcgctg gacgacttct tcgaatctgg tggtaactct 2940

ctgatggcgg ttgcgctggt taacaaaatc aacgcggcgt tcaacatccg tctgccgctg 3000

caaatcctgt tccagtctcc gaccatcgcg gaactggcgc ctttaatatt cgttttccgt 3060

tacagatact tttcaatctc cctaatatag cagaattggc taagtggatt gaacagacag 3120

actctaaaac aatatcaaga ttaattttat tgaatcaggc aagcaaagac cccatttact 3180

gttggccggg tttgggcgga tatcctatga gtttgagatt gcttgctaat aaagtcgttc 3240

ctgatcgggc attttatgga atacaggcat atgggataaa cgagagtgaa ataccgtttt 3300

cttctatcca gagaatggca gaagaggata ttaaagagat aaagaaaata cagccagaag 3360

ggccatatat attgtgggga tattcatttg gtgcccgagt agcatttgaa gttgcatacc 3420

agcttgaaca agcgggagaa gaagttaacg cattgaattt attggctccg ggatctcctc 3480

atcttgatat gaagcaagcg gaatatatgg ataaaggcgc tgaatttact aatccggctt 3540

ttgttaaaat actttttctt gtatttctcg gttcaatcaa cagcccaatg gttaaaactt 3600

gcttagaaca agtaaatagt gaaacgacat ttattaactt tatatgtagt cgttttaaaa 3660

acttggaacc atcattagta aaacgtatcg ttaggattgt gactttgact tatgatttca 3720

agtacagtat tgatgagctt tatcacagac acctaaaggc acctataact attttcaagg 3780

cgaatagaga taatgattca tttatcgagg aatcggatgt gatttcatca atgtcgccta 3840

aaataattga attaatatcg gatcactatc aactgttgga aagtgaaggt gttgctgaga 3900

ttgagaaaat aatctaa 3917

<210> 7
<211> 3917
<212> DNA
<213> Artificial Sequence

<220>
<223> engineered and functional indC from P. luminescens where we
      replaced the native T-domain with a synthetic T-domain of our own
      design (variant 3)

<400> 7
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540

ggtagcacgg gtaagccaaa gggagtgatg attgaacatc acagtattgt aaatcaaatg 600

agatttcttg caaaagcgtt caaattagga tgtcattccc ggattttaca gaaaacacca 660

atgagttttg atgcggctca atgggaaatt ctagcgcctg caattggtgg tcaagtgatt 720

atgggtcctt taggttgcta tcgcgatccg gatgcaatta ttaaaaccat tcttcagcat 780

caagtaacga ctttgcaatg tgttcctact ttgctacaag cgttactgga taatcctaat 840

tttttggatt gcttatcatt gactcaagta ttcagtgggg gagaagcgct gacaaccaaa 900

ttagccacgc aatttttgaa tagttttact cactgtgaat taatcaattt atatggcccg 960

acagaatgta cgattaattc atcatttttc cgggtgacaa atgagacttt gccgaattat 1020

caaacctcta tttcgattgg tgcacctgta gataataccg aatactacgt tcttgatgat 1080

gatagattac ctgtggcggt tggcgaaatt ggcgagcttt atatttcggg tgctcaatta 1140

gcacgtggtt atttgcataa accagaaatg acaaaagata aatttatttg taatcacctt 1200

gtatcaggaa ctcaacatca atggttatat cgaacgggag atctggtaac cagaggggct 1260

gatggtaata cttattttgt tggtcgggtt gatagccagg tcaaattacg aggttaccgt 1320

attgagcttg atgaaatacg ccatgcgatt gaagaacata gctggataaa gacggcggca 1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaagtgg attatcaatc tttatcagaa tctaaagccg tggagaatgt ttcaacacag 2820

cgtctattgg tgccattaca tacagatact gaaatccgtc tgggtaaaat ctggatggaa 2880

gttctgaaat gggactctgt tggtgcgctg gacgacttct tcgaactggg tggtcactct 2940

ctgatggcgg ttgcgatggt taacaaaatc aacgcggcgt tcaacatccg tctgccgctg 3000

caaatcctgt tccagtctcc gaccatcgcg gaactggcgc ctttaatatt cgttttccgt 3060

tacagatact ttttcaatct cctaatatag cagaattggc taagtggatt gaacagacag 3120

actctaaaac aatatcaaga ttaattttat tgaatcaggc aagcaaagac cccatttact 3180

gttggccggg tttgggcgga tatcctatga gtttgagatt gcttgctaat aaagtcgttc 3240

ctgatcgggc attttatgga atacaggcat atgggataaa cgagagtgaa ataccgtttt 3300

cttctatcca gagaatggca gaagaggata ttaaagagat aaagaaaata cagccagaag 3360

ggccatatat attgtgggga tattcatttg gtgcccgagt agcatttgaa gttgcatacc 3420

agcttgaaca agcgggagaa gaagttaacg cattgaattt attggctccg ggatctcctc 3480

atcttgatat gaagcaagcg gaatatatgg ataaaggcgc tgaatttact aatccggctt 3540

ttgttaaaat acttttttct gtattttctc gttcaatcaa cagcccaatg gttaaaactt 3600

gcttagaaca agtaaatagt gaaacgacat ttattaactt tatatgtagt cgttttaaaa 3660

acttggaacc atcattagta aaacgtatcg ttaggattgt gactttgact tatgatttca 3720

agtacagtat tgatgagctt tatcacagac acctaaaggc acctataact attttcaagg 3780

cgaatagaga taatgattca tttatcgagg aatcggatgt gatttcatca atgtcgccta 3840

aaataattga attaatatcg gatcactatc aactgttgga aagtgaaggt gttgctgaga 3900

ttgagaaaat aatctaa 3917

<210> 8
<211> 3917
<212> DNA
<213> Artificial Sequence

<220>
<223> engineered and functional indC from P. luminescens where we
      replaced the native T-domain with a synthetic T-domain of our own
      design (variant 4)

<400> 8
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540

ggtagcacgg gtaagccaaa gggagtgatg attgaacatc acagtattgt aaatcaaatg 600

agatttcttg caaaagcgtt caaattagga tgtcattccc ggattttaca gaaaacacca 660

atgagttttg atgcggctca atgggaaatt ctagcgcctg caattggtgg tcaagtgatt 720

atgggtcctt taggttgcta tcgcgatccg gatgcaatta ttaaaaccat tcttcagcat 780

caagtaacga ctttgcaatg tgttcctact ttgctacaag cgttactgga taatcctaat 840

tttttggatt gcttatcatt gactcaagta ttcagtgggg gagaagcgct gacaaccaaa 900

ttagccacgc aatttttgaa tagttttact cactgtgaat taatcaattt atatggcccg 960

acagaatgta cgattaattc atcatttttc cgggtgacaa atgagacttt gccgaattat 1020

caaacctcta tttcgattgg tgcacctgta gataataccg aatactacgt tcttgatgat 1080

gatagattac ctgtggcggt tggcgaaatt ggcgagcttt atatttcggg tgctcaatta 1140

gcacgtggtt atttgcataa accagaaatg acaaaagata aatttatttg taatcacctt 1200

gtatcaggaa ctcaacatca atggttatat cgaacgggag atctggtaac cagaggggct 1260

gatggtaata cttattttgt tggtcgggtt gatagccagg tcaaattacg aggttaccgt 1320

attgagcttg atgaaatacg ccatgcgatt gaagaacata gctggataaa gacggcggca 1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaagtgg attatcaatc tttatcagaa tctaaagccg tggagaatgt ttcaacacag 2820

cgtctattgg tgccattaca tacagatact gaaatccgtc tggcgaaaat ctggatggaa 2880

gttctgggtt gggactctgt ttctgcgctg gacgacttct tcgaactggg tggtaactct 2940

ctgatggcgg ttgcgatggt taacaaaatc aacgcggcgt tcaacatccg tttcccgctg 3000

caaatcctgt tccagtctcc gaccatcgcg gaactggcgc ctttaatatt cgttttccgt 3060

tacagatact ttttcaatct cctaatatag cagaattggc taagtggatt gaacagacag 3120

actctaaaac aatatcaaga ttaattttat tgaatcaggc aagcaaagac cccatttact 3180

gttggccggg tttgggcgga tatcctatga gtttgagatt gcttgctaat aaagtcgttc 3240

ctgatcgggc attttatgga atacaggcat atgggataaa cgagagtgaa ataccgtttt 3300

cttctatcca gagaatggca gaagaggata ttaaagagat aaagaaaata cagccagaag 3360

ggccatatat attgtgggga tattcatttg gtgcccgagt agcatttgaa gttgcatacc 3420

agcttgaaca agcgggagaa gaagttaacg cattgaattt attggctccg ggatctcctc 3480

atcttgatat gaagcaagcg gaatatatgg ataaaggcgc tgaatttact aatccggctt 3540

ttgttaaaat acttttttct gtattttctc gttcaatcaa cagcccaatg gttaaaactt 3600

gcttagaaca agtaaatagt gaaacgacat ttattaactt tatatgtagt cgttttaaaa 3660

acttggaacc atcattagta aaacgtatcg ttaggattgt gactttgact tatgatttca 3720

agtacagtat tgatgagctt tatcacagac acctaaaggc acctataact attttcaagg 3780

cgaatagaga taatgattca tttatcgagg aatcggatgt gatttcatca atgtcgccta 3840

aaataattga attaatatcg gatcactatc aactgttgga aagtgaaggt gttgctgaga 3900

ttgagaaaat aatctaa 3917

<210> 9
<211> 3917
<212> DNA
<213> Artificial Sequence

<220>
<223> engineered and functional indC from P. luminescens where we
      replaced the native T-domain with a synthetic T-domain of our own
      design (variant 5)

<400> 9
atgttagaaa ataatattac acaatgtgac tcaatcaatg atgtttatct taaagaagaa 60

gcaataacat tgatggatat gcttgagagt caacttaagc accaggcaga tggatatgtt 120

gttattgatc aagaagaatc tctcagttac gctgatttct atttgagggt gaaagagata 180

gggtattgtc tgtcagaaat tagctcaaag aattcggtgg gtattgggct tttttgtgat 240

ccttctatag atttaatttg tggtgcatgg ggtattttgt cagcggataa agcttatttg 300

ccgttatcgc ctgactatcc aactgaacgc ctcaaatata tgatagaaga ttctggtatt 360

gatgtgattt ttacgcaatc gcacttaaaa gcacagctac aggacattgc accaaaatca 420

gtattaatta tgacaccaga agatgtcgct ctgacgataa aaacacgaac aatagaagat 480

attctgggca cagttcaagt tcctaaaccc actagtctgg cttatattat ttatacctct 540

ggtagcacgg gtaagccaaa gggagtgatg attgaacatc acagtattgt aaatcaaatg 600

agatttcttg caaaagcgtt caaattagga tgtcattccc ggattttaca gaaaacacca 660

atgagttttg atgcggctca atgggaaatt ctagcgcctg caattggtgg tcaagtgatt 720

atgggtcctt taggttgcta tcgcgatccg gatgcaatta ttaaaaccat tcttcagcat 780

caagtaacga ctttgcaatg tgttcctact ttgctacaag cgttactgga taatcctaat 840

tttttggatt gcttatcatt gactcaagta ttcagtgggg gagaagcgct gacaaccaaa 900

ttagccacgc aatttttgaa tagttttact cactgtgaat taatcaattt atatggcccg 960

acagaatgta cgattaattc atcatttttc cgggtgacaa atgagacttt gccgaattat 1020

caaacctcta tttcgattgg tgcacctgta gataataccg aatactacgt tcttgatgat 1080

gatagattac ctgtggcggt tggcgaaatt ggcgagcttt atatttcggg tgctcaatta 1140

gcacgtggtt atttgcataa accagaaatg acaaaagata aatttatttg taatcacctt 1200

gtatcaggaa ctcaacatca atggttatat cgaacgggag atctggtaac cagaggggct 1260

gatggtaata cttattttgt tggtcgggtt gatagccagg tcaaattacg aggttaccgt 1320

attgagcttg atgaaatacg ccatgcgatt gaagaacata gctggataaa gacggcggca 1380

atgttaatta agaaggatgc cagaacgggt ttccaaaatc tcatcgcgtg tgtggaatta 1440

gatgagaaag aagctgcatt gatggatcaa ggtaatagta gctcacatca caaatcaaaa 1500

gccgataaac tacaggtgaa agcccaactt tctaattctg gttgtcgaag tgaagagtta 1560

tgtgaaaatc gccctacatt cttacttcct tatcaagaag gggagataaa acagagagaa 1620

tatgcatttg gacgcaagac atatcgctat tttgagggaa cagaaataac ggtagagaaa 1680

ttaaaaaaat tgctgacagc cactcaatcg aatgaaatta gctctttgcc actgagtcat 1740

ctaaccctga atgatttcgg ttatgcattg cgttattttg gtcagtttac cagccatcaa 1800

cgtttattgc ccaaatatgc ctatgcttca ccgggtgctc tctatgcgac acaaatgtat 1860

tttgaattgc ataatgttct cggtttggat gcggggattt actattatca tccagtgaca 1920

cataagttaa taaaaatttc aacattgagt cgtcggcaaa tgccaacgat aaaagtgcat 1980

tttattggca agcatgaagc cattgagccc gtttataaga acaatataca agaagttctg 2040

gaaatggaag cgggccatat gatgggtctt tttgatgacg tattaccgga aattggcttg 2100

agtattggta aaagtgaata tcaagatgaa tgtccagatt ggtatgatgg tgatattcag 2160

gattattatc ttggtgcatt tgaaatatgt agctatgaac atggattgcc gccatttgag 2220

actgatattt atttacaaac acatgcccat aaaatacctg agatgccgtg tggtttatat 2280

cacttttcta acggggaatt tgtacgaata agtgatgata ttgtccgaaa aaaggatgtt 2340

attgcgatta atcagcaagt ttatgatcgc tccagttttg gcgtgtcaat tattccacgc 2400

tgtgtccctg aatggcatta ttatataaca ctgggtcgtc ggttacatgc gttacaaagt 2460

aatccattgt atattggatt aatgtcatct ggttacagtt cgaagagcaa taacgattta 2520

ccttcggcga aaaggatgcg atctattctc aatgcacttg atagacctat ggcggcattt 2580

tatttctgca taggtggggg tattagccaa gcgcaatata tgtgtgaagg catgaaagaa 2640

gatgttgttc atatgaaagg gccagttgaa atcattaaag atgatcttca acaacaactc 2700

cctcaatata tgattccaaa taaggtatta gttttcgata aattaccttt gacggccaat 2760

ggaaaagtgg attatcaatc tttatcagaa tctaaagccg tggagaatgt ttcaacacag 2820

cgtctattgg tgccattaca tacagatact gaatctcgtc tggcggacgt ttggggtcgt 2880

gcgctgaaat acgacgacgt ttctgcgcac gacgacttct tcgaatctgg tggtaactct 2940

ctgtctgcgg tttctctgat caacgaaatc aaccgtgcgt tcggtctgac cctgccgatc 3000

caggttgttt tccaggcgcc gaaagttcgt gaactggcgc ctttaatatt cgttttccgt 3060

tacagatact ttttcaatct cctaatatag cagaattggc taagtggatt gaacagacag 3120

actctaaaac aatatcaaga ttaattttat tgaatcaggc aagcaaagac cccatttact 3180

gttggccggg tttgggcgga tatcctatga gtttgagatt gcttgctaat aaagtcgttc 3240

ctgatcgggc attttatgga atacaggcat atgggataaa cgagagtgaa ataccgtttt 3300

cttctatcca gagaatggca gaagaggata ttaaagagat aaagaaaata cagccagaag 3360

ggccatatat attgtgggga tattcatttg gtgcccgagt agcatttgaa gttgcatacc 3420

agcttgaaca agcgggagaa gaagttaacg cattgaattt attggctccg ggatctcctc 3480

atcttgatat gaagcaagcg gaatatatgg ataaaggcgc tgaatttact aatccggctt 3540

ttgttaaaat acttttttct gtattttctc gttcaatcaa cagcccaatg gttaaaactt 3600

gcttagaaca agtaaatagt gaaacgacat ttattaactt tatatgtagt cgttttaaaa 3660

acttggaacc atcattagta aaacgtatcg ttaggattgt gactttgact tatgatttca 3720

agtacagtat tgatgagctt tatcacagac acctaaaggc acctataact attttcaagg 3780

cgaatagaga taatgattca tttatcgagg aatcggatgt gatttcatca atgtcgccta 3840

aaataattga attaatatcg gatcactatc aactgttgga aagtgaaggt gttgctgaga 3900

ttgagaaaat aatctaa 3917

<210>  10
<211>  2751
<212>  DNA
<213>  Artificial Sequence

<220>
<223>  gene of unknown function from P. luminescens laumondii TT01; Pfam
       prediction suggests a single module NRPS with the domain sequence
       A-T-TE. We used the T-domain of this module to successfully
       engineer the indC indigoidine synthetase

<400>  10
atgcaatcaa ctctcccaat aataaaatgg cgcaatatat taaaaacagg acagtatcga      60

aaatacgata tctccagcgc tcaaccggca aatgaaaatt ggataacgtt aaacaacatt     120

aagttaccag cgagttttca acgaaaagag tgcctaccgg gattactctt ttcacacgtc     180

agatcaactc cctgggctac agcagtcatt cacggtgaag agcaactcag ttatttggaa     240

atggcaattg gcagtgtaca tctggcctgc tatctgcaaa acctgggatg tttagcgggt     300

gattgcgtcg gtatatttgt tgaaccgtcg attgagcaga tgatcggagt ttggggaact     360

ctttttgccg gtggtgcata tctgccattg tctcatgatt atccagagga acgacttcgt     420

tacatgatcc acgatagcaa tctgaaaatg atatttaccc aagaaaaatt aaaggaaaaa     480

ttggtcaggt tggttgcaga aaatatccat atcgtgactc ttgaagacgt agagaaatca     540

tttgaatcca gtgccattac caacaacacc ctccatgact accttagccc agataacttg     600

gcttatgtca tttatacctc tggaagtaca gggaaaccga aaggtgtaat gattgagcac     660

cgcagtatcg ttaaccaaat gtgttggtta aatgaaaaat gcgatttaaa tattgaaaaa     720

acaattattc agaaaacgcc catcagcttt gatgctgctc aatgggaaat attatcagtc     780

agttgtggta gtcgggttgt tattagctca tctggaacac acaggaatat tccccaactc     840

attgacctga ttattcgcca caatgtgacc acgttacagt gtgttcccac gctattacaa     900

gcactgatcg ataatcatca attccgggaa tgccacaccc ttcggcaaat attcatcgga     960

gcagagagcc tatcaagaaa actcgccact caatgtatcc atacactacc aaactgtcta    1020

ctgattaata tgtatggccc ggcggaatgt acaattaatg cttcagtttt ccttgttaat    1080

cactacccaa tatctgacga agttaattca gtccctattg gtaagccggt atccaatacg    1140

gaatttttta ttctcgatca ccactatcag ctcgcctcag aatatgaaat tggagagatt    1200

tatattgcgg gcactcaagt cgcaagagga tatctgaatc gtcaggatct cacagaaaag    1260

cactttcttg aaattgcaat accaccaaat acgcaaaaaa tccggcttta tagaaccgga    1320

gacctggctt attgggataa agagggtaat gcccactttg ctggtcggaa agataatcaa    1380

attaaagtga gagggatgcg ggtcgaatta gaagaaataa aaaatgcaat agaggttatc    1440

gatcaagtga aacacgctgc aattttggca gaaaaagacc ctcaacaccg ttcgacacga    1500

ttaaccgcct gtattgaatt agccgatgaa acaatacgcc agcaagcaaa gtatgacatt 1560

acttcaattc tgcggagtga acttagcaaa acattaccgg actatatgtt acctgacaga 1620

tttttgttcc tggataccat gccgctaact tccagtggaa aaatcgattt cgacacatta 1680

caagtactgg tcagcacagt atcacacagt ccacaggtac tcccaagcac ctcgacagaa 1740

acacagatcg taaagatatg ggaagaagtg ctaacgcgag aaagcatatc taccgaagat 1800

gacttctttg ctttaggtgg caattctctg atagccgtcc atctgataca acgtttaaat 1860

gaagaatttg cgttatcgct acctctccat actctatttg aggccgcaac ggttaaacaa 1920

ttggcaaaaa tcgttgaagg tgaagtaacc agattatctt cacgattggc ctgtttacag 1980

gaaaaagatg ctggattacc tgtcttttgc tggccaggat tgggtggata cccaatgaat 2040

ttacacctgc tggctacaca gatctgcact gatcgatcat tttatggcat ccaagcttac 2100

ggaattaatg aaggagaggt tccatactcc accatagccg aaatggtgat tcaagacata 2160

acagaaatca aaaaattaca acctactggc ccatacacgc tatggggata ttcttttggc 2220

tctgtattgg ctttcgaagc ggcttaccaa ttagaacgag ccggagaaca tgtcgaaaag 2280

gtggttctaa tcgctccagg gctctcgaag ataaaatatc acgttaattc cacgggaaca 2340

gaaaacgggt ctacttacca aaacaatgag ttgatatcac tattattctc tgtatttgcg 2400

ggcacttctc acagttcagc gttgaatgaa tgcctggcta acgttatcga tgagcaaagc 2460

tttgtctctt ttgttcataa gcactaccca actctggccc ctacgttaat tgttcgaatt 2520

gccaggatag ttattcaaac ctacggacag aaatattcag caacagaact gcaagaacga 2580

ataatcaagg cgccaattac ggtgtttaat gcacgtgacg atgccgtttc ttttatcgaa 2640

gaagcaacac cctacctaaa acatccccca gaaaatatca atcttaacgt tgatcatttc 2700

gaggtactta aggaatcagg tgttaacgaa ttagctcgat tcttgagttg a 2751

<210> 11
<211> 6984
<212> DNA
<213> Artificial Sequence

<220>
<223> NRPS being a synthetase of a fusion peptide consisting of
Asparagine and Indigoidine

<400> 11
atgcagacga acaaacaaca gacgttcagc gagctgctgc aaaccgtgca aaagcaagcc 60

ctggcgtctg ccacctacga tttcgcgccg ctgtacgaaa ttcagagcac aacagtgctg 120

aaacaggaat tgatcgatca tttggtcacg tttgaaaatt accccgatca ttcgatgaag 180

catctggaag aatcattagg gtttcaattc accgtagaaa gcggagatga gcagacctcc 240

tatgatttga acgtggtcgt cgccctcgct ccctcgaacg agctgtacgt gaagctaagc 300

tacaatgccg cggtgtatga atcgtcattc gtaaacagaa tcgaagggca tctccgcacc 360

gtcatcgacc aggtgatcgg caatccgcat gtacacctgc acgagatcgg catcatcacc 420

gaagaggaaa agcagcaact gctcgtcgcc tacaacgaca cggctgctga atatccgcgg 480

gacaaaacga ttttcgagct gatcgcggaa caagcgagcc ggacaccagc gaaagcagca 540

gttgtttgcg gcgaggacac cctgacctat caggagctga tggagcgttc tgcccagctt 600

gccaatgctt tgcgcgaaaa aggaatcgcc agcggcagca tcgtctcgat tatggcggaa 660

cattcactgg agctgatcgt ggcgatcatg gctgtcttgc ggtcaggtgc tgcctacttg 720

ccgattgatc ccgagtaccc gcaagatcgc atccagtatt tgctcgatga cagccagacc 780

acgctgctgt taacccagtc gcatctgcaa ccaaacatcc ggtttgcagg cagcgtgctt 840

tatttggacg atcgttcctt gtacgaaggc ggcagcacat ccttcgcacc cgagagcaag 900

cctgatgatt tggcgtacat gatctacact tccggttcta ccggcaatcc aaaaggggcg 960

atgattactc atcaaggcct ggtcaattac atctggtggg ccaacaaggt gtacgtccaa 1020

ggcgaagcgg tggactttcc gctgtactca tctatttcgt tcgatttgac cgtcacctcg 1080

atcttcacgc cgcttctgtc cggcaacacg attcatgtgt acagaggggc agacaaggta 1140

caggtcattt tggacatcat caaagataac aaagtcggga tcatcaagct gacgccgaca 1200

cacctgaagc tgattgaaca catcgacggc aaggccagca gcatcagacg gttcatcgtc 1260

ggcggcgaga acttgccgac aaagctggcg aagcaaatat acgaccattt cggagagaac 1320

gtgcaaattt tcaacgagta cggaccgacc gaaaccgttg tcggttgcat gatttacttg 1380

tatgacccgc aaacaacgac ccaggagtcg gtgccaatcg gtgtcccggc agacaacgtc 1440

cagctttatt tgctcgatgc ttccatgcag ccggtgcccg tcggctcgct tggcgaaatg 1500

tacatagccg gagacggcgt agccaaaggg tatttcaaca gaccggagct gacgaaggaa 1560

aagtttatcg acaacccgtt ccgtccggga accaaaatgt atcgaacagg cgacctggca 1620

aaatggctgc ctgatggaaa catggagtat gcaggcagaa tggactatca agtgaagatt 1680

cgcggccatc ggatcgagat gggcgaaatc gaaacgcgcc tgacgcagca tgaggcggtc 1740

aaggaagcgg tcgtgatcgt ggaaaaggat gagagcggcc aaaacgtgtt gtacgcgtac 1800

cttgtttccg agcgggaact gacggtagct gagctgagag aattttgggg gcgcacgctg 1860

ccttcctata tgattccttc cttctttatt cgcttggcgg aaattccgct gaccgcgaac 1920

ggaaaagtag agcgaaaaaa attgccgaag ccagctggcg cagtcgttac aggcaccgcg 1980

tatgcagctc cgcaaaatga aatcgaggca aagctggccg agatatggca gcaagtgctg 2040

ggcataagcc aggtagggat tcacgacgat ttctttgact tgggcggaca ctcgttgaag 2100

gcgatgactg tcgttttcca agtctcgaaa gcgctggaag tggaattgcc cgtaaaggcc 2160

ttgttcgaac atccaaccgt tgcggagctg gcccgcttcc tttcgcggtc ggaaaaaacc 2220

gagtacaccg cgattcaacc cgtggcagcg caggagtttt acccggtttc atctgcgcaa 2280

aaaagaatgt atatcctgca acagttcgaa ggcaacggaa tcagctacaa catttcgggt 2340

gcgattctcc tggaaggaaa gctggactac gcccggtttg ccagcgctgt gcaacagctg 2400

gcagagcgcc acgaagcttt gcgcacctcg ttccaccgga tcgacggcga gcctgtgcaa 2460

aaagtgcacg aggaagtaga agtgccgctt ttcatgctgg aggctcccga agaccaggcg 2520

gagaaaatca tgcgcgagtt tgtccgtccg tttgatctcg ggtcgctccc gctgatgcga 2580

acaggtttgc tcaagctggg caaagaccgc catttgtttt tgctcgacat gcaccatatc 2640

atctcggacg gcgtttcttc gcaaattttg ctgcgtgaat ttgccgagtt gtaccaggga 2700

gcagacttgc agccgctttc gctgcaatac aaagatttcg ctgcttggca aaatgagctg 2760

tttcagacgg aggcatacaa gaagcaggag cagcactggc tgaacacgtt tgctgatgaa 2820

attccgctct tgaacctgcc gactgactat ccgcgcccta gcgtgcaaag ctttgcaggc 2880

gatctcgtcc tttttgccgc cggaaaagaa ctgctggagc ggttgcaaca ggtagcgtca 2940

gaaacaggca ccaccttgta catgattttg cttgccgcct acaatgtgct gctgtccaag 3000

tataccggcc aggaagacat catcgtcggg acgcctgtcg ctggacgttc ccatgcggac 3060

gtggaaaaca tcatgggcat attcgtgaac acattggcgc tgcgcaacca gcctgccagc 3120

agcaaaacga tgttagaaaa taatattaca caatgtgact caatcaatga tgtttatctt 3180

aaagaagaag caataacatt gatggatatg cttgagagtc aacttaagca ccaggcagat 3240

ggatatgttg ttattgatca agaagaatct ctcagttacg ctgatttcta tttgagggtg 3300

aaagagatag ggtattgtct gtcagaaatt agctcaaaga attcggtggg tattgggctt 3360

ttttgtgatc cttctataga tttaatttgt ggtgcatggg gtattttgtc agcggataaa 3420

gcttatttgc cgttatcgcc tgactatcca actgaacgcc tcaaatatat gatagaagat 3480

tctggtattg atgtgatttt tacgcaatcg cacttaaaag cacagctaca ggacattgca 3540

ccaaaatcag tattaattat gacaccagaa gatgtcgctc tgacgataaa aacacgaaca 3600

atagaagata ttctgggcac agttcaagtt cctaaaccca ctagtctggc ttatattatt 3660

tatacctctg gtagcacggg taagccaaag ggagtgatga ttgaacatca cagtattgta 3720

aatcaaatga gatttcttgc aaaagcgttc aaattaggat gtcattcccg gattttacag 3780

aaaacaccaa tgagttttga tgcggctcaa tgggaaattc tagcgcctgc aattggtggt 3840

caagtgatta tgggtccttt aggttgctat cgcgatccgg atgcaattat taaaaccatt 3900

cttcagcatc aagtaacgac tttgcaatgt gttcctactt tgctacaagc gttactggat 3960

aatcctaatt ttttggattg cttatcattg actcaagtat tcagtggggg agaagcgctg 4020

acaaccaaat tagccacgca atttttgaat agttttactc actgtgaatt aatcaattta 4080

tatggcccga cagaatgtac gattaattca tcatttttcc gggtgacaaa tgagactttg 4140

ccgaattatc aaacctctat ttcgattggt gcacctgtag ataataccga atactacgtt 4200

cttgatgatg atagattacc tgtggcggtt ggcgaaattg gcgagcttta tatttcgggt 4260

gctcaattag cacgtggtta tttgcataaa ccagaaatga caaaagataa atttatttgt 4320

aatcaccttg tatcaggaac tcaacatcaa tggttatatc gaacgggaga tctggtaacc 4380

agaggggctg atggtaatac ttattttgtt ggtcgggttg atagccaggt caaattacga 4440

ggttaccgta ttgagcttga tgaaatacgc catgcgattg aagaacatag ctggataaag 4500

acggcggcaa tgttaattaa gaaggatgcc agaacgggtt tccaaaatct catcgcgtgt 4560

gtggaattag atgagaaaga agctgcattg atggatcaag gtaatagtag ctcacatcac 4620

aaatcaaaag ccgataaact acaggtgaaa gcccaacttt ctaattctgg ttgtcgaagt 4680

gaagagttat gtgaaaatcg ccctacattc ttacttcctt atcaagaagg ggagataaaa 4740

cagagagaat atgcatttgg acgcaagaca tatcgctatt ttgagggaac agaaataacg 4800

gtagagaaat taaaaaaatt gctgacagcc actcaatcga atgaaattag ctctttgcca 4860

ctgagtcatc taaccctgaa tgatttcggt tatgcattgc gttattttgg tcagtttacc 4920

agccatcaac gtttattgcc caaatatgcc tatgcttcac cgggtgctct ctatgcgaca 4980

caaatgtatt ttgaattgca taatgttctc ggtttggatg cggggattta ctattatcat 5040

ccagtgacac ataagttaat aaaaatttca acattgagtc gtcggcaaat gccaacgata 5100

aaagtgcatt ttattggcaa gcatgaagcc attgagcccg tttataagaa caatatacaa 5160

gaagttctgg aaatggaagc gggccatatg atgggtcttt ttgatgacgt attaccggaa 5220

attggcttga gtattggtaa aagtgaatat caagatgaat gtccagattg gtatgatggt 5280

gatattcagg attattatct tggtgcattt gaaatatgta gctatgaaca tggattgccg 5340

ccatttgaga ctgatattta tttacaaaca catgcccata aaatacctga gatgccgtgt 5400

ggtttatatc acttttctaa cggggaattt gtacgaataa gtgatgatat tgtccgaaaa 5460

aaggatgtta ttgcgattaa tcagcaagtt tatgatcgct ccagttttgg cgtgtcaatt 5520

attccacgct gtgtccctga atggcattat tatataacac tgggtcgtcg gttacatgcg 5580

ttacaaagta atccattgta tattggatta atgtcatctg gttacagttc gaagagcaat 5640

aacgatttac cttcggcgaa aaggatgcga tctattctca atgcacttga tagacctatg 5700

gcggcatttt atttctgcat aggtgggggt attagccaag cgcaatatat gtgtgaaggc 5760

atgaaagaag atgttgttca tatgaaaggg ccagttgaaa tcattaaaga tgatcttcaa 5820

caacaactcc ctcaatatat gattccaaat aaggtattag ttttcgataa attacctttg 5880

acggccaatg gaaaagtgga ttatcaatct ttatcagaat ctaaagccgt ggagaatgtt 5940

tcaacacagc gtctattggt gccattacat acagatactg aaataaggct tggaaaaatt 6000

tggatggaag tactgaaatg ggattcagta tctgccctcg atgatttttt cgaaagtggg 6060

ggtaattctt tgatggccgt tgcaatggtt aataagatca atgcggcctt taatattcgt 6120

tttccgttac agatactttt tcaatctcct aatatagcag aattggctaa gtggattgaa 6180

cagacagact ctaaaacaat atcaagatta attttattga atcaggcaag caaagacccc 6240

atttactgtt ggccgggttt gggcggatat cctatgagtt tgagattgct tgctaataaa 6300

gtcgttcctg atcgggcatt ttatggaata caggcatatg ggataaacga gagtgaaata 6360

ccgttttctt ctatccagag aatggcagaa gaggatatta aagagataaa gaaaatacag 6420

ccagaagggc catatatatt gtggggatat tcatttggtg cccgagtagc atttgaagtt 6480

gcataccagc ttgaacaagc gggagaagaa gttaacgcat tgaatttatt ggctccggga 6540

tctcctcatc ttgatatgaa gcaagcggaa tatatggata aaggcgctga atttactaat 6600

ccggcttttg ttaaaatact tttttctgta ttttctcgtt caatcaacag cccaatggtt 6660

aaaacttgct tagaacaagt aaatagtgaa acgacattta ttaactttat atgtagtcgt 6720

tttaaaaact tggaaccatc attagtaaaa cgtatcgtta ggattgtgac tttgacttat 6780

gatttcaagt acagtattga tgagctttat cacagacacc taaaggcacc tataactatt 6840

ttcaaggcga atagagataa tgattcattt atcgaggaat cggatgtgat ttcatcaatg 6900

tcgcctaaaa taattgaatt aatatcggat cactatcaac tgttggaaag tgaaggtgtt 6960

gctgagattg agaaaataat ctaa 6984

<210> 12
<211> 5450
<212> DNA
<213> Artificial Sequence

<220>
<223> Construct that enables easy cloning of NRPS modules in front of
      Indigoidine module through the exchange of ccdB.

<400> 12
actggctgtg tataagggag cctgacattt atattcccca gaacatcagg ttaatggcgt 60

ttttgatgtc attttcgcgg tggctgagat cagccacttc ttccccgata acggagaccg 120

gcacactggc catatcggtg gtcatcatgc gccagctttc atccccgata tgcaccaccg 180

ggtaaagttc acgggagact ttatctgaca gcagacgtgc actggccagg gggatcacca 240

tccgtcgccc gggcgtgtca ataatatcac tctgtacatc cacaaacaga cgataacggc 300

tctctctttt ataggtgtaa accttaaact gcatttcacc agcccctgtt ctcgtcagca 360

aaagagccgt tcatttcaat aaaccgggcg acctcagcca tcccttcctg attttccgct 420

ttccagcgtt cggcacgcag acgacgggct tcattctgca tggttgtgct taccagaccg 480

gagatattga catcatatat gccttgagca actgatagct gtcgctgtca actgtcactg 540
taatacgctg cttcatagca tacctctttt tgacatactt cgggtataca tatcagtata 600
tattcttata ccgcaaaaat cagcgcgcaa atacgcatac tgttatctgg cttttagtaa 660
gccggatcca cgcgtcggaa aaaaccgagt acaccgcgat tcaacccgtg gcagcgcagg 720
agttttaccc ggtttcatct gcgcaaaaaa gaatgtatat cctgcaacag ttcgaaggca 780
acggaatcag ctacaacatt tcgggtgcga ttctcctgga aggaaagctg gactacgccc 840
ggtttgccag cgctgtgcaa cagctggcag agcgccacga agctttgcgc acctcgttcc 900
accggatcga cggcgagcct gtgcaaaaag tgcacgagga agtagaagtg ccgcttttca 960
tgctggaggc tcccgaagac caggcggaga aaatcatgcg cgagtttgtc cgtccgtttg 1020
atctcggggt cgctccgctg atgcgaacag gtttgctcaa gctgggcaaa gaccgccatt 1080
tgtttttgct cgacatgcac catatcatct cggacggcgt ttcttcgcaa attttgctgc 1140
gtgaatttgc cgagttgtac cagggagcag acttgcagcc gctttcgctg caatacaaag 1200
atttcgctgc ttggcaaaat gagctgtttc agacggaggc atacaagaag caggagcagc 1260
actggctgaa cacgtttgct gatgaaattc cgctcttgaa cctgccgact gactatccgc 1320
gccctagcgt gcaaagcttt gcaggcgatc tcgtcctttt tgccgccgga aaagaactgc 1380
tggagcggtt gcaacaggta gcgtcagaaa caggcaccac cttgtacatg attttgcttg 1440
ccgcctacaa tgtgctgctg tccaagtata ccggccagga agacatcatc gtcgggacgc 1500
ctgtcgctgg acgttcccat gcggacgtgg aaaacatcat gggcatattc gtgaacacat 1560
tggcgctgcg caaccagcct gccagcagca aaacgatgtt agaaaataat attacacaat 1620
gtgactcaat caatgatgtt tatcttaaag aagaagcaat aacattgatg gatatgcttg 1680
agagtcaact taagcaccag gcagatggat atgttgttat tgatcaagaa gaatctctca 1740
gttacgctga tttctatttg agggtgaaag agatagggta ttgtctgtca gaaattagct 1800
caaagagttc ggtgggtatt gggctttttt gtgatccttc tatagattta atttgtggtg 1860
catggggtat tttgtcagcg gataaagctt atttgccgtt atcgcctgac tatccaactg 1920
aacgcctcaa atatatgata gaagattctg gtattgatgt gatttttacg caatcgcact 1980
taaaagcaca gctacaggac attgcaccaa aatcagtatt aattatgaca ccagaagatg 2040
tcgctctgac gataaaaaca cgaacaatag aagatattct gggcacagtt caagttccta 2100
aacccacgag tctggcttat attatttata cctctggtag cacgggtaag ccaaagggag 2160
tgatgattga acatcacagt attgtaaatc aaatgagatt tcttgcaaaa gcgttcaaat 2220
taggatgtca ttcccggatt ttacagaaaa caccaatgag ttttgatgcg gctcaatggg 2280
aaattctagc gcctgcaatt ggtggtcaag tgattatggg tcctttaggt tgctatcgcg 2340
atccggatgc aattattaaa accattcttc agcatcaagt aacgactttg caatgtgttc 2400

ctactttgct acaagcgtta ctggataatc ctaatttttt ggattgctta tcattgactc 2460

aagtattcag tgggggagaa gcgctgacaa ccaaattagc cacgcaattt ttgaatagtt 2520

ttactcactg tgaattaatc aatttatatg gcccgacaga atgtacgatt aattcatcat 2580

ttttccgggt gacaaatgag actttgccga attatcaaac ctctatttcg attggtgcac 2640

ctgtagataa taccgaatac tacgttcttg atgatgatag attacctgtg gcggttggcg 2700

aaattggcga gctttatatt tcgggtgctc aattagcacg tggttatttg cataaaccag 2760

aaatgacaaa agataaattt atttgtaatc accttgtatc aggaactcaa catcaatggt 2820

tatatcgaac gggagatctg gtaaccagag gggctgatgg taatacttat tttgttggtc 2880

gggttgatag ccaggtcaaa ttacgaggtt accgtattga gcttgatgaa atacgccatg 2940

cgattgaaga acatagctgg ataaagacgg cggcaatgtt aattaagaag gatgccagaa 3000

cgggtttcca aaatctcatc gcgtgtgtgg aattagatga gaaagaagct gcattgatgg 3060

atcaaggtaa tagtagctca catcacaaat caaaagccga taaactacag gtgaaagccc 3120

aactttctaa ttctggttgt cgaagtgaag agttatgtga aaatcgccct acattcttac 3180

ttccttatca agaaggggag ataaaacaga gagaatatgc atttggacgc aagacatatc 3240

gctattttga gggaacagaa ataacggtag agaaattaaa aaaattgctg acagccactc 3300

aatcgaatga aattagctct ttgccactga gtcatctaac cctgaatgat ttcggttatg 3360

cattgcgtta ttttggtcag tttaccagcc atcaacgttt attgcccaaa tatgcctatg 3420

cttcaccggg tgctctctat gcgacacaaa tgtattttga attgcataat gttctcggtt 3480

tggatgcggg gatttactat tatcatccag tgacacataa gttaataaaa atttcaacat 3540

tgagtcgtcg gcaaatgcca acgataaaag tgcattttat tggcaagcat gaagccattg 3600

agcccgttta taagaacaat atacaagaag ttctggaaat ggaagcgggc catatgatgg 3660

gtctttttga tgacgtatta ccggaaattg gcttgagtat tggtaaaagt gaatatcaag 3720

atgaatgtcc agattggtat gatggtgata ttcaggatta ttatcttggt gcatttgaaa 3780

tatgtagcta tgaacatgga ttgccgccat ttgagactga tatttattta caaacacatg 3840

cccataaaat acctgagatg ccgtgtggtt tatatcactt ttctaacggg gaatttgtac 3900

gaataagtga tgatattgtc cgaaaaaagg atgttattgc gattaatcag caagtttatg 3960

atcgctccag ttttggcgtg tcaattattc cacgctgtgt ccctgaatgg cattattata 4020

taacactggg tcgtcggtta catgcgttac aaagtaatcc attgtatatt ggattaatgt 4080

catctggtta cagttcgaag agcaataacg atttaccttc ggcgaaaagg atgcgatcta 4140

ttctcaatgc acttgataga cctatggcgg catttatatt ctgcataggt gggggtatta 4200

gccaagcgca atatatgtgt gaaggcatga aagaagatgt tgttcatatg aaagggccag 4260

ttgaaatcat taaagatgat cttcaacaac aactccctca atatatgatt ccaaataagg 4320

tattagtttt cgataaatta cctttgacgg ccaatggaaa agtggattat caatctttat 4380

cagaatctaa agccgtggag aatgtttcaa cacagcgtct attggtgcca ttacatacag 4440

atactgaaat aaggcttgga aaaatttgga tggaagtact gaaatgggat tcagtatctg 4500

ccctcgatga ttttttcgaa agtgggggta attctttgat ggccgttgca atggttaata 4560

agatcaatgc ggcctttaat attcgttttc cgttacagat actttttcaa tctcctaata 4620

tagcagaatt ggctaagtgg attgaacaga cagactctaa aacaatatca agattaattt 4680

tattgaatca ggcaagcaaa gaccccattt actgttggcc gggtttgggc ggatatccta 4740

tgagtttgag attgcttgct aataaagtcg ttcctgatcg ggcattttat ggaatacagg 4800

catatgggat aaacgagagt gaaataccgt tttcttctat ccagagaatg gcagaagagg 4860

atattaaaga gataaagaaa atacagccag aagggccata tatattgtgg ggatattcat 4920

ttggtgcccg agtagcattt gaagttgcat accagcttga acaagcggga gaagaagtta 4980

acgcattgaa tttattggct ccgggatctc ctcatcttga tatgaagcaa gcggaatata 5040

tggataaagg cgctgaattt actaatccgg cttttgttaa aatacttttt tctgtatttt 5100

ctcgttcaat caacagccca atggttaaaa cttgcttaga acaagtaaat agtgaaacga 5160

catttattaa ctttatatgt agtcgtttta aaaacttgga accatcatta gtaaaacgta 5220

tcgttaggat tgtgactttg acttatgatt tcaagtacag tattgatgag ctttatcaca 5280

gacacctaaa ggcacctata actattttca aggcgaatag agataatgat tcatttatcg 5340

aggaatcgga tgtgatttca tcaatgtcgc ctaaaataat tgaattaata tcggatcact 5400

atcaactgtt ggaaagtgaa ggtgttgctg agattgagaa aataatctaa 5450

<210> 13
<211> 9666
<212> DNA
<213> Artificial Sequence

<220>
<223> NRPS synthesizing a Indigoidine-tagged Dipeptide consisting of
      Ornithine and Valine

<400> 13
atgctgcaca gcttcctcgc aaccaaaaca gcctatccga cggacaaaac gttccagaag 60

ctgttcgagg agcaagtgga aaaaacaccg aacgagattg ccgttctgtt cggcaatgaa 120

cagctgacct atcaggagtt gaatgcaaaa gcaaaccagc tcgcccgcgt cctgcggcga 180

aaaggcgtca agccggagag caccgtcggc atcctcgtag accgctcgct ctacatggtc 240

atcggcatgc tggccgtgtt gaaagcaggc ggaacattcg tcccgattga tccggactac 300

ccgctggagc gccaagcgtt catgctcgaa gacagcgagg cgaagctgct gctcaccttg 360

caaaaaatga acagtcaagt tgccttccct tatgaaacct tttatctgga tacagagaca 420

gtggatcagg aggagacggg caatctggag cacgttgcgc agccggagaa cgtcgcttac 480

atcatctaca catccggtac gacgggcaag ccaaaagggg tcgtcatcga gcaccgcagc 540

tatgccaatg tcgcatttgc ctggaaagac gaatatcacc tggacagctt cccggtccgt 600

ttgctgcaaa tggcgagctt cgcctttgac gtctcgacgg gcgattttgc cagggcgctg 660

ctgacaggcg ggcaactggt catctgcccg aatggggtca aaatggaccc agcttcgctg 720

tacgagacca tcaggcgtca cgaaattacc attttcgaag cgacacccgc cttgatcatg 780

ccgttgatgc actacgttta cgaaaacgaa ctggatatga gccaaatgaa gctgctgatt 840

ctcggagcag acagctgccc ggcggaagac ttcaaaacgt tgctcgcgcg cttcggtcag 900

aagatgcgca ttatcaacag ctacggcgtg acagaggcgt gcattgacac cagctactac 960

gaagaaacag acgtcaccgc catccgctcg ggaacggtgc cgatcggcaa accgcttccg 1020

aacatgacga tgtacgtggt cgatgcgcat ttgaatttgc agcctgtcgg cgtcgtaggc 1080

gaattgtgca tcggcggagc aggggttgcg cgcggttatt tgaacagacc tgagctgacg 1140

gaagagaagt tcgtgccgaa tccgttcgcc ccaggtgaac gattgtaccg cacaggtgat 1200

ctggcgaagt ggcgcgcaga tggcaatgtc gagttcctcg gacgcaatga ccaccaggta 1260

aaaatcaggg gtgtccgcat cgagctgggc gagatcgaga cacaactgcg caagctggac 1320

ggaattacgg aagcagtcgt ggttgcgaga gaagatcgcg ggcaggaaaa ggaattgtgc 1380

gcatacgtcg tggcggacca caagcttgac accgcagaat tgcgggcgaa tttgctgaag 1440

gaactgccgc aagcgatgat tccagcgtat ttcgtcacct tggatgcgct gccgctgact 1500

gccaatggca aagtagacag acgttccttg ccagcgccgg atgtcaccat gctgagaacg 1560

accgagtatg tagcgccgcg ctccgtctgg gaagcccgat tggcccaagt atgggagcag 1620

gtgctgaatg ttccgcaagt gggtgcgcta gacgactttt tcgcgctcgg cggtcactca 1680

ttgcgtgcca tgcgcgtcct ttccagcatg cacaacgaat accaggtcga catcccgctg 1740

cgcatcttgt tcgaaaaacc gacgattcag gaactggcgg cgttcatcga aacgagcgga 1800

aaagagacgt atgtgccgat cgagcctgca ccgttgcaag agtattatcc tgtttcatct 1860

gcgcaaaagc ggatgtatgt cctgcgccag tttgcggaca caggcacggt ttataacatg 1920

ccgagcgcgt tgtatatcga aggcgatctg gatcggaagc gttttgaagc cgccatccac 1980

ggattggtcg agcggcacga atcgctgcgc acatccttcc acaccgtaaa tggcgagcct 2040

gtccagcgcg tacacgagca tgtcgagctg aatgtgcagt acgcggaagt gacggaagcg 2100

caagtggagc caaccgtcga gtcgttcgtg caagcatttg atctgacaaa agctccgcta 2160

ttgcgggtcg gacttttcaa gctggcagcg aaacggcatc tgttcctgct ggatatgcat 2220

cacatcatct cggatggcgt ctcggccgga atcattatgg aagagttctc gaagctgtat 2280

cgaggcgaag aactgcctgc gctttccgtc cattacaaag atttcgccgt ctggcagtct 2340

gaactgttcc agagcgacgt ctataccgag catgaaaact actggctgaa cgcgttttct 2400

ggcgacattc cggtgcttaa cttgccagcc gatttttctc gtccgctgac acagagcttt 2460

gaaggagatt gcgtttcgtt ccaggcagac aaagcgttgc tggacgatct tcacaagctc 2520

gctcaggaga gccaatcgac gttgttcatg gtattgctgg cggcttacaa tgtgctgctt 2580

gccaagtaca gcggacagga agacatcgtc gtcggcacac cgattgcggg cagatcgcac 2640

gccgatatcg agaacgttct ggggatgttt gtcaacacgc tcgctttgcg caactatccg 2700

gtcgagacga aacacttcca ggcatttttg gaagaggtca agcaaaatac gctgcaagca 2760

tacgcccatc aagattatcc gttcgaagca ctggtcgaaa agctggacat ccagcgggat 2820

ctcagccgca atccgctgtt tgacaccatg tttattttgc aaaacctgga ccaaaaagct 2880

tacgagctgg atgggctgaa actggaggca tatccggcac aagcaggcaa cgccaaattc 2940

gatctcacgc tggaagcgca cgaggacgag acaggcattc attttgcgct cgtctactcg 3000

accaaattgt tccagcgaga atcaatcgaa agaatggcgg gtcacttcct gcaagtgctg 3060

cgccaagtcg ttgccgacca agcaactgcc ttgcgcgaga tcagcctgct cagcgaggaa 3120

gagcgccgaa ttgtgaccgt tgatttcaac aacacgtttg cctatccgcg cgatctgacg 3180

attcaggagc tgttcgagca gcaggcagca aaaactccgg agcatgcagc ggtcgtgatg 3240

gacggacaga tgctgacgta tcgggagctg aacgaaaaag cgaaccagct cgcccatgtc 3300

cttcgtcaaa acggagtcgg gaaagagagc atcgtcggtc tgctcgcaga tcgttcgctg 3360

gaaatgatta caggcatcat ggggattctc aaagcgggcg gcgcctacct gggactggac 3420

ccggagcatc cgtccgaacg cctggcttac atgttggaag atggcggcgt gaaagttgtc 3480

ctcgtgcaaa agcacttgct gccgctcgtc ggcgaagggc tgatgccaat cgttttggaa 3540

gaggagagcc tgcgcccgga agattgcggc aatccggcga ttgtcaacgg tgcgagtgac 3600

ctggcttatg tgatgtacac ctcaggctct acaggcaagc caaaaggagt catggtcgag 3660

catcgcaacg tcacccgctt ggtcatgcat acgaattacg tgcaagtgcg cgagagcgac 3720

cggatgattc aaaccggcgc gattggcttc gacgccatga catttgagat ttttggagcc 3780

ttgctgcacg gggccagcct gtatttggtg agcaaggacg tcttgctgga tgccgaaaag 3840

ctgggcgact tcctgcggac gaatcagatt acgaccatgt ggctgacctc gccgctcttc 3900

aaccagcttt cgcaagacaa tccggcgatg tttgacagct tgcgcgcctt gatcgtcggt 3960

ggcgaagcgt tgtcgccgaa gcacatcaac cgggtaaaaa gtgcccttcc tgacctggaa 4020

atctggaacg gatacggccc gaccgaaaac acgaccttct cgacgtgcta tttgattgag 4080

cagcattttg aagagcagat tccgatcggc aagccgattg caaactccac cgcgtatatc 4140

gtcgacggca acaatcagcc gcagccgatc ggcgtaccgg gtgaactgtg cgtcggtggt 4200

gacggtgtcg caagaggcta tgtgaacaag ccggaattaa ccgccgaaaa gtttgtgccc 4260

aatccgtttg cgcctggcga aacgatgtat cgcaccggag atttggcgag atggctgccg 4320

gatgggacga ttgagtattt gggccgaatc gaccagcagg tcaaaatcag gggataccgg 4380

atcgagcttg gggaaatcga gacggtcttg tcccagcagg cacaagtaaa agaagcagtc 4440

gtggccgtga tcgaggaggc gaacgggcaa aaagctctct gcgcttactt tgtgccagaa 4500

caggccgtcg acgccgcaga gctgcgagaa gcgatgtcca aacaattgcc tggctacatg 4560

gtccctgctt actatgtgca aatggaaaag ctgccgttga ccgcgaacgg aaaggtcgac 4620

cgccgggcat tgccgcagcc atccggcgag cggacgacag gaagcgcctt tgtcgctgcg 4680

caaaatgata ccgaagcgaa gctgcaacag atttggcaag aagttttggg cattccggca 4740

atcggcattc acgacaactt ctttgaaatc ggcggtcatt ccttgaaggc gatgaacgtc 4800

atcacgcaag tccataaaac attccaggtg gagctgccgt taaaagcgct gtttgccact 4860

ccgacgatcc atgagttggc tgcgcatatt tcggaaaaaa ccgagtacac cgcgattcaa 4920

cccgtggcag cgcaggagtt ttacccggtt tcatctgcgc aaaaaagaat gtatatcctg 4980

caacagttcg aaggcaacgg aatcagctac aacatttcgg gtgcgattct cctggaagga 5040

aagctggact acgcccggtt tgccagcgct gtgcaacagc tggcagagcg ccacgaagct 5100

ttgcgcacct cgttccaccg gatcgacggc gagcctgtgc aaaaagtgca cgaggaagta 5160

gaagtgccgc ttttcatgct ggaggctccc gaagaccagg cggagaaaat catgcgcgag 5220

tttgtccgtc cgtttgatct cggggtcgct ccgctgatgc gaacaggttt gctcaagctg 5280

ggcaaagacc gccatttgtt tttgctcgac atgcaccata tcatctcgga cggcgtttct 5340

tcgcaaattt tgctgcgtga atttgccgag ttgtaccagg gagcagactt gcagccgctt 5400

tcgctgcaat acaaagattt cgctgcttgg caaaatgagc tgtttcagac ggaggcatac 5460

aagaagcagg agcagcactg gctgaacacg tttgctgatg aaattccgct cttgaacctg 5520

ccgactgact atccgcgccc tagcgtgcaa agctttgcag gcgatctcgt cctttttgcc 5580

gccggaaaag aactgctgga gcggttgcaa caggtagcgt cagaaacagg caccaccttg 5640

tacatgattt tgcttgccgc ctacaatgtg ctgctgtcca agtataccgg ccaggaagac 5700

atcatcgtcg ggacgcctgt cgctggacgt tcccatgcgg acgtggaaaa catcatgggc 5760

atattcgtga acacattggc gctgcgcaac cagcctgcca gcagcaaaac gatgttagaa 5820

aataatatta cacaatgtga ctcaatcaat gatgtttatc ttaaagaaga agcaataaca 5880

ttgatggata tgcttgagag tcaacttaag caccaggcag atggatatgt tgttattgat 5940

caagaagaat ctctcagtta cgctgatttc tatttgaggg tgaaagagat agggtattgt 6000

ctgtcagaaa ttagctcaaa gaattcggtg ggtattgggc ttttttgtga tccttctata 6060

gatttaattt gtggtgcatg gggtattttg tcagcggata aagcttattt gccgttatcg 6120

cctgactatc caactgaacg cctcaaatat atgatagaag attctggtat tgatgtgatt 6180

tttacgcaat cgcacttaaa agcacagcta caggacattg caccaaaatc agtattaatt 6240

atgacaccag aagatgtcgc tctgacgata aaaacacgaa caatagaaga tattctgggc 6300

acagttcaag ttcctaaacc cactagtctg gcttatatta tttatacctc tggtagcacg 6360

ggtaagccaa agggagtgat gattgaacat cacagtattg taaatcaaat gagatttctt 6420

gcaaaagcgt tcaaattagg atgtcattcc cggattttac agaaaacacc aatgagtttt 6480

gatgcggctc aatgggaaat tctagcgcct gcaattggtg gtcaagtgat tatgggtcct 6540

ttaggttgct atcgcgatcc ggatgcaatt attaaaacca ttcttcagca tcaagtaacg 6600

actttgcaat gtgttcctac tttgctacaa gcgttactgg ataatcctaa ttttttggat 6660

tgcttatcat tgactcaagt attcagtggg ggagaagcgc tgacaaccaa attagccacg 6720

caatttttga atagttttac tcactgtgaa ttaatcaatt tatatggccc gacagaatgt 6780

acgattaatt catcattttt ccgggtgaca aatgagactt tgccgaatta tcaaacctct 6840

atttcgattg gtgcacctgt agataatacc gaatactacg ttcttgatga tgatagatta 6900

cctgtggcgg ttggcgaaat tggcgagctt tatatttcgg gtgctcaatt agcacgtggt 6960

tatttgcata aaccagaaat gacaaaagat aaatttattt gtaatcacct tgtatcagga 7020

actcaacatc aatggttata tcgaacggga gatctggtaa ccagaggggc tgatggtaat 7080

acttattttg ttggtcgggt tgatagccag gtcaaattac gaggttaccg tattgagctt 7140

gatgaaatac gccatgcgat tgaagaacat agctggataa agacggcggc aatgttaatt 7200

aagaaggatg ccagaacggg tttccaaaat ctcatcgcgt gtgtggaatt agatgagaaa 7260

gaagctgcat tgatggatca aggtaatagt agctcacatc acaaatcaaa agccgataaa 7320

ctacaggtga aagcccaact ttctaattct ggttgtcgaa gtgaagagtt atgtgaaaat 7380

cgccctacat tcttacttcc ttatcaagaa ggggagataa aacagagaga atatgcattt 7440

ggacgcaaga catatcgcta ttttgaggga acagaaataa cggtagagaa attaaaaaaa 7500

ttgctgacag ccactcaatc gaatgaaatt agctctttgc cactgagtca tctaaccctg 7560

aatgatttcg gttatgcatt gcgttatttt ggtcagttta ccagccatca acgtttattg 7620

cccaaatatg cctatgcttc accgggtgct ctctatgcga cacaaatgta ttttgaattg 7680

cataatgttc tcggtttgga tgcggggatt tactattatc atccagtgac acataagtta 7740

ataaaaattt caacattgag tcgtcggcaa atgccaacga taaaagtgca ttttattggc 7800

aagcatgaag ccattgagcc cgtttataag aacaatatac aagaagttct ggaaatggaa 7860

gcgggccata tgatgggtct ttttgatgac gtattaccgg aaattggctt gagtattggt 7920

aaaagtgaat atcaagatga atgtccagat tggtatgatg gtgatattca ggattattat 7980

cttggtgcat ttgaaatatg tagctatgaa catggattgc cgccatttga gactgatatt 8040

tatttacaaa cacatgccca taaaatacct gagatgccgt gtggtttata tcacttttct 8100

aacggggaat ttgtacgaat aagtgatgat attgtccgaa aaaaggatgt tattgcgatt 8160

aatcagcaag tttatgatcg ctccagtttt ggcgtgtcaa ttattccacg ctgtgtccct 8220

gaatggcatt attatataac actgggtcgt cggttacatg cgttacaaag taatccattg 8280

tatattggat taatgtcatc tggttacagt tcgaagagca ataacgattt accttcggcg 8340

aaaaggatgc gatctattct caatgcactt gatagaccta tggcggcatt ttatttctgc 8400

ataggtgggg gtattagcca agcgcaatat atgtgtgaag gcatgaaaga agatgttgtt 8460

catatgaaag ggccagttga aatcattaaa gatgatcttc aacaacaact ccctcaatat 8520

atgattccaa ataaggtatt agttttcgat aaattacctt tgacggccaa tggaaaagtg 8580

gattatcaat ctttatcaga atctaaagcc gtggagaatg tttcaacaca gcgtctattg 8640

gtgccattac atacagatac tgaaataagg cttggaaaaa tttggatgga agtactgaaa 8700

tgggattcag tatctgccct cgatgatttt ttcgaaagtg ggggtaattc tttgatggcc 8760

gttgcaatgg ttaataagat caatgcggcc tttaatattc gttttccgtt acagatactt 8820

tttcaatctc ctaatatagc agaattggct aagtggattg aacagacaga ctctaaaaca 8880

atatcaagat taattttatt gaatcaggca agcaaagacc ccatttactg ttggccgggt 8940

ttgggcggat atcctatgag tttgagattg cttgctaata aagtcgttcc tgatcgggca 9000

ttttatggaa tacaggcata tgggataaac gagagtgaaa taccgttttc ttctatccag 9060

agaatggcag aagaggatat taaagagata aagaaaatac agccagaagg gccatatata 9120

ttgtggggat attcatttgg tgcccgagta gcatttgaag ttgcatacca gcttgaacaa 9180

gcgggagaag aagttaacgc attgaattta ttggctccgg gatctcctca tcttgatatg 9240

aagcaagcgg aatatatgga taaaggcgct gaatttacta atccggcttt tgttaaaata 9300

cttttttctg tattttctcg ttcaatcaac agcccaatgg ttaaaacttg cttagaacaa 9360

gtaaatagtg aaacgacatt tattaacttt atatgtagtc gttttaaaaa cttggaacca 9420

tcattagtaa aacgtatcgt taggattgtg actttgactt atgatttcaa gtacagtatt 9480

gatgagcttt atcacagaca cctaaaggca cctataacta ttttcaaggc gaatagagat 9540

aatgattcat ttatcgagga atcggatgtg atttcatcaa tgtcgcctaa aataattgaa 9600

ttaatatcgg atcactatca actgttggaa agtgaaggtg ttgctgagat tgagaaaata 9660

atctaa 9666

<210> 14
<211> 12771

<212>  DNA
<213>  Artificial Sequence

<220>
<223>  NRPS synthesizing a Indigoidine-tagged Tripeptide consisting of
       Ornithine and two Valines

<400>  14
atgctgcaca gcttcctcgc aaccaaaaca gcctatccga cggacaaaac gttccagaag      60

ctgttcgagg agcaagtgga aaaaacaccg aacgagattg ccgttctgtt cggcaatgaa     120

cagctgacct atcaggagtt gaatgcaaaa gcaaaccagc tcgcccgcgt cctgcggcga     180

aaaggcgtca agccggagag caccgtcggc atcctcgtag accgctcgct ctacatggtc     240

atcggcatgc tggccgtgtt gaaagcaggc ggaacattcg tcccgattga tccggactac     300

ccgctggagc gccaagcgtt catgctcgaa gacagcgagg cgaagctgct gctcaccttg     360

caaaaaatga acagtcaagt tgccttccct tatgaaacct tttatctgga tacagagaca     420

gtggatcagg aggagacggg caatctggag cacgttgcgc agccggagaa cgtcgcttac     480

atcatctaca catccggtac gacgggcaag ccaaaagggg tcgtcatcga gcaccgcagc     540

tatgccaatg tcgcatttgc ctggaaagac gaatatcacc tggacagctt cccggtccgt     600

ttgctgcaaa tggcgagctt cgcctttgac gtctcgacgg gcgattttgc cagggcgctg     660

ctgacaggcg ggcaactggt catctgcccg aatggggtca aaatggaccc agcttcgctg     720

tacgagacca tcaggcgtca cgaaattacc attttcgaag cgacacccgc cttgatcatg     780

ccgttgatgc actacgttta cgaaaacgaa ctggatatga gccaaatgaa gctgctgatt     840

ctcggagcag acagctgccc ggcggaagac ttcaaaacgt tgctcgcgcg cttcggtcag     900

aagatgcgca ttatcaacag ctacggcgtg acagaggcgt gcattgacac cagctactac     960

gaagaaacag acgtcaccgc catccgctcg ggaacggtgc cgatcggcaa accgcttccg    1020

aacatgacga tgtacgtggt cgatgcgcat ttgaatttgc agcctgtcgg cgtcgtaggc    1080

gaattgtgca tcggcggagc aggggttgcg cgcggttatt tgaacagacc tgagctgacg    1140

gaagagaagt tcgtgccgaa tccgttcgcc ccaggtgaac gattgtaccg cacaggtgat    1200

ctggcgaagt ggcgcgcaga tggcaatgtc gagttcctcg gacgcaatga ccaccaggta    1260

aaaatcaggg gtgtccgcat cgagctgggc gagatcgaga cacaactgcg caagctggac    1320

ggaattacgg aagcagtcgt ggttgcgaga gaagatcgcg ggcaggaaaa ggaattgtgc    1380

gcatacgtcg tggcggacca caagcttgac accgcagaat tgcgggcgaa tttgctgaag    1440

gaactgccgc aagcgatgat tccagcgtat ttcgtcacct tggatgcgct gccgctgact    1500

gccaatggca aagtagacag acgttccttg ccagcgccgg atgtcaccat gctgagaacg    1560

accgagtatg tagcgccgcg ctccgtctgg gaagcccgat tggcccaagt atgggagcag    1620

gtgctgaatg ttccgcaagt gggtgcgcta gacgactttt tcgcgctcgg cggtcactca    1680

ttgcgtgcca tgcgcgtcct ttccagcatg cacaacgaat accaggtcga catcccgctg 1740

cgcatcttgt tcgaaaaacc gacgattcag gaactggcgg cgttcatcga aacgagcgga 1800

aaagagacgt atgtgccgat cgagcctgca ccgttgcaag agtattatcc tgtttcatct 1860

gcgcaaaagc ggatgtatgt cctgcgccag tttgcggaca caggcacggt ttataacatg 1920

ccgagcgcgt tgtatatcga aggcgatctg gatcggaagc gttttgaagc cgccatccac 1980

ggattggtcg agcggcacga atcgctgcgc acatccttcc acaccgtaaa tggcgagcct 2040

gtccagcgcg tacacgagca tgtcgagctg aatgtgcagt acgcggaagt gacggaagcg 2100

caagtggagc caaccgtcga gtcgttcgtg caagcatttg atctgacaaa agctccgcta 2160

ttgcgggtcg gacttttcaa gctggcagcg aaacggcatc tgttcctgct ggatatgcat 2220

cacatcatct cggatggcgt ctcggccgga atcattatgg aagagttctc gaagctgtat 2280

cgaggcgaag aactgcctgc gctttccgtc cattacaaag atttcgccgt ctggcagtct 2340

gaactgttcc agagcgacgt ctataccgag catgaaaact actggctgaa cgcgttttct 2400

ggcgacattc cggtgcttaa cttgccagcc gatttttctc gtccgctgac acagagcttt 2460

gaaggagatt gcgtttcgtt ccaggcagac aaagcgttgc tggacgatct tcacaagctc 2520

gctcaggaga gccaatcgac gttgttcatg gtattgctgg cggcttacaa tgtgctgctt 2580

gccaagtaca gcggacagga agacatcgtc gtcggcacac cgattgcggg cagatcgcac 2640

gccgatatcg agaacgttct ggggatgttt gtcaacacgc tcgctttgcg caactatccg 2700

gtcgagacga aacacttcca ggcatttttg gaagaggtca agcaaaatac gctgcaagca 2760

tacgcccatc aagattatcc gttcgaagca ctggtcgaaa agctggacat ccagcgggat 2820

ctcagccgca atccgctgtt tgacaccatg tttattttgc aaaacctgga ccaaaaagct 2880

tacgagctgg atgggctgaa actggaggca tatccggcac aagcaggcaa cgccaaattc 2940

gatctcacgc tggaagcgca cgaggacgag acaggcattc attttgcgct cgtctactcg 3000

accaaattgt tccagcgaga atcaatcgaa agaatggcgg gtcacttcct gcaagtgctg 3060

cgccaagtcg ttgccgacca agcaactgcc ttgcgcgaga tcagcctgct cagcgaggaa 3120

gagcgccgaa ttgtgaccgt tgatttcaac aacacgtttg ccgcgtatcc gcgcgatctg 3180

acgattcagg agctgttcga gcagcaggca gcaaaaactc cggagcatgc agcggtcgtg 3240

atggacggac agatgctgac gtatcgggag ctgaacgaaa aagcgaacca gctcgcccat 3300

gtccttcgtc aaaacggagt cgggaaagag agcatcgtcg gtctgctcgc agatcgttcg 3360

ctggaaatga ttacaggcat catggggatt ctcaaagcgg gcggcgccta cctgggactg 3420

gacccggagc atccgtccga acgcctggct tacatgttgg aagatggcgg cgtgaaagtt 3480

gtcctcgtgc aaaagcactt gctgccgctc gtcggcgaag ggctgatgcc aatcgttttg 3540

gaagaggaga gcctgcgccc ggaagattgc ggcaatccgg cgattgtcaa cggtgcgagt 3600

gacctggctt atgtgatgta cacctcaggc tctacaggca agccaaaagg agtcatggtc 3660

gagcatcgca acgtcacccg cttggtcatg catacgaatt acgtgcaagt gcgcgagagc 3720

gaccggatga ttcaaaccgg cgcgattggc ttcgacgcca tgacatttga gatttttgga 3780

gccttgctgc acggggccag cctgtatttg gtgagcaagg acgtcttgct ggatgccgaa 3840

aagctgggcg acttcctgcg gacgaatcag attacgacca tgtggctgac ctcgccgctc 3900

ttcaaccagc tttcgcaaga caatccggcg atgtttgaca gcttgcgcgc cttgatcgtc 3960

ggtggcgaag cgttgtcgcc gaagcacatc aaccgggtaa aaagtgccct tcctgacctg 4020

gaaatctgga acggatacgg cccgaccgaa aacacgacct tctcgacgtg ctatttgatt 4080

gagcagcatt ttgaagagca gattccgatc ggcaagccga ttgcaaactc caccgcgtat 4140

atcgtcgacg gcaacaatca gccgcagccg atcggcgtac cgggtgaact gtgcgtcggt 4200

ggtgacggtg tcgcaagagg ctatgtgaac aagccggaat taaccgccga aaagtttgtg 4260

cccaatccgt ttgcgcctgg cgaaacgatg tatcgcaccg gagatttggc gagatggctg 4320

ccggatggga cgattgagta tttgggccga atcgaccagc aggtcaaaat caggggatac 4380

cggatcgagc ttggggaaat cgagacggtc ttgtcccagc aggcacaagt aaaagaagca 4440

gtcgtggccg tgatcgagga ggcgaacggg caaaaagctc tctgcgctta ctttgtgcca 4500

gaacaggccg tcgacgccgc agagctgcga gaagcgatgt ccaaacaatt gcctggctac 4560

atggtccctg cttactatgt gcaaatggaa aagctgccgt tgaccgcgaa cggaaaggtc 4620

gaccgccggg cattgccgca gccatccggc gagcggacga caggaagcgc ctttgtcgct 4680

gcgcaaaatg ataccgaagc gaagctgcaa cagatttggc aagaagtttt gggcattccg 4740

gcaatcggca ttcacgacaa cttctttgaa atcggcggtc attccttgaa ggcgatgaac 4800

gtcatcacgc aagtccataa aacattccag gtggagctgc cgttaaaagc gctgtttgcc 4860

actccgacga tccatgagtt ggctgcgcat attgccacga gcggaaaaga gacgtatgtg 4920

ccgatcgagc ctgcaccgtt gcaagagtat tatcctgttt catctgcgca aaagcggatg 4980

tatgtcctgc gccagtttgc ggacacaggc acggtttata acatgccgag cgcgttgtat 5040

atcgaaggcg atctggatcg gaagcgtttt gaagccgcca tccacggatt ggtcgagcgg 5100

cacgaatcgc tgcgcacatc cttccacacc gtaaatggcg agcctgtcca gcgcgtacac 5160

gagcatgtcg agctgaatgt gcagtacgcg gaagtgacgg aagcgcaagt ggagccaacc 5220

gtcgagtcgt tcgtgcaagc atttgatctg acaaaagctc cgctattgcg ggtcggactt 5280

ttcaagctgg cagcgaaacg gcatctgttc ctgctggata tgcatcacat catctcggat 5340

ggcgtctcgg ccggaatcat tatggaagag ttctcgaagc tgtatcgagg cgaagaactg 5400

cctgcgcttt ccgtccatta caaagatttc gccgtctggc agtctgaact gttccagagc 5460

gacgtctata ccgagcatga aaactactgg ctgaacgcgt tttctggcga cattccggtg 5520

cttaacttgc cagccgattt ttctcgtccg ctgacacaga gctttgaagg agattgcgtt 5580

tcgttccagg cagacaaagc gttgctggac gatcttcaca agctcgctca ggagagccaa 5640

tcgacgttgt tcatggtatt gctggcggct tacaatgtgc tgcttgccaa gtacagcgga 5700

caggaagaca tcgtcgtcgg cacaccgatt gcgggcagat cgcacgccga tatcgagaac 5760

gttctgggga tgtttgtcaa cacgctcgct ttgcgcaact atccggtcga gacgaaacac 5820

ttccaggcat ttttggaaga ggtcaagcaa aatacgctgc aagcatacgc ccatcaagat 5880

tatccgttcg aagcactggt cgaaaagctg gacatccagc gggatctcag ccgcaatccg 5940

ctgtttgaca ccatgtttat tttgcaaaac ctggaccaaa aagcttacga gctggatggg 6000

ctgaaactgg aggcatatcc ggcacaagca ggcaacgcca aattcgatct cacgctggaa 6060

gcgcacgagg acgagacagg cattcatttt gcgctcgtct actcgaccaa attgttccag 6120

cgagaatcaa tcgaaagaat ggcgggtcac ttcctgcaag tgctgcgcca agtcgttgcc 6180

gaccaagcaa ctgccttgcg cgagatcagc ctgctcagcg aggaagagcg ccgaattgtg 6240

accgttgatt tcaacaacac gtttgcctat ccgcgcgatc tgacgattca ggagctgttc 6300

gagcagcagg cagcaaaaac tccggagcat gcagcggtcg tgatggacgg acagatgctg 6360

acgtatcggg agctgaacga aaaagcgaac cagctcgccc atgtccttcg tcaaaacgga 6420

gtcgggaaag agagcatcgt cggtctgctc gcagatcgtt cgctggaaat gattacaggc 6480

atcatgggga ttctcaaagc gggcggcgcc tacctgggac tggacccgga gcatccgtcc 6540

gaacgcctgg cttacatgtt ggaagatggc ggcgtgaaag ttgtcctcgt gcaaaagcac 6600

ttgctgccgc tcgtcggcga agggctgatg ccaatcgttt tggaagagga gagcctgcgc 6660

ccggaagatt gcggcaatcc ggcgattgtc aacggtgcga gtgacctggc ttatgtgatg 6720

tacacctcag gctctacagg caagccaaaa ggagtcatgg tcgagcatcg caacgtcacc 6780

cgcttggtca tgcatacgaa ttacgtgcaa gtgcgcgaga gcgaccggat gattcaaacc 6840

ggcgcgattg gcttcgacgc catgacattt gagatttttg gagccttgct gcacggggcc 6900

agcctgtatt tggtgagcaa ggacgtcttg ctggatgccg aaaagctggg cgacttcctg 6960

cggacgaatc agattacgac catgtggctg acctcgccgc tcttcaacca gctttcgcaa 7020

gacaatccgg cgatgtttga cagcttgcgc gccttgatcg tcggtggcga agcgttgtcg 7080

ccgaagcaca tcaaccgggt aaaaagtgcc cttcctgacc tggaaatctg gaacggatac 7140

ggcccgaccg aaaacacgac cttctcgacg tgctatttga ttgagcagca ttttgaagag 7200

cagattccga tcggcaagcc gattgcaaac tccaccgcgt atatcgtcga cggcaacaat 7260

cagccgcagc cgatcggcgt accgggtgaa ctgtgcgtcg gtggtgacgg tgtcgcaaga 7320

ggctatgtga acaagccgga attaaccgcc gaaaagtttg tgcccaatcc gtttgcgcct 7380

ggcgaaacga tgtatcgcac cggagatttg gcgagatggc tgccggatgg gacgattgag 7440

tatttgggcc gaatcgacca gcaggtcaaa atcaggggat accggatcga gcttggggaa 7500

atcgagacgg tcttgtccca gcaggcacaa gtaaaagaag cagtcgtggc cgtgatcgag 7560

gaggcgaacg ggcaaaaagc tctctgcgct tactttgtgc cagaacaggc cgtcgacgcc 7620

gcagagctgc gagaagcgat gtccaaacaa ttgcctggct acatggtccc tgcttactat 7680

gtgcaaatgg aaaagctgcc gttgaccgcg aacggaaagg tcgaccgccg ggcattgccg 7740

cagccatccg gcgagcggac gacaggaagc gcctttgtcg ctgcgcaaaa tgataccgaa 7800

gcgaagctgc aacagatttg gcaagaagtt ttgggcattc cggcaatcgg cattcacgac 7860

aacttctttg aaatcggcgg tcattccttg aaggcgatga acgtcatcac gcaagtccat 7920

aaaacattcc aggtggagct gccgttaaaa gcgctgtttg ccactccgac gatccatgag 7980

ttggctgcgc atatttcgga aaaaaccgag tacaccgcga ttcaacccgt ggcagcgcag 8040

gagttttacc cggtttcatc tgcgcaaaaa agaatgtata tcctgcaaca gttcgaaggc 8100

aacggaatca gctacaacat ttcgggtgcg attctcctgg aaggaaagct ggactacgcc 8160

cggtttgcca gcgctgtgca acagctggca gagcgccacg aagctttgcg cacctcgttc 8220

caccggatcg acggcgagcc tgtgcaaaaa gtgcacgagg aagtagaagt gccgcttttc 8280

atgctggagg ctcccgaaga ccaggcggag aaaatcatgc gcgagtttgt ccgtccgttt 8340

gatctcgggg tcgctccgct gatgcgaaca ggtttgctca agctgggcaa agaccgccat 8400

ttgtttttgc tcgacatgca ccatatcatc tcggacggcg tttcttcgca aattttgctg 8460

cgtgaatttg ccgagttgta ccagggagca gacttgcagc cgctttcgct gcaatacaaa 8520

gatttcgctg cttggcaaaa tgagctgttt cagacggagg catacaagaa gcaggagcag 8580

cactggctga acacgtttgc tgatgaaatt ccgctcttga acctgccgac tgactatccg 8640

cgccctagcg tgcaaagctt tgcaggcgat ctcgtccttt ttgccgccgg aaaagaactg 8700

ctggagcggt tgcaacaggt agcgtcagaa acaggcacca ccttgtacat gattttgctt 8760

gccgcctaca atgtgctgct gtccaagtat accggccagg aagacatcat cgtcgggacg 8820

cctgtcgctg gacgttccca tgcggacgtg gaaaacatca tgggcatatt cgtgaacaca 8880

ttggcgctgc gcaaccagcc tgccagcagc aaaacgatgt tagaaaataa tattacacaa 8940

tgtgactcaa tcaatgatgt ttatcttaaa gaagaagcaa taacattgat ggatatgctt 9000

gagagtcaac ttaagcacca ggcagatgga tatgttgtta ttgatcaaga agaatctctc 9060

agttacgctg atttctattt gagggtgaaa gagatagggt attgtctgtc agaaattagc 9120

tcaaagaatt cggtgggtat tgggcttttt tgtgatcctt ctatagattt aatttgtggt 9180

gcatggggta ttttgtcagc ggataaagct tatttgccgt tatcgcctga ctatccaact 9240

gaacgcctca aatatatgat agaagattct ggtattgatg tgatttttac gcaatcgcac 9300

ttaaaagcac agctacagga cattgcacca aaatcagtat taattatgac accagaagat 9360

gtcgctctga cgataaaaac acgaacaata gaagatattc tgggcacagt tcaagttcct 9420

aaacccacta gtctggctta tattatttat acctctggta gcacgggtaa gccaaaggga 9480

gtgatgattg aacatcacag tattgtaaat caaatgagat ttcttgcaaa agcgttcaaa 9540

ttaggatgtc attcccggat tttacagaaa acaccaatga gttttgatgc ggctcaatgg 9600

gaaattctag cgcctgcaat tggtggtcaa gtgattatgg gtcctttagg ttgctatcgc 9660

gatccggatg caattattaa aaccattctt cagcatcaag taacgacttt gcaatgtgtt 9720

cctactttgc tacaagcgtt actggataat cctaattttt tggattgctt atcattgact 9780

caagtattca gtgggggaga agcgctgaca accaaattag ccacgcaatt tttgaatagt 9840

tttactcact gtgaattaat caatttatat ggcccgacag aatgtacgat taattcatca 9900

tttttccggg tgacaaatga gactttgccg aattatcaaa cctctatttc gattggtgca 9960

cctgtagata ataccgaata ctacgttctt gatgatgata gattacctgt ggcggttggc 10020

gaaattggcg agctttatat ttcgggtgct caattagcac gtggttattt gcataaacca 10080

gaaatgacaa aagataaatt tatttgtaat caccttgtat caggaactca acatcaatgg 10140

ttatatcgaa cgggagatct ggtaaccaga ggggctgatg gtaatactta ttttgttggt 10200

cgggttgata gccaggtcaa attacgaggt taccgtattg agcttgatga aatacgccat 10260

gcgattgaag aacatagctg gataaagacg gcggcaatgt taattaagaa ggatgccaga 10320

acgggtttcc aaaatctcat cgcgtgtgtg gaattagatg agaaagaagc tgcattgatg 10380

gatcaaggta atagtagctc acatcacaaa tcaaaagccg ataaactaca ggtgaaagcc 10440

caactttcta attctggttg tcgaagtgaa gagttatgtg aaaatcgccc tacattctta 10500

cttccttatc aagaagggga gataaaacag agagaatatg catttggacg caagacatat 10560

cgctattttg agggaacaga aataacggta gagaaattaa aaaaattgct gacagccact 10620

caatcgaatg aaattagctc tttgccactg agtcatctaa ccctgaatga tttcggttat 10680

gcattgcgtt attttggtca gtttaccagc catcaacgtt tattgcccaa atatgcctat 10740

gcttcaccgg gtgctctcta tgcgacacaa atgtattttg aattgcataa tgttctcggt 10800

ttggatgcgg ggatttacta ttatcatcca gtgacacata agttaataaa aatttcaaca 10860

ttgagtcgtc ggcaaatgcc aacgataaaa gtgcatttta ttggcaagca tgaagccatt 10920

gagcccgttt ataagaacaa tatacaagaa gttctggaaa tggaagcggg ccatatgatg 10980

ggtctttttg atgacgtatt accggaaatt ggcttgagta ttggtaaaag tgaatatcaa 11040

gatgaatgtc cagattggta tgatggtgat attcaggatt attatcttgg tgcatttgaa 11100

atatgtagct atgaacatgg attgccgcca tttgagactg atatttattt acaaacacat 11160

gcccataaaa tacctgagat gccgtgtggt ttatatcact tttctaacgg ggaatttgta 11220

cgaataagtg atgatattgt ccgaaaaaag gatgttattg cgattaatca gcaagtttat 11280

gatcgctcca gttttggcgt gtcaattatt ccacgctgtg tccctgaatg gcattattat 11340

ataacactgg gtcgtcggtt acatgcgtta caaagtaatc cattgtatat tggattaatg 11400

tcatctggtt acagttcgaa gagcaataac gatttacctt cggcgaaaag gatgcgatct 11460

attctcaatg cacttgatag acctatggcg gcattttatt tctgcatagg tgggggtatt 11520

agccaagcgc aatatatgtg tgaaggcatg aaagaagatg ttgttcatat gaaagggcca 11580

gttgaaatca ttaaagatga tcttcaacaa caactccctc aatatatgat tccaaataag 11640

gtattagttt tcgataaatt acctttgacg gccaatggaa aagtggatta tcaatcttta 11700

tcagaatcta aagccgtgga gaatgtttca acacagcgtc tattggtgcc attacataca 11760

gatactgaaa taaggcttgg aaaaatttgg atggaagtac tgaaatggga ttcagtatct 11820

gccctcgatg attttttcga aagtgggggt aattctttga tggccgttgc aatggttaat 11880

aagatcaatg cggcctttaa tattcgtttt ccgttacaga tactttttca atctcctaat 11940

atagcagaat tggctaagtg gattgaacag acagactcta aaacaatatc aagattaatt 12000

ttattgaatc aggcaagcaa agaccccatt tactgttggc cgggtttggg cggatatcct 12060

atgagtttga gattgcttgc taataaagtc gttcctgatc gggcatttta tggaatacag 12120

gcatatggga taaacgagag tgaaataccg ttttcttcta tccagagaat ggcagaagag 12180

gatattaaag agataaagaa aatacagcca gaagggccat atatattgtg gggatattca 12240

tttggtgccc gagtagcatt tgaagttgca taccagcttg aacaagcggg agaagaagtt 12300

aacgcattga atttattggc tccgggatct cctcatcttg atatgaagca agcggaatat 12360

atggataaag gcgctgaatt tactaatccg gcttttgtta aaatactttt ttctgtattt 12420

tctcgttcaa tcaacagccc aatggttaaa acttgcttag aacaagtaaa tagtgaaacg 12480

acatttatta actttatatg tagtcgtttt aaaaacttgg aaccatcatt agtaaaacgt 12540

atcgttagga ttgtgacttt gacttatgat ttcaagtaca gtattgatga gctttatcac 12600

agacacctaa aggcacctat aactattttc aaggcgaata gagataatga ttcatttatc 12660

gaggaatcgg atgtgatttc atcaatgtcg cctaaaataa ttgaattaat atcggatcac 12720

tatcaactgt tggaaagtga aggtgttgct gagattgaga aaataatcta a 12771

<210> 15
<211> 6585
<212> DNA
<213> Artificial Sequence

<220>

<223>  NRPS being a putative synthetase of a fusion peptide consisting
       of Phenylalanine and Indigoidine

<400>  15
atgttagcaa atcaggccaa tctcatcgac aacaagcggg aactggagca gcatgcgcta      60

gttccatatg cacagggcaa gtcgatccat caattgttcg aggaacaagc agaggctttt     120

ccagaccgcg ttgccatcgt ttttgaaaac aggcggcttt cgtatcagga gttgaacagg     180

aaagccaatc aactggcaag agccttgctc gaaaaagggg tgcaaacaga cagcatcgtc     240

ggtgtgatga tggagaagtc catcgaaaat gtcatcgcga ttctggccgt tcttaaagca     300

ggcggagcct atgtgcccat cgacatcgaa tatccccgcg atcgcatcca atatattttg     360

caggatagtc aaacgaaaat cgtgcttacc caaaaaagcg tcagccagct cgtgcatgac     420

gtcgggtaca gcggagaggt agttgtactc gacgaagaac agttggacgc tcgcgagact     480

gccaatctgc accagcccag caagcctacg gatcttgcct atgtcattta cacctcaggc     540

acgacaggca agccaaaagg caccatgctt gaacataaag gcatcgccaa tttgcaatcc     600

tttttccaaa attcgtttgg cgtcaccgag caagacagga tcgggctttt tgccagcatg     660

tcgttcgacg catccgtttg ggaaatgttc atggctttgc tgtctggcgc cagcctgtac     720

atcctttcca aacagacgat ccatgatttc gctgcatttg aacactattt gagtgaaaat     780

gaattgacca tcatcacact gccgccgact tatttgactc acctcacccc agagcgcatc     840

acctcgctac gcatcatgat tacggcagga tcagcttcct ccgcaccctt ggtaaacaaa     900

tggaaagaca aactcaggta cataaatgca tacggcccga cggaaacgag catttgcgcg     960

acgatctggg aagccccgtc caatcagctc tccgtgcaat cggttccgat cggcaaaccg    1020

attcaaaata cacatattta tatcgtcaat gaagacttgc agctactgcc gactggcagc    1080

gaaggcgaat tgtgcatcgg cggagtcggc ttggcaagag gctattggaa tcggcccgac    1140

ttgaccgcag aaaaattcgt agacaatccg ttcgtaccag gcgaaaaaat gtaccgcaca    1200

ggtgacttgg ccaaatggct gacggatgga acgatcgagt ttctcggcag aatcgaccat    1260

caggtgaaaa tcagaggtca tcgcatcgag cttggcgaaa tcgagtctgt tttgttggca    1320

catgaacaca tcacagaggc cgtggtcatt gccagagagg atcaacacgc gggacagtat    1380

ttgtgcgcct attatatttc gcaacaagaa gcaactcctg cgcagctcag agactacgcc    1440

gcccagaagc ttccggctta catgctgcca tcttatttcg tcaagctgga caaaatgccg    1500

cttacgccaa atgacaagat cgaccgcaaa gcgttgcccg agcctgatct tacggcaaac    1560

caaagccagg ctgcctacca tcctccgaga accgagacag aatcgattct cgtctccatc    1620

tggcaaaacg ttttgggaat tgaaaagatc gggattcgcg ataattttta ctcgctcggc    1680

ggagattcga tccaagcgat ccaggtcgtg gctcgtctgc attcctatca attgaagcta    1740

gagacgaaag acttgctgaa ttacccgacg atcgagcagg ttgctgagct ggcccgcttc    1800

ctttcgcggt cggaaaaaac cgagtacacc gcgattcaac ccgtggcagc gcaggagttt 1860

tacccggttt catctgcgca aaaaagaatg tatatcctgc aacagttcga aggcaacgga 1920

atcagctaca acatttcggg tgcgattctc ctggaaggaa agctggacta cgcccggttt 1980

gccagcgctg tgcaacagct ggcagagcgc cacgaagctt tgcgcacctc gttccaccgg 2040

atcgacggcg agcctgtgca aaaagtgcac gaggaagtag aagtgccgct tttcatgctg 2100

gaggctcccg aagaccaggc ggagaaaatc atgcgcgagt ttgtccgtcc gtttgatctc 2160

ggggtcgctc cgctgatgcg aacaggtttg ctcaagctgg gcaaagaccg ccatttgttt 2220

ttgctcgaca tgcaccatat catctcggac ggcgtttctt cgcaaatttt gctgcgtgaa 2280

tttgccgagt tgtaccaggg agcagacttg cagccgcttt cgctgcaata caaagatttc 2340

gctgcttggc aaaatgagct gtttcagacg gaggcataca agaagcagga gcagcactgg 2400

ctgaacacgt ttgctgatga aattccgctc ttgaacctgc cgactgacta tccgcgccct 2460

agcgtgcaaa gctttgcagg cgatctcgtc ctttttgccg ccggaaaaga actgctggag 2520

cggttgcaac aggtagcgtc agaaacaggc accaccttgt acatgatttt gcttgccgcc 2580

tacaatgtgc tgctgtccaa gtataccggc caggaagaca tcatcgtcgg gacgcctgtc 2640

gctggacgtt cccatgcgga cgtggaaaac atcatgggca tattcgtgaa cacattggcg 2700

ctgcgcaacc agcctgccag cagcaaaacg atgttagaaa ataatattac acaatgtgac 2760

tcaatcaatg atgtttatct taaagaagaa gcaataacat tgatggatat gcttgagagt 2820

caacttaagc accaggcaga tggatatgtt gttattgatc aagaagaatc tctcagttac 2880

gctgatttct atttgagggt gaaagagata gggtattgtc tgtcagaaat tagctcaaag 2940

aattcggtgg gtattgggct tttttgtgat ccttctatag atttaatttg tggtgcatgg 3000

ggtattttgt cagcggataa agcttatttg ccgttatcgc ctgactatcc aactgaacgc 3060

ctcaaatata tgatagaaga ttctggtatt gatgtgattt ttacgcaatc gcacttaaaa 3120

gcacagctac aggacattgc accaaaatca gtattaatta tgacaccaga agatgtcgct 3180

ctgacgataa aaacacgaac aatagaagat attctgggca cagttcaagt tcctaaaccc 3240

actagtctgg cttatattat ttatacctct ggtagcacgg gtaagccaaa gggagtgatg 3300

attgaacatc acagtattgt aaatcaaatg agatttcttg caaaagcgtt caaattagga 3360

tgtcattccc ggattttaca gaaaacacca atgagttttg atgcggctca atgggaaatt 3420

ctagcgcctg caattggtgg tcaagtgatt atgggtcctt taggttgcta tcgcgatccg 3480

gatgcaatta ttaaaaccat tcttcagcat caagtaacga ctttgcaatg tgttcctact 3540

ttgctacaag cgttactgga taatcctaat tttttggatt gcttatcatt gactcaagta 3600

ttcagtgggg gagaagcgct gacaaccaaa ttagccacgc aatttttgaa tagttttact 3660

cactgtgaat taatcaattt atatggcccg acagaatgta cgattaattc atcatttttc 3720

cgggtgacaa atgagacttt gccgaattat caaacctcta tttcgattgg tgcacctgta 3780

gataataccg aatactacgt tcttgatgat gatagattac ctgtggcggt tggcgaaatt 3840

ggcgagcttt atatttcggg tgctcaatta gcacgtggtt atttgcataa accagaaatg 3900

acaaaagata aatttatttg taatcacctt gtatcaggaa ctcaacatca atggttatat 3960

cgaacgggag atctggtaac cagaggggct gatggtaata cttattttgt tggtcgggtt 4020

gatagccagg tcaaattacg aggttaccgt attgagcttg atgaaatacg ccatgcgatt 4080

gaagaacata gctggataaa gacggcggca atgttaatta agaaggatgc cagaacgggt 4140

ttccaaaatc tcatcgcgtg tgtggaatta gatgagaaag aagctgcatt gatggatcaa 4200

ggtaatagta gctcacatca caaatcaaaa gccgataaac tacaggtgaa agcccaactt 4260

tctaattctg gttgtcgaag tgaagagtta tgtgaaaatc gccctacatt cttacttcct 4320

tatcaagaag gggagataaa acagagagaa tatgcatttg gacgcaagac atatcgctat 4380

tttgagggaa cagaaataac ggtagagaaa ttaaaaaaat tgctgacagc cactcaatcg 4440

aatgaaatta gctctttgcc actgagtcat ctaaccctga atgatttcgg ttatgcattg 4500

cgttattttg gtcagtttac cagccatcaa cgtttattgc ccaaatatgc ctatgcttca 4560

ccgggtgctc tctatgcgac acaaatgtat tttgaattgc ataatgttct cggtttggat 4620

gcggggattt actattatca tccagtgaca cataagttaa taaaaatttc aacattgagt 4680

cgtcggcaaa tgccaacgat aaaagtgcat tttattggca agcatgaagc cattgagccc 4740

gtttataaga acaatataca agaagttctg gaaatggaag cgggccatat gatgggtctt 4800

tttgatgacg tattaccgga aattggcttg agtattggta aaagtgaata tcaagatgaa 4860

tgtccagatt ggtatgatgg tgatattcag gattattatc ttggtgcatt tgaaatatgt 4920

agctatgaac atggattgcc gccatttgag actgatattt atttacaaac acatgcccat 4980

aaaatacctg agatgccgtg tggtttatat cacttttcta acggggaatt tgtacgaata 5040

agtgatgata ttgtccgaaa aaaggatgtt attgcgatta atcagcaagt ttatgatcgc 5100

tccagttttg gcgtgtcaat tattccacgc tgtgtccctg aatggcatta ttatataaca 5160

ctgggtcgtc ggttacatgc gttacaaagt aatccattgt atattggatt aatgtcatct 5220

ggttacagtt cgaagagcaa taacgattta ccttcggcga aaaggatgcg atctattctc 5280

aatgcacttg atagacctat ggcggcattt tatttctgca taggtggggg tattagccaa 5340

gcgcaatata tgtgtgaagg catgaaagaa gatgttgttc atatgaaagg gccagttgaa 5400

atcattaaag atgatcttca acaacaactc cctcaatata tgattccaaa taaggtatta 5460

gttttcgata aattaccttt gacggccaat ggaaaagtgg attatcaatc tttatcagaa 5520

tctaaagccg tggagaatgt ttcaacacag cgtctattgg tgccattaca tacagatact 5580

gaaataaggc ttggaaaaat ttggatggaa gtactgaaat gggattcagt atctgccctc 5640

gatgattttt tcgaaagtgg gggtaattct ttgatggccg ttgcaatggt taataagatc 5700

aatgcggcct ttaatattcg ttttccgtta cagatacttt ttcaatctcc taatatagca 5760

gaattggcta agtggattga acagacagac tctaaaacaa tatcaagatt aattttattg 5820

aatcaggcaa gcaaagaccc catttactgt tggccgggtt tgggcggata tcctatgagt 5880

ttgagattgc ttgctaataa agtcgttcct gatcgggcat tttatggaat acaggcatat 5940

gggataaacg agagtgaaat accgttttct tctatccaga gaatggcaga agaggatatt 6000

aaagagataa agaaaataca gccagaaggg ccatatatat tgtggggata ttcatttggt 6060

gcccgagtag catttgaagt tgcataccag cttgaacaag cgggagaaga agttaacgca 6120

ttgaatttat tggctccggg atctcctcat cttgatatga agcaagcgga atatatggat 6180

aaaggcgctg aatttactaa tccggctttt gttaaaatac ttttttctgt attttctcgt 6240

tcaatcaaca gcccaatggt taaaacttgc ttagaacaag taaatagtga aacgacattt 6300

attaacttta tatgtagtcg ttttaaaaac ttggaaccat cattagtaaa acgtatcgtt 6360

aggattgtga ctttgactta tgatttcaag tacagtattg atgagcttta tcacagacac 6420

ctaaaggcac ctataactat tttcaaggcg aatagagata atgattcatt tatcgaggaa 6480

tcggatgtga tttcatcaat gtcgcctaaa ataattgaat taatatcgga tcactatcaa 6540

ctgttggaaa gtgaaggtgt tgctgagatt gagaaaataa tctaa 6585

<210> 16
<211> 14235
<212> DNA
<213> Artificial Sequence

<220>
<223> NRPS synthesizing a Indigoidine-tagged Tripeptide consisting of
 Phenylalanine, Ornithine and Leucine

<400> 16
atgttagcaa atcaggccaa tctcatcgac aacaagcggg aactggagca gcatgcgcta 60

gttccatatg cacagggcaa gtcgatccat caattgttcg aggaacaagc agaggctttt 120

ccagaccgcg ttgccatcgt ttttgaaaac aggcggcttt cgtatcagga gttgaacagg 180

aaagccaatc aactggcaag agccttgctc gaaaaagggg tgcaaacaga cagcatcgtc 240

ggtgtgatga tggagaagtc catcgaaaat gtcatcgcga ttctggccgt tcttaaagca 300

ggcggagcct atgtgcccat cgacatcgaa tatccccgcg atcgcatcca atatattttg 360

caggatagtc aaacgaaaat cgtgcttacc caaaaaagcg tcagccagct cgtgcatgac 420

gtcgggtaca gcggagaggt agttgtactc gacgaagaac agttggacgc tcgcgagact 480

gccaatctgc accagcccag caagcctacg gatcttgcct atgtcattta cacctcaggc 540

acgacaggca agccaaaagg caccatgctt gaacataaag gcatcgccaa tttgcaatcc 600

tttttccaaa attcgtttgg cgtcaccgag caagacagga tcgggctttt tgccagcatg 660

tcgttcgacg catccgtttg ggaaatgttc atggctttgc tgtctggcgc cagcctgtac 720

atcctttcca aacagacgat ccatgatttc gctgcatttg aacactattt gagtgaaaat 780

gaattgacca tcatcacact gccgccgact tatttgactc acctcacccc agagcgcatc 840

acctcgctac gcatcatgat tacggcagga tcagcttcct ccgcaccctt ggtaaacaaa 900

tggaaagaca aactcaggta cataaatgca tacggcccga cggaaacgag catttgcgcg 960

acgatctggg aagccccgtc caatcagctc tccgtgcaat cggttccgat cggcaaaccg 1020

attcaaaata cacatattta tatcgtcaat gaagacttgc agctactgcc gactggcagc 1080

gaaggcgaat tgtgcatcgg cggagtcggc ttggcaagag gctattggaa tcggcccgac 1140

ttgaccgcag aaaaattcgt agacaatccg ttcgtaccag gcgaaaaaat gtaccgcaca 1200

ggtgacttgg ccaaatggct gacggatgga acgatcgagt ttctcggcag aatcgaccat 1260

caggtgaaaa tcagaggtca tcgcatcgag cttggcgaaa tcgagtctgt tttgttggca 1320

catgaacaca tcacagaggc cgtggtcatt gccagagagg atcaacacgc gggacagtat 1380

ttgtgcgcct attatatttc gcaacaagaa gcaactcctg cgcagctcag agactacgcc 1440

gcccagaagc ttccggctta catgctgcca tcttatttcg tcaagctgga caaaatgccg 1500

cttacgccaa atgacaagat cgaccgcaaa gcgttgcccg agcctgatct tacggcaaac 1560

caaagccagg ctgcctacca tcctccgaga accgagacag aatcgattct cgtctccatc 1620

tggcaaaacg ttttgggaat tgaaaagatc gggattcgcg ataattttta ctcgctcggc 1680

ggagattcga tccaagcgat ccaggtcgtg gctcgtctgc attcctatca attgaagcta 1740

gagacgaaag acttgctgaa ttacccgacg atcgagcagg ttgctctttt tgtcaagagc 1800

acgacgagaa aaagcgatca gggcatcatc gctggaaacg taccgcttac acccattcag 1860

aagtggtttt tcgggaaaaa ctttacgaat acaggccatt ggaaccaatc gtctgtgctc 1920

tatcgcccgg aaggctttga tcctaaagtc atccaaagtg tcatggacaa aatcatcgaa 1980

caccacgacg cgctccgcat ggtctatcag cacgaaaacg gaaatgtcgt tcagcacaac 2040

cgcggcttgg gtggacaatt atacgatttc ttctcttata atctgaccgc gcaaccagac 2100

gtccagcagg cgatcgaagc agagacgcaa cgtctgcaca gcagcatgaa tttgcaggaa 2160

ggacctctgg tgaaggttgc cttatttcag acgttacatg gcgatcattt gtttctcgca 2220

attcatcatt tggtcgtgga tggcatttcc tggcgcattt tgtttgaaga tttggcaacc 2280

ggatacgcgc aggcacttgc agggcaagcg atcagtctgc ccgaaaaaac ggattctttt 2340

caaagctggt cacaatggtt gcaagaatat gcgaacgagg cggatttgct gagcgagatt 2400

ccgtactggg agagtctcga atcgcaagca aaaaatgtgt ccctgccgaa agactatgaa 2460

gtgaccgact gcaaacaaaa gagcgtgcga aacatgcgga tacggctgca cccggaagag 2520

accgagcagt tgttgaagca cgccaatcag gcctatcaaa cggaaatcaa cgatctgttg 2580

ttggcggcgc tcggcttggc ttttgcggag tggagcaagc ttgcgcaaat cgtcattcat 2640

ttggaggggc acgggcgcga ggacatcatc gaacaggcaa acgtggccag aacggtcgga 2700

tggtttacgt cgcaatatcc ggtattgctc gacttgaagc aaaccgctcc cttgtccgac 2760

tatatcaagc tcaccaaaga gaatatgcgg aagattcctc gtaaagggat cggttacgac 2820

atcttgaagc atgtgacact tccagaaaat cgcggttcct tatccttccg cgtgcagccg 2880

gaagtgacgt tcaactactt gggacagttt gatgcggaca tgagaacgga actgtttacc 2940

cgctcaccct acagcggcgg caacacgtta ggcgcagatg gcaaaaacaa tctgagtcct 3000

gagtcagagg tgtacaccgc tttgaatata accggattga ttgaaggcgg agagctcgtc 3060

ctcacattct cttacagctc ggagcagtat cgggaagagt ccatccagca attgagccaa 3120

agttatcaaa agcatctgct tgccatcatc gcgcattgca ccgagaaaaa agaagtagag 3180

cgaacggcgc atattgccga gagcgcattc gagcagttcg agacgatcca gccagtcgag 3240

cctgccgcgt tttatcccgt gtcgtttgcc caaaagcgaa tgtacatcct gcatcagttc 3300

gaaggaagcg ggatcagcta caacgtgccg agtgtgctgg tgctggaagg caagctcgat 3360

tatgaccgct ttgctgctgc catccagagc ctggttaaac ggcatgaatc tttgcgcacc 3420

tcgttccatt cggtaaacgg ggaaccgctg caacgagtac atccggatgt cgagctgcct 3480

gtccgccttt tggaggcgac agaagatcag agcgaatcgc tcatccagga gctaatccag 3540

ccgtttgatc tggagatagc cccgttgttc agagtgaatc tgatcaagct tggcgcagag 3600

cggcacttgt tcttcatgga tatgcaccac attatttccg atggcgtatc gcttgcggtc 3660

atcgtcgagg aaattgccag cttgtatgca ggaaaacagc tttccgacct gcgcatccag 3720

tacaaagact ttgctgtgtg gcagaccaag ctggctcagt cggatcgctt ccaaaaacag 3780

gaggattttt ggacccggac gtttgccggg gagattcctt tgctgaatct gccccatgat 3840

tatccaagac cttctgtgca gagctttgac ggtgacacgg tcgcgcttgg caccggacat 3900

cacctgctgg aacaactgcg caagctcgct gccgagactg gcacgacctt gttcatggtg 3960

ctgctggctg cctaccatgt gttgctctcc aagtacgccg gacaggaaga aatcgtcgtc 4020

ggcacaccga tcgcaggccg ctcgcacgca gatgtcgagc gcattgtcgg gatgttcgtc 4080

aacacgctcg ctttgaaaaa tacggccgct ggcagcctga gcttccgcgc ctttttggaa 4140

gacgtgaagc aaaatgcgct ccatgccttc gagcatcaag actatccgtt cgagcatctg 4200

gtcgagaagc tgcaagtgcg gcgcgatctg agcagaaacc cgctgtttga tacgatgttc 4260

agcctggggc ttgccgaatc agccgaagga gaagtagcgg atctgaaagt gtcgccttat 4320

ccggtgaacg gccacatcgc caaattcgac ctttccctgg atgcgatgga aaaacaggat 4380

ggacttcttg ttcaattcag ctattgcacg aagctgttcg caaaagaaac ggttgatcga 4440

ctggccgccc attacgttca gcttttgcaa acaatcacag ccgatcccga catcgagctc 4500

gcccggatca gcgtgttgtc caaagcagag acggagcaca tgctgcacag cttcctcgca 4560

accaaaacag cctatccgac ggacaaaacg ttccagaagc tgttcgagga gcaagtggaa 4620

aaaacaccga acgagattgc cgttctgttc ggcaatgaac agctgaccta tcaggagttg 4680

aatgcaaaag caaaccagct cgcccgcgtc ctgcggcgaa aaggcgtcaa gccggagagc 4740

accgtcggca tcctcgtaga ccgctcgctc tacatggtca tcggcatgct ggccgtgttg 4800

aaagcaggcg gaacattcgt cccgattgat ccggactacc cgctggagcg ccaagcgttc 4860

atgctcgaag acagcgaggc gaagctgctg ctcaccttgc aaaaaatgaa cagtcaagtt 4920

gccttccctt atgaaacctt ttatctggat acagagacag tggatcagga ggagacgggc 4980

aatctggagc acgttgcgca gccggagaac gtcgcttaca tcatctacac atccggtacg 5040

acgggcaagc caaaaggggt cgtcatcgag caccgcagct atgccaatgt cgcatttgcc 5100

tggaaagacg aatatcacct ggacagcttc ccggtccgtt tgctgcaaat ggcgagcttc 5160

gcctttgacg tctcgacggg cgattttgcc agggcgctgc tgacaggcgg gcaactggtc 5220

atctgcccga atggggtcaa aatggaccca gcttcgctgt acgagaccat caggcgtcac 5280

gaaattacca ttttcgaagc gacacccgcc ttgatcatgc cgttgatgca ctacgtttac 5340

gaaaacgaac tggatatgag ccaaatgaag ctgctgattc tcggagcaga cagctgcccg 5400

gcggaagact tcaaaacgtt gctcgcgcgc ttcggtcaga agatgcgcat tatcaacagc 5460

tacggcgtga cagaggcgtg cattgacacc agctactacg aagaaacaga cgtcaccgcc 5520

atccgctcgg gaacggtgcc gatcggcaaa ccgcttccga acatgacgat gtacgtggtc 5580

gatgcgcatt tgaatttgca gcctgtcggc gtcgtaggcg aattgtgcat cggcggagca 5640

ggggttgcgc gcggttattt gaacagacct gagctgacgg aagagaagtt cgtgccgaat 5700

ccgttcgccc caggtgaacg attgtaccgc acaggtgatc tggcgaagtg gcgcgcagat 5760

ggcaatgtcg agttcctcgg acgcaatgac caccaggtaa aaatcagggg tgtccgcatc 5820

gagctgggcg agatcgagac acaactgcgc aagctggacg gaattacgga agcagtcgtg 5880

gttgcgagag aagatcgcgg gcaggaaaag gaattgtgcg catacgtcgt ggcggaccac 5940

aagcttgaca ccgcagaatt gcgggcgaat ttgctgaagg aactgccgca agcgatgatt 6000

ccagcgtatt tcgtcacctt ggatgcgctg ccgctgactg ccaatggcaa agtagacaga 6060

cgttccttgc cagcgccgga tgtcaccatg ctgagaacga ccgagtatgt agcgccgcgc 6120

tccgtctggg aagcccgatt ggcccaagta tgggagcagg tgctgaatgt tccgcaagtg 6180

ggtgcgctag acgacttttt cgcgctcggc ggtcactcat tgcgtgccat gcgcgtcctt 6240

tccagcatgc acaacgaata ccaggtcgac atcccgctgc gcatcttgtt cgaaaaaccg 6300

acgattcagg aactggcggc gttcatcgaa gagacagcca aggggaatgt cttctcgatc 6360

gagcctgtgc aaaagcaagc gtactatccg gtctcctcgg cacaaaagcg catgtacatc 6420

ctcgatcaat ttgagggagt cggcatcagc tacaacatgc cgtcgactat gctgatcgaa 6480

ggcaagctgg agcgaacacg ggtagaagcg gcgttccagc gcttgattgc gcgacatgaa 6540

agcctgcgca cttcgtttgc cgtcgtcaac ggagagcctg tgcaaaacat tcacgaggac 6600

gttccgtttg cgcttgccta ttcggaagtc acagaacagg aggcgcgcga actcgtttct 6660

tctctcgtgc agccgttcga tctggaggtc gcaccactca tccgcgtgtc gctgctgaaa 6720

atcggcgagg atcgttacgt gctctttacc gacatgcatc acagcatttc cgatggcgta 6780

tcctccggca ttcttttggc agagtgggtg cagctgtacc agggtgacgt tttgccggag 6840

ctgcgtatcc agtacaagga ctttgctgtg tggcaacaag agttttccca gtcggctgcc 6900

ttccacaagc aggaagcgta ctggttgcaa acgtttgccg atgacattcc tgtgctgaac 6960

ttgccgaccg atttcacccg ccccagcacc caaagctttg ccggggatca gtgcacgatc 7020

ggcgcgggca aagcgctcac ggaaggcttg caccagttgg cgcaggcgac gggaacgact 7080

ttgtacatgg ttttgctcgc cgcgtacaac gtgctgctcg ccaagtatgc cgggcaggag 7140

gacatcatcg tcggcacgcc gattacaggc agatcccatg ccgatctcga accgatcgtc 7200

ggcatgttcg tgaacacctt ggcgatgcga aacaaaccgc agcgcgaaaa gacttttagc 7260

gagtttttgc aagaagtcaa gcaaaatgcg ctggatgcgt acggccatca ggattacccg 7320

tttgaagaac tggtggaaaa gctcgcgatc gcgcgcgatt tgagccgaaa tccgctgttt 7380

gacaccgtgt ttacgttcca aaacagcacg gaagaggtca tgacgctgcc tgaatgcacg 7440

cttgcgccgt ttatgacgga cgaaacaggc cagcacgcca agttcgactt gactttcagc 7500

gctacggaag agcgggaaga aatgacgatt ggcgtggagt acagcacaag cttgtttacg 7560

cgggaaacga tggaacggtt cagccgccac ttcctgacga ttgcagcgag catcgtgcaa 7620

aatccgcaca tccgtctggg cgagatcgac atgcttttgc cagaagaaaa acagcagatt 7680

ttggccgggt tcaacgatac ggcagtcagc tatgcgctgg acaaaacgct gcaccagcta 7740

ttcgaagagc aggtcgacaa aacaccggat caggcagcgc ttctctttag cgagcaatcg 7800

ctgacgtaca gcgaactgaa cgagcgagca aacagactgg caagggtcct gcgcgcaaaa 7860

ggagtcggac cggaccgtct ggtagcgatc atggcggagc gctcgccgga aatggtgatc 7920

ggtattctcg gtattttgaa ggcaggcggc gcttatgttc ccgtcgatcc cggctatccg 7980

caggagcgca ttcagtacct gctcgaagat agcaacgcag ccctgctgct cagccaggcg 8040

catctgttgc cgctgttggc ccaggtgtca agcgagctgc cggagtgcct tgatctgaac 8100

gctgaactgg atgccggact gagcggctcc aacctgccag ctgtcaacca accgactgac 8160

cttgcctacg tcatctatac atccggtacg accggcaagc cgaagggtgt catgatcccg 8220

catcaaggaa tcgtgaactg cttgcagtgg agaagagacg aatacgggtt cgggccgagt 8280

gacaaggcgt tgcaagtgtt ctcctttgcc ttcgacggtt ttgtagccag cttgttcgct 8340

ccgctgctcg gaggggcaac gtgcgtgttg ccgcaagaag cagctgccaa agacccggtc 8400

gcgctgaaaa aactgatggc cgcaacggaa gtcacccatt actacggcgt accgagtctg 8460

ttccaggcca ttctcgattg ctcgacgaca accgacttca atcagttgcg ttgcgtcact 8520

ttgggcggcg agaagctgcc tgtgcagctt gtgcaaaaaa caaaagaaaa gcatccggca 8580

atcgagatca acaacgagta cggcccgacg gaaaacagcg tcgtcaccac catctcgcgc 8640

tcgattgaag cggggcaagc gatcacgatt ggccgaccgc ttgcgaacgt ccaagtctac 8700

attgtagatg agcagcatca cttgcagccg attggcgtgg tcggtgagct gtgcatcggc 8760

ggagccgggc ttgccagagg ctatctgaac aaaccggagc tgaccgcaga gaagtttgtc 8820

gcaaatccgt tccgaccagg cgagcgcatg tacaaaacag gcgacttggt aaaatggcgg 8880

acggatggca cgatcgagta catcggccgc gcagacgaac aggtcaaggt gagagggtat 8940

cgcatcgaga tcggcgagat cgagagcgcc gtactcgctt accagggcat cgatcaagcg 9000

gtggtcgttg cgcgagacga tgacgctacg gctggttcct atctttgcgc ctactttgtc 9060

gcagcaacag ccgtgtccgt atccggcttg agaagccatc tggccaaaga gctgcctgct 9120

tacatgattc cgagctattt cgtcgagctg gatcagctgc cgctttccgc caatggaaaa 9180

gtggatcgca aagctttgcc gaagccgcaa cagtccgatg cgaccacgcg cgaatacgtg 9240

gccccgagga atgcgaccga acagcaactg gcagccatct ggcaagaagt tttgggagta 9300

gagccaatcg gcatcaccga ccagttcttt gaactcggag gacattcctt aaaagctacg 9360

ctgttgattg ccaaagtgta tgagtacatg caaatcgagc tgccgctgaa tctcatcttc 9420

cagtatccga cgatcgaaaa ggtggccgat ttcatcacgt cggaaaaaac cgagtacacc 9480

gcgattcaac ccgtggcagc gcaggagttt tacccggttt catctgcgca aaaaagaatg 9540

tatatcctgc aacagttcga aggcaacgga atcagctaca acatttcggg tgcgattctc 9600

ctggaaggaa agctggacta cgcccggttt gccagcgctg tgcaacagct ggcagagcgc 9660

cacgaagctt tgcgcacctc gttccaccgg atcgacggcg agcctgtgca aaaagtgcac 9720

gaggaagtag aagtgccgct tttcatgctg gaggctcccg aagaccaggc ggagaaaatc 9780

atgcgcgagt ttgtccgtcc gtttgatctc ggggtcgctc cgctgatgcg aacaggtttg 9840

ctcaagctgg gcaaagaccg ccatttgttt ttgctcgaca tgcaccatat catctcggac 9900

ggcgtttctt cgcaaatttt gctgcgtgaa tttgccgagt tgtaccaggg agcagacttg 9960

cagccgcttt cgctgcaata caaagatttc gctgcttggc aaaatgagct gtttcagacg 10020

gaggcataca agaagcagga gcagcactgg ctgaacacgt ttgctgatga aattccgctc 10080

ttgaacctgc cgactgacta tccgcgccct agcgtgcaaa gctttgcagg cgatctcgtc 10140

ctttttgccg ccggaaaaga actgctggag cggttgcaac aggtagcgtc agaaacaggc 10200

accaccttgt acatgatttt gcttgccgcc tacaatgtgc tgctgtccaa gtataccggc 10260

caggaagaca tcatcgtcgg gacgcctgtc gctggacgtt cccatgcgga cgtggaaaac 10320

atcatgggca tattcgtgaa cacattggcg ctgcgcaacc agcctgccag cagcaaaacg 10380

atgttagaaa ataatattac acaatgtgac tcaatcaatg atgtttatct taaagaagaa 10440

gcaataacat tgatggatat gcttgagagt caacttaagc accaggcaga tggatatgtt 10500

gttattgatc aagaagaatc tctcagttac gctgatttct atttgagggt gaaagagata 10560

gggtattgtc tgtcagaaat tagctcaaag aattcggtgg gtattgggct tttttgtgat 10620

ccttctatag atttaatttg tggtgcatgg ggtattttgt cagcggataa agcttatttg 10680

ccgttatcgc ctgactatcc aactgaacgc ctcaaatata tgatagaaga ttctggtatt 10740

gatgtgattt ttacgcaatc gcacttaaaa gcacagctac aggacattgc accaaatcaa 10800

gtattaatta tgacaccaga agatgtcgct ctgacgataa aaacacgaac aatagaagat 10860

attctgggca cagttcaagt tcctaaaccc actagtctgg cttatattat ttatacctct 10920

ggtagcacgg gtaagccaaa gggagtgatg attgaacatc acagtattgt aaatcaaatg 10980

agatttcttg caaaagcgtt caaattagga tgtcattccc ggattttaca gaaaacacca 11040

atgagttttg atgcggctca atgggaaatt ctagcgcctg caattggtgg tcaagtgatt 11100

atgggtcctt taggttgcta tcgcgatccg gatgcaatta ttaaaaccat tcttcagcat 11160

caagtaacga ctttgcaatg tgttcctact ttgctacaag cgttactgga taatcctaat 11220

tttttggatt gcttatcatt gactcaagta ttcagtgggg gagaagcgct gacaaccaaa 11280

ttagccacgc aatttttgaa tagttttact cactgtgaat taatcaattt atatggcccg 11340

acagaatgta cgattaattc atcatttttc cgggtgacaa atgagacttt gccgaattat 11400

caaacctcta tttcgattgg tgcacctgta gataataccg aatactacgt tcttgatgat 11460

gatagattac ctgtggcggt tggcgaaatt ggcgagcttt atatttcggg tgctcaatta 11520

gcacgtggtt atttgcataa accagaaatg acaaaagata aatttatttg taatcacctt 11580

gtatcaggaa ctcaacatca atggttatat cgaacgggag atctggtaac cagaggggct 11640

gatggtaata cttattttgt tggtcgggtt gatagccagg tcaaattacg aggttaccgt 11700

attgagcttg atgaaatacg ccatgcgatt gaagaacata gctggataaa gacggcggca 11760

atgttaatta agaaggatgc cagaacgggt ttccaaaatc tcatcgcgtg tgtggaatta 11820

gatgagaaag aagctgcatt gatggatcaa ggtaatagta gctcacatca caaatcaaaa 11880

gccgataaac tacaggtgaa agcccaactt tctaattctg gttgtcgaag tgaagagtta 11940

tgtgaaaatc gccctacatt cttacttcct tatcaagaag gggagataaa acagagagaa 12000

tatgcatttg gacgcaagac atatcgctat tttgagggaa cagaaataac ggtagagaaa 12060

ttaaaaaaat tgctgacagc cactcaatcg aatgaaatta gctctttgcc actgagtcat 12120

ctaaccctga atgatttcgg ttatgcattg cgttattttg gtcagtttac cagccatcaa 12180

cgtttattgc ccaaatatgc ctatgcttca ccgggtgctc tctatgcgac acaaatgtat 12240

tttgaattgc ataatgttct cggtttggat gcggggattt actattatca tccagtgaca 12300

cataagttaa taaaaatttc aacattgagt cgtcggcaaa tgccaacgat aaaagtgcat 12360

tttattggca agcatgaagc cattgagccc gtttataaga acaatataca agaagttctg 12420

gaaatggaag cgggccatat gatgggtctt tttgatgacg tattaccgga aattggcttg 12480

agtattggta aaagtgaata tcaagatgaa tgtccagatt ggtatgatgg tgatattcag 12540

gattattatc ttggtgcatt tgaaatatgt agctatgaac atggattgcc gccatttgag 12600

actgatattt atttacaaac acatgcccat aaaatacctg agatgccgtg tggtttatat 12660

cacttttcta acggggaatt tgtacgaata agtgatgata ttgtccgaaa aaaggatgtt 12720

attgcgatta atcagcaagt ttatgatcgc tccagttttg gcgtgtcaat tattccacgc 12780

tgtgtccctg aatggcatta ttatataaca ctgggtcgtc ggttacatgc gttacaaagt 12840

aatccattgt atattggatt aatgtcatct ggttacagtt cgaagagcaa taacgattta 12900

ccttcggcga aaaggatgcg atctattctc aatgcacttg atagacctat ggcggcattt 12960

tatttctgca taggtggggg tattagccaa gcgcaatata tgtgtgaagg catgaaagaa 13020

gatgttgttc atatgaaagg gccagttgaa atcattaaag atgatcttca acaacaactc 13080

cctcaatata tgattccaaa taaggtatta gttttcgata aattaccttt gacggccaat 13140

ggaaaagtgg attatcaatc tttatcagaa tctaaagccg tggagaatgt ttcaacacag 13200

cgtctattgg tgccattaca tacagatact gaaataaggc ttggaaaaat ttggatggaa 13260

gtactgaaat gggattcagt atctgccctc gatgattttt tcgaaagtgg gggtaattct 13320

ttgatggccg ttgcaatggt taataagatc aatgcggcct ttaatattcg ttttccgtta 13380

cagatacttt ttcaatctcc taatatagca gaattggcta agtggattga acagacagac 13440

tctaaaacaa tatcaagatt aattttattg aatcaggcaa gcaaagaccc catttactgt 13500

tggccgggtt tgggcggata tcctatgagt ttgagattgc ttgctaataa agtcgttcct 13560

gatcgggcat tttatggaat acaggcatat gggataaacg agagtgaaat accgttttct 13620

tctatccaga gaatggcaga agaggatatt aaagagataa agaaaataca gccagaaggg 13680

ccatatatat tgtggggata ttcatttggt gcccgagtag catttgaagt tgcataccag 13740

cttgaacaag cgggagaaga agttaacgca ttgaatttat tggctccggg atctcctcat 13800

cttgatatga agcaagcgga atatatggat aaaggcgctg aatttactaa tccggctttt 13860

gttaaaatac ttttttctgt attttctcgt tcaatcaaca gcccaatggt taaaacttgc 13920

ttagaacaag taaatagtga aacgacattt attaacttta tatgtagtcg ttttaaaaac 13980

ttggaaccat cattagtaaa acgtatcgtt aggattgtga ctttgactta tgatttcaag 14040

tacagtattg atgagcttta tcacagacac ctaaaggcac ctataactat tttcaaggcg 14100

aatagagata atgattcatt tatcgaggaa tcggatgtga tttcatcaat gtcgcctaaa 14160

ataattgaat taatatcgga tcactatcaa ctgttggaaa gtgaaggtgt tgctgagatt 14220

gagaaaataa tctaa 14235

<210> 17
<211> 17334
<212> DNA
<213> Artificial Sequence

<220>
<223> NRPS synthesizing a Valine-Indigoidine-tagged Tripeptide
consisting of Phenylalanine, Ornithine and Leucine. Valine is
here used as spacer.

<400> 17
atgttagcaa atcaggccaa tctcatcgac aacaagcggg aactggagca gcatgcgcta 60

gttccatatg cacagggcaa gtcgatccat caattgttcg aggaacaagc agaggctttt 120

ccagaccgcg ttgccatcgt ttttgaaaac aggcggcttt cgtatcagga gttgaacagg 180

aaagccaatc aactggcaag agccttgctc gaaaaagggg tgcaaacaga cagcatcgtc 240

ggtgtgatga tggagaagtc catcgaaaat gtcatcgcga ttctggccgt tcttaaagca 300

ggcggagcct atgtgcccat cgacatcgaa tatccccgcg atcgcatcca atatattttg 360

caggatagtc aaacgaaaat cgtgcttacc caaaaaagcg tcagccagct cgtgcatgac 420

gtcgggtaca gcggagaggt agttgtactc gacgaagaac agttggacgc tcgcgagact 480

gccaatctgc accagcccag caagcctacg gatcttgcct atgtcattta cacctcaggc 540

acgacaggca agccaaaagg caccatgctt gaacataaag gcatcgccaa tttgcaatcc 600

tttttccaaa attcgtttgg cgtcaccgag caagacagga tcgggctttt tgccagcatg 660

tcgttcgacg catccgtttg ggaaatgttc atggctttgc tgtctggcgc cagcctgtac 720

atcctttcca aacagacgat ccatgatttc gctgcatttg aacactattt gagtgaaaat 780

gaattgacca tcatcacact gccgccgact tatttgactc acctcacccc agagcgcatc 840

acctcgctac gcatcatgat tacggcagga tcagcttcct ccgcaccctt ggtaaacaaa 900

tggaaagaca aactcaggta cataaatgca tacggcccga cggaaacgag catttgcgcg 960

acgatctggg aagccccgtc caatcagctc tccgtgcaat cggttccgat cggcaaaccg 1020

attcaaaata cacatattta tatcgtcaat gaagacttgc agctactgcc gactggcagc 1080

gaaggcgaat tgtgcatcgg cggagtcggc ttggcaagag gctattggaa tcggcccgac 1140

ttgaccgcag aaaaattcgt agacaatccg ttcgtaccag gcgaaaaaat gtaccgcaca 1200

ggtgacttgg ccaaatggct gacggatgga acgatcgagt ttctcggcag aatcgaccat 1260

caggtgaaaa tcagaggtca tcgcatcgag cttggcgaaa tcgagtctgt tttgttggca 1320

catgaacaca tcacagaggc cgtggtcatt gccagagagg atcaacacgc gggacagtat 1380

ttgtgcgcct attatatttc gcaacaagaa gcaactcctg cgcagctcag agactacgcc 1440

gcccagaagc ttccggctta catgctgcca tcttatttcg tcaagctgga caaaatgccg 1500

cttacgccaa atgacaagat cgaccgcaaa gcgttgcccg agcctgatct tacggcaaac 1560

caaagccagg ctgcctacca tcctccgaga accgagacag aatcgattct cgtctccatc 1620

tggcaaaacg ttttgggaat tgaaaagatc gggattcgcg ataattttta ctcgctcggc 1680

ggagattcga tccaagcgat ccaggtcgtg gctcgtctgc attcctatca attgaagcta 1740

gagacgaaag acttgctgaa ttacccgacg atcgagcagg ttgctctttt tgtcaagagc 1800

acgacgagaa aaagcgatca gggcatcatc gctggaaacg taccgcttac acccattcag 1860

aagtggtttt tcgggaaaaa ctttacgaat acaggccatt ggaaccaatc gtctgtgctc 1920

tatcgcccgg aaggctttga tcctaaagtc atccaaagtg tcatggacaa aatcatcgaa 1980

caccacgacg cgctccgcat ggtctatcag cacgaaaacg gaaatgtcgt tcagcacaac 2040

cgcggcttgg gtggacaatt atacgatttc ttctcttata atctgaccgc gcaaccagac 2100

gtccagcagg cgatcgaagc agagacgcaa cgtctgcaca gcagcatgaa tttgcaggaa 2160

ggacctctgg tgaaggttgc cttatttcag acgttacatg gcgatcattt gtttctcgca 2220

attcatcatt tggtcgtgga tggcatttcc tggcgcattt tgtttgaaga tttggcaacc 2280

ggatacgcgc aggcacttgc agggcaagcg atcagtctgc ccgaaaaaac ggattctttt 2340

caaagctggt cacaatggtt gcaagaatat gcgaacgagg cggatttgct gagcgagatt 2400

ccgtactggg agagtctcga atcgcaagca aaaaatgtgt ccctgccgaa agactatgaa 2460

gtgaccgact gcaaacaaaa gagcgtgcga aacatgcgga tacggctgca cccggaagag 2520

accgagcagt tgttgaagca cgccaatcag gcctatcaaa cggaaatcaa cgatctgttg 2580

ttggcggcgc tcggcttggc ttttgcggag tggagcaagc ttgcgcaaat cgtcattcat 2640

ttggaggggc acgggcgcga ggacatcatc gaacaggcaa acgtggccag aacggtcgga 2700

tggtttacgt cgcaatatcc ggtattgctc gacttgaagc aaaccgctcc cttgtccgac 2760

tatatcaagc tcaccaaaga gaatatgcgg aagattcctc gtaaagggat cggttacgac 2820

atcttgaagc atgtgacact tccagaaaat cgcggttcct tatccttccg cgtgcagccg 2880

gaagtgacgt tcaactactt gggacagttt gatgcggaca tgagaacgga actgtttacc 2940

cgctcaccct acagcggcgg caacacgtta ggcgcagatg gcaaaaacaa tctgagtcct 3000

gagtcagagg tgtacaccgc tttgaatata accggattga ttgaaggcgg agagctcgtc 3060

ctcacattct cttacagctc ggagcagtat cgggaagagt ccatccagca attgagccaa 3120

agttatcaaa agcatctgct tgccatcatc gcgcattgca ccgagaaaaa agaagtagag 3180

cgaacggcgc atattgccga gagcgcattc gagcagttcg agacgatcca gccagtcgag 3240

cctgccgcgt tttatcccgt gtcgtttgcc caaaagcgaa tgtacatcct gcatcagttc 3300

gaaggaagcg ggatcagcta caacgtgccg agtgtgctgg tgctggaagg caagctcgat 3360

tatgaccgct ttgctgctgc catccagagc ctggttaaac ggcatgaatc tttgcgcacc 3420

tcgttccatt cggtaaacgg ggaaccgctg caacgagtac atccggatgt cgagctgcct 3480

gtccgccttt tggaggcgac agaagatcag agcgaatcgc tcatccagga gctaatccag 3540

ccgtttgatc tggagatagc cccgttgttc agagtgaatc tgatcaagct tggcgcagag 3600

cggcacttgt tcttcatgga tatgcaccac attatttccg atggcgtatc gcttgcggtc 3660

atcgtcgagg aaattgccag cttgtatgca ggaaaacagc tttccgacct gcgcatccag 3720

tacaaagact ttgctgtgtg gcagaccaag ctggctcagt cggatcgctt ccaaaaacag 3780

gaggattttt ggacccggac gtttgccggg gagattcctt tgctgaatct gccccatgat 3840

tatccaagac cttctgtgca gagctttgac ggtgacacgg tcgcgcttgg caccggacat 3900

cacctgctgg aacaactgcg caagctcgct gccgagactg gcacgacctt gttcatggtg 3960

ctgctggctg cctaccatgt gttgctctcc aagtacgccg gacaggaaga aatcgtcgtc 4020

ggcacaccga tcgcaggccg ctcgcacgca gatgtcgagc gcattgtcgg gatgttcgtc 4080

aacacgctcg ctttgaaaaa tacggccgct ggcagcctga gcttccgcgc ctttttggaa 4140

gacgtgaagc aaaatgcgct ccatgccttc gagcatcaag actatccgtt cgagcatctg 4200

gtcgagaagc tgcaagtgcg gcgcgatctg agcagaaacc cgctgtttga tacgatgttc 4260

agcctggggc ttgccgaatc agccgaagga gaagtagcgg atctgaaagt gtcgccttat 4320

ccggtgaacg gccacatcgc caaattcgac ctttccctgg atgcgatgga aaaacaggat 4380

ggacttcttg ttcaattcag ctattgcacg aagctgttcg caaaagaaac ggttgatcga 4440

ctggccgccc attacgttca gcttttgcaa acaatcacag ccgatcccga catcgagctc 4500

gcccggatca gcgtgttgtc caaagcagag acggagcaca tgctgcacag cttcctcgca 4560

accaaaacag cctatccgac ggacaaaacg ttccagaagc tgttcgagga gcaagtggaa 4620

aaaacaccga acgagattgc cgttctgttc ggcaatgaac agctgaccta tcaggagttg 4680

aatgcaaaag caaaccagct cgcccgcgtc ctgcggcgaa aaggcgtcaa gccggagagc 4740

accgtcggca tcctcgtaga ccgctcgctc tacatggtca tcggcatgct ggccgtgttg 4800

aaagcaggcg gaacattcgt cccgattgat ccggactacc cgctggagcg ccaagcgttc 4860

atgctcgaag acagcgaggc gaagctgctg ctcaccttgc aaaaaatgaa cagtcaagtt 4920

gccttccctt atgaaacctt ttatctggat acagagacag tggatcagga ggagacgggc 4980

aatctggagc acgttgcgca gccggagaac gtcgcttaca tcatctacac atccggtacg 5040

acgggcaagc caaaaggggt cgtcatcgag caccgcagct atgccaatgt cgcatttgcc 5100

tggaaagacg aatatcacct ggacagcttc ccggtccgtt tgctgcaaat ggcgagcttc 5160

gcctttgacg tctcgacggg cgattttgcc agggcgctgc tgacaggcgg gcaactggtc 5220

atctgcccga atggggtcaa aatggaccca gcttcgctgt acgagaccat caggcgtcac 5280

gaaattacca ttttcgaagc gacacccgcc ttgatcatgc cgttgatgca ctacgtttac 5340

gaaaacgaac tggatatgag ccaaatgaag ctgctgattc tcggagcaga cagctgcccg 5400

gcggaagact tcaaaacgtt gctcgcgcgc ttcggtcaga agatgcgcat tatcaacagc 5460

tacggcgtga cagaggcgtg cattgacacc agctactacg aagaaacaga cgtcaccgcc 5520

atccgctcgg gaacggtgcc gatcggcaaa ccgcttccga acatgacgat gtacgtggtc 5580

gatgcgcatt tgaatttgca gcctgtcggc gtcgtaggcg aattgtgcat cggcggagca 5640

ggggttgcgc gcggttattt gaacagacct gagctgacgg aagagaagtt cgtgccgaat 5700

ccgttcgccc caggtgaacg attgtaccgc acaggtgatc tggcgaagtg gcgcgcagat 5760

ggcaatgtcg agttcctcgg acgcaatgac caccaggtaa aaatcagggg tgtccgcatc 5820

gagctgggcg agatcgagac acaactgcgc aagctggacg gaattacgga agcagtcgtg 5880

gttgcgagag aagatcgcgg gcaggaaaag gaattgtgcg catacgtcgt ggcggaccac 5940

aagcttgaca ccgcagaatt gcgggcgaat ttgctgaagg aactgccgca agcgatgatt 6000

ccagcgtatt tcgtcacctt ggatgcgctg ccgctgactg ccaatggcaa agtagacaga 6060

cgttccttgc cagcgccgga tgtcaccatg ctgagaacga ccgagtatgt agcgccgcgc 6120

tccgtctggg aagcccgatt ggcccaagta tgggagcagg tgctgaatgt tccgcaagtg 6180

ggtgcgctag acgacttttt cgcgctcggc ggtcactcat tgcgtgccat gcgcgtcctt 6240

tccagcatgc acaacgaata ccaggtcgac atcccgctgc gcatcttgtt cgaaaaaccg 6300

acgattcagg aactggcggc gttcatcgaa gagacagcca aagggaatgt cttctcgatc 6360

gagcctgtgc aaaagcaagc gtactatccg gtctcctcgg cacaaaagcg catgtacatc 6420

ctcgatcaat ttgagggagt cggcatcagc tacaacatgc cgtcgactat gctgatcgaa 6480

ggcaagctgg agcgaacacg ggtagaagcg gcgttccagc gcttgattgc gcgacatgaa 6540

agcctgcgca cttcgtttgc cgtcgtcaac ggagagcctg tgcaaaacat tcacgaggac 6600

gttccgtttg cgcttgccta ttcggaagtc acagaacagg aggcgcgcga actcgtttct 6660

tctctcgtgc agccgttcga tctggaggtc gcaccactca tccgcgtgtc gctgctgaaa 6720

atcggcgagg atcgttacgt gctctttacc gacatgcatc acagcatttc cgatggcgta 6780

tcctccggca ttcttttggc agagtgggtg cagctgtacc agggtgacgt tttgccggag 6840

ctgcgtatcc agtacaagga ctttgctgtg tggcaacaag agttttccca gtcggctgcc 6900

ttccacaagc aggaagcgta ctggttgcaa acgtttgccg atgacattcc tgtgctgaac 6960

ttgccgaccg atttcacccg ccccagcacc caaagctttg ccggggatca gtgcacgatc 7020

ggcgcgggca aagcgctcac ggaaggcttg caccagttgg cgcaggcgac gggaacgact 7080

ttgtacatgg ttttgctcgc cgcgtacaac gtgctgctcg ccaagtatgc cgggcaggag 7140

gacatcatcg tcggcacgcc gattacaggc agatcccatg ccgatctcga accgatcgtc 7200

ggcatgttcg tgaacacctt ggcgatgcga aacaaaccgc agcgcgaaaa gacttttagc 7260

gagtttttgc aagaagtcaa gcaaaatgcg ctggatgcgt acggccatca ggattacccg 7320

tttgaagaac tggtggaaaa gctcgcgatc gcgcgcgatt tgagccgaaa tccgctgttt 7380

gacaccgtgt ttacgttcca aaacagcacg gaagaggtca tgacgctgcc tgaatgcacg 7440

cttgcgccgt ttatgacgga cgaaacaggc cagcacgcca agttcgactt gactttcagc 7500

gctacggaag agcgggaaga aatgacgatt ggcgtggagt acagcacaag cttgtttacg 7560

cgggaaacga tggaacggtt cagccgccac ttcctgacga ttgcagcgag catcgtgcaa 7620

aatccgcaca tccgtctggg cgagatcgac atgcttttgc cagaagaaaa acagcagatt 7680

ttggccgggt tcaacgatac ggcagtcagc tatgcgctgg acaaaacgct gcaccagcta 7740

ttcgaagagc aggtcgacaa aacaccggat caggcagcgc ttctctttag cgagcaatcg 7800

ctgacgtaca gcgaactgaa cgagcgagca aacagactgg caagggtcct gcgcgcaaaa 7860

ggagtcggac cggaccgtct ggtagcgatc atggcggagc gctcgccgga aatggtgatc 7920

ggtattctcg gtattttgaa ggcaggcggc gcttatgttc ccgtcgatcc cggctatccg 7980

caggagcgca ttcagtacct gctcgaagat agcaacgcag ccctgctgct cagccaggcg 8040

catctgttgc cgctgttggc ccaggtgtca agcgagctgc cggagtgcct tgatctgaac 8100

gctgaactgg atgccggact gagcggctcc aacctgccag ctgtcaacca accgactgac 8160

cttgcctacg tcatctatac atccggtacg accggcaagc cgaagggtgt catgatcccg 8220

catcaaggaa tcgtgaactg cttgcagtgg agaagagacg aatacgggtt cgggccgagt 8280

gacaaggcgt tgcaagtgtt ctcctttgcc ttcgacggtt ttgtagccag cttgttcgct 8340

ccgctgctcg gaggggcaac gtgcgtgttg ccgcaagaag cagctgccaa agacccggtc 8400

gcgctgaaaa aactgatggc cgcaacggaa gtcacccatt actacggcgt accgagtctg 8460

ttccaggcca ttctcgattg ctcgacgaca accgacttca atcagttgcg ttgcgtcact 8520

ttgggcggcg agaagctgcc tgtgcagctt gtgcaaaaaa caaaagaaaa gcatccggca 8580

atcgagatca acaacgagta cggcccgacg gaaaacagcg tcgtcaccac catctcgcgc 8640

tcgattgaag cggggcaagc gatcacgatt ggccgaccgc ttgcgaacgt ccaagtctac 8700

attgtagatg agcagcatca cttgcagccg attggcgtgg tcggtgagct gtgcatcggc 8760

ggagccgggc ttgccagagg ctatctgaac aaaccggagc tgaccgcaga gaagtttgtc 8820

gcaaatccgt tccgaccagg cgagcgcatg tacaaaacag gcgacttggt aaaatggcgg 8880

acggatggca cgatcgagta catcggccgc gcagacgaac aggtcaaggt gagagggtat 8940

cgcatcgaga tcggcgagat cgagagcgcc gtactcgctt accagggcat cgatcaagcg 9000

gtggtcgttg cgcgagacga tgacgctacg gctggttcct atctttgcgc ctactttgtc 9060

gcagcaacag ccgtgtccgt atccggcttg agaagccatc tggccaaaga gctgcctgct 9120

tacatgattc cgagctattt cgtcgagctg gatcagctgc cgctttccgc caatggaaaa 9180

gtggatcgca aagctttgcc gaagccgcaa cagtccgatg cgaccacgcg cgaatacgtg 9240

gccccgagga atgcgaccga acagcaactg gcagccatct ggcaagaagt tttgggagta 9300

gagccaatcg gcatcaccga ccagttcttt gaactcggag gacattcctt aaaagctacg 9360

ctgttgattg ccaaagtgta tgagtacatg caaatcgagc tgccgctgaa tctcatcttc 9420

cagtatccga cgatcgaaaa ggtggccgat ttcatcacga cgagcggaaa agagacgtat 9480

gtgccgatcg agcctgcacc gttgcaagag tattatcctg tttcatctgc gcaaaagcgg 9540

atgtatgtcc tgcgccagtt tgcggacaca ggcacggttt ataacatgcc gagcgcgttg 9600

tatatcgaag gcgatctgga tcggaagcgt tttgaagccg ccatccacgg attggtcgag 9660

cggcacgaat cgctgcgcac atccttccac accgtaaatg gcgagcctgt ccagcgcgta 9720

cacgagcatg tcgagctgaa tgtgcagtac gcggaagtga cggaagcgca agtggagcca 9780

accgtcgagt cgttcgtgca agcatttgat ctgacaaaag ctccgctatt gcgggtcgga 9840

cttttcaagc tggcagcgaa acggcatctg ttcctgctgg atatgcatca catcatctcg 9900

gatggcgtct cggccggaat cattatggaa gagttctcga agctgtatcg aggcgaagaa 9960

ctgcctgcgc tttccgtcca ttacaaagat ttcgccgtct ggcagtctga actgttccag 10020

agcgacgtct ataccgagca tgaaaactac tggctgaacg cgttttctgg cgacattccg 10080

gtgcttaact tgccagccga tttttctcgt ccgctgacac agagctttga aggagattgc 10140

gtttcgttcc aggcagacaa agcgttgctg gacgatcttc acaagctcgc tcaggagagc 10200

caatcgacgt tgttcatggt attgctggcg gcttacaatg tgctgcttgc caagtacagc 10260

ggacaggaag acatcgtcgt cggcacaccg attgcgggca gatcgcacgc cgatatcgag 10320

aacgttctgg ggatgtttgt caacacgctc gctttgcgca actatccggt cgagacgaaa 10380

cacttccagg catttttgga agaggtcaag caaaatacgc tgcaagcata cgcccatcaa 10440

gattatccgt tcgaagcact ggtcgaaaag ctggacatcc agcgggatct cagccgcaat 10500

ccgctgtttg acaccatgtt tattttgcaa aacctggacc aaaaagctta cgagctggat 10560

gggctgaaac tggaggcata tccggcacaa gcaggcaacg ccaaattcga tctcacgctg 10620

gaagcgcacg aggacgagac aggcattcat tttgcgctcg tctactcgac caaattgttc 10680

cagcgagaat caatcgaaag aatggcgggt cacttcctgc aagtgctgcg ccaagtcgtt 10740

gccgaccaag caactgcctt gcgcgagatc agcctgctca gcgaggaaga gcgccgaatt 10800

gtgaccgttg atttcaacaa cacgtttgcc tatccgcgcg atctgacgat tcaggagctg 10860

ttcgagcagc aggcagcaaa aactccggag catgcagcgg tcgtgatgga cggacagatg 10920

ctgacgtatc gggagctgaa cgaaaaagcg aaccagctcg cccatgtcct tcgtcaaaac 10980

ggagtcggga aagagagcat cgtcggtctg ctcgcagatc gttcgctgga aatgattaca 11040

ggcatcatgg ggattctcaa agcgggcggc gcctacctgg gactggaccc ggagcatccg 11100

tccgaacgcc tggcttacat gttggaagat ggcggcgtga aagttgtcct cgtgcaaaag 11160

cacttgctgc cgctcgtcgg cgaagggctg atgccaatcg ttttggaaga ggagagcctg 11220

cgcccggaag attgcggcaa tccggcgatt gtcaacggtg cgagtgacct ggcttatgtg 11280

atgtacacct caggctctac aggcaagcca aaaggagtca tggtcgagca tcgcaacgtc 11340

acccgcttgg tcatgcatac gaattacgtg caagtgcgcg agagcgaccg gatgattcaa 11400

accggcgcga ttggcttcga cgccatgaca tttgagattt ttggagcctt gctgcacggg 11460

gccagcctgt atttggtgag caaggacgtc ttgctggatg ccgaaaagct gggcgacttc 11520

ctgcggacga atcagattac gaccatgtgg ctgacctcgc cgctcttcaa ccagctttcg 11580

caagacaatc cggcgatgtt tgacagcttg cgcgccttga tcgtcggtgg cgaagcgttg 11640

tcgccgaagc acatcaaccg ggtaaaaagt gcccttcctg acctggaaat ctggaacgga 11700

tacggcccga ccgaaaacac gaccttctcg acgtgctatt tgattgagca gcattttgaa 11760

gagcagattc cgatcggcaa gccgattgca aactccaccg cgtatatcgt cgacggcaac 11820

aatcagccgc agccgatcgg cgtaccgggt gaactgtgcg tcggtggtga cggtgtcgca 11880

agaggctatg tgaacaagcc ggaattaacc gccgaaaagt ttgtgcccaa tccgtttgcg 11940

cctggcgaaa cgatgtatcg caccggagat ttggcgagat ggctgccgga tgggacgatt 12000

gagtatttgg gccgaatcga ccagcaggtc aaaatcaggg gataccggat cgagcttggg 12060

gaaatcgaga cggtcttgtc ccagcaggca caagtaaaag aagcagtcgt ggccgtgatc 12120

gaggaggcga acgggcaaaa agctctctgc gcttactttg tgccagaaca ggccgtcgac 12180

gccgcagagc tgcgagaagc gatgtccaaa caattgcctg gctacatggt ccctgcttac 12240

tatgtgcaaa tggaaaagct gccgttgacc gcgaacggaa aggtcgaccg ccgggcattg 12300

ccgcagccat ccggcgagcg gacgacagga agcgcctttg tcgctgcgca aaatgatacc 12360

gaagcgaagc tgcaacagat ttggcaagaa gttttgggca ttccggcaat cggcattcac 12420

gacaacttct ttgaaatcgg cggtcattcc ttgaaggcga tgaacgtcat cacgcaagtc 12480

cataaaacat tccaggtgga gctgccgtta aaagcgctgt ttgccactcc gacgatccat 12540

gagttggctg cgcatatttc ggaaaaaacc gagtacaccg cgattcaacc cgtggcagcg 12600

caggagtttt acccggtttc atctgcgcaa aaaagaatgt atatcctgca acagttcgaa 12660

ggcaacggaa tcagctacaa catttcgggt gcgattctcc tggaaggaaa gctggactac 12720

gcccggtttg ccagcgctgt gcaacagctg gcagagcgcc acgaagcttt gcgcacctcg 12780

ttccaccgga tcgacggcga gcctgtgcaa aaagtgcacg aggaagtaga agtgccgctt 12840

ttcatgctgg aggctcccga agaccaggcg gagaaaatca tgcgcgagtt tgtccgtccg 12900

tttgatctcg gggtcgctcc gctgatgcga acaggtttgc tcaagctggg caaagaccgc 12960

catttgtttt tgctcgacat gcaccatatc atctcggacg gcgtttcttc gcaaattttg 13020

ctgcgtgaat ttgccgagtt gtaccaggga gcagacttgc agccgctttc gctgcaatac 13080

aaagatttcg ctgcttggca aaatgagctg tttcagacgg aggcatacaa gaagcaggag 13140

cagcactggc tgaacacgtt tgctgatgaa attccgctct tgaacctgcc gactgactat 13200

ccgcgcccta gcgtgcaaag ctttgcaggc gatctcgtcc tttttgccgc cggaaaagaa 13260

ctgctggagc ggttgcaaca ggtagcgtca gaaacaggca ccaccttgta catgattttg 13320

cttgccgcct acaatgtgct gctgtccaag tataccggcc aggaagacat catcgtcggg 13380

acgcctgtcg ctggacgttc ccatgcggac gtggaaaaca tcatgggcat attcgtgaac 13440

acattggcgc tgcgcaacca gcctgccagc agcaaaacga tgttagaaaa taatattaca 13500

caatgtgact caatcaatga tgtttatctt aaagaagaag caataacatt gatggatatg 13560

cttgagagtc aacttaagca ccaggcagat ggatatgttg ttattgatca agaagaatct 13620

ctcagttacg ctgatttcta tttgagggtg aaagagatag ggtattgtct gtcagaaatt 13680

agctcaaaga attcggtggg tattgggctt ttttgtgatc cttctataga tttaatttgt 13740

ggtgcatggg gtattttgtc agcggataaa gcttatttgc cgttatcgcc tgactatcca 13800

actgaacgcc tcaaatatat gatagaagat tctggtattg atgtgatttt tacgcaatcg 13860

cacttaaaag cacagctaca ggacattgca ccaaaatcag tattaattat gacaccagaa 13920

gatgtcgctc tgacgataaa aacacgaaca atagaagata ttctgggcac agttcaagtt 13980

cctaaaccca ctagtctggc ttatattatt tatacctctg gtagcacggg taagccaaag 14040

ggagtgatga ttgaacatca cagtattgta aatcaaatga gatttcttgc aaaagcgttc 14100

aaattaggat gtcattcccg gattttacag aaaacaccaa tgagttttga tgcggctcaa 14160

tgggaaattc tagcgcctgc aattggtggt caagtgatta tgggtccttt aggttgctat 14220

cgcgatccgg atgcaattat taaaaccatt cttcagcatc aagtaacgac tttgcaatgt 14280

gttcctactt tgctacaagc gttactggat aatcctaatt ttttggattg cttatcattg 14340

actcaagtat tcagtggggg agaagcgctg acaaccaaat tagccacgca atttttgaat 14400

agttttactc actgtgaatt aatcaattta tatggcccga cagaatgtac gattaattca 14460

tcatttttcc gggtgacaaa tgagactttg ccgaattatc aaacctctat ttcgattggt 14520

gcacctgtag ataataccga atactacgtt cttgatgatg atagattacc tgtggcggtt 14580

ggcgaaattg gcgagcttta tatttcgggt gctcaattag cacgtggtta tttgcataaa 14640

ccagaaatga caaaagataa atttatttgt aatcaccttg tatcaggaac tcaacatcaa 14700

tggttatatc gaacgggaga tctggtaacc agaggggctg atggtaatac ttattttgtt 14760

ggtcgggttg atagccaggt caaattacga ggttaccgta ttgagcttga tgaaatacgc 14820

catgcgattg aagaacatag ctggataaag acggcggcaa tgttaattaa gaaggatgcc 14880

agaacgggtt tccaaaatct catcgcgtgt gtggaattag atgagaaaga agctgcattg 14940

atggatcaag gtaatagtag ctcacatcac aaatcaaaag ccgataaact acaggtgaaa 15000

gcccaacttt ctaattctgg ttgtcgaagt gaagagttat gtgaaaatcg ccctacattc 15060

ttacttcctt atcaagaagg ggagataaaa cagagagaat atgcatttgg acgcaagaca 15120

tatcgctatt ttgagggaac agaaataacg gtagagaaat taaaaaaatt gctgacagcc 15180

actcaatcga atgaaattag ctctttgcca ctgagtcatc taaccctgaa tgatttcggt 15240

tatgcattgc gttattttgg tcagtttacc agccatcaac gtttattgcc caaatatgcc 15300

tatgcttcac cgggtgctct ctatgcgaca caaatgtatt ttgaattgca taatgttctc 15360

ggtttggatg cggggattta ctattatcat ccagtgacac ataagttaat aaaaatttca 15420

acattgagtc gtcggcaaat gccaacgata aaagtgcatt ttattggcaa gcatgaagcc 15480

attgagcccg tttataagaa caatatacaa gaagttctgg aaatggaagc gggccatatg 15540

atgggtcttt ttgatgacgt attaccggaa attggcttga gtattggtaa aagtgaatat 15600

caagatgaat gtccagattg gtatgatggt gatattcagg attattatct tggtgcattt 15660

gaaatatgta gctatgaaca tggattgccg ccatttgaga ctgatattta tttacaaaca 15720

catgcccata aaatacctga gatgccgtgt ggtttatatc acttttctaa cggggaattt 15780

gtacgaataa gtgatgatat tgtccgaaaa aaggatgtta ttgcgattaa tcagcaagtt 15840

tatgatcgct ccagttttgg cgtgtcaatt attccacgct gtgtccctga atggcattat 15900

tatataacac tgggtcgtcg gttacatgcg ttacaaagta atccattgta tattggatta 15960

atgtcatctg gttacagttc gaagagcaat aacgatttac cttcggcgaa aaggatgcga 16020

tctattctca atgcacttga tagacctatg gcggcatttt atttctgcat aggtgggggt 16080

attagccaag cgcaatatat gtgtgaaggc atgaaagaag atgttgttca tatgaaaggg 16140

ccagttgaaa tcattaaaga tgatcttcaa caacaactcc ctcaatatat gattccaaat 16200

aaggtattag ttttcgataa attacctttg acggccaatg gaaaagtgga ttatcaatct 16260

ttatcagaat ctaaagccgt ggagaatgtt tcaacacagc gtctattggt gccattacat 16320

acagatactg aaataaggct tggaaaaatt tggatggaag tactgaaatg ggattcagta 16380

tctgccctcg atgatttttt cgaaagtggg ggtaattctt tgatggccgt tgcaatggtt 16440

aataagatca atgcggcctt taatattcgt tttccgttac agatactttt tcaatctcct 16500

aatatagcag aattggctaa gtggattgaa cagacagact ctaaaacaat atcaagatta 16560

attttattga atcaggcaag caaagacccc atttactgtt ggccgggttt gggcggatat 16620

cctatgagtt tgagattgct tgctaataaa gtcgttcctg atcgggcatt ttatggaata 16680

caggcatatg ggataaacga gagtgaaata ccgttttctt ctatccagag aatggcagaa 16740

gaggatatta aagagataaa gaaaatacag ccagaagggc catatatatt gtggggatat 16800

tcatttggtg cccgagtagc atttgaagtt gcataccagc ttgaacaagc gggagaagaa 16860

gttaacgcat tgaatttatt ggctccggga tctcctcatc ttgatatgaa gcaagcggaa 16920

tatatggata aaggcgctga atttactaat ccggcttttg ttaaaatact tttttctgta 16980

ttttctcgtt caatcaacag cccaatggtt aaaacttgct tagaacaagt aaatagtgaa 17040

acgacattta ttaactttat atgtagtcgt tttaaaaact tggaaccatc attagtaaaa 17100

cgtatcgtta ggattgtgac tttgacttat gatttcaagt acagtattga tgagctttat 17160

cacagacacc taaaggcacc tataactatt ttcaaggcga atagagataa tgattcattt 17220

atcgaggaat cggatgtgat ttcatcaatg tcgcctaaaa taattgaatt aatatcggat 17280

cactatcaac tgttggaaag tgaaggtgtt gctgagattg agaaaataat ctaa 17334

<210> 18
<211> 9756
<212> DNA
<213> Artificial Sequence

<220>
<223> NRPS synthesizing a Indigoidine-tagged Dipeptide consisting of
      Proline and Leucine

<400> 18
atggattgcg tggcaaacaa ttcgggagtc gagctttgcc agattccgtt gctgacagaa 60

gcagaaacta gccagctgtt ggcaaagcgt acggaaacag cggctgacta tcctgccgca 120

accatgcacg agctgttttc gcggcaggca gaaaaaacgc ctgagcaagt ggcggtagtc 180

ttcgcggatc agcacctgac gtatcgggag ctggatgaaa aatccaatca gctcgcccgc 240

tttttgcgca aaaaaggcat tggcacgggc agtcttgtcg gcacgctgct ggatcgctcg 300

ctggacatga tcgtcggaat cctcggcgtc ttgaaggcag gcggcgcatt tgtgccgatc 360

gacccggagt tgcctgccga acgaatcgct tacatgctga cgcatagcag agttccattg 420

gtcgtgacgc aaaatcattt gcgggcaaaa gtgaccacgc ctacagaaac aattgacatc 480

aacacagcgg tgatcgggga agagagccgc gcccctatcg aatcgctcaa tcagccgcat 540

gacttgtttt acatcatcta tacgtccgga acgacagggc aaccgaaagg cgtcatgctg 600

gagcatcgca acatggcgaa cctgatgcat tttacgtttg atcagacgaa catcgctttt 660

catgaaaaag tgttgcagta taccacgtgc agctttgatg tttgctacca ggaaattttc 720

tccacgctgc tatccggggg ccagctctac ctgatcacga acgagctgag acggcatgtg 780

gaaaagctgt ttgctttcat ccaggaaaag cagatcagca ttttgtctct cccggtgtcc 840

ttcctgaaat ttatttttaa cgaacaagac tacgcgcaaa gcttcccgcg ttgtgtcaaa 900

catatcatca cggccgggga acaactcgtc gtcacacacg agctgcaaaa gtatctgcgc 960

cagcatcgcg tatttttgca caatcactac ggcccgtcgg agacgcatgt ggtgacgaca 1020

tgcacgatgg acccgggaca ggcgatacca gagctgccgc ccatcggaaa gccgatcagc 1080

aacacaggca tttacatttt ggatgaaggg ctgcaattga agccggaggg gatcgtcggg 1140

gagttgtaca tttccggcgc aaacgtagga agagggtatt tgcaccagcc ggagctgacc 1200

gcggagaagt ttctcgacaa tccgtatcag ccaggcgaaa gaatgtaccg aacgggtgat 1260

ctggcccttt ggttgccgga tggccagctc gaatttttgg gccgaatcga ccatcaggta 1320

aaaatcaggg gccatcgcat cgagctggga gagatcgaat cgcgcctgct caaccatccc 1380

gccatcaagg aagcggtggt tatcgaccga gcagacgaga caggcggcaa gtttttgtgc 1440

gcctatgtcg tcctgcaaaa agcgctcagc gacgaagaga tgcgggcata cttggcgcaa 1500

gcgttgccgg agtatatgat cccttccttt ttcgtgacgc tggagcggat tccagtcacg 1560

ccgaacggaa aaacagacag gcgagctttg ccgaagccgg aaggaagtgc caagacgaaa 1620

gcggattacg tcgccccgac gactgagctg gaacaaaagc tggtcgcgat ttgggagcaa 1680

attcttggcg tgtcgccgat cggcattcag gatcattttt tcacgctggg cggccattcg 1740

ttaaaagcga ttcagctcat ttcccgcatc caaaaggaat gccaggcgga tgtcccgctg 1800

cgcgtcctgt ttgagcaacc gacgattcaa gcgctggcag cgtatgtgga aggcggggag 1860

gaagggaatg tcttctcgat cgagcctgtg caaaagcaag cgtactatcc ggtctcctcg 1920

gcacaaaagc gcatgtacat cctcgatcaa tttgagggag tcggcatcag ctacaacatg 1980

ccgtcgacta tgctgatcga aggcaagctg gagcgaacac gggtagaagc ggcgttccag 2040

cgcttgattg cgcgacatga aagcctgcgc acttcgtttg ccgtcgtcaa cggagagcct 2100

gtgcaaaaca ttcacgagga cgttccgttt gcgcttgcct attcggaagt cacagaacag 2160

gaggcgcgcg aactcgtttc ttctctcgtg cagccgttcg atctggaggt cgcaccactc 2220

atccgcgtgt cgctgctgaa aatcggcgag gatcgttacg tgctctttac cgacatgcat 2280

cacagcattt ccgatggcgt atcctccggc attcttttgg cagagtgggt gcagctgtac 2340

cagggtgacg ttttgccgga gctgcgtatc cagtacaagg actttgctgt gtggcaacaa 2400

gagttttccc agtcggctgc cttccacaag caggaagcgt actggttgca aacgtttgcc 2460

gatgacattc ctgtgctgaa cttgccgacc gatttcaccc gccccagcac ccaaagcttt 2520

gccggggatc agtgcacgat cggcgcgggc aaagcgctca cggaaggctt gcaccagttg 2580

gcgcaggcga cgggaacgac tttgtacatg gttttgctcg ccgcgtacaa cgtgctgctc 2640

gccaagtatg ccgggcagga ggacatcatc gtcggcacgc cgattacagg cagatcccat 2700

gccgatctcg aaccgatcgt cggcatgttc gtgaacacct tggcgatgcg aaacaaaccg 2760

cagcgcgaaa agacttttag cgagtttttg caagaagtca agcaaaatgc gctggatgcg 2820

tacggccatc aggattaccc gtttgaagaa ctggtggaaa agctcgcgat cgcgcgcgat 2880

ttgagccgaa atccgctgtt tgacaccgtg tttacgttcc aaaacagcac ggaagaggtc 2940

atgacgctgc ctgaatgcac gcttgcgccg tttatgacgg acgaaacagg ccagcacgcc 3000

aagttcgact tgactttcag cgctacggaa gagcgggaag aaatgacgat tggcgtggag 3060

tacagcacaa gcttgtttac gcgggaaacg atggaacggt tcagccgcca cttcctgacg 3120

attgcagcga gcatcgtgca aaatccgcac atccgtctgg gcgagatcga catgcttttg 3180

ccagaagaaa aacagcagat tttggccggg ttcaacgata cggcagtcag ctatgcgctg 3240

gacaaaacgc tgcaccagct attcgaagag caggtcgaca aaacaccgga tcaggcagcg 3300

cttctcttta gcgagcaatc gctgacgtac agcgaactga acgagcgagc aaacagactg 3360

gcaagggtcc tgcgcgcaaa aggagtcgga ccggaccgtc tggtagcgat catggcggag 3420

cgctcgccgg aaatggtgat cggtattctc ggtattttga aggcaggcgg cgcttatgtt 3480

cccgtcgatc ccggctatcc gcaggagcgc attcagtacc tgctcgaaga tagcaacgca 3540

gccctgctgc tcagccaggc gcatctgttg ccgctgttgg cccaggtgtc aagcgagctg 3600

ccggagtgcc ttgatctgaa cgctgaactg gatgccggac tgagcggctc caacctgcca 3660

gctgtcaacc aaccgactga ccttgcctac gtcatctata catccggtac gaccggcaag 3720

ccgaagggtg tcatgatccc gcatcaagga atcgtgaact gcttgcagtg gagaagagac 3780

gaatacgggt tcgggccgag tgacaaggcg ttgcaagtgt tctcctttgc cttcgacggt 3840

tttgtagcca gcttgttcgc tccgctgctc ggaggggcaa cgtgcgtgtt gccgcaagaa 3900

gcagctgcca aagacccggt cgcgctgaaa aaactgatgg ccgcaacgga agtcacccat 3960

tactacggcg taccgagtct gttccaggcc attctcgatt gctcgacgac aaccgacttc 4020

aatcagttgc gttgcgtcac tttgggcggc gagaagctgc ctgtgcagct tgtgcaaaaa 4080

acaaaagaaa agcatccggc aatcgagatc aacaacgagt acggcccgac ggaaaacagc 4140

gtcgtcacca ccatctcgcg ctcgattgaa gcggggcaag cgatcacgat tggccgaccg 4200

cttgcgaacg tccaagtcta cattgtagat gagcagcatc acttgcagcc gattggcgtg 4260

gtcggtgagc tgtgcatcgg cggagccggg cttgccagag gctatctgaa caaaccggag 4320

ctgaccgcag agaagtttgt cgcaaatccg ttccgaccag gcgagcgcat gtacaaaaca 4380

ggcgacttgg taaaatggcg gacggatggc acgatcgagt acatcggccg cgcagacgaa 4440

caggtcaagg tgagagggta tcgcatcgag atcggcgaga tcgagagcgc cgtactcgct 4500

taccagggca tcgatcaagc ggtggtcgtt gcgcgagacg atgacgctac ggctggttcc 4560

tatctttgcg cctactttgt cgcagcaaca gccgtgtccg tatccggctt gagaagccat 4620

ctggccaaag agctgcctgc ttacatgatt ccgagctatt tcgtcgagct ggatcagctg 4680

ccgctttccg ccaatggaaa agtggatcgc aaagctttgc cgaagccgca acagtccgat 4740

gcgaccacgc gcgaatacgt ggccccgagg aatgcgaccg aacagcaact ggcagccatc 4800

tggcaagaag ttttgggagt agagccaatc ggcatcaccg accagttctt tgaactcgga 4860

ggacattcct taaaagctac gctgttgatt gccaaagtgt atgagtacat gcaaatcgag 4920

ctgccgctga atctcatctt ccagtatccg acgatcgaaa aggtggccga tttcatcacg 4980

tcggaaaaaa ccgagtacac cgcgattcaa cccgtggcag cgcaggagtt ttacccggtt 5040

tcatctgcgc aaaaaagaat gtatatcctg caacagttcg aaggcaacgg aatcagctac 5100

aacatttcgg gtgcgattct cctggaagga aagctggact acgcccggtt tgccagcgct 5160

gtgcaacagc tggcagagcg ccacgaagct ttgcgcacct cgttccaccg gatcgacggc 5220

gagcctgtgc aaaaagtgca cgaggaagta gaagtgccgc ttttcatgct ggaggctccc 5280

gaagaccagg cggagaaaat catgcgcgag tttgtccgtc cgtttgatct cggggtcgct 5340

ccgctgatgc gaacaggttt gctcaagctg ggcaaagacc gccatttgtt tttgctcgac 5400

atgcaccata tcatctcgga cggcgtttct tcgcaaattt tgctgcgtga atttgccgag 5460

ttgtaccagg gagcagactt gcagccgctt tcgctgcaat acaaagattt cgctgcttgg 5520

caaaatgagc tgtttcagac ggaggcatac aagaagcagg agcagcactg gctgaacacg 5580

tttgctgatg aaattccgct cttgaacctg ccgactgact atccgcgccc tagcgtgcaa 5640

agctttgcag gcgatctcgt cctttttgcc gccggaaaag aactgctgga gcggttgcaa 5700

caggtagcgt cagaaacagg caccaccttg tacatgattt tgcttgccgc ctacaatgtg 5760

ctgctgtcca agtataccgg ccaggaagac atcatcgtcg ggacgcctgt cgctggacgt 5820

tcccatgcgg acgtggaaaa catcatgggc atattcgtga acacattggc gctgcgcaac 5880

cagcctgcca gcagcaaaac gatgttagaa aataatatta cacaatgtga ctcaatcaat 5940

gatgtttatc ttaaagaaga agcaataaca ttgatggata tgcttgagag tcaacttaag 6000

caccaggcag atggatatgt tgttattgat caagaagaat ctctcagtta cgctgatttc 6060

tatttgaggg tgaaagagat agggtattgt ctgtcagaaa ttagctcaaa gaattcggtg 6120

ggtattgggc ttttttgtga tccttctata gatttaattt gtggtgcatg gggtattttg 6180

tcagcggata aagcttattt gccgttatcg cctgactatc caactgaacg cctcaaatat 6240

atgatagaag attctggtat tgatgtgatt tttacgcaat cgcacttaaa agcacagcta 6300

caggacattg caccaaaatc agtattaatt atgacaccag aagatgtcgc tctgacgata 6360

aaaacacgaa caatagaaga tattctgggc acagttcaag ttcctaaacc cactagtctg 6420

gcttatatta tttatacctc tggtagcacg ggtaagccaa agggagtgat gattgaacat 6480

cacagtattg taaatcaaat gagatttctt gcaaaagcgt tcaaattagg atgtcattcc 6540

cggattttac agaaaacacc aatgagtttt gatgcggctc aatgggaaat tctagcgcct 6600

gcaattggtg gtcaagtgat tatgggtcct ttaggttgct atcgcgatcc ggatgcaatt 6660

attaaaacca ttcttcagca tcaagtaacg actttgcaat gtgttcctac tttgctacaa 6720

gcgttactgg ataatcctaa ttttttggat tgcttatcat tgactcaagt attcagtggg 6780

ggagaagcgc tgacaaccaa attagccacg caatttttga atagttttac tcactgtgaa 6840

ttaatcaatt tatatggccc gacagaatgt acgattaatt catcattttt ccgggtgaca 6900

aatgagactt tgccgaatta tcaaacctct atttcgattg gtgcacctgt agataatacc 6960

gaatactacg ttcttgatga tgatagatta cctgtggcgg ttggcgaaat tggcgagctt 7020

tatatttcgg gtgctcaatt agcacgtggt tatttgcata aaccagaaat gacaaaagat 7080

aaatttattt gtaatcacct tgtatcagga actcaacatc aatggttata tcgaacggga 7140

gatctggtaa ccagaggggc tgatggtaat acttattttg ttggtcgggt tgatagccag 7200

gtcaaattac gaggttaccg tattgagctt gatgaaatac gccatgcgat tgaagaacat 7260

agctggataa agacggcggc aatgttaatt aagaaggatg ccagaacggg tttccaaaat 7320

ctcatcgcgt gtgtggaatt agatgagaaa gaagctgcat tgatggatca aggtaatagt 7380

agctcacatc acaaatcaaa agccgataaa ctacaggtga aagcccaact ttctaattct 7440

ggttgtcgaa gtgaagagtt atgtgaaaat cgccctacat tcttacttcc ttatcaagaa 7500

ggggagataa aacagagaga atatgcattt ggacgcaaga catatcgcta ttttgaggga 7560

acagaaataa cggtagagaa attaaaaaaa ttgctgacag ccactcaatc gaatgaaatt 7620

agctctttgc cactgagtca tctaaccctg aatgatttcg gttatgcatt gcgttatttt 7680

ggtcagttta ccagccatca acgtttattg cccaaatatg cctatgcttc accgggtgct 7740

ctctatgcga cacaaatgta ttttgaattg cataatgttc tcggtttgga tgcggggatt 7800

tactattatc atccagtgac acataagtta ataaaaattt caacattgag tcgtcggcaa 7860

atgccaacga taaaagtgca ttttattggc aagcatgaag ccattgagcc cgtttataag 7920

aacaatatac aagaagttct ggaaatggaa gcgggccata tgatgggtct ttttgatgac 7980

gtattaccgg aaattggctt gagtattggt aaaagtgaat atcaagatga atgtccagat 8040

tggtatgatg gtgatattca ggattattat cttggtgcat ttgaaatatg tagctatgaa 8100

catggattgc cgccatttga gactgatatt tatttacaaa cacatgccca taaaatacct 8160

gagatgccgt gtggtttata tcacttttct aacggggaat ttgtacgaat aagtgatgat 8220

attgtccgaa aaaaggatgt tattgcgatt aatcagcaag tttatgatcg ctccagtttt 8280

ggcgtgtcaa ttattccacg ctgtgtccct gaatggcatt attatataac actgggtcgt 8340

cggttacatg cgttacaaag taatccattg tatattggat taatgtcatc tggttacagt 8400

tcgaagagca ataacgattt accttcggcg aaaaggatgc gatctattct caatgcactt 8460

gatagaccta tggcggcatt ttatttctgc ataggtgggg gtattagcca agcgcaatat 8520

atgtgtgaag gcatgaaaga agatgttgtt catatgaaag ggccagttga aatcattaaa 8580

gatgatcttc aacaacaact ccctcaatat atgattccaa ataaggtatt agttttcgat 8640

aaattacctt tgacggccaa tggaaaagtg gattatcaat ctttatcaga atctaaagcc 8700

gtggagaatg tttcaacaca gcgtctattg gtgccattac atacagatac tgaaataagg 8760

cttggaaaaa tttggatgga agtactgaaa tgggattcag tatctgccct cgatgatttt 8820

ttcgaaagtg ggggtaattc tttgatggcc gttgcaatgg ttaataagat caatgcggcc 8880

tttaatattc gttttccgtt acagatactt tttcaatctc ctaatatagc agaattggct 8940

aagtggattg aacagacaga ctctaaaaca atatcaagat taattttatt gaatcaggca 9000

agcaaagacc ccatttactg ttggccgggt ttgggcggat atcctatgag tttgagattg 9060

cttgctaata aagtcgttcc tgatcgggca ttttatggaa tacaggcata tgggataaac 9120

gagagtgaaa taccgttttc ttctatccag agaatggcag aagaggatat taaagagata 9180

aagaaaatac agccagaagg gccatatata ttgtggggat attcatttgg tgcccgagta 9240

gcatttgaag ttgcatacca gcttgaacaa gcgggagaag aagttaacgc attgaattta 9300

ttggctccgg gatctcctca tcttgatatg aagcaagcgg aatatatgga taaaggcgct 9360

gaatttacta atccggcttt tgttaaaata cttttttctg tattttctcg ttcaatcaac 9420

agcccaatgg ttaaaacttg cttagaacaa gtaaatagtg aaacgacatt tattaacttt 9480

atatgtagtc gttttaaaaa cttggaacca tcattagtaa aacgtatcgt taggattgtg 9540

actttgactt atgatttcaa gtacagtatt gatgagcttt atcacagaca cctaaaggca 9600

cctataacta ttttcaaggc gaatagagat aatgattcat ttatcgagga atcggatgtg 9660

atttcatcaa tgtcgcctaa aataattgaa ttaatatcgg atcactatca actgttggaa 9720

agtgaaggtg ttgctgagat tgagaaaata atctaa 9756

<210>  19
<211>  12855
<212>  DNA
<213>  Artificial Sequence

<220>
<223>  NRPS synthesizing a Valine-Indigoidine-tagged Dipeptide
       consisting of Proline and Leucine. Valine is here used as spacer.

<400>  19
   atggattgcg tggcaaacaa ttcgggagtc gagctttgcc agattccgtt gctgacagaa      60
   gcagaaacta gccagctgtt ggcaaagcgt acggaaacag cggctgacta tcctgccgca     120
   accatgcacg agctgttttc gcggcaggca gaaaaaacgc ctgagcaagt ggcggtagtc     180
   ttcgcggatc agcacctgac gtatcgggag ctggatgaaa aatccaatca gctcgcccgc     240
   tttttgcgca aaaaaggcat tggcacgggc agtcttgtcg gcacgctgct ggatcgctcg     300
   ctggacatga tcgtcggaat cctcggcgtc ttgaaggcag gcggcgcatt tgtgccgatc     360
   gacccggagt tgcctgccga acgaatcgct tacatgctga cgcatagcag agttccattg     420
   gtcgtgacgc aaaatcattt gcgggcaaaa gtgaccacgc ctacagaaac aattgacatc     480
   aacacagcgg tgatcgggga agagagccgc gcccctatcg aatcgctcaa tcagccgcat     540
   gacttgtttt acatcatcta tacgtccgga acgacagggc aaccgaaagg cgtcatgctg     600
   gagcatcgca acatggcgaa cctgatgcat tttacgtttg atcagacgaa catcgctttt     660
   catgaaaaag tgttgcagta taccacgtgc agctttgatg tttgctacca ggaaattttc     720
   tccacgctgc tatccggggg ccagctctac ctgatcacga acgagctgag acggcatgtg     780
   gaaaagctgt ttgctttcat ccaggaaaag cagatcagca ttttgtctct cccggtgtcc     840
   ttcctgaaat ttatttttaa cgaacaagac tacgcgcaaa gcttcccgcg ttgtgtcaaa     900
   catatcatca cggccgggga acaactcgtc gtcacacacg agctgcaaaa gtatctgcgc     960
   cagcatcgcg tatttttgca caatcactac ggcccgtcgg agacgcatgt ggtgacgaca    1020
   tgcacgatgg acccgggaca ggcgatacca gagctgccgc ccatcggaaa gccgatcagc    1080
   aacacaggca tttacatttt ggatgaaggg ctgcaattga agccggaggg gatcgtcggg    1140
   gagttgtaca tttccggcgc aaacgtagga agagggtatt tgcaccagcc ggagctgacc    1200
   gcggagaagt ttctcgacaa tccgtatcag ccaggcgaaa gaatgtaccg aacgggtgat    1260
   ctggcccttt ggttgccgga tggccagctc gaatttttgg gccgaatcga ccatcaggta    1320
   aaaatcaggg gccatcgcat cgagctggga gagatcgaat cgcgcctgct caaccatccc    1380
   gccatcaagg aagcggtggt tatcgaccga gcagacgaga caggcggcaa gtttttgtgc    1440
   gcctatgtcg tcctgcaaaa agcgctcagc gacgaagaga tgcgggcata cttggcgcaa    1500
   gcgttgccgg agtatatgat cccttccttt ttcgtgacgc tggagcggat tccagtcacg    1560
   ccgaacggaa aaacagacag gcgagctttg ccgaagccgg aaggaagtgc caagacgaaa 1620
   gcggattacg tcgccccgac gactgagctg gaacaaaagc tggtcgcgat ttgggagcaa 1680
   attcttggcg tgtcgccgat cggcattcag gatcattttt tcacgctggg cggccattcg 1740
   ttaaaagcga ttcagctcat ttcccgcatc caaaaggaat gccaggcgga tgtcccgctg 1800
   cgcgtcctgt ttgagcaacc gacgattcaa gcgctggcag cgtatgtgga aggcggggag 1860
   gaagggaatg tcttctcgat cgagcctgtg caaaagcaag cgtactatcc ggtctcctcg 1920
   gcacaaaagc gcatgtacat cctcgatcaa tttgagggag tcggcatcag ctacaacatg 1980
   ccgtcgacta tgctgatcga aggcaagctg gagcgaacac gggtagaagc ggcgttccag 2040
   cgcttgattg cgcgacatga aagcctgcgc acttcgtttg ccgtcgtcaa cggagagcct 2100
   gtgcaaaaca ttcacgagga cgttccgttt gcgcttgcct attcggaagt cacagaacag 2160
   gaggcgcgcg aactcgtttc ttctctcgtg cagccgttcg atctggaggt cgcaccactc 2220
   atccgcgtgt cgctgctgaa aatcggcgag gatcgttacg tgctctttac cgacatgcat 2280
   cacagcattt ccgatggcgt atcctccggc attcttttgg cagagtgggt gcagctgtac 2340
   cagggtgacg ttttgccgga gctgcgtatc cagtacaagg actttgctgt gtggcaacaa 2400
   gagttttccc agtcggctgc cttccacaag caggaagcgt actggttgca aacgtttgcc 2460
   gatgacattc ctgtgctgaa cttgccgacc gatttcaccc gccccagcac ccaaagcttt 2520
   gccggggatc agtgcacgat cggcgcgggc aaagcgctca cggaaggctt gcaccagttg 2580
   gcgcaggcga cgggaacgac tttgtacatg gttttgctcg ccgcgtacaa cgtgctgctc 2640
   gccaagtatg ccgggcagga ggacatcatc gtcggcacgc cgattacagg cagatcccat 2700
   gccgatctcg aaccgatcgt cggcatgttc gtgaacacct tggcgatgcg aaacaaaccg 2760
   cagcgcgaaa agacttttag cgagtttttg caagaagtca agcaaaatgc gctggatgcg 2820
   tacggccatc aggattaccc gtttgaagaa ctggtggaaa agctcgcgat cgcgcgcgat 2880
   ttgagccgaa atccgctgtt tgacaccgtg tttacgttcc aaaacagcac ggaagaggtc 2940
   atgacgctgc ctgaatgcac gcttgcgccg tttatgacgg acgaaacagg ccagcacgcc 3000
   aagttcgact tgactttcag cgctacggaa gagcgggaag aaatgacgat tggcgtggag 3060
   tacagcacaa gcttgtttac gcgggaaacg atggaacggt tcagccgcca cttcctgacg 3120
   attgcagcga gcatcgtgca aaatccgcac atccgtctgg gcgagatcga catgcttttg 3180
   ccagaagaaa aacagcagat tttggccggg ttcaacgata cggcagtcag ctatgcgctg 3240
   gacaaaacgc tgcaccagct attcgaagag caggtcgaca aaacaccgga tcaggcagcg 3300
   cttctcttta gcgagcaatc gctgacgtac agcgaactga acgagcgagc aaacagactg 3360
   gcaagggtcc tgcgcgcaaa aggagtcgga ccggaccgtc tggtagcgat catggcggag 3420
   cgctcgccgg aaatggtgat cggtattctc ggtattttga aggcaggcgg cgcttatgtt 3480
   cccgtcgatc ccggctatcc gcaggagcgc attcagtacc tgctcgaaga tagcaacgca 3540
   gccctgctgc tcagccaggc gcatctgttg ccgctgttgg cccaggtgtc aagcgagctg 3600
   ccggagtgcc ttgatctgaa cgctgaactg gatgccggac tgagcggctc caacctgcca 3660
   gctgtcaacc aaccgactga ccttgcctac gtcatctata catccggtac gaccggcaag 3720
   ccgaagggtg tcatgatccc gcatcaagga atcgtgaact gcttgcagtg gagaagagac 3780
   gaatacgggt tcggcccgag tgacaaggcg ttgcaagtgt tctcctttgc cttcgacggt 3840
   tttgtagcca gcttgttcgc tccgctgctc ggaggggcaa cgtgcgtgtt gccgcaagaa 3900
   gcagctgcca aagacccggt cgcgctgaaa aaactgatgg ccgcaacgga agtcacccat 3960
   tactacggcg taccgagtct gttccaggcc attctcgatt gctcgacgac aaccgacttc 4020
   aatcagttgc gttgcgtcac tttgggcggc gagaagctgc ctgtgcagct tgtgcaaaaa 4080
   acaaaagaaa agcatccggc aatcgagatc aacaacgagt acggcccgac ggaaaacagc 4140
   gtcgtcacca ccatctcgcg ctcgattgaa gcggggcaag cgatcacgat tggccgaccg 4200
   cttgcgaacg tccaagtcta cattgtagat gagcagcatc acttgcagcc gattggcgtg 4260
   gtcggtgagc tgtgcatcgg cggagccggg cttgccagag gctatctgaa caaaccggag 4320
   ctgaccgcag agaagtttgt cgcaaatccg ttccgaccag gcgagcgcat gtacaaaaca 4380
   ggcgacttgg taaaatggcg gacggatggc acgatcgagt acatcggccg cgcagacgaa 4440
   caggtcaagg tgagagggta tcgcatcgag atcggcgaga tcgagagcgc cgtactcgct 4500
   taccagggca tcgatcaagc ggtggtcgtt gcgcgagacg atgacgctac ggctggttcc 4560
   tatctttgcg cctactttgt cgcagcaaca gccgtgtccg tatccggctt gagaagccat 4620
   ctggccaaag agctgcctgc ttacatgatt ccgagctatt tcgtcgagct ggatcagctg 4680
   ccgctttccg ccaatggaaa agtggatcgc aaagctttgc cgaagccgca acagtccgat 4740
   gcgaccacgc gcgaatacgt ggccccgagg aatgcgaccg aacagcaact ggcagccatc 4800
   tggcaagaag ttttgggagt agagccaatc ggcatcaccg accagttctt tgaactcgga 4860
   ggacattcct taaaagctac gctgttgatt gccaaagtgt atgagtacat gcaaatcgag 4920
   ctgccgctga atctcatctt ccagtatccg acgatcgaaa aggtggccga tttcatcacg 4980
   acgagcggaa aagagacgta tgtgccgatc gagcctgcac cgttgcaaga gtattatcct 5040
   gtttcatctg cgcaaaagcg gatgtatgtc ctgcgccagt ttgcggacac aggcacggtt 5100
   tataacatgc cgagcgcgtt gtatatcgaa ggcgatctgg atcggaagcg ttttgaagcc 5160
   gccatccacg gattggtcga gcggcacgaa tcgctgcgca catccttcca caccgtaaat 5220
   ggcgagcctg tccagcgcgt acacgagcat gtcgagctga atgtgcagta cgcggaagtg 5280
   acggaagcgc aagtggagcc aaccgtcgag tcgttcgtgc aagcatttga tctgacaaaa 5340
   gctccgctat tgcgggtcgg acttttcaag ctggcagcga aacggcatct gttcctgctg 5400
   gatatgcatc acatcatctc ggatggcgtc tcggccggaa tcattatgga agagttctcg 5460
   aagctgtatc gaggcgaaga actgcctgcg ctttccgtcc attacaaaga tttcgccgtc 5520
   tggcagtctg aactgttcca gagcgacgtc tataccgagc atgaaaacta ctggctgaac 5580
   gcgttttctg gcgacattcc ggtgcttaac ttgccagccg atttttctcg tccgctgaca 5640
   cagagctttg aaggagattg cgtttcgttc caggcagaca aagcgttgct ggacgatctt 5700
   cacaagctcg ctcaggagag ccaatcgacg ttgttcatgg tattgctggc ggcttacaat 5760
   gtgctgcttg ccaagtacag cggacaggaa gacatcgtcg tcggcacacc gattgcgggc 5820
   agatcgcacg ccgatatcga gaacgttctg gggatgtttg tcaacacgct cgctttgcgc 5880
   aactatccgg tcgagacgaa acacttccag gcatttttgg aagaggtcaa gcaaaatacg 5940
   ctgcaagcat acgcccatca agattatccg ttcgaagcac tggtcgaaaa gctggacatc 6000
   cagcgggatc tcagccgcaa tccgctgttt gacaccatgt ttattttgca aaacctggac 6060
   caaaaagctt acgagctgga tgggctgaaa ctggaggcat atccggcaca agcaggcaac 6120
   gccaaattcg atctcacgct ggaagcgcac gaggacgaga caggcattca ttttgcgctc 6180
   gtctactcga ccaaattgtt ccagcgagaa tcaatcgaaa gaatggcggg tcacttcctg 6240
   caagtgctgc gccaagtcgt tgccgaccaa gcaactgcct tgcgcgagat cagcctgctc 6300
   agcgaggaag agcgccgaat tgtgaccgtt gatttcaaca acacgtttgc ctatccgcgc 6360
   gatctgacga ttcaggagct gttcgagcag caggcagcaa aaactccgga gcatgcagcg 6420
   gtcgtgatgg acggacagat gctgacgtat cgggagctga acgaaaaagc gaaccagctc 6480
   gcccatgtcc ttcgtcaaaa cggagtcggg aaagagagca tcgtcggtct gctcgcagat 6540
   cgttcgctgg aaatgattac aggcatcatg gggattctca aagcgggcgg cgcctacctg 6600
   ggactggacc cggagcatcc gtccgaacgc ctggcttaca tgttggaaga tggcggcgtg 6660
   aaagttgtcc tcgtgcaaaa gcacttgctg ccgctcgtcg gcgaagggct gatgccaatc 6720
   gttttggaag aggagagcct gcgcccggaa gattgcggca atccggcgat tgtcaacggt 6780
   gcgagtgacc tggcttatgt gatgtacacc tcaggctcta caggcaagcc aaaaggagtc 6840
   atggtcgagc atcgcaacgt cacccgcttg gtcatgcata cgaattacgt gcaagtgcgc 6900
   gagagcgacc ggatgattca aaccggcgcg attggcttcg acgccatgac atttgagatt 6960
   tttggagcct tgctgcacgg ggccagcctg tatttggtga gcaaggacgt cttgctggat 7020
   gccgaaaagc tgggcgactt cctgcggacg aatcagatta cgaccatgtg gctgacctcg 7080
   ccgctcttca accagctttc gcaagacaat ccggcgatgt ttgacagctt gcgcgccttg 7140
   atcgtcggtg gcgaagcgtt gtcgccgaag cacatcaacc gggtaaaaag tgcccttcct 7200
   gacctggaaa tctggaacgg atacggcccg accgaaaaca cgaccttctc gacgtgctat 7260
   ttgattgagc agcattttga agagcagatt ccgatcggca agccgattgc aaactccacc 7320
   gcgtatatcg tcgacggcaa caatcagccg cagccgatcg gcgtaccggg tgaactgtgc 7380
   gtcggtggtg acggtgtcgc aagaggctat gtgaacaagc cggaattaac cgccgaaaag 7440
   tttgtgccca atccgtttgc gcctggcgaa acgatgtatc gcaccggaga tttggcgaga 7500
   tggctgccgg atgggacgat tgagtatttg ggccgaatcg accagcaggt caaaatcagg 7560
   ggataccgga tcgagcttgg ggaaatcgag acggtcttgt cccagcaggc acaagtaaaa 7620
   gaagcagtcg tggccgtgat cgaggaggcg aacgggcaaa aagctctctg cgcttacttt 7680
   gtgccagaac aggccgtcga cgccgcagag ctgcgagaag cgatgtccaa acaattgcct 7740
   ggctacatgg tccctgctta ctatgtgcaa atggaaaagc tgccgttgac cgcgaacgga 7800
   aaggtcgacc gccgggcatt gccgcagcca tccggcgagc ggacgacagg aagcgccttt 7860
   gtcgctgcgc aaaatgatac cgaagcgaag ctgcaacaga tttggcaaga agttttgggc 7920
   attccggcaa tcggcattca cgacaacttc tttgaaatcg gcggtcattc cttgaaggcg 7980
   atgaacgtca tcacgcaagt ccataaaaca ttccaggtgg agctgccgtt aaaagcgctg 8040
   tttgccactc cgacgatcca tgagttggct gcgcatattt cggaaaaaac cgagtacacc 8100
   gcgattcaac ccgtggcagc gcaggagttt tacccggttt catctgcgca aaaaagaatg 8160
   tatatcctgc aacagttcga aggcaacgga atcagctaca acatttcggg tgcgattctc 8220
   ctggaaggaa agctggacta cgcccggttt gccagcgctg tgcaacagct ggcagagcgc 8280
   cacgaagctt tgcgcacctc gttccaccgg atcgacggcg agcctgtgca aaaagtgcac 8340
   gaggaagtag aagtgccgct tttcatgctg gaggctcccg aagaccaggc ggagaaaatc 8400
   atgcgcgagt ttgtccgtcc gtttgatctc ggggtcgctc cgctgatgcg aacaggtttg 8460
   ctcaagctgg gcaaagaccg ccatttgttt ttgctcgaca tgcaccatat catctcggac 8520
   ggcgtttctt cgcaaatttt gctgcgtgaa tttgccgagt tgtaccaggg agcagacttg 8580
   cagccgcttt cgctgcaata caaagatttc gctgcttggc aaaatgagct gtttcagacg 8640
   gaggcataca agaagcagga gcagcactgg ctgaacacgt ttgctgatga aattccgctc 8700
   ttgaacctgc cgactgacta tccgcgccct agcgtgcaaa gctttgcagg cgatctcgtc 8760
   ctttttgccg ccggaaaaga actgctggag cggttgcaac aggtagcgtc agaaacaggc 8820
   accaccttgt acatgatttt gcttgccgcc tacaatgtgc tgctgtccaa gtataccggc 8880
   caggaagaca tcatcgtcgg gacgcctgtc gctggacgtt cccatgcgga cgtggaaaac 8940
   atcatgggca tattcgtgaa cacattggcg ctgcgcaacc agcctgccag cagcaaaacg 9000
   atgttagaaa ataatattac acaatgtgac tcaatcaatg atgtttatct taaagaagaa 9060
   gcaataacat tgatggatat gcttgagagt caacttaagc accaggcaga tggatatgtt 9120
   gttattgatc aagaagaatc tctcagttac gctgatttct atttgagggt gaaagagata 9180
   gggtattgtc tgtcagaaat tagctcaaag aattcggtgg gtattgggct tttttgtgat 9240
   ccttctatag atttaatttg tggtgcatgg ggtattttgt cagcggataa agcttatttg 9300
   ccgttatcgc ctgactatcc aactgaacgc ctcaaatata tgatagaaga ttctggtatt 9360
   gatgtgattt ttacgcaatc gcacttaaaa gcacagctac aggacattgc accaaaatca 9420
   gtattaatta tgacaccaga agatgtcgct ctgacgataa aaacacgaac aatagaagat 9480
   attctgggca cagttcaagt tcctaaaccc actagtctgg cttatattat ttatacctct 9540
   ggtagcacgg gtaagccaaa gggagtgatg attgaacatc acagtattgt aaatcaaatg 9600
   agatttcttg caaaagcgtt caaattagga tgtcattccc ggattttaca gaaaacacca 9660
   atgagttttg atgcggctca atgggaaatt ctagcgcctg caattggtgg tcaagtgatt 9720
   atgggtcctt taggttgcta tcgcgatccg gatgcaatta ttaaaaccat tcttcagcat 9780
   caagtaacga ctttgcaatg tgttcctact ttgctacaag cgttactgga taatcctaat 9840
   tttttggatt gcttatcatt gactcaagta ttcagtgggg gagaagcgct gacaaccaaa 9900
   ttagccacgc aatttttgaa tagttttact cactgtgaat taatcaattt atatggcccg 9960
   acagaatgta cgattaattc atcatttttc cgggtgacaa atgagacttt gccgaattat 10020
   caaacctcta tttcgattgg tgcacctgta gataataccg aatactacgt tcttgatgat 10080
   gatagattac ctgtggcggt tggcgaaatt ggcgagcttt atatttcggg tgctcaatta 10140
   gcacgtggtt atttgcataa accagaaatg acaaaagata aatttatttg taatcacctt 10200
   gtatcaggaa ctcaacatca atggttatat cgaacgggag atctggtaac cagaggggct 10260
   gatggtaata cttattttgt tggtcgggtt gatagccagg tcaaattacg aggttaccgt 10320
   attgagcttg atgaaatacg ccatgcgatt gaagaacata gctggataaa gacggcggca 10380
   atgttaatta agaaggatgc cagaacgggt ttccaaaatc tcatcgcgtg tgtggaatta 10440
   gatgagaaag aagctgcatt gatggatcaa ggtaatagta gctcacatca caaatcaaaa 10500
   gccgataaac tacaggtgaa agcccaactt tctaattctg gttgtcgaag tgaagagtta 10560
   tgtgaaaatc gccctacatt cttacttcct tatcaagaag gggagataaa acagagagaa 10620
   tatgcatttg gacgcaagac atatcgctat tttgagggaa cagaaataac ggtagagaaa 10680
   ttaaaaaaat tgctgacagc cactcaatcg aatgaaatta gctctttgcc actgagtcat 10740
   ctaaccctga atgatttcgg ttatgcattg cgttattttg gtcagtttac cagccatcaa 10800
   cgtttattgc ccaaatatgc ctatgcttca ccgggtgctc tctatgcgac acaaatgtat 10860
   tttgaattgc ataatgttct cggtttggat gcggggattt actattatca tccagtgaca 10920
   cataagttaa taaaaatttc aacattgagt cgtcggcaaa tgccaacgat aaaagtgcat 10980
   tttattggca agcatgaagc cattgagccc gtttataaga acaatataca agaagttctg 11040
   gaaatggaag cgggccatat gatgggtctt tttgatgacg tattaccgga aattggcttg 11100
   agtattggta aaagtgaata tcaagatgaa tgtccagatt ggtatgatgg tgatattcag 11160
   gattattatc ttggtgcatt tgaaatatgt agctatgaac atggattgcc gccatttgag 11220
   actgatattt atttacaaac acatgcccat aaaatacctg agatgccgtg tggtttatat 11280
   cacttttcta acggggaatt tgtacgaata agtgatgata ttgtccgaaa aaaggatgtt 11340
   attgcgatta atcagcaagt ttatgatcgc tccagttttg gcgtgtcaat tattccacgc 11400
   tgtgtccctg aatggcatta ttatataaca ctgggtcgtc ggttacatgc gttacaaagt 11460
   aatccattgt atattggatt aatgtcatct ggttacagtt cgaagagcaa taacgattta 11520
   ccttcggcga aaaggatgcg atctattctc aatgcacttg atagacctat ggcggcattt 11580
   tatttctgca taggtggggg tattagccaa gcgcaatata tgtgtgaagg catgaaagaa 11640
   gatgttgttc atatgaaagg gccagttgaa atcattaaag atgatcttca acaacaactc 11700
   cctcaatata tgattccaaa taaggtatta gttttcgata aattaccttt gacggccaat 11760
   ggaaaagtgg attatcaatc tttatcagaa tctaaagccg tggagaatgt ttcaacacag 11820
   cgtctattgg tgccattaca tacagatact gaaataaggc ttggaaaaat ttggatggaa 11880
   gtactgaaat gggattcagt atctgccctc gatgattttt tcgaaagtgg gggtaattct 11940
   ttgatggccg ttgcaatggt taataagatc aatgcggcct ttaatattcg ttttccgtta 12000
   cagatacttt ttcaatctcc taatatagca gaattggcta agtggattga acagacagac 12060
   tctaaaacaa tatcaagatt aattttattg aatcaggcaa gcaaagaccc catttactgt 12120
   tggccgggtt tgggcggata tcctatgagt ttgagattgc ttgctaataa agtcgttcct 12180
   gatcgggcat tttatggaat acaggcatat gggataaacg agagtgaaat accgttttct 12240
   tctatccaga gaatggcaga agaggatatt aaagagataa agaaaataca gccagaaggg 12300
   ccatatatat tgtggggata ttcatttggt gcccgagtag catttgaagt tgcataccag 12360
   cttgaacaag cgggagaaga agttaacgca ttgaatttat tggctccggg atctcctcat 12420
   cttgatatga agcaagcgga atatatggat aaaggcgctg aatttactaa tccggctttt 12480
   gttaaaatac ttttttctgt attttctcgt tcaatcaaca gcccaatggt taaaacttgc 12540
   ttagaacaag taaatagtga aacgacattt attaacttta tatgtagtcg ttttaaaaac 12600
   ttggaaccat cattagtaaa acgtatcgtt aggattgtga ctttgactta tgatttcaag 12660
   tacagtattg atgagcttta tcacagacac ctaaaggcac ctataactat tttcaaggcg 12720
   aatagagata atgattcatt tatcgaggaa tcggatgtga tttcatcaat gtcgcctaaa 12780
   ataattgaat taatatcgga tcactatcaa ctgttggaaa gtgaaggtgt tgctgagatt 12840
   gagaaaataa tctaa 12855

<210>  20
<211>  3267
<212>  DNA
<213>  Brevibacillus parabrevis

<400>  20
atgttagcaa atcaggccaa tctcatcgac aacaagcggg aactggagca gcatgcgcta      60

gttccatatg cacagggcaa gtcgatccat caattgttcg aggaacaagc agaggctttt     120

ccagaccgcg ttgccatcgt ttttgaaaac aggcggcttt cgtatcagga gttgaacagg     180

aaagccaatc aactggcaag agccttgctc gaaaaagggg tgcaaacaga cagcatcgtc     240

ggtgtgatga tggagaagtc catcgaaaat gtcatcgcga ttctggccgt tcttaaagca     300

ggcggagcct atgtgcccat cgacatcgaa tatccccgcg atcgcatcca atatattttg     360

caggatagtc aaacgaaaat cgtgcttacc caaaaaagcg tcagccagct cgtgcatgac     420

gtcgggtaca gcggagaggt agttgtactc gacgaagaac agttggacgc tcgcgagact     480

gccaatctgc accagcccag caagcctacg gatcttgcct atgtcattta cacctcaggc     540

acgacaggca agccaaaagg caccatgctt gaacataaag gcatcgccaa tttgcaatcc     600

tttttccaaa attcgtttgg cgtcaccgag caagacagga tcgggctttt tgccagcatg     660

tcgttcgacg catccgtttg ggaaatgttc atggctttgc tgtctggcgc cagcctgtac     720

atcctttcca aacagacgat ccatgatttc gctgcatttg aacactattt gagtgaaaat     780

gaattgacca tcatcacact gccgccgact tatttgactc acctcacccc agagcgcatc     840

acctcgctac gcatcatgat tacggcagga tcagcttcct ccgcaccctt ggtaaacaaa     900

tggaaagaca aactcaggta cataaatgca tacggcccga cggaaacgag catttgcgcg     960

acgatctggg aagccccgtc caatcagctc tccgtgcaat cggttccgat cggcaaaccg    1020

attcaaaata cacatattta tatcgtcaat gaagacttgc agctactgcc gactggcagc    1080

gaaggcgaat tgtgcatcgg cggagtcggc ttggcaagag gctattggaa tcggcccgac    1140

ttgaccgcag aaaaattcgt agacaatccg ttcgtaccag gcgaaaaaat gtaccgcaca    1200

ggtgacttgg ccaaatggct gacggatgga acgatcgagt ttctcggcag aatcgaccat    1260

caggtgaaaa tcagaggtca tcgcatcgag cttggcgaaa tcgagtctgt tttgttggca    1320

catgaacaca tcacagaggc cgtggtcatt gccagagagg atcaacacgc gggacagtat    1380

ttgtgcgcct attatatttc gcaacaagaa gcaactcctg cgcagctcag agactacgcc    1440

gcccagaagc ttccggctta catgctgcca tcttatttcg tcaagctgga caaaatgccg    1500

cttacgccaa atgacaagat cgaccgcaaa gcgttgcccg agcctgatct tacggcaaac    1560

caaagccagg ctgcctacca tcctccgaga accgagacag aatcgattct cgtctccatc    1620

tggcaaaacg ttttgggaat tgaaaagatc gggattcgcg ataattttta ctcgctcggc    1680

ggagattcga tccaagcgat ccaggtcgtg gctcgtctgc attcctatca attgaagcta 1740

gagacgaaag acttgctgaa ttacccgacg atcgagcagg ttgctctttt tgtcaagagc 1800

acgacgagaa aaagcgatca gggcatcatc gctggaaacg taccgcttac acccattcag 1860

aagtggtttt tcgggaaaaa ctttacgaat acaggccatt ggaaccaatc gtctgtgctc 1920

tatcgcccgg aaggctttga tcctaaagtc atccaaagtg tcatggacaa aatcatcgaa 1980

caccacgacg cgctccgcat ggtctatcag cacgaaaacg gaaatgtcgt tcagcacaac 2040

cgcggcttgg gtggacaatt atacgatttc ttctcttata atctgaccgc gcaaccagac 2100

gtccagcagg cgatcgaagc agagacgcaa cgtctgcaca gcagcatgaa tttgcaggaa 2160

ggacctctgg tgaaggttgc cttatttcag acgttacatg gcgatcattt gtttctcgca 2220

attcatcatt tggtcgtgga tggcatttcc tggcgcattt tgtttgaaga tttggcaacc 2280

ggatacgcgc aggcacttgc agggcaagcg atcagtctgc ccgaaaaaac ggattctttt 2340

caaagctggt cacaatggtt gcaagaatat gcgaacgagg cggatttgct gagcgagatt 2400

ccgtactggg agagtctcga atcgcaagca aaaaatgtgt ccctgccgaa agactatgaa 2460

gtgaccgact gcaaacaaaa gagcgtgcga aacatgcgga tacggctgca cccggaagag 2520

accgagcagt tgttgaagca cgccaatcag gcctatcaaa cggaaatcaa cgatctgttg 2580

ttggcggcgc tcggcttggc ttttgcggag tggagcaagc ttgcgcaaat cgtcattcat 2640

ttggaggggc acgggcgcga ggacatcatc gaacaggcaa acgtggccag aacggtcgga 2700

tggtttacgt cgcaatatcc ggtattgctc gacttgaagc aaaccgctcc cttgtccgac 2760

tatatcaagc tcaccaaaga gaatatgcgg aagattcctc gtaaagggat cggttacgac 2820

atcttgaagc atgtgacact tccagaaaat cgcggttcct tatccttccg cgtgcagccg 2880

gaagtgacgt tcaactactt gggacagttt gatgcggaca tgagaacgga actgtttacc 2940

cgctcaccct acagcggcgg caacacgtta ggcgcagatg gcaaaaacaa tctgagtcct 3000

gagtcagagg tgtacaccgc tttgaatata accggattga ttgaaggcgg agagctcgtc 3060

ctcacattct cttacagctc ggagcagtat cgggaagagt ccatccagca attgagccaa 3120

agttatcaaa agcatctgct tgccatcatc gcgcattgca ccgagaaaaa agaagtagag 3180

cgaacgccca gcgatttcag cgtcaaaggt ctccaaatgg aagaaatgga cgatatcttc 3240

gaattgcttg caaatacact gcgctaa 3267

<210> 21
<211> 10764
<212> DNA
<213> Brevibacillus parabrevis

<400> 21
atgagtgtat ttagcaaaga acaagttcag gatatgtatg cgttgacccc gatgcaagag 60

gggatgctgt ttcacgcctt gctcgatcaa gagcacaact cgcatctggt acagatgtcg 120
atttcgttgc agggcgatct tgacgttggg ctatttacgg atagcctgca tgtgctggta 180
gagagatacg atgtattccg cacgttgttt ctctatgaaa agctgaagca gcctttgcaa 240
gttgtcttga agcaacggcc tattccgatc gaattttacg acttgtctgc ctgcgacgag 300
tccgagaaac aacttcgcta tacgcaatac aaaagggcgg atcaggagcg gacgtttcat 360
ctggcaaaag acccgttgat gcgggtcgcc cttttccaaa tgtcccagca cgactaccag 420
gtcatctgga gctttcatca catcctcatg gacggctggt gcttcagcat tatttttgat 480
gacttgcttg ccatctactt gtccttgcaa aacaagacgg cactctccct ggagcccgta 540
cagccataca gtcgctttat caactggctg gaaaaacaaa ataaacaggc cgctctcaac 600
tattggagcg actatctgga agcctatgaa caaaagacta ccttgccgaa gaaggaagct 660
gccttcgcca aagcatttca accaacccaa taccgctttt cgctgaaccg caccttgacc 720
aagcagctcg ggaccatcgc cagtcaaaat caagtgacgc tatcgacggt gattcaaacg 780
atctggggag ttctcctgca aaaatacaat gcggcccatg atgtgctgtt cggctctgtt 840
gtatccggac gccctacaga catcgtcgga atcgacaaaa tggttggctt gtttatcaat 900
acgattccat tccgggtgca agcgaaagct ggtcaaacgt tttccgagct gttgcaagct 960
gtgcacaaaa gaactttgca atcacagccg tatgagcacg tgcctttgta cgacattcaa 1020
actcagtccg tcttgaagca ggagctgatt gaccacctgc tggtcatcga aaattacccg 1080
ctggtagagg ctttgcagaa aaaagcattg aaccagcaga tcggcttcac gattactgct 1140
gtggaaatgt tcgagccgac caattacgac ttgactgtca tggtgatgcc aaaagaagag 1200
cttgccttcc gttttgacta caatgcggct ctgtttgacg aacaggtcgt gcaaaaactg 1260
gcggggcacc tccaacagat cgcggattgc gtggcaaaca attcgggagt cgagctttgc 1320
cagattccgt tgctgacaga agcagaaact agccagctgt tggcaaagcg tacggaaaca 1380
gcggctgact atcctgccgc aaccatgcac gagctgtttt cgcggcaggc agaaaaaacg 1440
cctgagcaag tggcggtagt cttcgcggat cagcacctga cgtatcggga gctggatgaa 1500
aaatccaatc agctcgcccg ctttttgcgc aaaaaaggca ttggcacggg cagtcttgtc 1560
ggcacgctgc tggatcgctc gctggacatg atcgtcggaa tcctcggcgt cttgaaggca 1620
ggcggcgcat ttgtgccgat cgacccggag ttgcctgccg aacgaatcgc ttacatgctg 1680
acgcatagca gagttccatt ggtcgtgacg caaaatcatt tgcgggcaaa agtgaccacg 1740
cctacagaaa caattgacat caacacagcg gtgatcgggg aagagagccg cgcccctatc 1800
gaatcgctca atcagccgca tgacttgttt tacatcatct atacgtccgg aacgacaggg 1860
caaccgaaag gcgtcatgct ggagcatcgc aacatggcga acctgatgca ttttacgttt 1920
gatcagacga acatcgcttt tcatgaaaaa gtgttgcagt ataccacgtg cagctttgat 1980

gtttgctacc aggaaatttt ctccacgctg ctatccgggg gccagctcta cctgatcacg 2040

aacgagctga gacggcatgt ggaaaagctg tttgctttca tccaggaaaa gcagatcagc 2100

attttgtctc tcccggtgtc cttcctgaaa tttattttta acgaacaaga ctacgcgcaa 2160

agcttcccgc gttgtgtcaa acatatcatc acggccgggg aacaactcgt cgtcacacac 2220

gagctgcaaa agtatctgcg ccagcatcgc gtatttttgc acaatcacta cggcccgtcg 2280

gagacgcatg tggtgacgac atgcacgatg gacccgggac aggcgatacc agagctgccg 2340

cccatcggaa agccgatcag caacacaggc atttacattt tggatgaagg gctgcaattg 2400

aagccggagg ggatcgtcgg ggagttgtac atttccggcg caaacgtagg aagagggtat 2460

ttgcaccagc cggagctgac cgcggagaag tttctcgaca atccgtatca gccaggcgaa 2520

agaatgtacc gaacgggtga tctggccctt tggttgccgg atggccagct cgaatttttg 2580

ggccgaatcg accatcaggt aaaaatcagg ggccatcgca tcgagctggg agagatcgaa 2640

tcgcgcctgc tcaaccatcc cgccatcaag gaagcggtgg ttatcgaccg agcagacgag 2700

acaggcggca agtttttgtg cgcctatgtc gtcctgcaaa aagcgctcag cgacgaagag 2760

atgcgggcat acttggcgca agcgttgccg gagtatatga tcccttcctt tttcgtgacg 2820

ctggagcgga ttccagtcac gccgaacgga aaaacagaca ggcgagcttt gccgaagccg 2880

gaaggaagtg ccaagacgaa agcggattac gtcgccccga cgactgagct ggaacaaaag 2940

ctggtcgcga tttgggagca aattcttggc gtgtcgccga tcggcattca ggatcatttt 3000

ttcacgctgg gcggccattc gttaaaagcg attcagctca tttcccgcat ccaaaaggaa 3060

tgccaggcgg atgtcccgct gcgcgtcctg tttgagcaac cgacgattca agcgctggca 3120

gcgtatgtgg aaggcgggga ggaaagcgcg tatctcgcca ttccccaggc cgagccgcaa 3180

gcgtattatc ccgtatcgtc tgcgcaaaaa cgcatgctca tcttaaacca gctcgatccg 3240

cacagcacgg tttacaacct gcctgtcgcg atgatcctcg aaggaacgct ggataaagct 3300

cggctggagc acgccatttc caacctggtg gctcgccatg agtcgttgcg cacgtcgttt 3360

catacgatca acggggagcc agtttcccgc atccatgagc aaggccactt gccgattgtt 3420

tacttggaaa cggcggaaga gcaagtgaac gaggtcattt tggggttcat gcagccgttt 3480

gatctggtaa cagctccgct atgccgggtt ggcttggtga agctcgcaga gaaccgtcac 3540

gtcctgatca tcgacatgca ccatatcatt tcggacggag tctcttctca gctcatcctg 3600

aatgactttt cccgtttgta tcaaaacaaa gctttgccag agcagcgcat tcactataaa 3660

gacttcgccg tttgggaaaa agcgtggaca caaacgaccg attaccaaaa acaggaaaaa 3720

tattggctcg atcgatttgc gggcgaaatc ccggttttga acctgccgat ggattacccg 3780

cggccagctg ttcaaagctt tgagggcgaa cgttatttgt tccgcacaga aaaacagttg 3840

ttggaaagtt tgcaggacgt agcccaaaag acaggcacga ccttgtacat ggtgcttctc 3900

gcagcctatc atgtgctgct ttccaaatac tccgggcagg atgacgtgat gatcggcacc 3960

gtgactgccg gcagggtgca cccggatacg gagagcatga cggggatgtt cgtcaacacg 4020

ctggcgatgc gcaatcagtc tgcgccgacc aaaacgttcc ggcaattttt gctggaggta 4080

aaagacaaca cgctggccgc ttttgaacac gggcaatatc cgtttgaaga gcttgtcgaa 4140

aagttggcga tccagcgaaa ccggagccga aacccgctgt tcgacacctt gttcattttg 4200

caaaacatgg atgccgacct gatcgagctg gatggactga ccgtgacgcc ttatgtgcca 4260

gagggggaag tcgccaagtt cgatctgtcg ctggaagcaa gcgaaaacca ggcgggactt 4320

tccttctgct tcgaattttg caccaagctg ttcgcacgcg agacgatcga gcgcatgtcg 4380

cttcattact tgcaaatttt gcaggcagtc agcgcaaaca cggagcagga gctggcgcaa 4440

atcgagatgc tgactgcgca tgagaagcag gagctgctcg ttcacttcaa cgacacggcc 4500

gccctgtatc cagcggagag cacgctgtcg cagctgtttg aagatcaggc acagaaaact 4560

cctgagcaaa ccgccgtcgt cttcggtgac aaacgactga cgtaccgcga actgaacgag 4620

cgggccaacc agctcgcgca cactttgcgg gcaaaaggcg tgcaggctga gcaaagcgta 4680

gggatcatgg cgcaaagatc gttggaaatg gcgatcggaa ttatcgctat tctcaaagcg 4740

ggcggggcgt atgtgccgat cgatccggat tatccgaatg agcggattgc ttacatgctg 4800

gaagattgcc gccgtctggt gctgacccag cagcagctcg ccgaaaagat gaccgcaaac 4860

gtggaatgcc tgtatctgga tgaggagggc agctactcgc ctcagacgga aaacatcgag 4920

ccgatccata ccgctgctga tctcgcttac atcatctaca catccggtac gacaggcagg 4980

ccaaaaggcg tcatggtaga gcatcgggga atcgtcaaca gtgtgacgtg gaacagggac 5040

gagtttgccc tttctgtccg ggacagtgga acgctgtcgc tatcttttgc cttcgatgcc 5100

tttgccctta ctttctttac gttgattgta tcaggctcca cggtcgtcct gatgccggat 5160

cacgaagcca aagatccgat cgcgctacgc aacctgattg ccgcttggga atgcagctac 5220

gtcgttttcg tgcccagtat gttccaggcg atattggagt gcagcactcc ggcagacatc 5280

cgctccatcc aggcagtcat gctcgggggc gaaaagctgt cgccgaagct tgttcagctg 5340

tgcaaagcga tgcatccgca gatgagcgtg atgaatgcat acggcccgac ggagagcagc 5400

gtcatggcca cctacctgcg agatacacag ccagatcagc cgatcaccat cgggcggccg 5460

attgccaaca ccgccattta catcgtagac cagcaccatc aactgctgcc tgtcggggtg 5520

gtaggggaaa tctgcatcgg cggtcacggc ttggcgcggg gctattggaa aaagccggag 5580

cttactgcgg agaaattcgt ggccaatcca gctgttccgg gagagcgcat gtacaaaaca 5640

ggcgatctgg gcagatggct ccacgacggc acgattgatt ttataggccg cgtcgatgac 5700

caaatcaagg tgagaggata ccggattgag gtcggggaga ttgaagcggt tttgctcgct 5760

tacgatcaga cgaatgaagc tatcgtcgtc gcttatcagg acgatcgcgg cgattcctat 5820

ctggctgcgt atgtcacggg aaaaacggcg atagaggaat ccgagcttcg cgcgcatctg 5880

ttgcgagagc ttccggccta catggtgccg acctacctga ttcaactgga cgctttcccg 5940

ctcacgccaa acggcaaggt cgaccgcaag gcactgccca agccggaagg aaagcctgca 6000

acaggagcag cttatgtcgc acccgctaca gaagtggagg cgaagctggt cgccatttgg 6060

gagaatgcgc tggggatttc cggcgtcggg gtgttggatc acttttttga gctgggcggt 6120

cattccttga aagcgatgac ggttgtggcg caagtgcatc gcgagtttca aatcgacctt 6180

ttgctgaagc agttttttgc agcgccaacc atccgggact tggcccgctt gatcgaacat 6240

agcgaacagg cagccggcgc cgccattcaa ccggcagagc cgcaagcgta ttatccggta 6300

tcttctgctc agcagcggat gtacttgctc catcagcttg aaggtgccgg aatcagctac 6360

aacacaccgg gcatcatcat gctggaaggc aagctcgatc gcgagcaatt ggcgaatgcg 6420

ctgcaagcgt tggtagatcg tcacgatatt ttgcggacgt cttttgagat ggtcggagac 6480

gagctggtgc aaaaaattca tgaccgcgtg gccgtgaaca tggagtatgt gacggcagaa 6540

gagcagcaga tcgatgacct tttccacgcg ttcgtccgtc cgtttgatct ttctgtgccg 6600

ccattgctcc gcatgagcct ggtgaaactc gcggatgagc gtcacctgct cctgtacgac 6660

atgcaccata ttgctgccga tgccgcatcg atcacgatcc tgttcgatga actggctgaa 6720

ttgtaccagg gaagagaact gccggaaatg cgcatccagt acaaagattt tgctgtctgg 6780

caaaaagcct tgcatgagtc ggatgccttc aagcagcagg aagcgtattg gctgagcacg 6840

ttcgctggaa atatcaccgc tgtcgatgtg ccgacagatt ttccgcgccc agccgtgaaa 6900

agttttgcag gggggcaagt caccctgtcc atggaccaag agctgctcag tgctttgcac 6960

gagttggctg cgcatacgaa tacgacgctg tttatggttt tgctggccgc ctacaacgtg 7020

ctgctcgcaa aatacgctgg gcaagacgac atcatcgtgg gaacgccgat ctccggcagg 7080

tcacgcgccg agcttgcgcc tgtcgtcggc atgttcgtcc atacgctggc gatccgcaac 7140

aaaccgaccg ccgagaagac attcaagcag tttttgcagg aggtcaagca aaacgcgctc 7200

gatgctttcg accaccagga ctacccgttt gaaagccttg tggaaaagct gggcattccg 7260

cgcgatccgg ggcgcaatcc gctgtttgac accatgttca tcctgcaaaa cgatgagttg 7320

cacgcaaaaa cgctggatca gctcgtctat cgcccttatg aatcggacag cgcgcttgac 7380

gtggcgaaat tcgacttgtc gttccatctg accgagcggg aaaccgacct gttcttgcgc 7440

ctggaatact gcaccaagct gttcaagcaa caaacggtag aacgaatggc gcaccacttc 7500

ttgcaaattt tgcgagcggt cacggccaat ccggaaaatg aattgcaaga gatcgagatg 7560

ctgacagcag cagaaaagca aatgctgctg gtggcgttca acgatacgca cagagaatac 7620

cgggcagatc aaacaatcca gcaacttttt gaagagctgg cggaaaaaat gcctgagcac 7680
acggcgctcg tattcgaaga aaagcgcatg tcgttccggg agctgaatga aagagcgaac 7740
cagctcgcag ccgttttgcg ggaaaaagga gtcgggccag cgcagatcgt cgctttgctg 7800
gtagagcgtt ccgccgagat ggtcattgcc acgcttgcca cgttaaaagc gggcggcgcc 7860
tttttgcccg tcgatcctga ttatccggaa gagcgaatcc gctacatgct ggaggacagc 7920
caggcaaaac tggtggtgac ccatgcgcac ttgctgcaca aagtgagcag tcagtccgaa 7980
gtcgttgatg tggatgaccc tggaagctac gcaacacaga cagacaacct gccgtgcgca 8040
aacacaccgt ctgatttggc ttatatcatt tacacgtccg gtacgacggg caagccaaaa 8100
ggcgtcatgc tggagcacaa aggggtagcg aatctgcaag cggtatttgc ccatcatcta 8160
ggcgtcacgc cgcaagatcg ggcagggcat tttgccagca tctcgtttga cgcatcggtg 8220
tgggatatgt ttggcccgtt gctgtcggga gcgaccttgt acgtcttgtc ccgagacgtc 8280
atcaacgatt ttcaacgatt cgccgaatac gttcgcgata acgcgatcac cttcctcact 8340
ttgccgccga cgtacgcgat ttatctggag ccggagcagg tgccgtcgtt acgcaccctg 8400
attacagccg gatcggcttc ctccgttgca ttggtggata aatggaaaga aaaagtcacc 8460
tatgtcaatg gatacggccc aacagagagc accgtttgcg cgacactgtg gaaagccaaa 8520
ccggatgagc cagtcgaaac gatcacgatt ggcaaaccga ttcagaacac caagctgtac 8580
atcgtggatg accagttgca gttgaaagcg ccggggcaga tgggagaact gtgcatcagc 8640
ggcttgtcgc tggcgagagg ctattggaat cgtccagagc tgaccgccga gaagttcgtc 8700
gacaacccgt ttgtgccagg aacaaagatg taccggacag gcgacctggc aagatggctg 8760
ccagatggaa ctatcgagta tctgggcaga atcgatcacc aagtgaaaat tcgcggacat 8820
cgtgtggaac tcggcgaagt ggaaagcgtg ctgctgcggt atgacacggt caaagaggca 8880
gctgccatca cacatgagga cgaccgcggc caagcttact tgtgcgccta ctacgtagcg 8940
gagggagaag ccacgcctgc gcaacttcga gcctatatgg aaaacgagtt gccgaactac 9000
atggttcccg ccttctttat ccagttggaa aagatgccgc tgacaccgaa tgacaagatt 9060
gaccgaaaag cgctgccgaa gccgaaccag gaggagaacc ggactgagca atatgcagcg 9120
ccgcaaaccg agctggaaca gttgctggct ggcatctggg cagatgtact ggggatcaag 9180
caagtcggga cgcaagacaa cttctttgaa ttgggcggcg attcgattaa agcgatccag 9240
gtatccaccc gcctgaatgc gtcaggctgg acgcttgcga tgaaagaact gttccagtac 9300
ccgacgattg aagaagctgc tctgcgcgtc atcccgaaca gccgagagag cgagcagggt 9360
gtcgtagaag gcgagattgc cttgacaccg atccagaaat ggttcttcgc gaacaacttc 9420
acggatcgtc accattggaa tcaggctgtc atgctgtttc gcgaggacgg ctttgacgag 9480
ggactcgtgc ggcaagcgtt ccagcaaatc gtcgagcatc acgatgcgct gcgcatggtc 9540

tacaagcaag aggacggggc gatcaagcaa atcaaccgcg ggctgaccga cgagcgcttc 9600

cgtttctact cttatgactt gaaaaatcac gcgaacagcg aagctcgcat cctggagctg 9660

tctgatcaga tccagagcag catcgatttg gagcacggcc cactcgttca cgtggctctg 9720

ttcgccacaa aagacgggga tcatttgctg gtcgcgatcc accatcttgt cgtggatggc 9780

gtctcctggc gcattttgtt cgaagatttt tcctcagcct actcgcaggc tctccatcag 9840

caggagatcg tcttgccgaa aaagacggac tccttcaaag actgggcggc tcaattgcaa 9900

aagtacgcgg acagcgacga gctgttgcgg gaagtggcat attggcacaa cttggagact 9960

acaacgacga ctgcggcact gccaacagat tttgtcacgg cagatcgcaa gcaaaaacat 10020

acgcggacac tgtcgttcgc gttgacagtc ccgcagacag aaaacctttt gcgtcacgtt 10080

catcatgcct atcacacaga gatgaacgac ctgctgctga cagcgctcgg cttggccgta 10140

aaagactggg cacatacgaa tggcgtcgtc atcaatctgg aaggccatgg gcgcgaagac 10200

atccagaacg aaatgaacgt cacgcgcacg attggctggt tcacttcgca atatccggtg 10260

gtgctcgaca tggaaaaagc cgaggacttg ccgtaccaga tcaagcaaac caaagaaaac 10320

ttgcgacgga ttccgaaaaa agggatcggc tacgagattt tgcgcacgct gacgacaagc 10380

cagttgcagc cgccattagc ctttacgctg cggccggaaa tcagctttaa ctatctcggt 10440

caattcgagt cggacggaaa aacaggcggg tttacattct cgccgctcgg aacagggcag 10500

ttgttcagcc cggaatcgga gcgagtgttc ctgctggaca tttccgccat gatcgaggac 10560

ggcgagctgc ggatcagcgt ggggtacagc cgtctccaat atgaggaaaa aacgattgcc 10620

agcctggcag acagctaccg gaagcacttg ctaggcatca tcgagcattg catggcaaaa 10680

gaagaaggcg agtacacccc gagcgacctg ggggatgaag agctgtccat ggaggagctg 10740

gaaaacatcc tggaatggat ttga 10764

<210> 22
<211> 19461
<212> DNA
<213> Brevibacillus parabrevis

<400> 22
atgaaaaagc aggaaaacat cgcaaaaatt tacccgctaa ccccattgca agagggtatg 60

ttgtttcacg ctgtcacaga cacgggcagc agcgcctatt gcctccagat gtctgcaacg 120

atcgagggcg attttcacct gccgcttttt gaaaagagtc tgaacaagct cgtggaaaac 180

tacgaagtat tgcgcacggc ttttgtatac caaaacatgc agcgacctcg ccaagtcgtg 240

ttcaaggaaa gaaaagtgac cgttccttgc gaaaacatcg cgcatttgcc aagcgcagag 300

caggacgcgt acatacaagc gtacacgaag caacatcatg cattcgacct gacaaaagac 360

aacttgatga aagcagccat ttttcaaacg gccgagaaca agtaccgatt ggtttgggcc 420

ttccatcata ttatcgtgga cggttggaca ttgggcgtct tgctgcataa gctgctgacc 480

tattacgcag cgctgcgaaa aggcgagccg attccgcggg aagcgacgaa gccgtacagc 540

gaatatatca agtggctgga taagcaaaac aaggacgagg ccctcgctta ttggcaaaac 600

tacctggcag ggtatgacca tcaggctgct tttccgaaaa agaagcttgg aacggaagca 660

agccgctatg aacatgtcga ggcgatgttc acgatcgctc ccgagaagac gcagcagctg 720

atccagatcg cgaaccaaaa tcaggcgacg atgagcagcg tgtttcaagc tctttggggc 780

attttggcca gcacatacaa aaatgcggac gatgtcgttt tcggctcggt cgtatcaggc 840

cgcccgccgc aaatccaagg aattgagagc atggtcggct tgttcatcaa cacgattccg 900

acccgcgtcc agacgaacaa acaacagacg ttcagcgagc tgctgcaaac cgtgcaaaag 960

caagccctgg cgtctgccac ctacgatttc gcgccgctgt acgaaattca gagcacaaca 1020

gtgctgaaac aggaattgat cgatcatttg gtcacgtttg aaaattaccc cgatcattcg 1080

atgaagcatc tggaagaatc attagggttt caattcaccg tagaaagcgg agatgagcag 1140

acctcctatg atttgaacgt ggtcgtcgcc ctcgctccct cgaacgagct gtacgtgaag 1200

ctaagctaca atgccgcggt gtatgaatcg tcattcgtaa acagaatcga agggcatctc 1260

cgcaccgtca tcgaccaggt gatcggcaat ccgcatgtac acctgcacga gatcggcatc 1320

atcaccgaag aggaaaagca gcaactgctc gtcgcctaca acgacacggc tgctgaatat 1380

ccgcgggaca aaacgatttt cgagctgatc gcggaacaag cgagccggac accagcgaaa 1440

gcagcagttg tttgcggcga ggacaccctg acctatcagg agctgatgga gcgttctgcc 1500

cagcttgcca atgctttgcg cgaaaaagga atcgccagcg gcagcatcgt ctcgattatg 1560

gcggaacatt cactggagct gatcgtggcg atcatggctg tcttgcggtc aggtgctgcc 1620

tacttgccga ttgatcccga gtacccgcaa gatcgcatcc agtatttgct cgatgacagc 1680

cagaccacgc tgctgttaac ccagtcgcat ctgcaaccaa acatccggtt tgcaggcagc 1740

gtgctttatt tggacgatcg ttccttgtac gaaggcggca gcacatcctt cgcacccgag 1800

agcaagcctg atgatttggc gtacatgatc tacacttccg gttctaccgg caatccaaaa 1860

ggggcgatga ttactcatca aggcctggtc aattacatct ggtgggccaa caaggtgtac 1920

gtccaaggcg aagcggtgga ctttccgctg tactcatcta tttcgttcga tttgaccgtc 1980

acctcgatct tcacgccgct tctgtccggc aacacgattc atgtgtacag aggggcagac 2040

aaggtacagg tcattttgga catcatcaaa gataacaaag tcgggatcat caagctgacg 2100

ccgacacacc tgaagctgat tgaacacatc gacggcaagg ccagcagcat cagacggttc 2160

atcgtcggcg gcgagaactt gccgacaaag ctggcgaagc aaatatacga ccatttcgga 2220

gagaacgtgc aaattttcaa cgagtacgga ccgaccgaaa ccgttgtcgg ttgcatgatt 2280

tacttgtatg acccgcaaac aacgacccag gagtcggtgc caatcggtgt cccggcagac 2340

aacgtccagc tttatttgct cgatgcttcc atgcagccgg tgcccgtcgg ctcgcttggc 2400

gaaatgtaca tagccggaga cggcgtagcc aaagggtatt tcaacagacc ggagctgacg 2460

aaggaaaagt ttatcgacaa cccgttccgt ccgggaacca aaatgtatcg aacaggcgac 2520

ctggcaaaat ggctgcctga tggaaacatg gagtatgcag gcagaatgga ctatcaagtg 2580

aagattcgcg gccatcggat cgagatgggc gaaatcgaaa cgcgcctgac gcagcatgag 2640

gcggtcaagg aagcggtcgt gatcgtggaa aaggatgaga gcggccaaaa cgtgttgtac 2700

gcgtaccttg tttccgagcg ggaactgacg gtagctgagc tgagagaatt tttggggcgc 2760

acgctgcctt cctatatgat tccttccttc tttattcgct tggcggaaat tccgctgacc 2820

gcgaacggaa aagtagagcg aaaaaaattg ccgaagccag ctggcgcagt cgttacaggc 2880

accgcgtatg cagctccgca aaatgaaatc gaggcaaagc tggccgagat atggcagcaa 2940

gtgctgggca taagccaggt agggattcac gacgatttct ttgacttggg cggacactcg 3000

ttgaaggcga tgactgtcgt tttccaagtc tcgaaagcgc tggaagtgga attgcccgta 3060

aaggccttgt tcgaacatcc aaccgttgcg gagctggccc gcttcctttc gcggtcggaa 3120

aaaaccgagt acaccgcgat tcaacccgtg gcagcgcagg agttttaccc ggtttcatct 3180

gcgcaaaaaa gaatgtatat cctgcaacag ttcgaaggca acggaatcag ctacaacatt 3240

tcgggtgcga ttctcctgga aggaaagctg gactacgccc ggtttgccag cgctgtgcaa 3300

cagctggcag agcgccacga agctttgcgc acctcgttcc accggatcga cggcgagcct 3360

gtgcaaaaag tgcacgagga agtagaagtg ccgcttttca tgctggaggc tcccgaagac 3420

caggcggaga aatcatgcgc cgagtttgtc cgtccgtttg atctcggggt cgctccgctg 3480

atgcgaacag gtttgctcaa gctgggcaaa gaccgccatt tgtttttgct cgacatgcac 3540

catatcatct cggacggcgt ttcttcgcaa attttgctgc gtgaatttgc cgagttgtac 3600

cagggagcag acttgcagcc gctttcgctg caatacaaag atttcgctgc ttggcaaaat 3660

gagctgtttc agacggaggc atacaagaag caggagcagc actggctgaa cacgtttgct 3720

gatgaaattc cgctcttgaa cctgccgact gactatccgc gccctagcgt gcaaagcttt 3780

gcaggcgatc tcgtcctttt tgccgccgga aaagaactgc tggagcggtt gcaacaggta 3840

gcgtcagaaa caggcaccac cttgtacatg attttgcttg ccgcctacaa tgtgctgctg 3900

tccaagtata ccggccagga agacatcatc gtcgggacgc ctgtcgctgg acgttcccat 3960

gcggacgtgg aaaacatcat gggcatattc gtgaacacat tggcgctgcg caaccagcct 4020

gccagcagca aaacgtttgc gcaatttttg caggaagtca agcaaaacgc gcttgcagcc 4080

tatgaccatc aagattatcc atttgaagaa ctcgtggaaa aactggcgat tcagcgggat 4140

attagccgaa atccgttgtt tgacacgttg ttttctttgg aaaacgcgaa ccagcagtcg 4200

cttgccatcg ccgagctgac agcgtcgccc tatgagctgt tcaacaaaat ttccaagttt 4260

gatcttgctt tgaacgcaag cgaatcgcca gcggacattc agttccagct cacattcgca 4320

accaagctgt tcaagaaaga aacggtcgag cgaatggccc ggcattacct ggaaattttg 4380

cgctggatca gtgagcagcc aacggcaagc ctcgcggaca tcgacatgat gacggaagcg 4440

gaaaaacgca cactccttct gaacgtgaac gatacgtttg tcgagcggac tgccgcgacc 4500

gctttgcatc aattagtgga ggagcaagca gcacgcacgc ctgatgaagt ggccgtcgtg 4560

tacgaagaat atgccttgac ctatcgcgag ctgaacgcca gggcgaacca gctggcccgt 4620

ttgctgcgca gtcacggaac cggaccagat acgttgatcg gcattatggt ggaccgttcg 4680

ccaggcatgg tcgtcgggat gctggctgtg ctcaaagcag gcggcgcgta cacgccaatc 4740

gacccaagct atccgccaga acgaatccag tacatgctca gcgacagcca ggcgccgatt 4800

ttgctgacgc agcgtcattt gcaggagctg gctgcttatc aaggggagat catcgacgta 4860

gacgaggaag cgatttacac cggagccgac acgaacttgg acaacgtcgc tggcaaagac 4920

gacttggcct atgtgatcta cacatcggga tcgacgggca atccgaaagg cgtcatgatc 4980

tcccatcagg cgatttgcaa tcacatgttg tggatgagag agacgttccc gctgacgacc 5040

gaggatgctg tcctgcaaaa aacgccgttc agcttcgacg cttccgtatg ggagttttat 5100

ttgccgctca tcaccggagg acaactggtg ttggcaaagc cggacgggca tcgcgacatc 5160

gcctacatga ctcgtctcat tcgagatgag aaaatcacga ccttgcagat ggttccgtcc 5220

ttgctggatc tggtcatgac cgacccgggc tggagcgcat gcacgagctt gcagcgagtg 5280

ttctgcggcg gggaagcatt gacgcctgcc ctcgtctcgc gtttttacga gacacagcaa 5340

gctcagttga tcaacttgta cggccctaca gagacaacca tcgatgcgac ttattggcct 5400

tgcccgcgcc agcaggaata cagcgcaatt ccgatcggca aaccgatcga caacgtccgg 5460

ctgtatgtcg tcaatgccag caaccagctt cagccagtag gcgtagcggg agagctgtgc 5520

attgccggag acggtttggc ccgcggctat tggcagcgcg aggagctgac gaaagcaagt 5580

tttgtcgaca acccgtttga gccgggcggc accatgtacc gtaccggaga catggtccgc 5640

tatttgccag atggccatat cgagtatttg ggacgcatcg accatcaagt caaaatcaga 5700

ggtcaccgca tcgagctggg ggaaatcgaa gccacgcttt tgcagcatga agcggtcaaa 5760

gcggtcgtcg tcatggcccg ccaggatggc aaagggcaaa acagcctgta cgcctatgtc 5820

gtagcggagc aggacatcca gacagcggag ctgagaacgt acctgtctgc caccttgcca 5880

gcctacatgg ttccgtccgc ttttgttttc ttggagcagc tgccgctttc agcgaacggc 5940

aaagtggatc gcaaggcatt gcctcaaccg gaggatgccg ccgcctctgc tgccgtgtat 6000

gtggcgccgc gcaacgaatg ggaagccaag ctcgcagcga tatgggaaag tgtgcttgga 6060

gtcgagccga tcggggttca cgatcatttc tttgaactgg gcggacattc tttgaaagcg 6120

atgcacgtca tttctttgct ccagcgcagc ttccaggtgg acgtaccgtt gaaagtcctg 6180

tttgaatcgc caacgatcgc gggcctggcc ccacttgttg cggctgcccg caaaggcacg 6240

tacacagcga tcccccctgt cgaaaagcag gagtattacc cggtttccgc ggcacagaag 6300

cgaatgttca ttctgcagca aatggaagga gcaggtatca gctacaacat gccaggcttc 6360

atgtatctcg acggcaagct ggatacagag cggctgcaac aggcgctgaa aagtttggtg 6420

caacgccacg aatcgttgcg cacctcgttc cactccgtgc aaggcgagac ggttcagcgt 6480

gtgcatgacg atgtcgatct ggccatctcg tttggcgaag cgaccgaagc agagacccgg 6540

caaatagccg agcagtttat ccagccgttc gatctgggaa cagccccgct gttgcgtgcc 6600

ggactcatca agctggcgcc ggaacgccac ctgttcatgc tcgatttgca ccatattgtc 6660

gtcgatggcg tctccatcgg cctgctcatc gaggaatttg cccagctcta tcacggggaa 6720

gagctgccag cgctgcgcat tcagtacaaa gattttgcca agtggcagca ggactggttc 6780

cagaccgagg aatttgccga gcaggaagcc tactggctca acacctttac gggagaaatc 6840

cccgtgctta atctgccgac ggattatcca agaccgtctg tgaaaagctt tgcgggagat 6900

cgcttcgtct ttggctccgg cactgctttg ccaaaacaat tgcatcagct cgcccaagag 6960

acaggcacga cgctctacat ggttctgttg gccgcctaca acgtgctcct gtccaaatac 7020

tccaggcaag aggacatcat cgtcggcgct cctacggctg gcaggtccca tgccgaaacg 7080

gagtccatcg tcggaatgtt tgtcaacaca ctggccttgc gcaacgagcc agccgggggc 7140

aaaactttcc gcgacttttt ggccgaagtg aaaatcaata cgttgggagc gtttgagcat 7200

caagattatc cgctcgatga actcgtcgac aagctggaca tgcaacggga tttgagccgc 7260

aaccctttgt ttgacacggt tttcattttg caaaacatgg agcaaaagcc gttcgaaatg 7320

gagcagttga cgattactcc ttattcggca gaggtgaaac aggccaagtt tgacctgtcg 7380

ctggaggcgt acgaagaaaa cgcggaaatc atctttagcc tggattacag caccaagctg 7440

ttttcgcgcg agacgatcga aaaaatagcg acccatttta tccaaatctt gcgggcggtc 7500

attgcggaac cggaaatgcc gttgtccgag atcaccatgc tcacagaggc ggaaaagcag 7560

cgcttgctgg tcgacttcaa cggtgcgcac aaagatttcc cgcaaaacaa aacgcttcag 7620

gcgctttttg aagaacaagc ggaaaagtcg ccgcaggcaa cagccgtgga aatcagcggg 7680

cagcccctgt cctatcagga gctgaatgag cgagccaacc agcttgccgc tacgctgcgg 7740

gagcggggag tacagcctga ccaacctgta gggattatgg cgaaccgctc tgtggagatg 7800

gtcgtcggca tcctcgccat cttgaaagca ggcggagctt acgtgccgat cgacccggaa 7860

tatccggagg agcgtgtcgc ctacatgctg acggattgcc aagcccgcct ggtgctgacg 7920

caaaagcatc tgggagcgaa gcttggttcc agcgtgaccg cggaatgcct gtatctcgac 7980

gacgagagca actatggtgt gcaccgctcg aatttgcagc cgatcaatac cgcttccgat 8040

ctggcttaca tcatctacac atcgggtacg actggcaagc caaaaggggt catggtcgag 8100

caccggggca tcgtcaacaa cgtgctgtgg aagaaagcgg agtaccaaat gaaggttggc 8160

gacagaagct tgctgtctct gtcctttgcc tttgacgctt tcgttctgtc cttctttacg 8220

cctgtgcttt ccggggcaac tgtcgtactg gcggaggatg aagaagccaa ggacccagtc 8280

tctttgaaaa agctcatcgc cgcttcgcgc tgcaccttga tgacaggcgt gccgagcttg 8340

ttccaggcca ttctggaatg cagcacgcca gcggatatcc gtccgctgca aaccgtcaca 8400

ctcggcggag aaaaaattac ggcgcagctt gttgaaaaat gcaagcagct gaatcccgat 8460

ctggtcatcg tcaacgagta cggcccgaca gaaagcagtg tcgtcgccac ctggcagcgc 8520

cttgcgggtc cggatgctgc catcaccatc gggcggccga ttgccaacac cagcctgtac 8580

atcgtgaacc aatatcacca gctacagcca atcggcgtgg tcggggagat ttgcatcggc 8640

ggccgcggct tggcacgagg ctattggaac aagccagcgc tcacggaaga gaagttcgtt 8700

tcccatccgt ttgcggcagg cgagcgcatg tacaagacgg gcgatcttgg caagtggctc 8760

ccggacggaa cgattgaata cattgggcgc atcgacgaac aggtcaaagt ccgaggctac 8820

cgaattgaaa tcggcgagat cgagtcggct ctgctggctg cggaaaagct gacagcggct 8880

gttgtggtcg tctatgagga tcagcttggc cagtcggctc tggcagcgta ttttaccgcc 8940

gacgaacagc ttgatgtcac gaagctgtgg tcgcatctgt cgaagcgact cccgtcgtac 9000

atgattcctg cgcattttgt gcagctcgat cagcttccgc ttacgccaaa cggcaaagtc 9060

gacaagaaag ccttgccgaa gccagaaggc aagcccgtaa ccgaagcgca atatgtcgcg 9120

ccgacaaatg cggtggaaag caagctggca gagatttggg aacgcgtgct cggggttagc 9180

ggcatcggca ttctcgacaa ctttttccag atcggcggac attccttgaa agcgatggct 9240

gtcgctgcac aggtgcatcg cgagtatcag gttgagcttc cgctgaaagt gctgttcgcg 9300

cagcctacga tcaaggcgtt ggcccagtat gtcgccacga gcggaaaaga gacgtatgtg 9360

ccgatcgagc ctgcaccgtt gcaagagtat tatcctgttt catctgcgca aaagcggatg 9420

tatgtcctgc gccagtttgc ggacacaggc acggtttata acatgccgag cgcgttgtat 9480

atcgaaggcg atctggatcg gaagcgtttt gaagccgcca tccacggatt ggtcgagcgg 9540

cacgaatcgc tgcgcacatc cttccacacc gtaaatggcg agcctgtcca gcgcgtacac 9600

gagcatgtcg agctgaatgt gcagtacgcg gaagtgacgg aagcgcaagt ggagccaacc 9660

gtcgagtcgt tcgtgcaagc atttgatctg acaaaagctc cgctattgcg ggtcggactt 9720

ttcaagctgg cagcgaaacg gcatctgttc ctgctggata tgcatcacat catctcggat 9780

ggcgtctcgg ccggaatcat tatggaagag ttctcgaagc tgtatcgagg cgaagaactg 9840

cctgcgcttt ccgtccatta caaagatttc gccgtctggc agtctgaact gttccagagc 9900
gacgtctata ccgagcatga aaactactgg ctgaacgcgt tttctggcga cattccggtg 9960
cttaacttgc cagccgattt ttctcgtccg ctgacacaga gctttgaagg agattgcgtt 10020
tcgttccagg cagacaaagc gttgctggac gatcttcaca agctcgctca ggagagccaa 10080
tcgacgttgt tcatggtatt gctggcggct tacaatgtgc tgcttgccaa gtacagcgga 10140
caggaagaca tcgtcgtcgg cacaccgatt gcgggcagat cgcacgccga tatcgagaac 10200
gttctgggga tgtttgtcaa cacgctcgct ttgcgcaact atccggtcga gacgaaacac 10260
ttccaggcat ttttggaaga ggtcaagcaa aatacgctgc aagcatacgc ccatcaagat 10320
tatccgttcg aagcactggt cgaaaagctg gacatccagc gggatctcag ccgcaatccg 10380
ctgtttgaca ccatgtttat tttgcaaaac ctggaccaaa aagcttacga gctggatggg 10440
ctgaaactgg aggcatatcc ggcacaagca ggcaacgcca aattcgatct cacgctggaa 10500
gcgcacgagg acgagacagg cattcatttt gcgctcgtct actcgaccaa attgttccag 10560
cgagaatcaa tcgaaagaat ggcgggtcac ttcctgcaag tgctgcgcca agtcgttgcc 10620
gaccaagcaa ctgccttgcg cgagatcagc ctgctcagcg aggaagagcg ccgaattgtg 10680
accgttgatt tcaacaacac gtttgccgcg tatccgcgcg atctgacgat tcaggagctg 10740
ttcgagcagc aggcagcaaa aactccggag catgcagcgg tcgtgatgga cggacagatg 10800
ctgacgtatc gggagctgaa cgaaaaagcg aaccagctcg cccatgtcct tcgtcaaaac 10860
ggagtcggga aagagagcat cgtcggtctg ctcgcagatc gttcgctgga aatgattaca 10920
ggcatcatgg ggattctcaa agcgggcggc gcctacctgg gactggaccc ggagcatccg 10980
tccgaacgcc tggcttacat gttggaagat ggcggcgtga aagttgtcct cgtgcaaaag 11040
cacttgctgc cgctcgtcgg cgaagggctg atgccaatcg ttttggaaga ggagagcctg 11100
cgcccggaag attgcggcaa tccggcgatt gtcaacggtg cgagtgacct ggcttatgtg 11160
atgtacacct caggctctac aggcaagcca aaaggagtca tggtcgagca tcgcaacgtc 11220
acccgcttgg tcatgcatac gaattacgtg caagtgcgcg agagcgaccg gatgattcaa 11280
accggcgcga ttggcttcga cgccatgaca tttgagattt ttggagcctt gctgcacggg 11340
gccagcctgt atttggtgag caaggacgtc ttgctggatg ccgaaaagct gggcgacttc 11400
ctgcggacga atcagattac gaccatgtgg ctgacctcgc cgctcttcaa ccagctttcg 11460
caagacaatc cggcgatgtt tgacagcttg cgcgccttga tcgtcggtgg cgaagcgttg 11520
tcgccgaagc acatcaaccg ggtaaaaagt gcccttcctg acctggaaat ctggaacgga 11580
tacggcccga ccgaaaacac gaccttctcg acgtgctatt tgattgagca gcattttgaa 11640
gagcagattc cgatcggcaa gccgattgca aactccaccg cgtatatcgt cgacggcaac 11700
aatcagccgc agccgatcgg cgtaccgggt gaactgtgcg tcggtggtga cggtgtcgca 11760

agaggctatg tgaacaagcc ggaattaacc gccgaaaagt ttgtgcccaa tccgtttgcg 11820

cctggcgaaa cgatgtatcg caccggagat ttggcgagat ggctgccgga tgggacgatt 11880

gagtatttgg gccgaatcga ccagcaggtc aaaatcaggg gataccggat cgagcttggg 11940

gaaatcgaga cggtcttgtc ccagcaggca caagtaaaag aagcagtcgt ggccgtgatc 12000

gaggaggcga acgggcaaaa agctctctgc gcttactttg tgccagaaca ggccgtcgac 12060

gccgcagagc tgcgagaagc gatgtccaaa caattgcctg gctacatggt ccctgcttac 12120

tatgtgcaaa tggaaaagct gccgttgacc gcgaacggaa aggtcgaccg ccgggcattg 12180

ccgcagccat ccggcgagcg gacgacagga agcgcctttg tcgctgcgca aaatgatacc 12240

gaagcgaagc tgcaacagat ttggcaagaa gttttgggca ttccggcaat cggcattcac 12300

gacaacttct ttgaaatcgg cggtcattcc ttgaaggcga tgaacgtcat cacgcaagtc 12360

cataaaacat tccaggtgga gctgccgtta aaagcgctgt ttgccactcc gacgatccat 12420

gagttggctg cgcatattgc cgagagcgca ttcgagcagt tcgagacgat ccagccagtc 12480

gagcctgccg cgttttatcc cgtgtcgttt gcccaaaagc gaatgtacat cctgcatcag 12540

ttcgaaggaa gcgggatcag ctacaacgtg ccgagtgtgc tggtgctgga aggcaagctc 12600

gattatgacc gctttgctgc tgccatccag agcctggtta aacggcatga atctttgcgc 12660

acctcgttcc attcggtaaa cggggaaccg ctgcaacgag tacatccgga tgtcgagctg 12720

cctgtccgcc ttttggaggc gacagaagat cagagcgaat cgctcatcca ggagctaatc 12780

cagccgtttg atctggagat agccccgttg ttcagagtga atctgatcaa gcttggcgca 12840

gagcggcact tgttcttcat ggatatgcac cacattattt ccgatggcgt atcgcttgcg 12900

gtcatcgtcg aggaaattgc cagcttgtat gcaggaaaac agctttccga cctgcgcatc 12960

cagtacaaag actttgctgt gtggcagacc aagctggctc agtcggatcg cttccaaaaa 13020

caggaggatt tttggacccg gacgtttgcc ggggagattc ctttgctgaa tctgccccat 13080

gattatccaa gaccttctgt gcagagcttt gacggtgaca cggtcgcgct tggcaccgga 13140

catcacctgc tggaacaact gcgcaagctc gctgccgaga ctggcacgac cttgttcatg 13200

gtgctgctgg ctgcctacca tgtgttgctc tccaagtacg ccggacagga agaaatcgtc 13260

gtcggcacac cgatcgcagg ccgctcgcac gcagatgtcg agcgcattgt cgggatgttc 13320

gtcaacacgc tcgctttgaa aaatacggcc gctggcagcc tgagcttccg cgcctttttg 13380

gaagacgtga agcaaaatgc gctccatgcc ttcgagcatc aagactatcc gttcgagcat 13440

ctggtcgaga agctgcaagt gcggcgcgat ctgagcagaa acccgctgtt tgatacgatg 13500

ttcagcctgg ggcttgccga atcagccgaa ggagaagtag cggatctgaa agtgtcgcct 13560

tatccggtga acggccacat cgccaaattc gacctttccc tggatgcgat ggaaaaacag 13620

gatggacttc ttgttcaatt cagctattgc acgaagctgt tcgcaaaaga aacggttgat 13680

cgactggccg cccattacgt tcagcttttg caaacaatca cagccgatcc cgacatcgag 13740

ctcgcccgga tcagcgtgtt gtccaaagca gagacggagc acatgctgca cagcttcctc 13800

gcaaccaaaa cagcctatcc gacggacaaa acgttccaga agctgttcga ggagcaagtg 13860

gaaaaaacac cgaacgagat tgccgttctg ttcggcaatg aacagctgac ctatcaggag 13920

ttgaatgcaa aagcaaacca gctcgcccgc gtcctgcggc gaaaaggcgt caagccggag 13980

agcaccgtcg gcatcctcgt agaccgctcg ctctacatgg tcatcggcat gctggccgtg 14040

ttgaaagcag gcggaacatt cgtcccgatt gatccggact acccgctgga gcgccaagcg 14100

ttcatgctcg aagacagcga ggcgaagctg ctgctcacct tgcaaaaaat gaacagtcaa 14160

gttgccttcc cttatgaaac cttttatctg gatacagaga cagtggatca ggaggagacg 14220

ggcaatctgg agcacgttgc gcagccggag aacgtcgctt acatcatcta cacatccggt 14280

acgacgggca agccaaaagg ggtcgtcatc gagcaccgca gctatgccaa tgtcgcattt 14340

gcctggaaag acgaatatca cctggacagc ttcccggtcc gtttgctgca aatggcgagc 14400

ttcgcctttg acgtctcgac gggcgatttt gccagggcgc tgctgacagg cgggcaactg 14460

gtcatctgcc cgaatggggt caaaatggac ccagcttcgc tgtacgagac catcaggcgt 14520

cacgaaatta ccattttcga agcgacaccc gccttgatca tgccgttgat gcactacgtt 14580

tacgaaaacg aactggatat gagccaaatg aagctgctga ttctcggagc agacagctgc 14640

ccggcggaag acttcaaaac gttgctcgcg cgcttcggtc agaagatgcg cattatcaac 14700

agctacggcg tgacagaggc gtgcattgac accagctact acgaagaaac agacgtcacc 14760

gccatccgct cgggaacggt gccgatcggc aaaccgcttc cgaacatgac gatgtacgtg 14820

gtcgatgcgc atttgaattt gcagcctgtc ggcgtcgtag gcgaattgtg catcggcgga 14880

gcaggggttg cgcgcggtta tttgaacaga cctgagctga cggaagagaa gttcgtgccg 14940

aatccgttcg ccccaggtga acgattgtac cgcacaggtg atctggcgaa gtggcgcgca 15000

gatggcaatg tcgagttcct cggacgcaat gaccaccagg taaaaatcag gggtgtccgc 15060

atcgagctgg gcgagatcga gacacaactg cgcaagctgg acggaattac ggaagcagtc 15120

gtggttgcga gagaagatcg cgggcaggaa aaggaattgt gcgcatacgt cgtggcggac 15180

cacaagcttg acaccgcaga attgcgggcg aatttgctga aggaactgcc gcaagcgatg 15240

attccagcgt atttcgtcac cttggatgcg ctgccgctga ctgccaatgg caaagtagac 15300

agacgttcct tgccagcgcc ggatgtcacc atgctgagaa cgaccgagta tgtagcgccg 15360

cgctccgtct gggaagcccg attggcccaa gtatgggagc aggtgctgaa tgttccgcaa 15420

gtgggtgcgc tagacgactt tttcgcgctc ggcggtcact cattgcgtgc catgcgcgtc 15480

ctttccagca tgcacaacga ataccaggtc gacatcccgc tgcgcatctt gttcgaaaaa 15540

ccgacgattc aggaactggc ggcgttcatc gaagagacag ccaaagggaa tgtcttctcg 15600

atcgagcctg tgcaaaagca agcgtactat ccggtctcct cggcacaaaa gcgcatgtac 15660

atcctcgatc aatttgaggg agtcggcatc agctacaaca tgccgtcgac tatgctgatc 15720

gaaggcaagc tggagcgaac acgggtagaa gcggcgttcc agcgcttgat tgcgcgacat 15780

gaaagcctgc gcacttcgtt tgccgtcgtc aacggagagc ctgtgcaaaa cattcacgag 15840

gacgttccgt ttgcgcttgc ctattcggaa gtcacagaac aggaggcgcg cgaactcgtt 15900

tcttctctcg tgcagccgtt cgatctggag gtcgcaccac tcatccgcgt gtcgctgctg 15960

aaaatcggcg aggatcgtta cgtgctcttt accgacatgc atcacagcat ttccgatggc 16020

gtatcctccg gcattctttt ggcagagtgg gtgcagctgt accaggtgga cgttttgccg 16080

gagctgcgta tccagtacaa ggactttgct gtgtggcaac aagagttttc ccagtcggct 16140

gccttccaca agcaggaagc gtactggttg caaacgtttg ccgatgacat tcctgtgctg 16200

aacttgccga ccgatttcac ccgccccagc acccaaagct ttgccgggga tcagtgcacg 16260

atcggcgcgg gcaaagcgct cacggaaggc ttgcaccagt tggcgcaggc gacgggaacg 16320

actttgtaca tggttttgct cgccgcgtac aacgtgctgc tcgccaagta tgccgggcag 16380

gaggacatca tcgtcggcac gccgattaca ggcagatccc atgccgatct cgaaccgatc 16440

gtcggcatgt tcgtgaacac cttggcgatg cgaaacaaac cgcagcgcga aaagactttt 16500

agcgagtttt tgcaagaagt caagcaaaat gcgctggatg cgtacggcca tcaggattac 16560

ccgtttgaag aactggtgga aaagctcgcg atcgcgcgcg atttgagccg aaatccgctg 16620

tttgacaccg tgtttacgtt ccaaaacagc acggaagagg tcatgacgct gcctgaatgc 16680

acgcttgcgc cgtttatgac ggacgaaaca ggccagcacg ccaagttcga cttgactttc 16740

agcgctacgg aagagcggga agaaatgacg attggcgtgg agtacagcac aagcttgttt 16800

acgcgggaaa cgatggaacg gttcagccgc cacttcctga cgattgcagc gagcatcgtg 16860

caaaatccgc acatccgtct gggcgagatc gacatgcttt tgccagaaga aaaacagcag 16920

attttggccg ggttcaacga tacggcagtc agctatgcgc tggacaaaac gctgcaccag 16980

ctattcgaag agcaggtcga caaaacaccg gatcaggcag cgcttctctt tagcgagcaa 17040

tcgctgacgt acagcgaact gaacgagcga gcaaacagac tggcaagggt cctgcgcgca 17100

aaaggagtcg gaccggaccg tctggtagcg atcatggcgg agcgctcgcc ggaaatggtg 17160

atcggtattc tcggtatttt gaaggcaggc ggcgcttatg ttcccgtcga tcccggctat 17220

ccgcaggagc gcattcagta cctgctcgaa gatagcaacg cagccctgct gctcagccag 17280

gcgcatctgt tgccgctgtt ggcccaggtg tcaagcgagc tgccggagtg ccttgatctg 17340

aacgctgaac tggatgccgg actgagcggc tccaacctgc cagctgtcaa ccaaccgact 17400

gaccttgcct acgtcatcta tacatccggt acgaccggca agccgaaggg tgtcatgatc 17460

ccgcatcaag gaatcgtgaa ctgcttgcag tggagaagag acgaatacgg gttcgggccg 17520

agtgacaagg cgttgcaagt gttctccttt gccttcgacg gttttgtagc cagcttgttc 17580

gctccgctgc tcggaggggc aacgtgcgtg ttgccgcaag aagcagctgc caaagacccg 17640

gtcgcgctga aaaaactgat ggccgcaacg gaagtcaccc attactacgg cgtaccgagt 17700

ctgttccagg ccattctcga ttgctcgacg acaaccgact tcaatcagtt gcgttgcgtc 17760

actttgggcg gcgagaagct gcctgtgcag cttgtgcaaa aaacaaaaga aaagcatccg 17820

gcaatcgaga tcaacaacga gtacggcccg acggaaaaca gcgtcgtcac caccatctcg 17880

cgctcgattg aagcggggca agcgatcacg attggccgac cgcttgcgaa cgtccaagtc 17940

tacattgtag atgagcagca tcacttgcag ccgattggcg tggtcggtga gctgtgcatc 18000

ggcggagccg ggcttgccag aggctatctg aacaaaccgg agctgaccgc agagaagttt 18060

gtcgcaaatc cgttccgacc aggcgagcgc atgtacaaaa caggcgactt ggtaaaatgg 18120

cggacggatg gcacgatcga gtacatcggc cgcgcagacg aacaggtcaa ggtgagaggg 18180

tatcgcatcg agatcggcga gatcgagagc gccgtactcg cttaccaggg catcgatcaa 18240

gcggtggtcg ttgcgcgaga cgatgacgct acggctggtt cctatctttg cgcctacttt 18300

gtcgcagcaa cagccgtgtc cgtatccggc ttgagaagcc atctggccaa agagctgcct 18360

gcttacatga ttccgagcta tttcgtcgag ctggatcagc tgccgctttc cgccaatgga 18420

aaagtggatc gcaaagcttt gccgaagccg caacagtccg atgcgaccac gcgcgaatac 18480

gtggccccga ggaatgcgac cgaacagcaa ctggcagcca tctggcaaga agttttggga 18540

gtagagccaa tcggcatcac cgaccagttc tttgaactcg gaggacattc cttaaaagct 18600

acgctgttga ttgccaaagt gtatgagtac atgcaaatcg agctgccgct gaatctcatc 18660

ttccagtatc cgacgatcga aaaggtggcc gatttcatca cgcataagcg ctttgagagc 18720

agatacggca cagccatttt gttaaatcag gagacggcgc gaaacgtatt ttgcttcacg 18780

ccgatcggcg cacaaagcgt gtactaccag aagcttgcgg cggaaattca aggcgtctct 18840

ttgtacagct ttgatttcat ccaggatgac aaccggatgg agcagtatat cgcggcgatc 18900

accgcaattg atccaagcgg tccgtacacg ctcatgggct actcctcggg aggcaatctg 18960

gcttttgaag tggcgaaaga actggaggag cggggctatg gcgtcaccga catcatcttg 19020

ttcgactcgt actggaaaga caaggcgatt gagcggactg tcgcggaaac agaaaacgac 19080

attgcccagc tattcgccga gattggcgaa aacaccgaga tgttcaacat gacgcaagaa 19140

gacttccagc tgtacgccgc caatgagttt gtcaagcaaa gcttcgttcg caaaacggtc 19200

agctatgtga tgttccataa caatctggtc aataccggaa tgaccactgc cgcgatccac 19260

ctcatccaat ccgagctgga agcagacgag gaagctccgg tggcagccaa gtggaacgaa 19320

tcagcctggg caaacgcaac gcaacgactg ctgacgtaca gcgggcacgg aatccactcg 19380

cgcatgctgg cgggcgatta cgcgtcgcaa aatgcttcga ttttgcaaaa catcctgcaa 19440

gaactgttca tcctgaaata a 19461

<210> 23
<211> 6507
<212> DNA
<213> Artificial Sequence

<220>
<223> NRPS being a synthetase of a fusion peptide consisting of Valine
      and Indigoidine. Due to its sterical advantages, Valine may be
      used as a spacer for other tags.

<400> 23
atgtatccgc gcgatctgac gattcaggag ctgttcgagc agcaggcagc aaaaactccg 60

gagcatgcag cggtcgtgat ggacggacag atgctgacgt atcgggagct gaacgaaaaa 120

gcgaaccagc tcgcccatgt ccttcgtcaa aacggagtcg ggaaagagag catcgtcggt 180

ctgctcgcag atcgttcgct ggaaatgatt acaggcatca tggggattct caaagcgggc 240

ggcgcctacc tgggactgga cccggagcat ccgtccgaac gcctggctta catgttggaa 300

gatggcggcg tgaaagttgt cctcgtgcaa aagcacttgc tgccgctcgt cggcgaaggg 360

ctgatgccaa tcgttttgga agaggagagc ctgcgcccgg aagattgcgg caatccggcg 420

attgtcaacg gtgcgagtga cctggcttat gtgatgtaca cctcaggctc tacaggcaag 480

ccaaaaggag tcatggtcga gcatcgcaac gtcacccgct tggtcatgca tacgaattac 540

gtgcaagtgc gcgagagcga ccggatgatt caaaccggcg cgattggctt cgacgccatg 600

acatttgaga tttttggagc cttgctgcac ggggccagcc tgtatttggt gagcaaggac 660

gtcttgctgg atgccgaaaa gctgggcgac ttcctgcgga cgaatcagat tacgaccatg 720

tggctgacct cgccgctctt caaccagctt tcgcaagaca atccggcgat gtttgacagc 780

ttgcgcgcct tgatcgtcgg tggcgaagcg ttgtcgccga agcacatcaa ccgggtaaaa 840

agtgcccttc ctgacctgga aatctggaac ggatacggcc cgaccgaaaa cacgaccttc 900

tcgacgtgct atttgattga gcagcatttt gaagagcaga ttccgatcgg caagccgatt 960

gcaaactcca ccgcgtatat cgtcgacggc aacaatcagc cgcagccgat cggcgtaccg 1020

ggtgaactgt gcgtcggtgg tgacggtgtc gcaagaggct atgtgaacaa gccggaatta 1080

accgccgaaa agtttgtgcc caatccgttt gcgcctggcg aaacgatgta tcgcaccgga 1140

gatttggcga gatggctgcc ggatgggacg attgagtatt tgggccgaat cgaccagcag 1200

gtcaaaatca ggggataccg gatcgagctt ggggaaatcg agacggtctt gtcccagcag 1260

gcacaagtaa aagaagcagt cgtggccgtg atcgaggagg cgaacggcca aaaagctctc 1320

tgcgcttact ttgtgccaga acaggccgtc gacgccgcag agctgcgaga agcgatgtcc 1380

aaacaattgc ctggctacat ggtccctgct tactatgtgc aaatggaaaa gctgccgttg 1440

accgcgaacg gaaaggtcga ccgccgggca ttgccgcagc catccggcga gcggacgaca 1500

ggaagcgcct ttgtcgctgc gcaaaatgat accgaagcga agctgcaaca gatttggcaa 1560

gaagttttgg gcattccggc aatcggcatt cacgacaact tctttgaaat cggcggtcat 1620

tccttgaagg cgatgaacgt catcacgcaa gtccataaaa cattccaggt ggagctgccg 1680

ttaaaagcgc tgtttgccac tccgacgatc catgagttgg ctgcgcatat ttcggaaaaa 1740

accgagtaca ccgcgattca acccgtggca gcgcaggagt tttacccggt ttcatctgcg 1800

caaaaaagaa tgtatatcct gcaacagttc gaaggcaacg gaatcagcta caacatttcg 1860

ggtgcgattc tcctggaagg aaagctggac tacgcccggt ttgccagcgc tgtgcaacag 1920

ctggcagagc gccacgaagc tttgcgcacc tcgttccacc ggatcgacgg cgagcctgtg 1980

caaaaagtgc acgaggaagt agaagtgccg cttttcatgc tggaggctcc cgaagaccag 2040

gcggagaaaa tcatgcgcga gtttgtccgt ccgtttgatc tcggggtcgc tccgctgatg 2100

cgaacaggtt tgctcaagct gggcaaagac cgccatttgt ttttgctcga catgcaccat 2160

atcatctcgg acggcgtttc ttcgcaaatt ttgctgcgtg aatttgccga gttgtaccag 2220

ggagcagact tgcagccgct ttcgctgcaa tacaaagatt tcgctgcttg gcaaaatgag 2280

ctgtttcaga cggaggcata caagaagcag gagcagcact ggctgaacac gtttgctgat 2340

gaaattccgc tcttgaacct gccgactgac tatccgcgcc ctagcgtgca aagctttgca 2400

ggcgatctcg tcctttttgc cgccggaaaa gaactgctgg agcggttgca acaggtagcg 2460

tcagaaacag gcaccacctt gtacatgatt ttgcttgccg cctacaatgt gctgctgtcc 2520

aagtataccg gccaggaaga catcatcgtc gggacgcctg tcgctggacg ttcccatgcg 2580

gacgtggaaa acatcatggg catattcgtg aacacattgg cgctgcgcaa ccagcctgcc 2640

agcagcaaaa cgatgttaga aaataatatt acacaatgtg actcaatcaa tgatgtttat 2700

cttaaagaag aagcaataac attgatggat atgcttgaga gtcaacttaa gcaccaggca 2760

gatggatatg ttgttattga tcaagaagaa tctctcagtt acgctgattt ctatttgagg 2820

gtgaaagaga tagggtattg tctgtcagaa attagctcaa agagttcggt gggtattggg 2880

cttttttgtg atccttctat agatttaatt tgtggtgcat ggggtatttt gtcagcggat 2940

aaagcttatt tgccgttatc gcctgactat ccaactgaac gcctcaaata tatgatagaa 3000

gattctggta ttgatgtgat ttttacgcaa tcgcacttaa aagcacagct acaggacatt 3060

gcaccaaaat cagtattaat tatgacacca gaagatgtcg ctctgacgat aaaaacacga 3120

acaatagaag atattctggg cacagttcaa gttcctaaac ccacgagtct ggcttatatt 3180

atttatacct ctggtagcac gggtaagcca aagggagtga tgattgaaca tcacagtatt 3240

gtaaatcaaa tgagatttct tgcaaaagcg ttcaaattag gatgtcattc ccggatttta 3300

cagaaaacac caatgagttt tgatgcggct caatgggaaa ttctagcgcc tgcaattggt 3360

ggtcaagtga ttatgggtcc tttaggttgc tatcgcgatc cggatgcaat tattaaaacc 3420

attcttcagc atcaagtaac gactttgcaa tgtgttccta ctttgctaca agcgttactg 3480

gataatccta attttttgga ttgcttatca ttgactcaag tattcagtgg gggagaagcg 3540

ctgacaacca aattagccac gcaatttttg aatagtttta ctcactgtga attaatcaat 3600

ttatatggcc cgacagaatg tacgattaat tcatcatttt tccgggtgac aaatgagact 3660

ttgccgaatt atcaaacctc tatttcgatt ggtgcacctg tagataatac cgaatactac 3720

gttcttgatg atgatagatt acctgtggcg gttggcgaaa ttggcgagct ttatatttcg 3780

ggtgctcaat tagcacgtgg ttatttgcat aaaccagaaa tgacaaaaga taaatttatt 3840

tgtaatcacc ttgtatcagg aactcaacat caatggttat atcgaacggg agatctggta 3900

accagagggg ctgatggtaa tacttatttt gttggtcggg ttgatagcca ggtcaaatta 3960

cgaggttacc gtattgagct tgatgaaata cgccatgcga ttgaagaaca tagctggata 4020

aagacggcgg caatgttaat taagaaggat gccagaacgg gtttccaaaa tctcatcgcg 4080

tgtgtggaat tagatgagaa agaagctgca ttgatggatc aaggtaatag tagctcacat 4140

cacaaatcaa aagccgataa actacaggtg aaagcccaac tttctaattc tggttgtcga 4200

agtgaagagt tatgtgaaaa tcgccctaca ttcttacttc cttatcaaga aggggagata 4260

aaacagagag aatatgcatt tggacgcaag acatatcgct attttgaggg aacagaaata 4320

acggtagaga aattaaaaaa attgctgaca gccactcaat cgaatgaaat tagctctttg 4380

ccactgagtc atctaaccct gaatgatttc ggttatgcat tgcgttattt tggtcagttt 4440

accagccatc aacgtttatt gcccaaatat gcctatgctt caccgggtgc tctctatgcg 4500

acacaaatgt attttgaatt gcataatgtt ctcggtttgg atgcggggat ttactattat 4560

catccagtga cacataagtt aataaaaatt tcaacattga gtcgtcggca aatgccaacg 4620

ataaaagtgc attttattgg caagcatgaa gccattgagc ccgtttataa gaacaatata 4680

caagaagttc tggaaatgga agcgggccat atgatgggtc tttttgatga cgtattaccg 4740

gaaattggct tgagtattgg taaaagtgaa tatcaagatg aatgtccaga ttggtatgat 4800

ggtgatattc aggattatta tcttggtgca tttgaaatat gtagctatga acatggattg 4860

ccgccatttg agactgatat ttatttacaa acacatgccc ataaaatacc tgagatgccg 4920

tgtggtttat atcacttttc taacggggaa tttgtacgaa taagtgatga tattgtccga 4980

aaaaaggatg ttattgcgat taatcagcaa gtttatgatc gctccagttt tggcgtgtca 5040

attattccac gctgtgtccc tgaatggcat tattatataa cactgggtcg tcggttacat 5100

gcgttacaaa gtaatccatt gtatattgga ttaatgtcat ctggttacag ttcgaagagc 5160

aataacgatt taccttcggc gaaaaggatg cgatctattc tcaatgcact tgatagacct 5220

atggcggcat tttatttctg cataggtggg ggtattagcc aagcgcaata tatgtgtgaa 5280

ggcatgaaag aagatgttgt tcatatgaaa gggccagttg aaatcattaa agatgatctt 5340

caacaacaac tccctcaata tatgattcca aataaggtat tagttttcga taaattacct 5400

ttgacggcca atggaaaagt ggattatcaa tctttatcag aatctaaagc cgtggagaat 5460

gtttcaacac agcgtctatt ggtgccatta catacagata ctgaaataag gcttggaaaa 5520

atttggatgg aagtactgaa atgggattca gtatctgccc tcgatgattt tttcgaaagt 5580

gggggtaatt ctttgatggc cgttgcaatg gttaataaga tcaatgcggc ctttaatatt 5640

cgttttccgt tacagatact ttttcaatct cctaatatag cagaattggc taagtggatt 5700

gaacagacag actctaaaac aatatcaaga ttaattttat tgaatcaggc aagcaaagac 5760

cccatttact gttggccggg tttgggcgga tatcctatga gtttgagatt gcttgctaat 5820

aaagtcgttc ctgatcgggc attttatgga atacaggcat atgggataaa cgagagtgaa 5880

ataccgtttt cttctatcca gagaatggca gaagaggata ttaaagagat aaagaaaata 5940

cagccagaag ggccatatat attgtgggga tattcatttg gtgcccgagt agcatttgaa 6000

gttgcatacc agcttgaaca agcgggagaa gaagttaacg cattgaattt attggctccg 6060

ggatctcctc atcttgatat gaagcaagcg gaatatatgg ataaaggcgc tgaatttact 6120

aatccggctt ttgttaaaat acttttttct gtattttctc gttcaatcaa cagcccaatg 6180

gttaaaactt gcttagaaca agtaaatagt gaaacgacat ttattaactt tatatgtagt 6240

cgttttaaaa acttggaacc atcattagta aaacgtatcg ttaggattgt gactttgact 6300

tatgatttca agtacagtat tgatgagctt tatcacagac acctaaaggc acctataact 6360

attttcaagg cgaatagaga taatgattca tttatcgagg aatcggatgt gatttcatca 6420

atgtcgccta aaataattga attaatatcg gatcactatc aactgttgga aagtgaaggt 6480

gttgctgaga ttgagaaaat aatctaa 6507

<210> 24
<211> 9609
<212> DNA
<213> Artificial Sequence

<220>
<223> NRPS synthesizing a Indigoidine-tagged Dipeptide consisting of
      two Valine-monomers.

<400> 24
atgtatccgc gcgatctgac gattcaggag ctgttcgagc agcaggcagc aaaaactccg 60

gagcatgcag cggtcgtgat ggacggacag atgctgacgt atcgggagct gaacgaaaaa 120

gcgaaccagc tcgcccatgt ccttcgtcaa aacggagtcg ggaaagagag catcgtcggt 180

ctgctcgcag atcgttcgct ggaaatgatt acaggcatca tggggattct caaagcgggc 240

ggcgcctacc tgggactgga cccggagcat ccgtccgaac gcctggctta catgttggaa 300

gatggcggcg tgaaagttgt cctcgtgcaa aagcacttgc tgccgctcgt cggcgaaggg 360

ctgatgccaa tcgttttgga agaggagagc ctgcgcccgg aagattgcgg caatccggcg 420

attgtcaacg gtgcgagtga cctggcttat gtgatgtaca cctcaggctc tacaggcaag 480

ccaaaaggag tcatggtcga gcatcgcaac gtcacccgct tggtcatgca tacgaattac 540

gtgcaagtgc gcgagagcga ccggatgatt caaaccggcg cgattggctt cgacgccatg 600

acatttgaga tttttggagc cttgctgcac ggggccagcc tgtatttggt gagcaaggac 660

gtcttgctgg atgccgaaaa gctgggcgac ttcctgcgga cgaatcagat tacgaccatg 720

tggctgacct cgccgctctt caaccagctt tcgcaagaca atccggcgat gtttgacagc 780

ttgcgcgcct tgatcgtcgg tggcgaagcg ttgtcgccga agcacatcaa ccgggtaaaa 840

agtgcccttc ctgacctgga aatctggaac ggatacggcc cgaccgaaaa cacgaccttc 900

tcgacgtgct atttgattga gcagcatttt gaagagcaga ttccgatcgg caagccgatt 960

gcaaactcca ccgcgtatat cgtcgacggc aacaatcagc cgcagccgat cggcgtaccg 1020

ggtgaactgt gcgtcggtgg tgacggtgtc gcaagaggct atgtgaacaa gccggaatta 1080

accgccgaaa agtttgtgcc caatccgttt gcgcctggcg aaacgatgta tcgcaccgga 1140

gatttggcga gatggctgcc ggatgggacg attgagtatt tgggccgaat cgaccagcag 1200

gtcaaaatca ggggataccg gatcgagctt ggggaaatcg agacggtctt gtcccagcag 1260

gcacaagtaa aagaagcagt cgtggccgtg atcgaggagg cgaacgggca aaaagctctc 1320

tgcgcttact ttgtgccaga acaggccgtc gacgccgcag agctgcgaga agcgatgtcc 1380

aaacaattgc ctggctacat ggtccctgct tactatgtgc aaatggaaaa gctgccgttg 1440

accgcgaacg gaaaggtcga ccgccgggca ttgccgcagc catccggcga gcggacgaca 1500

ggaagcgcct ttgtcgctgc gcaaaatgat accgaagcga agctgcaaca gatttggcaa 1560

gaagttttgg gcattccggc aatcggcatt cacgacaact tctttgaaat cggcggtcat 1620

tccttgaagg cgatgaacgt catcacgcaa gtccataaaa cattccaggt ggagctgccg 1680

ttaaaagcgc tgtttgccac tccgacgatc catgagttgg ctgcgcatat tgccacgagc 1740

ggaaaagaga cgtatgtgcc gatcgagcct gcaccgttgc aagagtatta tcctgtttca 1800

tctgcgcaaa agcggatgta tgtcctgcgc cagtttgcgg acacaggcac ggtttataac 1860

atgccgagcg cgttgtatat cgaaggcgat ctggatcgga agcgttttga agccgccatc 1920

cacggattgg tcgagcggca cgaatcgctg cgcacatcct tccacaccgt aaatggcgag 1980

cctgtccagc gcgtacacga gcatgtcgag ctgaatgtgc agtacgcgga agtgacggaa 2040

gcgcaagtgg agccaaccgt cgagtcgttc gtgcaagcat ttgatctgac aaaagctccg 2100

ctattgcggg tcggactttt caagctggca gcgaaacggc atctgttcct gctggatatg 2160

catcacatca tctcggatgg cgtctcggcc ggaatcatta tggaagagtt ctcgaagctg 2220

tatcgaggcg aagaactgcc tgcgctttcc gtccattaca aagatttcgc cgtctggcag 2280

tctgaactgt tccagagcga cgtctatacc gagcatgaaa actactggct gaacgcgttt 2340

tctggcgaca ttccggtgct taacttgcca gccgattttt ctcgtccgct gacacagagc 2400

tttgaaggag attgcgtttc gttccaggca gacaaagcgt tgctggacga tcttcacaag 2460

ctcgctcagg agagccaatc gacgttgttc atggtattgc tggcggctta caatgtgctg 2520

cttgccaagt acagcggaca ggaagacatc gtcgtcggca caccgattgc gggcagatcg 2580

cacgccgata tcgagaacgt tctggggatg tttgtcaaca cgctcgcttt gcgcaactat 2640

ccggtcgaga cgaaacactt ccaggcattt ttggaagagg tcaagcaaaa tacgctgcaa 2700

gcatacgccc atcaagatta tccgttcgaa gcactggtcg aaaagctgga catccagcgg 2760

gatctcagcc gcaatccgct gtttgacacc atgtttattt tgcaaaacct ggaccaaaaa 2820

gcttacgagc tggatgggct gaaactggag gcatatccgg cacaagcagg caacgccaaa 2880

ttcgatctca cgctggaagc gcacgaggac gagacaggca ttcattttgc gctcgtctac 2940

tcgaccaaat tgttccagcg agaatcaatc gaaagaatgg cgggtcactt cctgcaagtg 3000

ctgcgccaag tcgttgccga ccaagcaact gccttgcgcg agatcagcct gctcagcgag 3060

gaagagcgcc gaattgtgac cgttgatttc aacaacacgt ttgcctatcc gcgcgatctg 3120

acgattcagg agctgttcga gcagcaggca gcaaaaactc cggagcatgc agcggtcgtg 3180

atggacggac agatgctgac gtatcgggag ctgaacgaaa aagcgaacca gctcgcccat 3240

gtccttcgtc aaaacggagt cgggaaagag agcatcgtcg gtctgctcgc agatcgttcg 3300

ctggaaatga ttacaggcat catggggatt ctcaaagcgg gcggcgccta cctgggactg 3360

gacccggagc atccgtccga acgcctggct tacatgttgg aagatggcgg cgtgaaagtt 3420

gtcctcgtgc aaaagcactt gctgccgctc gtcggcgaag ggctgatgcc aatcgttttg 3480

gaagaggaga gcctgcgccc ggaagattgc ggcaatccgg cgattgtcaa cggtgcgagt 3540

gacctggctt atgtgatgta cacctcaggc tctacaggca agccaaaagg agtcatggtc 3600

gagcatcgca acgtcacccg cttggtcatg catacgaatt acgtgcaagt gcgcgagagc 3660

gaccggatga ttcaaaccgg cgcgattggc ttcgacgcca tgacatttga gatttttgga 3720

gccttgctgc acggggccag cctgtatttg gtgagcaagg acgtcttgct ggatgccgaa 3780

aagctgggcg acttcctgcg gacgaatcag attacgacca tgtggctgac ctcgccgctc 3840

ttcaaccagc tttcgcaaga caatccggcg atgtttgaca gcttgcgcgc cttgatcgtc 3900

ggtggcgaag cgttgtcgcc gaagcacatc aaccgggtaa aaagtgccct tcctgacctg 3960

gaaatctgga acggatacgg cccgaccgaa aacacgacct tctcgacgtg ctatttgatt 4020

gagcagcatt ttgaagagca gattccgatc ggcaagccga ttgcaaactc caccgcgtat 4080

atcgtcgacg gcaacaatca gccgcagccg atcggcgtac cgggtgaact gtgcgtcggt 4140

ggtgacggtg tcgcaagagg ctatgtgaac aagccggaat taaccgccga aaagtttgtg 4200

cccaatccgt ttgcgcctgg cgaaacgatg tatcgcaccg gagatttggc gagatggctg 4260

ccggatggga cgattgagta tttgggccga atcgaccagc aggtcaaaat caggggatac 4320

cggatcgagc ttggggaaat cgagacggtc ttgtcccagc aggcacaagt aaaagaagca 4380

gtcgtggccg tgatcgagga ggcgaacggg caaaaagctc tctgcgctta ctttgtgcca 4440

gaacaggccg tcgacgccgc agagctgcga gaagcgatgt ccaaacaatt gcctggctac 4500

atggtccctg cttactatgt gcaaatggaa aagctgccgt tgaccgcgaa cggaaaggtc 4560

gaccgccggg cattgccgca gccatccggc gagcggacga caggaagcgc ctttgtcgct 4620

gcgcaaaatg ataccgaagc gaagctgcaa cagatttggc aagaagtttt gggcattccg 4680

gcaatcggca ttcacgacaa cttctttgaa atcggcggtc attccttgaa ggcgatgaac 4740

gtcatcacgc aagtccataa aacattccag gtggagctgc cgttaaaagc gctgtttgcc 4800

actccgacga tccatgagtt ggctgcgcat atttcggaaa aaaccgagta caccgcgatt 4860

caacccgtgg cagcgcagga gttttacccg gtttcatctg cgcaaaaaag aatgtatatc 4920

ctgcaacagt tcgaaggcaa cggaatcagc tacaacattt cgggtgcgat tctcctggaa 4980

ggaaagctgg actacgcccg gtttgccagc gctgtgcaac agctggcaga gcgccacgaa 5040

gctttgcgca cctcgttcca ccggatcgac ggcgagcctg tgcaaaaagt gcacgaggaa 5100

gtagaagtgc cgcttttcat gctggaggct cccgaagacc aggcggagaa aatcatgcgc 5160

gagtttgtcc gtccgtttga tctcggggtc gctccgctga tgcgaacagg tttgctcaag 5220

ctgggcaaag accgccattt gtttttgctc gacatgcacc atatcatctc ggacggcgtt 5280

tcttcgcaaa ttttgctgcg tgaatttgcc gagttgtacc agggagcaga cttgcagccg 5340

ctttcgctgc aatacaaaga tttcgctgct tggcaaaatg agctgtttca gacggaggca 5400

tacaagaagc aggagcagca ctggctgaac acgtttgctg atgaaattcc gctcttgaac 5460

ctgccgactg actatccgcg ccctagcgtg caaagctttg caggcgatct cgtccttttt 5520

gccgccggaa aagaactgct ggagcggttg caacaggtag cgtcagaaac aggcaccacc 5580

ttgtacatga ttttgcttgc cgcctacaat gtgctgctgt ccaagtatac cggccaggaa 5640

gacatcatcg tcgggacgcc tgtcgctgga cgttcccatg cggacgtgga aaacatcatg 5700

ggcatattcg tgaacacatt ggcgctgcgc aaccagcctg ccagcagcaa aacgatgtta 5760

gaaaataata ttacacaatg tgactcaatc aatgatgttt atcttaaaga agaagcaata 5820

acattgatgg atatgcttga gagtcaactt aagcaccagg cagatggata tgttgttatt 5880

gatcaagaag aatctctcag ttacgctgat ttctatttga gggtgaaaga gatagggtat 5940

tgtctgtcag aaattagctc aaagaattcg gtgggtattg ggcttttttg tgatccttct 6000

atagatttaa tttgtggtgc atggggtatt ttgtcagcgg ataaagctta tttgccgtta 6060

tcgcctgact atccaactga acgcctcaaa tatatgatag aagattctgg tattgatgtg 6120

atttttacgc aatcgcactt aaaagcacag ctacaggaca ttgcaccaaa atcagtatta 6180

attatgacac cagaagatgt cgctctgacg ataaaaacac gaacaataga agatattctg 6240

ggcacagttc aagttcctaa acccactagt ctggcttata ttatttatac ctctggtagc 6300

acgggtaagc caaagggagt gatgattgaa catcacagta ttgtaaatca aatgagattt 6360

cttgcaaaag cgttcaaatt aggatgtcat tcccggattt tacagaaaac accaatgagt 6420

tttgatgcgg ctcaatggga aattctagcg cctgcaattg gtggtcaagt gattatgggt 6480

cctttaggtt gctatcgcga tccggatgca attattaaaa ccattcttca gcatcaagta 6540

acgactttgc aatgtgttcc tactttgcta caagcgttac tggataatcc taattttttg 6600

gattgcttat cattgactca agtattcagt gggggagaag cgctgacaac caaattagcc 6660

acgcaatttt tgaatagttt tactcactgt gaattaatca atttatatgg cccgacagaa 6720

tgtacgatta attcatcatt tttccgggtg acaaatgaga ctttgccgaa ttatcaaacc 6780

tctatttcga ttggtgcacc tgtagataat accgaatact acgttcttga tgatgataga 6840

ttacctgtgg cggttggcga aattggcgag ctttatattt cgggtgctca attagcacgt 6900

ggttatttgc ataaaccaga aatgacaaaa gataaattta tttgtaatca ccttgtatca 6960

ggaactcaac atcaatggtt atatcgaacg ggagatctgg taaccagagg ggctgatggt 7020

aatacttatt ttgttggtcg ggttgatagc caggtcaaat tacgaggtta ccgtattgag 7080

cttgatgaaa tacgccatgc gattgaagaa catagctgga taaagacggc ggcaatgtta 7140

attaagaagg atgccagaac gggtttccaa aatctcatcg cgtgtgtgga attagatgag 7200

aaagaagctg cattgatgga tcaaggtaat agtagctcac atcacaaatc aaaagccgat 7260

aaactacagg tgaaagccca actttctaat tctggttgtc gaagtgaaga gttatgtgaa 7320

aatcgcccta cattcttact tccttatcaa gaaggggaga taaaacagag agaatatgca 7380

tttggacgca agacatatcg ctattttgag ggaacagaaa taacggtaga gaaattaaaa 7440

aaattgctga cagccactca atcgaatgaa attagctctt tgccactgag tcatctaacc 7500

ctgaatgatt tcggttatgc attgcgttat tttggtcagt ttaccagcca tcaacgttta 7560

ttgcccaaat atgcctatgc ttcaccgggt gctctctatg cgacacaaat gtattttgaa 7620

ttgcataatg ttctcggttt ggatgcgggg atttactatt atcatccagt gacacataag 7680

ttaataaaaa tttcaacatt gagtcgtcgg caaatgccaa cgataaaagt gcattttatt 7740

ggcaagcatg aagccattga gcccgtttat aagaacaata tacaagaagt tctggaaatg 7800

gaagcgggcc atatgatggg tctttttgat gacgtattac cggaaattgg cttgagtatt 7860

ggtaaaagtg aatatcaaga tgaatgtcca gattggtatg atggtgatat tcaggattat 7920

tatcttggtg catttgaaat atgtagctat gaacatggat tgccgccatt tgagactgat 7980

atttatttac aaacacatgc ccataaaata cctgagatgc cgtgtggttt atatcacttt 8040

tctaacgggg aatttgtacg aataagtgat gatattgtcc gaaaaaagga tgttattgcg 8100

attaatcagc aagtttatga tcgctccagt tttggcgtgt caattattcc acgctgtgtc 8160

cctgaatggc attattatat aacactgggt cgtcggttac atgcgttaca aagtaatcca 8220

ttgtatattg gattaatgtc atctggttac agttcgaaga gcaataacga tttaccttcg 8280

gcgaaaagga tgcgatctat tctcaatgca cttgatagac ctatggcggc attttatttc 8340

tgcataggtg ggggtattag ccaagcgcaa tatatgtgtg aaggcatgaa agaagatgtt 8400

gttcatatga aagggccagt tgaaatcatt aaagatgatc ttcaacaaca actccctcaa 8460

tatatgattc caaataaggt attagttttc gataaattac ctttgacggc caatggaaaa 8520

gtggattatc aatctttatc agaatctaaa gccgtggaga atgtttcaac acagcgtcta 8580

ttggtgccat tacatacaga tactgaaata aggcttggaa aaatttggat ggaagtactg 8640

aaatgggatt cagtatctgc cctcgatgat tttttcgaaa gtgggggtaa ttctttgatg 8700

gccgttgcaa tggttaataa gatcaatgcg gcctttaata ttcgttttcc gttacagata 8760

ctttttcaat ctcctaatat agcagaattg gctaagtgga ttgaacagac agactctaaa 8820

acaatatcaa gattaatttt attgaatcag gcaagcaaag accccattta ctgttggccg 8880

ggtttgggcg gatatcctat gagtttgaga ttgcttgcta ataaagtcgt tcctgatcgg 8940

gcattttatg gaatacaggc atatgggata aacgagagtg aaataccgtt ttcttctatc 9000

cagagaatgg cagaagagga tattaaagag ataaagaaaa tacagccaga agggccatat 9060

atattgtggg gatattcatt tggtgcccga gtagcatttg aagttgcata ccagcttgaa 9120

caagcgggag aagaagttaa cgcattgaat ttattggctc cgggatctcc tcatcttgat 9180

atgaagcaag cggaatatat ggataaaggc gctgaattta ctaatccggc ttttgttaaa 9240

atactttttt ctgtattttc tcgttcaatc aacagcccaa tggttaaaac ttgcttagaa 9300

caagtaaata gtgaaacgac atttattaac tttatatgta gtcgttttaa aaacttggaa 9360

ccatcattag taaaacgtat cgttaggatt gtgactttga cttatgattt caagtacagt 9420

attgatgagc tttatcacag acacctaaag gcacctataa ctattttcaa ggcgaataga 9480

gataatgatt catttatcga ggaatcggat gtgatttcat caatgtcgcc taaaataatt 9540

gaattaatat cggatcacta tcaactgttg gaaagtgaag gtgttgctga gattgagaaa 9600

ataatctaa                                                          9609

<210>  25
<211>  1284
<212>  PRT
<213>  Photorhabdus luminescens

<400>  25

Met Leu Glu Asn Asn Ile Thr Gln Cys Asp Ser Ile Asn Asp Val Tyr
1               5                   10                  15

Leu Lys Glu Glu Ala Ile Thr Leu Met Asp Met Leu Glu Ser Gln Leu
            20                  25                  30

Lys His Gln Ala Asp Gly Tyr Val Val Ile Asp Gln Glu Glu Ser Leu
        35                  40                  45

Ser Tyr Ala Asp Phe Tyr Leu Arg Val Lys Glu Ile Gly Tyr Cys Leu
    50                  55                  60

Ser Glu Ile Ser Ser Lys Asn Ser Val Gly Ile Gly Leu Phe Cys Asp
65                  70                  75                  80

Pro Ser Ile Asp Leu Ile Cys Gly Ala Trp Gly Ile Leu Ser Ala Asp
                85                  90                  95

Lys Ala Tyr Leu Pro Leu Ser Pro Asp Tyr Pro Thr Glu Arg Leu Lys
            100                 105                 110

Tyr Met Ile Glu Asp Ser Gly Ile Asp Val Ile Phe Thr Gln Ser His
        115                 120                 125

Leu Lys Ala Gln Leu Gln Asp Ile Ala Pro Lys Ser Val Leu Ile Met
    130                 135                 140

Thr Pro Glu Asp Val Ala Leu Thr Ile Lys Thr Arg Thr Ile Glu Asp
145                 150                 155                 160

Ile Leu Gly Thr Val Gln Val Pro Lys Pro Thr Ser Leu Ala Tyr Ile
                165                 170                 175

Ile Tyr Thr Ser Gly Ser Thr Gly Lys Pro Lys Gly Val Met Ile Glu
            180                 185                 190

His His Ser Ile Val Asn Gln Met Arg Phe Leu Ala Lys Ala Phe Lys
        195                 200                 205

Leu Gly Cys His Ser Arg Ile Leu Gln Lys Thr Pro Met Ser Phe Asp
    210                 215                 220

Ala Ala Gln Trp Glu Ile Leu Ala Pro Ala Ile Gly Gly Gln Val Ile
225                 230                 235                 240

Met Gly Pro Leu Gly Cys Tyr Arg Asp Pro Asp Ala Ile Ile Lys Thr
                245                 250                 255

Ile Leu Gln His Gln Val Thr Thr Leu Gln Cys Val Pro Thr Leu Leu
            260                 265                 270

Gln Ala Leu Leu Asp Asn Pro Asn Phe Leu Asp Cys Leu Ser Leu Thr
        275                 280                 285

Gln Val Phe Ser Gly Gly Glu Ala Leu Thr Thr Lys Leu Ala Thr Gln
    290                 295                 300

Phe Leu Asn Ser Phe Thr His Cys Glu Leu Ile Asn Leu Tyr Gly Pro
305                 310                 315                 320

Thr Glu Cys Thr Ile Asn Ser Ser Phe Phe Arg Val Thr Asn Glu Thr
                325                 330                 335

Leu Pro Asn Tyr Gln Thr Ser Ile Ser Ile Gly Ala Pro Val Asp Asn
            340                 345                 350

Thr Glu Tyr Tyr Val Leu Asp Asp Asp Arg Leu Pro Val Ala Val Gly
        355                 360                 365

Glu Ile Gly Glu Leu Tyr Ile Ser Gly Ala Gln Leu Ala Arg Gly Tyr
    370                 375                 380

Leu His Lys Pro Glu Met Thr Lys Asp Lys Phe Ile Cys Asn His Leu
385                 390                 395                 400

Val Ser Gly Thr Gln His Gln Trp Leu Tyr Arg Thr Gly Asp Leu Val
                405                 410                 415

Thr Arg Gly Ala Asp Gly Asn Thr Tyr Phe Val Gly Arg Val Asp Ser
            420                 425                 430

Gln Val Lys Leu Arg Gly Tyr Arg Ile Glu Leu Asp Glu Ile Arg His
        435                 440                 445

Ala Ile Glu Glu His Ser Trp Ile Lys Thr Ala Ala Met Leu Ile Lys
    450                 455                 460

Lys Asp Ala Arg Thr Gly Phe Gln Asn Leu Ile Ala Cys Val Glu Leu
465                 470                 475                 480

Asp Glu Lys Glu Ala Ala Leu Met Asp Gln Gly Asn Ser Ser Ser His
                485                 490                 495

His Lys Ser Lys Ala Asp Lys Leu Gln Val Lys Ala Gln Leu Ser Asn
            500                 505                 510

Ser Gly Cys Arg Ser Glu Glu Leu Cys Glu Asn Arg Pro Thr Phe Leu
        515                 520                 525

Leu Pro Tyr Gln Glu Gly Glu Ile Lys Gln Arg Glu Tyr Ala Phe Gly
    530                 535                 540

Arg Lys Thr Tyr Arg Tyr Phe Glu Gly Thr Glu Ile Thr Val Glu Lys
545                 550                 555                 560

Leu Lys Lys Leu Leu Thr Ala Thr Gln Ser Asn Glu Ile Ser Ser Leu
                565                 570                 575

Pro Leu Ser His Leu Thr Leu Asn Asp Phe Gly Tyr Ala Leu Arg Tyr
            580                 585                 590

Phe Gly Gln Phe Thr Ser His Gln Arg Leu Leu Pro Lys Tyr Ala Tyr
        595                 600                 605

Ala Ser Pro Gly Ala Leu Tyr Ala Thr Gln Met Tyr Phe Glu Leu His
    610                 615                 620

Asn Val Leu Gly Leu Asp Ala Gly Ile Tyr Tyr Tyr His Pro Val Thr
625                 630                 635                 640

His Lys Leu Ile Lys Ile Ser Thr Leu Ser Arg Arg Gln Met Pro Thr
                645                 650                 655

Ile Lys Val His Phe Ile Gly Lys His Glu Ala Ile Glu Pro Val Tyr
            660                 665                 670

Lys Asn Asn Ile Gln Glu Val Leu Glu Met Glu Ala Gly His Met Met
        675                 680                 685

Gly Leu Phe Asp Asp Val Leu Pro Glu Ile Gly Leu Ser Ile Gly Lys
    690                 695                 700

Ser Glu Tyr Gln Asp Glu Cys Pro Asp Trp Tyr Asp Gly Asp Ile Gln
705                 710                 715                 720

Asp Tyr Tyr Leu Gly Ala Phe Glu Ile Cys Ser Tyr Glu His Gly Leu
                725                 730                 735

Pro Pro Phe Glu Thr Asp Ile Tyr Leu Gln Thr His Ala His Lys Ile
            740                 745                 750

Pro Glu Met Pro Cys Gly Leu Tyr His Phe Ser Asn Gly Glu Phe Val
        755                 760                 765

Arg Ile Ser Asp Asp Ile Val Arg Lys Lys Asp Val Ile Ala Ile Asn
    770                 775                 780

Gln Gln Val Tyr Asp Arg Ser Ser Phe Gly Val Ser Ile Ile Pro Arg
785                 790                 795                 800

Cys Val Pro Glu Trp His Tyr Tyr Ile Thr Leu Gly Arg Arg Leu His
                805                 810                 815

Ala Leu Gln Ser Asn Pro Leu Tyr Ile Gly Leu Met Ser Ser Gly Tyr
            820                 825                 830

Ser Ser Lys Ser Asn Asn Asp Leu Pro Ser Ala Lys Arg Met Arg Ser
        835                 840                 845

Ile Leu Asn Ala Leu Asp Arg Pro Met Ala Ala Phe Tyr Phe Cys Ile
    850                 855                 860

Gly Gly Gly Ile Ser Gln Ala Gln Tyr Met Cys Glu Gly Met Lys Glu
865                 870                 875                 880

Asp Val Val His Met Lys Gly Pro Val Glu Ile Ile Lys Asp Asp Leu
                885                 890                 895

Gln Gln Gln Leu Pro Gln Tyr Met Ile Pro Asn Lys Val Leu Val Phe
            900                 905                 910

Asp Lys Leu Pro Leu Thr Ala Asn Gly Lys Val Asp Tyr Gln Ser Leu
        915                 920                 925

Ser Glu Ser Lys Ala Val Glu Asn Val Ser Thr Gln Arg Leu Leu Val
    930                 935                 940

Pro Leu His Thr Asp Thr Glu Ile Arg Leu Gly Lys Ile Trp Met Glu
945                 950                 955                 960

Val Leu Lys Trp Asp Ser Val Ser Ala Leu Asp Asp Phe Phe Glu Ser

                965                 970                 975

Gly Gly Asn Ser Leu Met Ala Val Ala Met Val Asn Lys Ile Asn Ala
            980                 985                 990

Ala Phe Asn Ile Arg Phe Pro Leu Gln Ile Leu Phe Gln Ser Pro Asn
        995                 1000                1005

Ile Ala Glu Leu Ala Lys Trp Ile Glu Gln Thr Asp Ser Lys Thr
    1010                1015                1020

Ile Ser Arg Leu Ile Leu Leu Asn Gln Ala Ser Lys Asp Pro Ile
    1025                1030                1035

Tyr Cys Trp Pro Gly Leu Gly Gly Tyr Pro Met Ser Leu Arg Leu
    1040                1045                1050

Leu Ala Asn Lys Val Val Pro Asp Arg Ala Phe Tyr Gly Ile Gln
    1055                1060                1065

Ala Tyr Gly Ile Asn Glu Ser Glu Ile Pro Phe Ser Ser Ile Gln
    1070                1075                1080

Arg Met Ala Glu Glu Asp Ile Lys Glu Ile Lys Lys Ile Gln Pro
    1085                1090                1095

Glu Gly Pro Tyr Ile Leu Trp Gly Tyr Ser Phe Gly Ala Arg Val
    1100                1105                1110

Ala Phe Glu Val Ala Tyr Gln Leu Glu Gln Ala Gly Glu Glu Val
    1115                1120                1125

Asn Ala Leu Asn Leu Leu Ala Pro Gly Ser Pro His Leu Asp Met
    1130                1135                1140

Lys Gln Ala Glu Tyr Met Asp Lys Gly Ala Glu Phe Thr Asn Pro
    1145                1150                1155

Ala Phe Val Lys Ile Leu Phe Ser Val Phe Ser Arg Ser Ile Asn
    1160                1165                1170

Ser Pro Met Val Lys Thr Cys Leu Glu Gln Val Asn Ser Glu Thr
    1175                1180                1185

Thr Phe Ile Asn Phe Ile Cys Ser Arg Phe Lys Asn Leu Glu Pro
    1190                1195                1200

Ser Leu Val Lys Arg Ile Val Arg Ile Val Thr Leu Thr Tyr Asp
    1205                1210                1215

Phe Lys Tyr Ser Ile Asp Glu Leu Tyr His Arg His Leu Lys Ala
    1220                1225                1230

Pro Ile Thr Ile Phe Lys Ala Asn Arg Asp Asn Asp Ser Phe Ile
    1235                1240                1245

Glu Glu Ser Asp Val Ile Ser Ser Met Ser Pro Lys Ile Ile Glu
    1250                1255                1260

Leu Ile Ser Asp His Tyr Gln Leu Leu Glu Ser Glu Gly Val Ala
    1265                1270                1275

Glu Ile Glu Lys Ile Ile
    1280

<210> 26
<211> 4776
<212> DNA
<213> Artificial Sequence

<220>
<223> minimal construct C(of TycC2)-Ind

<400> 26
tcggaaaaaa ccgagtacac cgcgattcaa cccgtggcag cgcaggagtt ttacccggtt 60

tcatctgcgc aaaaaagaat gtatatcctg caacagttcg aaggcaacgg aatcagctac 120

aacatttcgg gtgcgattct cctggaagga aagctggact acgcccggtt tgccagcgct 180

gtgcaacagc tggcagagcg ccacgaagct ttgcgcacct cgttccaccg gatcgacggc 240

gagcctgtgc aaaaagtgca cgaggaagta gaagtgccgc ttttcatgct ggaggctccc 300

gaagaccagg cggagaaaat catgcgcgag tttgtccgtc cgtttgatct cggggtcgct 360

ccgctgatgc gaacaggttt gctcaagctg ggcaaagacc gccatttgtt tttgctcgac 420

atgcaccata tcatctcgga cggcgtttct tcgcaaattt tgctgcgtga atttgccgag 480

ttgtaccagg gagcagactt gcagccgctt tcgctgcaat acaaagattt cgctgcttgg 540

caaaatgagc tgtttcagac ggaggcatac aagaagcagg agcagcactg gctgaacacg 600

tttgctgatg aaattccgct cttgaacctg ccgactgact atccgcgccc tagcgtgcaa 660

agctttgcag gcgatctcgt cctttttgcc gccggaaaag aactgctgga gcggttgcaa 720

caggtagcgt cagaaacagg caccaccttg tacatgattt tgcttgccgc ctacaatgtg 780

ctgctgtcca agtataccgg ccaggaagac atcatcgtcg ggacgcctgt cgctggacgt 840

tcccatgcgg acgtggaaaa catcatgggc atattcgtga acacattggc gctgcgcaac 900

cagcctgcca gcagcaaaac gatgttagaa aataatatta cacaatgtga ctcaatcaat 960

gatgtttatc ttaaagaaga agcaataaca ttgatggata tgcttgagag tcaacttaag 1020

caccaggcag atggatatgt tgttattgat caagaagaat ctctcagtta cgctgatttc 1080

tatttgaggg tgaaagagat agggtattgt ctgtcagaaa ttagctcaaa gagttcggtg 1140

ggtattgggc ttttttgtga tccttctata gatttaattt gtggtgcatg gggtattttg 1200

tcagcggata aagcttattt gccgttatcg cctgactatc caactgaacg cctcaaatat 1260

atgatagaag attctggtat tgatgtgatt tttacgcaat cgcacttaaa agcacagcta 1320

caggacattg caccaaaatc agtattaatt atgacaccag aagatgtcgc tctgacgata 1380

aaaacacgaa caatagaaga tattctgggc acagttcaag ttcctaaacc cacgagtctg 1440

gcttatatta tttatacctc tggtagcacg ggtaagccaa agggagtgat gattgaacat 1500

cacagtattg taaatcaaat gagatttctt gcaaaagcgt tcaaattagg atgtcattcc 1560

cggattttac agaaaacacc aatgagtttt gatgcggctc aatgggaaat tctagcgcct 1620

gcaattggtg gtcaagtgat tatgggtcct ttaggttgct atcgcgatcc ggatgcaatt 1680

attaaaacca ttcttcagca tcaagtaacg actttgcaat gtgttcctac tttgctacaa 1740

gcgttactgg ataatcctaa ttttttggat tgcttatcat tgactcaagt attcagtggg 1800

ggagaagcgc tgacaaccaa attagccacg caatttttga atagttttac tcactgtgaa 1860

ttaatcaatt tatatggccc gacagaatgt acgattaatt catcattttt ccgggtgaca 1920

aatgagactt tgccgaatta tcaaacctct atttcgattg gtgcacctgt agataatacc 1980

gaatactacg ttcttgatga tgatagatta cctgtggcgg ttggcgaaat tggcgagctt 2040

tatatttcgg gtgctcaatt agcacgtggt tatttgcata aaccagaaat gacaaaagat 2100

aaatttattt gtaatcacct tgtatcagga actcaacatc aatggttata tcgaacggga 2160

gatctggtaa ccagaggggc tgatggtaat acttattttg ttggtcgggt tgatagccag 2220

gtcaaattac gaggttaccg tattgagctt gatgaaatac gccatgcgat tgaagaacat 2280

agctggataa agacggcggc aatgttaatt aagaaggatg ccagaacggg tttccaaaat 2340

ctcatcgcgt gtgtggaatt agatgagaaa gaagctgcat tgatggatca aggtaatagt 2400

agctcacatc acaaatcaaa agccgataaa ctacaggtga aagcccaact ttctaattct 2460

ggttgtcgaa gtgaagagtt atgtgaaaat cgccctacat tcttacttcc ttatcaagaa 2520

ggggagataa aacagagaga atatgcattt ggacgcaaga catatcgcta ttttgaggga 2580

acagaaataa cggtagagaa attaaaaaaa ttgctgacag ccactcaatc gaatgaaatt 2640

agctctttgc cactgagtca tctaaccctg aatgatttcg gttatgcatt gcgttatttt 2700

ggtcagttta ccagccatca acgtttattg cccaaatatg cctatgcttc accgggtgct 2760

ctctatgcga cacaaatgta ttttgaattg cataatgttc tcggtttgga tgcggggatt 2820

tactattatc atccagtgac acataagtta ataaaaattt caacattgag tcgtcggcaa 2880

atgccaacga taaaagtgca ttttattggc aagcatgaag ccattgagcc cgtttataag 2940

aacaatatac aagaagttct ggaaatggaa gcgggccata tgatgggtct ttttgatgac 3000

gtattaccgg aaattggctt gagtattggt aaaagtgaat atcaagatga atgtccagat 3060

tggtatgatg gtgatattca ggattattat cttggtgcat ttgaaatatg tagctatgaa 3120

catggattgc cgccatttga gactgatatt tatttacaaa cacatgccca taaaatacct 3180

gagatgccgt gtggtttata tcacttttct aacggggaat ttgtacgaat aagtgatgat 3240

attgtccgaa aaaaggatgt tattgcgatt aatcagcaag tttatgatcg ctccagtttt 3300

ggcgtgtcaa ttattccacg ctgtgtccct gaatggcatt attatataac actgggtcgt 3360

cggttacatg cgttacaaag taatccattg tatattggat taatgtcatc tggttacagt 3420

tcgaagagca ataacgattt accttcggcg aaaaggatgc gatctattct caatgcactt 3480

gatagaccta tggcggcatt ttatttctgc ataggtgggg gtattagcca agcgcaatat 3540

atgtgtgaag gcatgaaaga agatgttgtt catatgaaag ggccagttga aatcattaaa 3600

gatgatcttc aacaacaact ccctcaatat atgattccaa ataaggtatt agttttcgat 3660

aaattacctt tgacggccaa tggaaaagtg gattatcaat ctttatcaga atctaaagcc 3720

gtggagaatg tttcaacaca gcgtctattg gtgccattac atacagatac tgaaataagg 3780

cttggaaaaa tttggatgga agtactgaaa tgggattcag tatctgccct cgatgatttt 3840

ttcgaaagtg ggggtaattc tttgatggcc gttgcaatgg ttaataagat caatgcggcc 3900

tttaatattc gttttccgtt acagatactt tttcaatctc ctaatatagc agaattggct 3960

aagtggattg aacagacaga ctctaaaaca atatcaagat taattttatt gaatcaggca 4020

agcaaagacc ccatttactg ttggccgggt ttgggcggat atcctatgag tttgagattg 4080

cttgctaata aagtcgttcc tgatcgggca ttttatggaa tacaggcata tgggataaac 4140

gagagtgaaa taccgttttc ttctatccag agaatggcag aagaggatat taaagagata 4200

aagaaaatac agccagaagg gccatatata ttgtggggat attcatttgg tgcccgagta 4260

gcatttgaag ttgcatacca gcttgaacaa gcgggagaag aagttaacgc attgaattta 4320

ttggctccgg gatctcctca tcttgatatg aagcaagcgg aatatatgga taaaggcgct 4380

gaatttacta atccggcttt tgttaaaata cttttttctg tattttctcg ttcaatcaac 4440

agcccaatgg ttaaaacttg cttagaacaa gtaaatagtg aaacgacatt tattaacttt 4500

atatgtagtc gttttaaaaa cttggaacca tcattagtaa aacgtatcgt taggattgtg 4560

actttgactt atgatttcaa gtacagtatt gatgagcttt atcacagaca cctaaaggca 4620

cctataacta ttttcaaggc gaatagagat aatgattcat ttatcgagga atcggatgtg 4680

atttcatcaa tgtcgcctaa aataattgaa ttaatatcgg atcactatca actgttggaa 4740

agtgaaggtg ttgctgagat tgagaaaata atctaa 4776

<210> 27
<211> 2327
<212> PRT
<213> Artificial Sequence

<220>
<223> NRPSase of a fusion peptide consisting of Asparagine and
      Indigoidine

<400> 27

Met Gln Thr Asn Lys Gln Gln Thr Phe Ser Glu Leu Leu Gln Thr Val
1               5                   10                  15

Gln Lys Gln Ala Leu Ala Ser Ala Thr Tyr Asp Phe Ala Pro Leu Tyr
            20                  25                  30

Glu Ile Gln Ser Thr Thr Val Leu Lys Gln Glu Leu Ile Asp His Leu
        35                  40                  45

Val Thr Phe Glu Asn Tyr Pro Asp His Ser Met Lys His Leu Glu Glu
    50                  55                  60

Ser Leu Gly Phe Gln Phe Thr Val Glu Ser Gly Asp Glu Gln Thr Ser
65                  70                  75                  80

Tyr Asp Leu Asn Val Val Val Ala Leu Ala Pro Ser Asn Glu Leu Tyr
                85                  90                  95

Val Lys Leu Ser Tyr Asn Ala Ala Val Tyr Glu Ser Ser Phe Val Asn
            100                 105                 110

Arg Ile Glu Gly His Leu Arg Thr Val Ile Asp Gln Val Ile Gly Asn
        115                 120                 125

Pro His Val His Leu His Glu Ile Gly Ile Ile Thr Glu Glu Glu Lys
    130                 135                 140

Gln Gln Leu Leu Val Ala Tyr Asn Asp Thr Ala Ala Glu Tyr Pro Arg
145                 150                 155                 160

Asp Lys Thr Ile Phe Glu Leu Ile Ala Glu Gln Ala Ser Arg Thr Pro
                165                 170                 175

Ala Lys Ala Ala Val Val Cys Gly Glu Asp Thr Leu Thr Tyr Gln Glu
            180                 185                 190

Leu Met Glu Arg Ser Ala Gln Leu Ala Asn Ala Leu Arg Glu Lys Gly
        195                 200                 205

Ile Ala Ser Gly Ser Ile Val Ser Ile Met Ala Glu His Ser Leu Glu
    210                 215                 220

Leu Ile Val Ala Ile Met Ala Val Leu Arg Ser Gly Ala Ala Tyr Leu
225                 230                 235                 240

Pro Ile Asp Pro Glu Tyr Pro Gln Asp Arg Ile Gln Tyr Leu Leu Asp
                245                 250                 255

Asp Ser Gln Thr Thr Leu Leu Leu Thr Gln Ser His Leu Gln Pro Asn
            260                 265                 270

Ile Arg Phe Ala Gly Ser Val Leu Tyr Leu Asp Asp Arg Ser Leu Tyr
        275                 280                 285

Glu Gly Gly Ser Thr Ser Phe Ala Pro Glu Ser Lys Pro Asp Asp Leu
    290                 295                 300

Ala Tyr Met Ile Tyr Thr Ser Gly Ser Thr Gly Asn Pro Lys Gly Ala
305                 310                 315                 320

Met Ile Thr His Gln Gly Leu Val Asn Tyr Ile Trp Trp Ala Asn Lys
                325                 330                 335

Val Tyr Val Gln Gly Glu Ala Val Asp Phe Pro Leu Tyr Ser Ser Ile
            340                 345                 350

Ser Phe Asp Leu Thr Val Thr Ser Ile Phe Thr Pro Leu Leu Ser Gly
        355                 360                 365

Asn Thr Ile His Val Tyr Arg Gly Ala Asp Lys Val Gln Val Ile Leu
    370                 375                 380

Asp Ile Ile Lys Asp Asn Lys Val Gly Ile Ile Lys Leu Thr Pro Thr
385                 390                 395                 400

His Leu Lys Leu Ile Glu His Ile Asp Gly Lys Ala Ser Ser Ile Arg
                405                 410                 415

Arg Phe Ile Val Gly Gly Glu Asn Leu Pro Thr Lys Leu Ala Lys Gln
            420                 425                 430

Ile Tyr Asp His Phe Gly Glu Asn Val Gln Ile Phe Asn Glu Tyr Gly
        435                 440                 445

Pro Thr Glu Thr Val Val Gly Cys Met Ile Tyr Leu Tyr Asp Pro Gln
    450                 455                 460

Thr Thr Thr Gln Glu Ser Val Pro Ile Gly Val Pro Ala Asp Asn Val
465                 470                 475                 480

Gln Leu Tyr Leu Leu Asp Ala Ser Met Gln Pro Val Pro Val Gly Ser
                485                 490                 495

Leu Gly Glu Met Tyr Ile Ala Gly Asp Gly Val Ala Lys Gly Tyr Phe
            500                 505                 510

Asn Arg Pro Glu Leu Thr Lys Glu Lys Phe Ile Asp Asn Pro Phe Arg
        515                 520                 525

Pro Gly Thr Lys Met Tyr Arg Thr Gly Asp Leu Ala Lys Trp Leu Pro
    530                 535                 540

Asp Gly Asn Met Glu Tyr Ala Gly Arg Met Asp Tyr Gln Val Lys Ile
545                 550                 555                 560

Arg Gly His Arg Ile Glu Met Gly Glu Ile Glu Thr Arg Leu Thr Gln
                565                 570                 575

His Glu Ala Val Lys Glu Ala Val Val Ile Val Glu Lys Asp Glu Ser
            580                 585                 590

Gly Gln Asn Val Leu Tyr Ala Tyr Leu Val Ser Glu Arg Glu Leu Thr
        595                 600                 605

Val Ala Glu Leu Arg Glu Phe Leu Gly Arg Thr Leu Pro Ser Tyr Met
    610                 615                 620

Ile Pro Ser Phe Phe Ile Arg Leu Ala Glu Ile Pro Leu Thr Ala Asn
625                 630                 635                 640

Gly Lys Val Glu Arg Lys Lys Leu Pro Lys Pro Ala Gly Ala Val Val
                645                 650                 655

Thr Gly Thr Ala Tyr Ala Ala Pro Gln Asn Glu Ile Glu Ala Lys Leu
            660                 665                 670

Ala Glu Ile Trp Gln Gln Val Leu Gly Ile Ser Gln Val Gly Ile His
        675                 680                 685

Asp Asp Phe Phe Asp Leu Gly Gly His Ser Leu Lys Ala Met Thr Val

690                 695                 700

Val Phe Gln Val Ser Lys Ala Leu Glu Val Glu Leu Pro Val Lys Ala
705                 710                 715                 720

Leu Phe Glu His Pro Thr Val Ala Glu Leu Ala Arg Phe Leu Ser Arg
                725                 730                 735

Ser Glu Lys Thr Glu Tyr Thr Ala Ile Gln Pro Val Ala Ala Gln Glu
            740                 745                 750

Phe Tyr Pro Val Ser Ser Ala Gln Lys Arg Met Tyr Ile Leu Gln Gln
        755                 760                 765

Phe Glu Gly Asn Gly Ile Ser Tyr Asn Ile Ser Gly Ala Ile Leu Leu
    770                 775                 780

Glu Gly Lys Leu Asp Tyr Ala Arg Phe Ala Ser Ala Val Gln Gln Leu
785                 790                 795                 800

Ala Glu Arg His Glu Ala Leu Arg Thr Ser Phe His Arg Ile Asp Gly
                805                 810                 815

Glu Pro Val Gln Lys Val His Glu Glu Val Glu Val Pro Leu Phe Met
            820                 825                 830

Leu Glu Ala Pro Glu Asp Gln Ala Glu Lys Ile Met Arg Glu Phe Val
        835                 840                 845

Arg Pro Phe Asp Leu Gly Val Ala Pro Leu Met Arg Thr Gly Leu Leu
    850                 855                 860

Lys Leu Gly Lys Asp Arg His Leu Phe Leu Leu Asp Met His His Ile
865                 870                 875                 880

Ile Ser Asp Gly Val Ser Ser Gln Ile Leu Leu Arg Glu Phe Ala Glu
                885                 890                 895

Leu Tyr Gln Gly Ala Asp Leu Gln Pro Leu Ser Leu Gln Tyr Lys Asp
            900                 905                 910

Phe Ala Ala Trp Gln Asn Glu Leu Phe Gln Thr Glu Ala Tyr Lys Lys
        915                 920                 925

Gln Glu Gln His Trp Leu Asn Thr Phe Ala Asp Glu Ile Pro Leu Leu
    930                 935                 940

Asn Leu Pro Thr Asp Tyr Pro Arg Pro Ser Val Gln Ser Phe Ala Gly
945                 950                 955                 960

Asp Leu Val Leu Phe Ala Ala Gly Lys Glu Leu Leu Glu Arg Leu Gln
                965                 970                 975

Gln Val Ala Ser Glu Thr Gly Thr Thr Leu Tyr Met Ile Leu Leu Ala
            980                 985                 990

Ala Tyr Asn Val Leu Leu Ser Lys Tyr Thr Gly Gln Glu Asp Ile Ile
        995                 1000                1005

Val Gly Thr Pro Val Ala Gly Arg Ser His Ala Asp Val Glu Asn
    1010                1015                1020

Ile Met Gly Ile Phe Val Asn Thr Leu Ala Leu Arg Asn Gln Pro
    1025                1030                1035

Ala Ser Ser Lys Thr Met Leu Glu Asn Asn Ile Thr Gln Cys Asp
    1040                1045                1050

Ser Ile Asn Asp Val Tyr Leu Lys Glu Glu Ala Ile Thr Leu Met
    1055                1060                1065

Asp Met Leu Glu Ser Gln Leu Lys His Gln Ala Asp Gly Tyr Val
    1070                1075                1080

Val Ile Asp Gln Glu Glu Ser Leu Ser Tyr Ala Asp Phe Tyr Leu
    1085                1090                1095

Arg Val Lys Glu Ile Gly Tyr Cys Leu Ser Glu Ile Ser Ser Lys
    1100                1105                1110

Asn Ser Val Gly Ile Gly Leu Phe Cys Asp Pro Ser Ile Asp Leu
    1115                1120                1125

Ile Cys Gly Ala Trp Gly Ile Leu Ser Ala Asp Lys Ala Tyr Leu
    1130                1135                1140

Pro Leu Ser Pro Asp Tyr Pro Thr Glu Arg Leu Lys Tyr Met Ile
    1145                1150                1155

Glu Asp Ser Gly Ile Asp Val Ile Phe Thr Gln Ser His Leu Lys
    1160                1165                1170

Ala Gln Leu Gln Asp Ile Ala Pro Lys Ser Val Leu Ile Met Thr
    1175                1180                1185

Pro Glu Asp Val Ala Leu Thr Ile Lys Thr Arg Thr Ile Glu Asp
    1190                1195                1200

Ile Leu Gly Thr Val Gln Val Pro Lys Pro Thr Ser Leu Ala Tyr
    1205                1210                1215

Ile Ile Tyr Thr Ser Gly Ser Thr Gly Lys Pro Lys Gly Val Met
    1220                1225                1230

Ile Glu His His Ser Ile Val Asn Gln Met Arg Phe Leu Ala Lys
    1235                1240                1245

Ala Phe Lys Leu Gly Cys His Ser Arg Ile Leu Gln Lys Thr Pro
    1250                1255                1260

Met Ser Phe Asp Ala Ala Gln Trp Glu Ile Leu Ala Pro Ala Ile
    1265                1270                1275

Gly Gly Gln Val Ile Met Gly Pro Leu Gly Cys Tyr Arg Asp Pro
    1280                1285                1290

Asp Ala Ile Ile Lys Thr Ile Leu Gln His Gln Val Thr Thr Leu
    1295                1300                1305

Gln Cys Val Pro Thr Leu Leu Gln Ala Leu Leu Asp Asn Pro Asn
    1310                1315                1320

Phe Leu Asp Cys Leu Ser Leu Thr Gln Val Phe Ser Gly Gly Glu
    1325                1330                1335

Ala Leu Thr Thr Lys Leu Ala Thr Gln Phe Leu Asn Ser Phe Thr
    1340                1345                1350

His Cys Glu Leu Ile Asn Leu Tyr Gly Pro Thr Glu Cys Thr Ile
    1355                1360                1365

Asn Ser Ser Phe Phe Arg Val Thr Asn Glu Thr Leu Pro Asn Tyr
    1370                1375                1380

Gln Thr Ser Ile Ser Ile Gly Ala Pro Val Asp Asn Thr Glu Tyr
    1385                1390                1395

Tyr Val Leu Asp Asp Asp Arg Leu Pro Val Ala Val Gly Glu Ile
    1400                1405                1410

Gly Glu Leu Tyr Ile Ser Gly Ala Gln Leu Ala Arg Gly Tyr Leu
    1415                1420                1425

His Lys Pro Glu Met Thr Lys Asp Lys Phe Ile Cys Asn His Leu
    1430                1435                1440

Val Ser Gly Thr Gln His Gln Trp Leu Tyr Arg Thr Gly Asp Leu
    1445                1450                1455

Val Thr Arg Gly Ala Asp Gly Asn Thr Tyr Phe Val Gly Arg Val
    1460                1465                1470

Asp Ser Gln Val Lys Leu Arg Gly Tyr Arg Ile Glu Leu Asp Glu
    1475                1480                1485

Ile Arg His Ala Ile Glu Glu His Ser Trp Ile Lys Thr Ala Ala
    1490                1495                1500

Met Leu Ile Lys Lys Asp Ala Arg Thr Gly Phe Gln Asn Leu Ile
    1505                1510                1515

Ala Cys Val Glu Leu Asp Glu Lys Glu Ala Ala Leu Met Asp Gln
    1520                1525                1530

Gly Asn Ser Ser Ser His His Lys Ser Lys Ala Asp Lys Leu Gln
    1535                1540                1545

Val Lys Ala Gln Leu Ser Asn Ser Gly Cys Arg Ser Glu Glu Leu
    1550                1555                1560

Cys Glu Asn Arg Pro Thr Phe Leu Leu Pro Tyr Gln Glu Gly Glu
    1565                1570                1575

Ile Lys Gln Arg Glu Tyr Ala Phe Gly Arg Lys Thr Tyr Arg Tyr
    1580                1585                1590

Phe Glu Gly Thr Glu Ile Thr Val Glu Lys Leu Lys Lys Leu Leu
    1595                1600                1605

Thr Ala Thr Gln Ser Asn Glu Ile Ser Ser Leu Pro Leu Ser His
    1610                1615                1620

Leu Thr Leu Asn Asp Phe Gly Tyr Ala Leu Arg Tyr Phe Gly Gln
    1625                1630                1635

Phe Thr Ser His Gln Arg Leu Leu Pro Lys Tyr Ala Tyr Ala Ser
    1640                1645                1650

Pro Gly Ala Leu Tyr Ala Thr Gln Met Tyr Phe Glu Leu His Asn

1655                1660                1665

Val Leu Gly Leu Asp Ala Gly Ile Tyr Tyr Tyr His Pro Val Thr
    1670                1675                1680

His Lys Leu Ile Lys Ile Ser Thr Leu Ser Arg Arg Gln Met Pro
    1685                1690                1695

Thr Ile Lys Val His Phe Ile Gly Lys His Glu Ala Ile Glu Pro
    1700                1705                1710

Val Tyr Lys Asn Asn Ile Gln Glu Val Leu Glu Met Glu Ala Gly
    1715                1720                1725

His Met Met Gly Leu Phe Asp Asp Val Leu Pro Glu Ile Gly Leu
    1730                1735                1740

Ser Ile Gly Lys Ser Glu Tyr Gln Asp Glu Cys Pro Asp Trp Tyr
    1745                1750                1755

Asp Gly Asp Ile Gln Asp Tyr Tyr Leu Gly Ala Phe Glu Ile Cys
    1760                1765                1770

Ser Tyr Glu His Gly Leu Pro Pro Phe Glu Thr Asp Ile Tyr Leu
    1775                1780                1785

Gln Thr His Ala His Lys Ile Pro Glu Met Pro Cys Gly Leu Tyr
    1790                1795                1800

His Phe Ser Asn Gly Glu Phe Val Arg Ile Ser Asp Asp Ile Val
    1805                1810                1815

Arg Lys Lys Asp Val Ile Ala Ile Asn Gln Gln Val Tyr Asp Arg
    1820                1825                1830

Ser Ser Phe Gly Val Ser Ile Ile Pro Arg Cys Val Pro Glu Trp
    1835                1840                1845

His Tyr Tyr Ile Thr Leu Gly Arg Arg Leu His Ala Leu Gln Ser
    1850                1855                1860

Asn Pro Leu Tyr Ile Gly Leu Met Ser Ser Gly Tyr Ser Ser Lys
    1865                1870                1875

Ser Asn Asn Asp Leu Pro Ser Ala Lys Arg Met Arg Ser Ile Leu
    1880                1885                1890

Asn Ala Leu Asp Arg Pro Met Ala Ala Phe Tyr Phe Cys Ile Gly
    1895                1900                1905

Gly Gly Ile Ser Gln Ala Gln Tyr Met Cys Glu Gly Met Lys Glu
    1910                1915                1920

Asp Val Val His Met Lys Gly Pro Val Glu Ile Ile Lys Asp Asp
    1925                1930                1935

Leu Gln Gln Gln Leu Pro Gln Tyr Met Ile Pro Asn Lys Val Leu
    1940                1945                1950

Val Phe Asp Lys Leu Pro Leu Thr Ala Asn Gly Lys Val Asp Tyr
    1955                1960                1965

Gln Ser Leu Ser Glu Ser Lys Ala Val Glu Asn Val Ser Thr Gln
    1970                1975                1980

Arg Leu Leu Val Pro Leu His Thr Asp Thr Glu Ile Arg Leu Gly
    1985                1990                1995

Lys Ile Trp Met Glu Val Leu Lys Trp Asp Ser Val Ser Ala Leu
    2000                2005                2010

Asp Asp Phe Phe Glu Ser Gly Gly Asn Ser Leu Met Ala Val Ala
    2015                2020                2025

Met Val Asn Lys Ile Asn Ala Ala Phe Asn Ile Arg Phe Pro Leu
    2030                2035                2040

Gln Ile Leu Phe Gln Ser Pro Asn Ile Ala Glu Leu Ala Lys Trp
    2045                2050                2055

Ile Glu Gln Thr Asp Ser Lys Thr Ile Ser Arg Leu Ile Leu Leu
    2060                2065                2070

Asn Gln Ala Ser Lys Asp Pro Ile Tyr Cys Trp Pro Gly Leu Gly
    2075                2080                2085

Gly Tyr Pro Met Ser Leu Arg Leu Leu Ala Asn Lys Val Val Pro
    2090                2095                2100

Asp Arg Ala Phe Tyr Gly Ile Gln Ala Tyr Gly Ile Asn Glu Ser
    2105                2110                2115

Glu Ile Pro Phe Ser Ser Ile Gln Arg Met Ala Glu Glu Asp Ile
    2120                2125                2130

Lys Glu Ile Lys Lys Ile Gln Pro Glu Gly Pro Tyr Ile Leu Trp
    2135                2140                2145

Gly Tyr Ser Phe Gly Ala Arg Val Ala Phe Glu Val Ala Tyr Gln
    2150                2155                2160

Leu Glu Gln Ala Gly Glu Glu Val Asn Ala Leu Asn Leu Leu Ala
    2165                2170                2175

Pro Gly Ser Pro His Leu Asp Met Lys Gln Ala Glu Tyr Met Asp
    2180                2185                2190

Lys Gly Ala Glu Phe Thr Asn Pro Ala Phe Val Lys Ile Leu Phe
    2195                2200                2205

Ser Val Phe Ser Arg Ser Ile Asn Ser Pro Met Val Lys Thr Cys
    2210                2215                2220

Leu Glu Gln Val Asn Ser Glu Thr Thr Phe Ile Asn Phe Ile Cys
    2225                2230                2235

Ser Arg Phe Lys Asn Leu Glu Pro Ser Leu Val Lys Arg Ile Val
    2240                2245                2250

Arg Ile Val Thr Leu Thr Tyr Asp Phe Lys Tyr Ser Ile Asp Glu
    2255                2260                2265

Leu Tyr His Arg His Leu Lys Ala Pro Ile Thr Ile Phe Lys Ala
    2270                2275                2280

Asn Arg Asp Asn Asp Ser Phe Ile Glu Glu Ser Asp Val Ile Ser
    2285                2290                2295

Ser Met Ser Pro Lys Ile Ile Glu Leu Ile Ser Asp His Tyr Gln
    2300                2305                2310

Leu Leu Glu Ser Glu Gly Val Ala Glu Ile Glu Lys Ile Ile
    2315                2320                2325

<210>  28
<211>  3221
<212>  PRT
<213>  Artificial Sequence

<220>
<223>  NRPSase synthesizing a Indigoidine-tagged Dipeptide consisting of
       Ornithine and Valine

<400>  28

Met Leu His Ser Phe Leu Ala Thr Lys Thr Ala Tyr Pro Thr Asp Lys
1               5                   10                  15

Thr Phe Gln Lys Leu Phe Glu Glu Gln Val Glu Lys Thr Pro Asn Glu
            20                  25                  30

Ile Ala Val Leu Phe Gly Asn Glu Gln Leu Thr Tyr Gln Glu Leu Asn
        35                  40                  45

Ala Lys Ala Asn Gln Leu Ala Arg Val Leu Arg Arg Lys Gly Val Lys
    50                  55                  60

Pro Glu Ser Thr Val Gly Ile Leu Val Asp Arg Ser Leu Tyr Met Val
65                  70                  75                  80

Ile Gly Met Leu Ala Val Leu Lys Ala Gly Gly Thr Phe Val Pro Ile
                85                  90                  95

Asp Pro Asp Tyr Pro Leu Glu Arg Gln Ala Phe Met Leu Glu Asp Ser
            100                 105                 110

Glu Ala Lys Leu Leu Leu Thr Leu Gln Lys Met Asn Ser Gln Val Ala
        115                 120                 125

Phe Pro Tyr Glu Thr Phe Tyr Leu Asp Thr Glu Thr Val Asp Gln Glu
    130                 135                 140

Glu Thr Gly Asn Leu Glu His Val Ala Gln Pro Glu Asn Val Ala Tyr
145                 150                 155                 160

Ile Ile Tyr Thr Ser Gly Thr Thr Gly Lys Pro Lys Gly Val Val Ile
                165                 170                 175

Glu His Arg Ser Tyr Ala Asn Val Ala Phe Ala Trp Lys Asp Glu Tyr
            180                 185                 190

His Leu Asp Ser Phe Pro Val Arg Leu Leu Gln Met Ala Ser Phe Ala
        195                 200                 205

Phe Asp Val Ser Thr Gly Asp Phe Ala Arg Ala Leu Leu Thr Gly Gly
    210                 215                 220

Gln Leu Val Ile Cys Pro Asn Gly Val Lys Met Asp Pro Ala Ser Leu
225                 230                 235                 240

Tyr Glu Thr Ile Arg Arg His Glu Ile Thr Ile Phe Glu Ala Thr Pro
                245                 250                 255

Ala Leu Ile Met Pro Leu Met His Tyr Val Tyr Glu Asn Glu Leu Asp
        260                 265                 270

Met Ser Gln Met Lys Leu Leu Ile Leu Gly Ala Asp Ser Cys Pro Ala
    275                 280                 285

Glu Asp Phe Lys Thr Leu Leu Ala Arg Phe Gly Gln Lys Met Arg Ile
    290                 295                 300

Ile Asn Ser Tyr Gly Val Thr Glu Ala Cys Ile Asp Thr Ser Tyr Tyr
305                 310                 315                 320

Glu Glu Thr Asp Val Thr Ala Ile Arg Ser Gly Thr Val Pro Ile Gly
                325                 330                 335

Lys Pro Leu Pro Asn Met Thr Met Tyr Val Val Asp Ala His Leu Asn
        340                 345                 350

Leu Gln Pro Val Gly Val Val Gly Glu Leu Cys Ile Gly Gly Ala Gly
    355                 360                 365

Val Ala Arg Gly Tyr Leu Asn Arg Pro Glu Leu Thr Glu Glu Lys Phe
    370                 375                 380

Val Pro Asn Pro Phe Ala Pro Gly Glu Arg Leu Tyr Arg Thr Gly Asp
385                 390                 395                 400

Leu Ala Lys Trp Arg Ala Asp Gly Asn Val Glu Phe Leu Gly Arg Asn
                405                 410                 415

Asp His Gln Val Lys Ile Arg Gly Val Arg Ile Glu Leu Gly Glu Ile
        420                 425                 430

Glu Thr Gln Leu Arg Lys Leu Asp Gly Ile Thr Glu Ala Val Val Val
    435                 440                 445

Ala Arg Glu Asp Arg Gly Gln Glu Lys Glu Leu Cys Ala Tyr Val Val
    450                 455                 460

Ala Asp His Lys Leu Asp Thr Ala Glu Leu Arg Ala Asn Leu Leu Lys
465                 470                 475                 480

Glu Leu Pro Gln Ala Met Ile Pro Ala Tyr Phe Val Thr Leu Asp Ala
                485                 490                 495

Leu Pro Leu Thr Ala Asn Gly Lys Val Asp Arg Arg Ser Leu Pro Ala

500                 505                 510

Pro Asp Val Thr Met Leu Arg Thr Thr Glu Tyr Val Ala Pro Arg Ser
        515                 520                 525

Val Trp Glu Ala Arg Leu Ala Gln Val Trp Glu Gln Val Leu Asn Val
    530                 535                 540

Pro Gln Val Gly Ala Leu Asp Asp Phe Phe Ala Leu Gly Gly His Ser
545                 550                 555                 560

Leu Arg Ala Met Arg Val Leu Ser Ser Met His Asn Glu Tyr Gln Val
                565                 570                 575

Asp Ile Pro Leu Arg Ile Leu Phe Glu Lys Pro Thr Ile Gln Glu Leu
            580                 585                 590

Ala Ala Phe Ile Glu Thr Ser Gly Lys Glu Thr Tyr Val Pro Ile Glu
        595                 600                 605

Pro Ala Pro Leu Gln Glu Tyr Tyr Pro Val Ser Ser Ala Gln Lys Arg
    610                 615                 620

Met Tyr Val Leu Arg Gln Phe Ala Asp Thr Gly Thr Val Tyr Asn Met
625                 630                 635                 640

Pro Ser Ala Leu Tyr Ile Glu Gly Asp Leu Asp Arg Lys Arg Phe Glu
                645                 650                 655

Ala Ala Ile His Gly Leu Val Glu Arg His Glu Ser Leu Arg Thr Ser
            660                 665                 670

Phe His Thr Val Asn Gly Glu Pro Val Gln Arg Val His Glu His Val
        675                 680                 685

Glu Leu Asn Val Gln Tyr Ala Glu Val Thr Glu Ala Gln Val Glu Pro
    690                 695                 700

Thr Val Glu Ser Phe Val Gln Ala Phe Asp Leu Thr Lys Ala Pro Leu
705                 710                 715                 720

Leu Arg Val Gly Leu Phe Lys Leu Ala Ala Lys Arg His Leu Phe Leu
                725                 730                 735

Leu Asp Met His His Ile Ile Ser Asp Gly Val Ser Ala Gly Ile Ile
            740                 745                 750

Met Glu Glu Phe Ser Lys Leu Tyr Arg Gly Glu Glu Leu Pro Ala Leu
        755                 760                 765

Ser Val His Tyr Lys Asp Phe Ala Val Trp Gln Ser Glu Leu Phe Gln
    770                 775                 780

Ser Asp Val Tyr Thr Glu His Glu Asn Tyr Trp Leu Asn Ala Phe Ser
785                 790                 795                 800

Gly Asp Ile Pro Val Leu Asn Leu Pro Ala Asp Phe Ser Arg Pro Leu
                805                 810                 815

Thr Gln Ser Phe Glu Gly Asp Cys Val Ser Phe Gln Ala Asp Lys Ala
            820                 825                 830

Leu Leu Asp Asp Leu His Lys Leu Ala Gln Glu Ser Gln Ser Thr Leu
        835                 840                 845

Phe Met Val Leu Leu Ala Ala Tyr Asn Val Leu Leu Ala Lys Tyr Ser
    850                 855                 860

Gly Gln Glu Asp Ile Val Val Gly Thr Pro Ile Ala Gly Arg Ser His
865                 870                 875                 880

Ala Asp Ile Glu Asn Val Leu Gly Met Phe Val Asn Thr Leu Ala Leu
                885                 890                 895

Arg Asn Tyr Pro Val Glu Thr Lys His Phe Gln Ala Phe Leu Glu Glu
            900                 905                 910

Val Lys Gln Asn Thr Leu Gln Ala Tyr Ala His Gln Asp Tyr Pro Phe
        915                 920                 925

Glu Ala Leu Val Glu Lys Leu Asp Ile Gln Arg Asp Leu Ser Arg Asn
    930                 935                 940

Pro Leu Phe Asp Thr Met Phe Ile Leu Gln Asn Leu Asp Gln Lys Ala
945                 950                 955                 960

Tyr Glu Leu Asp Gly Leu Lys Leu Glu Ala Tyr Pro Ala Gln Ala Gly
                965                 970                 975

Asn Ala Lys Phe Asp Leu Thr Leu Glu Ala His Glu Asp Glu Thr Gly
            980                 985                 990

Ile His Phe Ala Leu Val Tyr Ser  Thr Lys Leu Phe Gln  Arg Glu Ser
        995                 1000                1005

Ile Glu Arg Met Ala Gly His Phe Leu Gln Val Leu Arg Gln Val
    1010                1015                1020

Val Ala Asp Gln Ala Thr Ala Leu Arg Glu Ile Ser Leu Leu Ser
    1025                1030                1035

Glu Glu Glu Arg Arg Ile Val Thr Val Asp Phe Asn Asn Thr Phe
    1040                1045                1050

Ala Tyr Pro Arg Asp Leu Thr Ile Gln Glu Leu Phe Glu Gln Gln
    1055                1060                1065

Ala Ala Lys Thr Pro Glu His Ala Ala Val Val Met Asp Gly Gln
    1070                1075                1080

Met Leu Thr Tyr Arg Glu Leu Asn Glu Lys Ala Asn Gln Leu Ala
    1085                1090                1095

His Val Leu Arg Gln Asn Gly Val Gly Lys Glu Ser Ile Val Gly
    1100                1105                1110

Leu Leu Ala Asp Arg Ser Leu Glu Met Ile Thr Gly Ile Met Gly
    1115                1120                1125

Ile Leu Lys Ala Gly Gly Ala Tyr Leu Gly Leu Asp Pro Glu His
    1130                1135                1140

Pro Ser Glu Arg Leu Ala Tyr Met Leu Glu Asp Gly Gly Val Lys
    1145                1150                1155

Val Val Leu Val Gln Lys His Leu Leu Pro Leu Val Gly Glu Gly
    1160                1165                1170

Leu Met Pro Ile Val Leu Glu Glu Glu Ser Leu Arg Pro Glu Asp
    1175                1180                1185

Cys Gly Asn Pro Ala Ile Val Asn Gly Ala Ser Asp Leu Ala Tyr
    1190                1195                1200

Val Met Tyr Thr Ser Gly Ser Thr Gly Lys Pro Lys Gly Val Met
    1205                1210                1215

Val Glu His Arg Asn Val Thr Arg Leu Val Met His Thr Asn Tyr
    1220                1225                1230

Val Gln Val Arg Glu Ser Asp Arg Met Ile Gln Thr Gly Ala Ile
    1235                1240                1245

Gly Phe Asp Ala Met Thr Phe Glu Ile Phe Gly Ala Leu Leu His
    1250                1255                1260

Gly Ala Ser Leu Tyr Leu Val Ser Lys Asp Val Leu Leu Asp Ala
    1265                1270                1275

Glu Lys Leu Gly Asp Phe Leu Arg Thr Asn Gln Ile Thr Thr Met
    1280                1285                1290

Trp Leu Thr Ser Pro Leu Phe Asn Gln Leu Ser Gln Asp Asn Pro
    1295                1300                1305

Ala Met Phe Asp Ser Leu Arg Ala Leu Ile Val Gly Gly Glu Ala
    1310                1315                1320

Leu Ser Pro Lys His Ile Asn Arg Val Lys Ser Ala Leu Pro Asp
    1325                1330                1335

Leu Glu Ile Trp Asn Gly Tyr Gly Pro Thr Glu Asn Thr Thr Phe
    1340                1345                1350

Ser Thr Cys Tyr Leu Ile Glu Gln His Phe Glu Glu Gln Ile Pro
    1355                1360                1365

Ile Gly Lys Pro Ile Ala Asn Ser Thr Ala Tyr Ile Val Asp Gly
    1370                1375                1380

Asn Asn Gln Pro Gln Pro Ile Gly Val Pro Gly Glu Leu Cys Val
    1385                1390                1395

Gly Gly Asp Gly Val Ala Arg Gly Tyr Val Asn Lys Pro Glu Leu
    1400                1405                1410

Thr Ala Glu Lys Phe Val Pro Asn Pro Phe Ala Pro Gly Glu Thr
    1415                1420                1425

Met Tyr Arg Thr Gly Asp Leu Ala Arg Trp Leu Pro Asp Gly Thr
    1430                1435                1440

Ile Glu Tyr Leu Gly Arg Ile Asp Gln Gln Val Lys Ile Arg Gly
    1445                1450                1455

Tyr Arg Ile Glu Leu Gly Glu Ile Glu Thr Val Leu Ser Gln Gln
    1460                1465                1470

Ala Gln Val Lys Glu Ala Val Val Ala Val Ile Glu Glu Ala Asn

1475                1480                1485

Gly Gln  Lys Ala Leu Cys Ala  Tyr Phe Val Pro Glu  Gln Ala Val
    1490                1495                1500

Asp Ala  Ala Glu Leu Arg Glu  Ala Met Ser Lys Gln  Leu Pro Gly
    1505                1510                1515

Tyr Met  Val Pro Ala Tyr Tyr  Val Gln Met Glu Lys  Leu Pro Leu
    1520                1525                1530

Thr Ala  Asn Gly Lys Val Asp  Arg Arg Ala Leu Pro  Gln Pro Ser
    1535                1540                1545

Gly Glu  Arg Thr Thr Gly Ser  Ala Phe Val Ala Ala  Gln Asn Asp
    1550                1555                1560

Thr Glu  Ala Lys Leu Gln Gln  Ile Trp Gln Glu Val  Leu Gly Ile
    1565                1570                1575

Pro Ala  Ile Gly Ile His Asp  Asn Phe Phe Glu Ile  Gly Gly His
    1580                1585                1590

Ser Leu  Lys Ala Met Asn Val  Ile Thr Gln Val His  Lys Thr Phe
    1595                1600                1605

Gln Val  Glu Leu Pro Leu Lys  Ala Leu Phe Ala Thr  Pro Thr Ile
    1610                1615                1620

His Glu  Leu Ala Ala His Ile  Ser Glu Lys Thr Glu  Tyr Thr Ala
    1625                1630                1635

Ile Gln  Pro Val Ala Ala Gln  Glu Phe Tyr Pro Val  Ser Ser Ala
    1640                1645                1650

Gln Lys  Arg Met Tyr Ile Leu  Gln Gln Phe Glu Gly  Asn Gly Ile
    1655                1660                1665

Ser Tyr  Asn Ile Ser Gly Ala  Ile Leu Leu Glu Gly  Lys Leu Asp
    1670                1675                1680

Tyr Ala  Arg Phe Ala Ser Ala  Val Gln Gln Leu Ala  Glu Arg His
    1685                1690                1695

Glu Ala  Leu Arg Thr Ser Phe  His Arg Ile Asp Gly  Glu Pro Val
    1700                1705                1710

Gln Lys Val His Glu Glu Val Glu Val Pro Leu Phe Met Leu Glu
    1715                1720                1725

Ala Pro Glu Asp Gln Ala Glu Lys Ile Met Arg Glu Phe Val Arg
    1730                1735                1740

Pro Phe Asp Leu Gly Val Ala Pro Leu Met Arg Thr Gly Leu Leu
    1745                1750                1755

Lys Leu Gly Lys Asp Arg His Leu Phe Leu Leu Asp Met His His
    1760                1765                1770

Ile Ile Ser Asp Gly Val Ser Ser Gln Ile Leu Leu Arg Glu Phe
    1775                1780                1785

Ala Glu Leu Tyr Gln Gly Ala Asp Leu Gln Pro Leu Ser Leu Gln
    1790                1795                1800

Tyr Lys Asp Phe Ala Ala Trp Gln Asn Glu Leu Phe Gln Thr Glu
    1805                1810                1815

Ala Tyr Lys Lys Gln Glu Gln His Trp Leu Asn Thr Phe Ala Asp
    1820                1825                1830

Glu Ile Pro Leu Leu Asn Leu Pro Thr Asp Tyr Pro Arg Pro Ser
    1835                1840                1845

Val Gln Ser Phe Ala Gly Asp Leu Val Leu Phe Ala Ala Gly Lys
    1850                1855                1860

Glu Leu Leu Glu Arg Leu Gln Gln Val Ala Ser Glu Thr Gly Thr
    1865                1870                1875

Thr Leu Tyr Met Ile Leu Leu Ala Ala Tyr Asn Val Leu Leu Ser
    1880                1885                1890

Lys Tyr Thr Gly Gln Glu Asp Ile Ile Val Gly Thr Pro Val Ala
    1895                1900                1905

Gly Arg Ser His Ala Asp Val Glu Asn Ile Met Gly Ile Phe Val
    1910                1915                1920

Asn Thr Leu Ala Leu Arg Asn Gln Pro Ala Ser Ser Lys Thr Met
    1925                1930                1935

Leu Glu Asn Asn Ile Thr Gln Cys Asp Ser Ile Asn Asp Val Tyr
    1940                1945                1950

Leu Lys Glu Glu Ala Ile Thr Leu Met Asp Met Leu Glu Ser Gln
    1955                1960                1965

Leu Lys His Gln Ala Asp Gly Tyr Val Val Ile Asp Gln Glu Glu
    1970                1975                1980

Ser Leu Ser Tyr Ala Asp Phe Tyr Leu Arg Val Lys Glu Ile Gly
    1985                1990                1995

Tyr Cys Leu Ser Glu Ile Ser Ser Lys Asn Ser Val Gly Ile Gly
    2000                2005                2010

Leu Phe Cys Asp Pro Ser Ile Asp Leu Ile Cys Gly Ala Trp Gly
    2015                2020                2025

Ile Leu Ser Ala Asp Lys Ala Tyr Leu Pro Leu Ser Pro Asp Tyr
    2030                2035                2040

Pro Thr Glu Arg Leu Lys Tyr Met Ile Glu Asp Ser Gly Ile Asp
    2045                2050                2055

Val Ile Phe Thr Gln Ser His Leu Lys Ala Gln Leu Gln Asp Ile
    2060                2065                2070

Ala Pro Lys Ser Val Leu Ile Met Thr Pro Glu Asp Val Ala Leu
    2075                2080                2085

Thr Ile Lys Thr Arg Thr Ile Glu Asp Ile Leu Gly Thr Val Gln
    2090                2095                2100

Val Pro Lys Pro Thr Ser Leu Ala Tyr Ile Ile Tyr Thr Ser Gly
    2105                2110                2115

Ser Thr Gly Lys Pro Lys Gly Val Met Ile Glu His His Ser Ile
    2120                2125                2130

Val Asn Gln Met Arg Phe Leu Ala Lys Ala Phe Lys Leu Gly Cys
    2135                2140                2145

His Ser Arg Ile Leu Gln Lys Thr Pro Met Ser Phe Asp Ala Ala
    2150                2155                2160

Gln Trp Glu Ile Leu Ala Pro Ala Ile Gly Gly Gln Val Ile Met
    2165                2170                2175

Gly Pro Leu Gly Cys Tyr Arg Asp Pro Asp Ala Ile Ile Lys Thr
    2180                2185                2190

Ile Leu Gln His Gln Val Thr Thr Leu Gln Cys Val Pro Thr Leu
    2195                2200                2205

Leu Gln Ala Leu Leu Asp Asn Pro Asn Phe Leu Asp Cys Leu Ser
    2210                2215                2220

Leu Thr Gln Val Phe Ser Gly Gly Glu Ala Leu Thr Thr Lys Leu
    2225                2230                2235

Ala Thr Gln Phe Leu Asn Ser Phe Thr His Cys Glu Leu Ile Asn
    2240                2245                2250

Leu Tyr Gly Pro Thr Glu Cys Thr Ile Asn Ser Ser Phe Phe Arg
    2255                2260                2265

Val Thr Asn Glu Thr Leu Pro Asn Tyr Gln Thr Ser Ile Ser Ile
    2270                2275                2280

Gly Ala Pro Val Asp Asn Thr Glu Tyr Tyr Val Leu Asp Asp Asp
    2285                2290                2295

Arg Leu Pro Val Ala Val Gly Glu Ile Gly Glu Leu Tyr Ile Ser
    2300                2305                2310

Gly Ala Gln Leu Ala Arg Gly Tyr Leu His Lys Pro Glu Met Thr
    2315                2320                2325

Lys Asp Lys Phe Ile Cys Asn His Leu Val Ser Gly Thr Gln His
    2330                2335                2340

Gln Trp Leu Tyr Arg Thr Gly Asp Leu Val Thr Arg Gly Ala Asp
    2345                2350                2355

Gly Asn Thr Tyr Phe Val Gly Arg Val Asp Ser Gln Val Lys Leu
    2360                2365                2370

Arg Gly Tyr Arg Ile Glu Leu Asp Glu Ile Arg His Ala Ile Glu
    2375                2380                2385

Glu His Ser Trp Ile Lys Thr Ala Ala Met Leu Ile Lys Lys Asp
    2390                2395                2400

Ala Arg Thr Gly Phe Gln Asn Leu Ile Ala Cys Val Glu Leu Asp
    2405                2410                2415

Glu Lys Glu Ala Ala Leu Met Asp Gln Gly Asn Ser Ser Ser His

2420                2425                2430

His Lys Ser Lys Ala Asp Lys Leu Gln Val Lys Ala Gln Leu Ser
    2435                2440                2445

Asn Ser Gly Cys Arg Ser Glu Glu Leu Cys Glu Asn Arg Pro Thr
    2450                2455                2460

Phe Leu Leu Pro Tyr Gln Glu Gly Glu Ile Lys Gln Arg Glu Tyr
    2465                2470                2475

Ala Phe Gly Arg Lys Thr Tyr Arg Tyr Phe Glu Gly Thr Glu Ile
    2480                2485                2490

Thr Val Glu Lys Leu Lys Lys Leu Leu Thr Ala Thr Gln Ser Asn
    2495                2500                2505

Glu Ile Ser Ser Leu Pro Leu Ser His Leu Thr Leu Asn Asp Phe
    2510                2515                2520

Gly Tyr Ala Leu Arg Tyr Phe Gly Gln Phe Thr Ser His Gln Arg
    2525                2530                2535

Leu Leu Pro Lys Tyr Ala Tyr Ala Ser Pro Gly Ala Leu Tyr Ala
    2540                2545                2550

Thr Gln Met Tyr Phe Glu Leu His Asn Val Leu Gly Leu Asp Ala
    2555                2560                2565

Gly Ile Tyr Tyr Tyr His Pro Val Thr His Lys Leu Ile Lys Ile
    2570                2575                2580

Ser Thr Leu Ser Arg Arg Gln Met Pro Thr Ile Lys Val His Phe
    2585                2590                2595

Ile Gly Lys His Glu Ala Ile Glu Pro Val Tyr Lys Asn Asn Ile
    2600                2605                2610

Gln Glu Val Leu Glu Met Glu Ala Gly His Met Met Gly Leu Phe
    2615                2620                2625

Asp Asp Val Leu Pro Glu Ile Gly Leu Ser Ile Gly Lys Ser Glu
    2630                2635                2640

Tyr Gln Asp Glu Cys Pro Asp Trp Tyr Asp Gly Asp Ile Gln Asp
    2645                2650                2655

Tyr Tyr Leu Gly Ala Phe Glu Ile Cys Ser Tyr Glu His Gly Leu
    2660                2665                2670

Pro Pro Phe Glu Thr Asp Ile Tyr Leu Gln Thr His Ala His Lys
    2675                2680                2685

Ile Pro Glu Met Pro Cys Gly Leu Tyr His Phe Ser Asn Gly Glu
    2690                2695                2700

Phe Val Arg Ile Ser Asp Asp Ile Val Arg Lys Lys Asp Val Ile
    2705                2710                2715

Ala Ile Asn Gln Gln Val Tyr Asp Arg Ser Ser Phe Gly Val Ser
    2720                2725                2730

Ile Ile Pro Arg Cys Val Pro Glu Trp His Tyr Tyr Ile Thr Leu
    2735                2740                2745

Gly Arg Arg Leu His Ala Leu Gln Ser Asn Pro Leu Tyr Ile Gly
    2750                2755                2760

Leu Met Ser Ser Gly Tyr Ser Ser Lys Ser Asn Asn Asp Leu Pro
    2765                2770                2775

Ser Ala Lys Arg Met Arg Ser Ile Leu Asn Ala Leu Asp Arg Pro
    2780                2785                2790

Met Ala Ala Phe Tyr Phe Cys Ile Gly Gly Gly Ile Ser Gln Ala
    2795                2800                2805

Gln Tyr Met Cys Glu Gly Met Lys Glu Asp Val Val His Met Lys
    2810                2815                2820

Gly Pro Val Glu Ile Ile Lys Asp Asp Leu Gln Gln Gln Leu Pro
    2825                2830                2835

Gln Tyr Met Ile Pro Asn Lys Val Leu Val Phe Asp Lys Leu Pro
    2840                2845                2850

Leu Thr Ala Asn Gly Lys Val Asp Tyr Gln Ser Leu Ser Glu Ser
    2855                2860                2865

Lys Ala Val Glu Asn Val Ser Thr Gln Arg Leu Leu Val Pro Leu
    2870                2875                2880

His Thr Asp Thr Glu Ile Arg Leu Gly Lys Ile Trp Met Glu Val
    2885                2890                2895

Leu Lys Trp Asp Ser Val Ser Ala Leu Asp Asp Phe Phe Glu Ser
    2900                2905                2910

Gly Gly Asn Ser Leu Met Ala Val Ala Met Val Asn Lys Ile Asn
    2915                2920                2925

Ala Ala Phe Asn Ile Arg Phe Pro Leu Gln Ile Leu Phe Gln Ser
    2930                2935                2940

Pro Asn Ile Ala Glu Leu Ala Lys Trp Ile Glu Gln Thr Asp Ser
    2945                2950                2955

Lys Thr Ile Ser Arg Leu Ile Leu Leu Asn Gln Ala Ser Lys Asp
    2960                2965                2970

Pro Ile Tyr Cys Trp Pro Gly Leu Gly Gly Tyr Pro Met Ser Leu
    2975                2980                2985

Arg Leu Leu Ala Asn Lys Val Val Pro Asp Arg Ala Phe Tyr Gly
    2990                2995                3000

Ile Gln Ala Tyr Gly Ile Asn Glu Ser Glu Ile Pro Phe Ser Ser
    3005                3010                3015

Ile Gln Arg Met Ala Glu Glu Asp Ile Lys Glu Ile Lys Lys Ile
    3020                3025                3030

Gln Pro Glu Gly Pro Tyr Ile Leu Trp Gly Tyr Ser Phe Gly Ala
    3035                3040                3045

Arg Val Ala Phe Glu Val Ala Tyr Gln Leu Glu Gln Ala Gly Glu
    3050                3055                3060

Glu Val Asn Ala Leu Asn Leu Leu Ala Pro Gly Ser Pro His Leu
    3065                3070                3075

Asp Met Lys Gln Ala Glu Tyr Met Asp Lys Gly Ala Glu Phe Thr
    3080                3085                3090

Asn Pro Ala Phe Val Lys Ile Leu Phe Ser Val Phe Ser Arg Ser
    3095                3100                3105

Ile Asn Ser Pro Met Val Lys Thr Cys Leu Glu Gln Val Asn Ser
    3110                3115                3120

Glu Thr Thr Phe Ile Asn Phe Ile Cys Ser Arg Phe Lys Asn Leu
    3125                3130                3135

Glu Pro Ser Leu Val Lys Arg Ile Val Arg Ile Val Thr Leu Thr
    3140                3145                3150

Tyr Asp Phe Lys Tyr Ser Ile Asp Glu Leu Tyr His Arg His Leu
    3155                3160                3165

Lys Ala Pro Ile Thr Ile Phe Lys Ala Asn Arg Asp Asn Asp Ser
    3170                3175                3180

Phe Ile Glu Glu Ser Asp Val Ile Ser Ser Met Ser Pro Lys Ile
    3185                3190                3195

Ile Glu Leu Ile Ser Asp His Tyr Gln Leu Leu Glu Ser Glu Gly
    3200                3205                3210

Val Ala Glu Ile Glu Lys Ile Ile
    3215                3220

<210> 29
<211> 4256
<212> PRT
<213> Artificial Sequence

<220>
<223> NRPSase synthesizing a Indigoidine-tagged Tripeptide consisting
of Ornithine and two Valines

<400> 29

Met Leu His Ser Phe Leu Ala Thr Lys Thr Ala Tyr Pro Thr Asp Lys
1               5                   10                  15

Thr Phe Gln Lys Leu Phe Glu Glu Gln Val Glu Lys Thr Pro Asn Glu
            20                  25                  30

Ile Ala Val Leu Phe Gly Asn Glu Gln Leu Thr Tyr Gln Glu Leu Asn
        35                  40                  45

Ala Lys Ala Asn Gln Leu Ala Arg Val Leu Arg Arg Lys Gly Val Lys
    50                  55                  60

Pro Glu Ser Thr Val Gly Ile Leu Val Asp Arg Ser Leu Tyr Met Val
65                  70                  75                  80

Ile Gly Met Leu Ala Val Leu Lys Ala Gly Gly Thr Phe Val Pro Ile
                85                  90                  95

Asp Pro Asp Tyr Pro Leu Glu Arg Gln Ala Phe Met Leu Glu Asp Ser
            100                 105                 110

Glu Ala Lys Leu Leu Leu Thr Leu Gln Lys Met Asn Ser Gln Val Ala
        115                 120                 125

Phe Pro Tyr Glu Thr Phe Tyr Leu Asp Thr Glu Thr Val Asp Gln Glu
    130                 135                 140

Glu Thr Gly Asn Leu Glu His Val Ala Gln Pro Glu Asn Val Ala Tyr
145                 150                 155                 160

Ile Ile Tyr Thr Ser Gly Thr Thr Gly Lys Pro Lys Gly Val Val Ile
                165                 170                 175

Glu His Arg Ser Tyr Ala Asn Val Ala Phe Ala Trp Lys Asp Glu Tyr
            180                 185                 190

His Leu Asp Ser Phe Pro Val Arg Leu Leu Gln Met Ala Ser Phe Ala
        195                 200                 205

Phe Asp Val Ser Thr Gly Asp Phe Ala Arg Ala Leu Leu Thr Gly Gly
    210                 215                 220

Gln Leu Val Ile Cys Pro Asn Gly Val Lys Met Asp Pro Ala Ser Leu
225                 230                 235                 240

Tyr Glu Thr Ile Arg Arg His Glu Ile Thr Ile Phe Glu Ala Thr Pro
                245                 250                 255

Ala Leu Ile Met Pro Leu Met His Tyr Val Tyr Glu Asn Glu Leu Asp
            260                 265                 270

Met Ser Gln Met Lys Leu Leu Ile Leu Gly Ala Asp Ser Cys Pro Ala
        275                 280                 285

Glu Asp Phe Lys Thr Leu Leu Ala Arg Phe Gly Gln Lys Met Arg Ile
    290                 295                 300

Ile Asn Ser Tyr Gly Val Thr Glu Ala Cys Ile Asp Thr Ser Tyr Tyr
305                 310                 315                 320

Glu Glu Thr Asp Val Thr Ala Ile Arg Ser Gly Thr Val Pro Ile Gly
                325                 330                 335

Lys Pro Leu Pro Asn Met Thr Met Tyr Val Val Asp Ala His Leu Asn
            340                 345                 350

Leu Gln Pro Val Gly Val Val Gly Glu Leu Cys Ile Gly Gly Ala Gly

355                 360                 365

Val Ala Arg Gly Tyr Leu Asn Arg Pro Glu Leu Thr Glu Glu Lys Phe
    370                 375                 380

Val Pro Asn Pro Phe Ala Pro Gly Glu Arg Leu Tyr Arg Thr Gly Asp
385                 390                 395                 400

Leu Ala Lys Trp Arg Ala Asp Gly Asn Val Glu Phe Leu Gly Arg Asn
                405                 410                 415

Asp His Gln Val Lys Ile Arg Gly Val Arg Ile Glu Leu Gly Glu Ile
            420                 425                 430

Glu Thr Gln Leu Arg Lys Leu Asp Gly Ile Thr Glu Ala Val Val Val
        435                 440                 445

Ala Arg Glu Asp Arg Gly Gln Glu Lys Glu Leu Cys Ala Tyr Val Val
    450                 455                 460

Ala Asp His Lys Leu Asp Thr Ala Glu Leu Arg Ala Asn Leu Leu Lys
465                 470                 475                 480

Glu Leu Pro Gln Ala Met Ile Pro Ala Tyr Phe Val Thr Leu Asp Ala
                485                 490                 495

Leu Pro Leu Thr Ala Asn Gly Lys Val Asp Arg Arg Ser Leu Pro Ala
            500                 505                 510

Pro Asp Val Thr Met Leu Arg Thr Thr Glu Tyr Val Ala Pro Arg Ser
        515                 520                 525

Val Trp Glu Ala Arg Leu Ala Gln Val Trp Glu Gln Val Leu Asn Val
    530                 535                 540

Pro Gln Val Gly Ala Leu Asp Asp Phe Phe Ala Leu Gly Gly His Ser
545                 550                 555                 560

Leu Arg Ala Met Arg Val Leu Ser Ser Met His Asn Glu Tyr Gln Val
                565                 570                 575

Asp Ile Pro Leu Arg Ile Leu Phe Glu Lys Pro Thr Ile Gln Glu Leu
            580                 585                 590

Ala Ala Phe Ile Glu Thr Ser Gly Lys Glu Thr Tyr Val Pro Ile Glu
        595                 600                 605

Pro Ala Pro Leu Gln Glu Tyr Tyr Pro Val Ser Ser Ala Gln Lys Arg
    610                 615                 620

Met Tyr Val Leu Arg Gln Phe Ala Asp Thr Gly Thr Val Tyr Asn Met
625                 630                 635                 640

Pro Ser Ala Leu Tyr Ile Glu Gly Asp Leu Asp Arg Lys Arg Phe Glu
                645                 650                 655

Ala Ala Ile His Gly Leu Val Glu Arg His Glu Ser Leu Arg Thr Ser
            660                 665                 670

Phe His Thr Val Asn Gly Glu Pro Val Gln Arg Val His Glu His Val
        675                 680                 685

Glu Leu Asn Val Gln Tyr Ala Glu Val Thr Glu Ala Gln Val Glu Pro
    690                 695                 700

Thr Val Glu Ser Phe Val Gln Ala Phe Asp Leu Thr Lys Ala Pro Leu
705                 710                 715                 720

Leu Arg Val Gly Leu Phe Lys Leu Ala Ala Lys Arg His Leu Phe Leu
                725                 730                 735

Leu Asp Met His His Ile Ile Ser Asp Gly Val Ser Ala Gly Ile Ile
            740                 745                 750

Met Glu Glu Phe Ser Lys Leu Tyr Arg Gly Glu Glu Leu Pro Ala Leu
        755                 760                 765

Ser Val His Tyr Lys Asp Phe Ala Val Trp Gln Ser Glu Leu Phe Gln
    770                 775                 780

Ser Asp Val Tyr Thr Glu His Glu Asn Tyr Trp Leu Asn Ala Phe Ser
785                 790                 795                 800

Gly Asp Ile Pro Val Leu Asn Leu Pro Ala Asp Phe Ser Arg Pro Leu
                805                 810                 815

Thr Gln Ser Phe Glu Gly Asp Cys Val Ser Phe Gln Ala Asp Lys Ala
            820                 825                 830

Leu Leu Asp Asp Leu His Lys Leu Ala Gln Glu Ser Gln Ser Thr Leu
        835                 840                 845

Phe Met Val Leu Leu Ala Ala Tyr Asn Val Leu Leu Ala Lys Tyr Ser
    850                 855                 860

Gly Gln Glu Asp Ile Val Val Gly Thr Pro Ile Ala Gly Arg Ser His
865                 870                 875                 880

Ala Asp Ile Glu Asn Val Leu Gly Met Phe Val Asn Thr Leu Ala Leu
                885                 890                 895

Arg Asn Tyr Pro Val Glu Thr Lys His Phe Gln Ala Phe Leu Glu Glu
            900                 905                 910

Val Lys Gln Asn Thr Leu Gln Ala Tyr Ala His Gln Asp Tyr Pro Phe
        915                 920                 925

Glu Ala Leu Val Glu Lys Leu Asp Ile Gln Arg Asp Leu Ser Arg Asn
    930                 935                 940

Pro Leu Phe Asp Thr Met Phe Ile Leu Gln Asn Leu Asp Gln Lys Ala
945                 950                 955                 960

Tyr Glu Leu Asp Gly Leu Lys Leu Glu Ala Tyr Pro Ala Gln Ala Gly
                965                 970                 975

Asn Ala Lys Phe Asp Leu Thr Leu Glu Ala His Glu Asp Glu Thr Gly
            980                 985                 990

Ile His Phe Ala Leu Val Tyr Ser  Thr Lys Leu Phe Gln  Arg Glu Ser
        995                 1000                1005

Ile Glu  Arg Met Ala Gly His  Phe Leu Gln Val Leu  Arg Gln Val
    1010                1015                1020

Val Ala  Asp Gln Ala Thr Ala  Leu Arg Glu Ile Ser  Leu Leu Ser
    1025                1030                1035

Glu Glu  Glu Arg Arg Ile Val  Thr Val Asp Phe Asn  Asn Thr Phe
    1040                1045                1050

Ala Ala  Tyr Pro Arg Asp Leu  Thr Ile Gln Glu Leu  Phe Glu Gln
    1055                1060                1065

Gln Ala  Ala Lys Thr Pro Glu  His Ala Ala Val Val  Met Asp Gly
    1070                1075                1080

Gln Met  Leu Thr Tyr Arg Glu  Leu Asn Glu Lys Ala  Asn Gln Leu
    1085                1090                1095

Ala His  Val Leu Arg Gln Asn  Gly Val Gly Lys Glu  Ser Ile Val
    1100                1105                1110

Gly Leu Leu Ala Asp Arg Ser Leu Glu Met Ile Thr Gly Ile Met
    1115                1120                1125

Gly Ile Leu Lys Ala Gly Gly Ala Tyr Leu Gly Leu Asp Pro Glu
    1130                1135                1140

His Pro Ser Glu Arg Leu Ala Tyr Met Leu Glu Asp Gly Gly Val
    1145                1150                1155

Lys Val Val Leu Val Gln Lys His Leu Leu Pro Leu Val Gly Glu
    1160                1165                1170

Gly Leu Met Pro Ile Val Leu Glu Glu Glu Ser Leu Arg Pro Glu
    1175                1180                1185

Asp Cys Gly Asn Pro Ala Ile Val Asn Gly Ala Ser Asp Leu Ala
    1190                1195                1200

Tyr Val Met Tyr Thr Ser Gly Ser Thr Gly Lys Pro Lys Gly Val
    1205                1210                1215

Met Val Glu His Arg Asn Val Thr Arg Leu Val Met His Thr Asn
    1220                1225                1230

Tyr Val Gln Val Arg Glu Ser Asp Arg Met Ile Gln Thr Gly Ala
    1235                1240                1245

Ile Gly Phe Asp Ala Met Thr Phe Glu Ile Phe Gly Ala Leu Leu
    1250                1255                1260

His Gly Ala Ser Leu Tyr Leu Val Ser Lys Asp Val Leu Leu Asp
    1265                1270                1275

Ala Glu Lys Leu Gly Asp Phe Leu Arg Thr Asn Gln Ile Thr Thr
    1280                1285                1290

Met Trp Leu Thr Ser Pro Leu Phe Asn Gln Leu Ser Gln Asp Asn
    1295                1300                1305

Pro Ala Met Phe Asp Ser Leu Arg Ala Leu Ile Val Gly Gly Glu
    1310                1315                1320

Ala Leu Ser Pro Lys His Ile Asn Arg Val Lys Ser Ala Leu Pro
    1325                1330                1335

Asp Leu Glu Ile Trp Asn Gly Tyr Gly Pro Thr Glu Asn Thr Thr

1340                1345                1350

Phe Ser Thr Cys Tyr Leu Ile Glu Gln His Phe Glu Glu Gln Ile
    1355                1360                1365

Pro Ile Gly Lys Pro Ile Ala Asn Ser Thr Ala Tyr Ile Val Asp
    1370                1375                1380

Gly Asn Asn Gln Pro Gln Pro Ile Gly Val Pro Gly Glu Leu Cys
    1385                1390                1395

Val Gly Gly Asp Gly Val Ala Arg Gly Tyr Val Asn Lys Pro Glu
    1400                1405                1410

Leu Thr Ala Glu Lys Phe Val Pro Asn Pro Phe Ala Pro Gly Glu
    1415                1420                1425

Thr Met Tyr Arg Thr Gly Asp Leu Ala Arg Trp Leu Pro Asp Gly
    1430                1435                1440

Thr Ile Glu Tyr Leu Gly Arg Ile Asp Gln Gln Val Lys Ile Arg
    1445                1450                1455

Gly Tyr Arg Ile Glu Leu Gly Glu Ile Glu Thr Val Leu Ser Gln
    1460                1465                1470

Gln Ala Gln Val Lys Glu Ala Val Val Ala Val Ile Glu Glu Ala
    1475                1480                1485

Asn Gly Gln Lys Ala Leu Cys Ala Tyr Phe Val Pro Glu Gln Ala
    1490                1495                1500

Val Asp Ala Ala Glu Leu Arg Glu Ala Met Ser Lys Gln Leu Pro
    1505                1510                1515

Gly Tyr Met Val Pro Ala Tyr Tyr Val Gln Met Glu Lys Leu Pro
    1520                1525                1530

Leu Thr Ala Asn Gly Lys Val Asp Arg Arg Ala Leu Pro Gln Pro
    1535                1540                1545

Ser Gly Glu Arg Thr Thr Gly Ser Ala Phe Val Ala Ala Gln Asn
    1550                1555                1560

Asp Thr Glu Ala Lys Leu Gln Gln Ile Trp Gln Glu Val Leu Gly
    1565                1570                1575

Ile Pro Ala Ile Gly Ile His Asp Asn Phe Phe Glu Ile Gly Gly
    1580                1585                1590

His Ser Leu Lys Ala Met Asn Val Ile Thr Gln Val His Lys Thr
    1595                1600                1605

Phe Gln Val Glu Leu Pro Leu Lys Ala Leu Phe Ala Thr Pro Thr
    1610                1615                1620

Ile His Glu Leu Ala Ala His Ile Ala Thr Ser Gly Lys Glu Thr
    1625                1630                1635

Tyr Val Pro Ile Glu Pro Ala Pro Leu Gln Glu Tyr Tyr Pro Val
    1640                1645                1650

Ser Ser Ala Gln Lys Arg Met Tyr Val Leu Arg Gln Phe Ala Asp
    1655                1660                1665

Thr Gly Thr Val Tyr Asn Met Pro Ser Ala Leu Tyr Ile Glu Gly
    1670                1675                1680

Asp Leu Asp Arg Lys Arg Phe Glu Ala Ala Ile His Gly Leu Val
    1685                1690                1695

Glu Arg His Glu Ser Leu Arg Thr Ser Phe His Thr Val Asn Gly
    1700                1705                1710

Glu Pro Val Gln Arg Val His Glu His Val Glu Leu Asn Val Gln
    1715                1720                1725

Tyr Ala Glu Val Thr Glu Ala Gln Val Glu Pro Thr Val Glu Ser
    1730                1735                1740

Phe Val Gln Ala Phe Asp Leu Thr Lys Ala Pro Leu Leu Arg Val
    1745                1750                1755

Gly Leu Phe Lys Leu Ala Ala Lys Arg His Leu Phe Leu Leu Asp
    1760                1765                1770

Met His His Ile Ile Ser Asp Gly Val Ser Ala Gly Ile Ile Met
    1775                1780                1785

Glu Glu Phe Ser Lys Leu Tyr Arg Gly Glu Glu Leu Pro Ala Leu
    1790                1795                1800

Ser Val His Tyr Lys Asp Phe Ala Val Trp Gln Ser Glu Leu Phe
    1805                1810                1815

Gln Ser Asp Val Tyr Thr Glu His Glu Asn Tyr Trp Leu Asn Ala
    1820                1825                1830

Phe Ser Gly Asp Ile Pro Val Leu Asn Leu Pro Ala Asp Phe Ser
    1835                1840                1845

Arg Pro Leu Thr Gln Ser Phe Glu Gly Asp Cys Val Ser Phe Gln
    1850                1855                1860

Ala Asp Lys Ala Leu Leu Asp Asp Leu His Lys Leu Ala Gln Glu
    1865                1870                1875

Ser Gln Ser Thr Leu Phe Met Val Leu Leu Ala Ala Tyr Asn Val
    1880                1885                1890

Leu Leu Ala Lys Tyr Ser Gly Gln Glu Asp Ile Val Val Gly Thr
    1895                1900                1905

Pro Ile Ala Gly Arg Ser His Ala Asp Ile Glu Asn Val Leu Gly
    1910                1915                1920

Met Phe Val Asn Thr Leu Ala Leu Arg Asn Tyr Pro Val Glu Thr
    1925                1930                1935

Lys His Phe Gln Ala Phe Leu Glu Glu Val Lys Gln Asn Thr Leu
    1940                1945                1950

Gln Ala Tyr Ala His Gln Asp Tyr Pro Phe Glu Ala Leu Val Glu
    1955                1960                1965

Lys Leu Asp Ile Gln Arg Asp Leu Ser Arg Asn Pro Leu Phe Asp
    1970                1975                1980

Thr Met Phe Ile Leu Gln Asn Leu Asp Gln Lys Ala Tyr Glu Leu
    1985                1990                1995

Asp Gly Leu Lys Leu Glu Ala Tyr Pro Ala Gln Ala Gly Asn Ala
    2000                2005                2010

Lys Phe Asp Leu Thr Leu Glu Ala His Glu Asp Glu Thr Gly Ile
    2015                2020                2025

His Phe Ala Leu Val Tyr Ser Thr Lys Leu Phe Gln Arg Glu Ser
    2030                2035                2040

Ile Glu Arg Met Ala Gly His Phe Leu Gln Val Leu Arg Gln Val
    2045                2050                2055

Val Ala Asp Gln Ala Thr Ala Leu Arg Glu Ile Ser Leu Leu Ser
    2060                2065                2070

Glu Glu Glu Arg Arg Ile Val Thr Val Asp Phe Asn Asn Thr Phe
    2075                2080                2085

Ala Tyr Pro Arg Asp Leu Thr Ile Gln Glu Leu Phe Glu Gln Gln
    2090                2095                2100

Ala Ala Lys Thr Pro Glu His Ala Ala Val Val Met Asp Gly Gln
    2105                2110                2115

Met Leu Thr Tyr Arg Glu Leu Asn Glu Lys Ala Asn Gln Leu Ala
    2120                2125                2130

His Val Leu Arg Gln Asn Gly Val Gly Lys Glu Ser Ile Val Gly
    2135                2140                2145

Leu Leu Ala Asp Arg Ser Leu Glu Met Ile Thr Gly Ile Met Gly
    2150                2155                2160

Ile Leu Lys Ala Gly Gly Ala Tyr Leu Gly Leu Asp Pro Glu His
    2165                2170                2175

Pro Ser Glu Arg Leu Ala Tyr Met Leu Glu Asp Gly Gly Val Lys
    2180                2185                2190

Val Val Leu Val Gln Lys His Leu Leu Pro Leu Val Gly Glu Gly
    2195                2200                2205

Leu Met Pro Ile Val Leu Glu Glu Glu Ser Leu Arg Pro Glu Asp
    2210                2215                2220

Cys Gly Asn Pro Ala Ile Val Asn Gly Ala Ser Asp Leu Ala Tyr
    2225                2230                2235

Val Met Tyr Thr Ser Gly Ser Thr Gly Lys Pro Lys Gly Val Met
    2240                2245                2250

Val Glu His Arg Asn Val Thr Arg Leu Val Met His Thr Asn Tyr
    2255                2260                2265

Val Gln Val Arg Glu Ser Asp Arg Met Ile Gln Thr Gly Ala Ile
    2270                2275                2280

Gly Phe Asp Ala Met Thr Phe Glu Ile Phe Gly Ala Leu Leu His

2285                2290                2295

Gly Ala Ser Leu Tyr Leu Val Ser Lys Asp Val Leu Leu Asp Ala
2300                2305                2310

Glu Lys Leu Gly Asp Phe Leu Arg Thr Asn Gln Ile Thr Thr Met
2315                2320                2325

Trp Leu Thr Ser Pro Leu Phe Asn Gln Leu Ser Gln Asp Asn Pro
2330                2335                2340

Ala Met Phe Asp Ser Leu Arg Ala Leu Ile Val Gly Gly Glu Ala
2345                2350                2355

Leu Ser Pro Lys His Ile Asn Arg Val Lys Ser Ala Leu Pro Asp
2360                2365                2370

Leu Glu Ile Trp Asn Gly Tyr Gly Pro Thr Glu Asn Thr Thr Phe
2375                2380                2385

Ser Thr Cys Tyr Leu Ile Glu Gln His Phe Glu Glu Gln Ile Pro
2390                2395                2400

Ile Gly Lys Pro Ile Ala Asn Ser Thr Ala Tyr Ile Val Asp Gly
2405                2410                2415

Asn Asn Gln Pro Gln Pro Ile Gly Val Pro Gly Glu Leu Cys Val
2420                2425                2430

Gly Gly Asp Gly Val Ala Arg Gly Tyr Val Asn Lys Pro Glu Leu
2435                2440                2445

Thr Ala Glu Lys Phe Val Pro Asn Pro Phe Ala Pro Gly Glu Thr
2450                2455                2460

Met Tyr Arg Thr Gly Asp Leu Ala Arg Trp Leu Pro Asp Gly Thr
2465                2470                2475

Ile Glu Tyr Leu Gly Arg Ile Asp Gln Gln Val Lys Ile Arg Gly
2480                2485                2490

Tyr Arg Ile Glu Leu Gly Glu Ile Glu Thr Val Leu Ser Gln Gln
2495                2500                2505

Ala Gln Val Lys Glu Ala Val Val Ala Val Ile Glu Glu Ala Asn
2510                2515                2520

Gly Gln Lys Ala Leu Cys Ala Tyr Phe Val Pro Glu Gln Ala Val
    2525                2530                2535

Asp Ala Ala Glu Leu Arg Glu Ala Met Ser Lys Gln Leu Pro Gly
    2540                2545                2550

Tyr Met Val Pro Ala Tyr Tyr Val Gln Met Glu Lys Leu Pro Leu
    2555                2560                2565

Thr Ala Asn Gly Lys Val Asp Arg Arg Ala Leu Pro Gln Pro Ser
    2570                2575                2580

Gly Glu Arg Thr Thr Gly Ser Ala Phe Val Ala Ala Gln Asn Asp
    2585                2590                2595

Thr Glu Ala Lys Leu Gln Gln Ile Trp Gln Glu Val Leu Gly Ile
    2600                2605                2610

Pro Ala Ile Gly Ile His Asp Asn Phe Phe Glu Ile Gly Gly His
    2615                2620                2625

Ser Leu Lys Ala Met Asn Val Ile Thr Gln Val His Lys Thr Phe
    2630                2635                2640

Gln Val Glu Leu Pro Leu Lys Ala Leu Phe Ala Thr Pro Thr Ile
    2645                2650                2655

His Glu Leu Ala Ala His Ile Ser Glu Lys Thr Glu Tyr Thr Ala
    2660                2665                2670

Ile Gln Pro Val Ala Ala Gln Glu Phe Tyr Pro Val Ser Ser Ala
    2675                2680                2685

Gln Lys Arg Met Tyr Ile Leu Gln Gln Phe Glu Gly Asn Gly Ile
    2690                2695                2700

Ser Tyr Asn Ile Ser Gly Ala Ile Leu Leu Glu Gly Lys Leu Asp
    2705                2710                2715

Tyr Ala Arg Phe Ala Ser Ala Val Gln Gln Leu Ala Glu Arg His
    2720                2725                2730

Glu Ala Leu Arg Thr Ser Phe His Arg Ile Asp Gly Glu Pro Val
    2735                2740                2745

Gln Lys Val His Glu Glu Val Glu Val Pro Leu Phe Met Leu Glu
    2750                2755                2760

Ala Pro Glu Asp Gln Ala Glu Lys Ile Met Arg Glu Phe Val Arg
    2765                2770                2775

Pro Phe Asp Leu Gly Val Ala Pro Leu Met Arg Thr Gly Leu Leu
    2780                2785                2790

Lys Leu Gly Lys Asp Arg His Leu Phe Leu Leu Asp Met His His
    2795                2800                2805

Ile Ile Ser Asp Gly Val Ser Ser Gln Ile Leu Leu Arg Glu Phe
    2810                2815                2820

Ala Glu Leu Tyr Gln Gly Ala Asp Leu Gln Pro Leu Ser Leu Gln
    2825                2830                2835

Tyr Lys Asp Phe Ala Ala Trp Gln Asn Glu Leu Phe Gln Thr Glu
    2840                2845                2850

Ala Tyr Lys Lys Gln Glu Gln His Trp Leu Asn Thr Phe Ala Asp
    2855                2860                2865

Glu Ile Pro Leu Leu Asn Leu Pro Thr Asp Tyr Pro Arg Pro Ser
    2870                2875                2880

Val Gln Ser Phe Ala Gly Asp Leu Val Leu Phe Ala Ala Gly Lys
    2885                2890                2895

Glu Leu Leu Glu Arg Leu Gln Gln Val Ala Ser Glu Thr Gly Thr
    2900                2905                2910

Thr Leu Tyr Met Ile Leu Leu Ala Ala Tyr Asn Val Leu Leu Ser
    2915                2920                2925

Lys Tyr Thr Gly Gln Glu Asp Ile Ile Val Gly Thr Pro Val Ala
    2930                2935                2940

Gly Arg Ser His Ala Asp Val Glu Asn Ile Met Gly Ile Phe Val
    2945                2950                2955

Asn Thr Leu Ala Leu Arg Asn Gln Pro Ala Ser Ser Lys Thr Met
    2960                2965                2970

Leu Glu Asn Asn Ile Thr Gln Cys Asp Ser Ile Asn Asp Val Tyr
    2975                2980                2985

Leu Lys Glu Glu Ala Ile Thr Leu Met Asp Met Leu Glu Ser Gln
    2990                2995                3000

Leu Lys His Gln Ala Asp Gly Tyr Val Val Ile Asp Gln Glu Glu
    3005                3010                3015

Ser Leu Ser Tyr Ala Asp Phe Tyr Leu Arg Val Lys Glu Ile Gly
    3020                3025                3030

Tyr Cys Leu Ser Glu Ile Ser Ser Lys Asn Ser Val Gly Ile Gly
    3035                3040                3045

Leu Phe Cys Asp Pro Ser Ile Asp Leu Ile Cys Gly Ala Trp Gly
    3050                3055                3060

Ile Leu Ser Ala Asp Lys Ala Tyr Leu Pro Leu Ser Pro Asp Tyr
    3065                3070                3075

Pro Thr Glu Arg Leu Lys Tyr Met Ile Glu Asp Ser Gly Ile Asp
    3080                3085                3090

Val Ile Phe Thr Gln Ser His Leu Lys Ala Gln Leu Gln Asp Ile
    3095                3100                3105

Ala Pro Lys Ser Val Leu Ile Met Thr Pro Glu Asp Val Ala Leu
    3110                3115                3120

Thr Ile Lys Thr Arg Thr Ile Glu Asp Ile Leu Gly Thr Val Gln
    3125                3130                3135

Val Pro Lys Pro Thr Ser Leu Ala Tyr Ile Ile Tyr Thr Ser Gly
    3140                3145                3150

Ser Thr Gly Lys Pro Lys Gly Val Met Ile Glu His His Ser Ile
    3155                3160                3165

Val Asn Gln Met Arg Phe Leu Ala Lys Ala Phe Lys Leu Gly Cys
    3170                3175                3180

His Ser Arg Ile Leu Gln Lys Thr Pro Met Ser Phe Asp Ala Ala
    3185                3190                3195

Gln Trp Glu Ile Leu Ala Pro Ala Ile Gly Gly Gln Val Ile Met
    3200                3205                3210

Gly Pro Leu Gly Cys Tyr Arg Asp Pro Asp Ala Ile Ile Lys Thr
    3215                3220                3225

Ile Leu Gln His Gln Val Thr Thr Leu Gln Cys Val Pro Thr Leu

3230                    3235                    3240

Leu Gln Ala Leu Leu Asp Asn Pro Asn Phe Leu Asp Cys Leu Ser
    3245                    3250                    3255

Leu Thr Gln Val Phe Ser Gly Gly Glu Ala Leu Thr Thr Lys Leu
    3260                    3265                    3270

Ala Thr Gln Phe Leu Asn Ser Phe Thr His Cys Glu Leu Ile Asn
    3275                    3280                    3285

Leu Tyr Gly Pro Thr Glu Cys Thr Ile Asn Ser Ser Phe Phe Arg
    3290                    3295                    3300

Val Thr Asn Glu Thr Leu Pro Asn Tyr Gln Thr Ser Ile Ser Ile
    3305                    3310                    3315

Gly Ala Pro Val Asp Asn Thr Glu Tyr Tyr Val Leu Asp Asp Asp
    3320                    3325                    3330

Arg Leu Pro Val Ala Val Gly Glu Ile Gly Glu Leu Tyr Ile Ser
    3335                    3340                    3345

Gly Ala Gln Leu Ala Arg Gly Tyr Leu His Lys Pro Glu Met Thr
    3350                    3355                    3360

Lys Asp Lys Phe Ile Cys Asn His Leu Val Ser Gly Thr Gln His
    3365                    3370                    3375

Gln Trp Leu Tyr Arg Thr Gly Asp Leu Val Thr Arg Gly Ala Asp
    3380                    3385                    3390

Gly Asn Thr Tyr Phe Val Gly Arg Val Asp Ser Gln Val Lys Leu
    3395                    3400                    3405

Arg Gly Tyr Arg Ile Glu Leu Asp Glu Ile Arg His Ala Ile Glu
    3410                    3415                    3420

Glu His Ser Trp Ile Lys Thr Ala Ala Met Leu Ile Lys Lys Asp
    3425                    3430                    3435

Ala Arg Thr Gly Phe Gln Asn Leu Ile Ala Cys Val Glu Leu Asp
    3440                    3445                    3450

Glu Lys Glu Ala Ala Leu Met Asp Gln Gly Asn Ser Ser Ser His
    3455                    3460                    3465

His Lys Ser Lys Ala Asp Lys Leu Gln Val Lys Ala Gln Leu Ser
    3470                3475                3480

Asn Ser Gly Cys Arg Ser Glu Glu Leu Cys Glu Asn Arg Pro Thr
    3485                3490                3495

Phe Leu Leu Pro Tyr Gln Glu Gly Glu Ile Lys Gln Arg Glu Tyr
    3500                3505                3510

Ala Phe Gly Arg Lys Thr Tyr Arg Tyr Phe Glu Gly Thr Glu Ile
    3515                3520                3525

Thr Val Glu Lys Leu Lys Lys Leu Leu Thr Ala Thr Gln Ser Asn
    3530                3535                3540

Glu Ile Ser Ser Leu Pro Leu Ser His Leu Thr Leu Asn Asp Phe
    3545                3550                3555

Gly Tyr Ala Leu Arg Tyr Phe Gly Gln Phe Thr Ser His Gln Arg
    3560                3565                3570

Leu Leu Pro Lys Tyr Ala Tyr Ala Ser Pro Gly Ala Leu Tyr Ala
    3575                3580                3585

Thr Gln Met Tyr Phe Glu Leu His Asn Val Leu Gly Leu Asp Ala
    3590                3595                3600

Gly Ile Tyr Tyr Tyr His Pro Val Thr His Lys Leu Ile Lys Ile
    3605                3610                3615

Ser Thr Leu Ser Arg Arg Gln Met Pro Thr Ile Lys Val His Phe
    3620                3625                3630

Ile Gly Lys His Glu Ala Ile Glu Pro Val Tyr Lys Asn Asn Ile
    3635                3640                3645

Gln Glu Val Leu Glu Met Glu Ala Gly His Met Met Gly Leu Phe
    3650                3655                3660

Asp Asp Val Leu Pro Glu Ile Gly Leu Ser Ile Gly Lys Ser Glu
    3665                3670                3675

Tyr Gln Asp Glu Cys Pro Asp Trp Tyr Asp Gly Asp Ile Gln Asp
    3680                3685                3690

Tyr Tyr Leu Gly Ala Phe Glu Ile Cys Ser Tyr Glu His Gly Leu
    3695                3700                3705

Pro Pro Phe Glu Thr Asp Ile Tyr Leu Gln Thr His Ala His Lys
    3710                3715                3720

Ile Pro Glu Met Pro Cys Gly Leu Tyr His Phe Ser Asn Gly Glu
    3725                3730                3735

Phe Val Arg Ile Ser Asp Asp Ile Val Arg Lys Lys Asp Val Ile
    3740                3745                3750

Ala Ile Asn Gln Gln Val Tyr Asp Arg Ser Ser Phe Gly Val Ser
    3755                3760                3765

Ile Ile Pro Arg Cys Val Pro Glu Trp His Tyr Tyr Ile Thr Leu
    3770                3775                3780

Gly Arg Arg Leu His Ala Leu Gln Ser Asn Pro Leu Tyr Ile Gly
    3785                3790                3795

Leu Met Ser Ser Gly Tyr Ser Ser Lys Ser Asn Asn Asp Leu Pro
    3800                3805                3810

Ser Ala Lys Arg Met Arg Ser Ile Leu Asn Ala Leu Asp Arg Pro
    3815                3820                3825

Met Ala Ala Phe Tyr Phe Cys Ile Gly Gly Gly Ile Ser Gln Ala
    3830                3835                3840

Gln Tyr Met Cys Glu Gly Met Lys Glu Asp Val Val His Met Lys
    3845                3850                3855

Gly Pro Val Glu Ile Ile Lys Asp Asp Leu Gln Gln Gln Leu Pro
    3860                3865                3870

Gln Tyr Met Ile Pro Asn Lys Val Leu Val Phe Asp Lys Leu Pro
    3875                3880                3885

Leu Thr Ala Asn Gly Lys Val Asp Tyr Gln Ser Leu Ser Glu Ser
    3890                3895                3900

Lys Ala Val Glu Asn Val Ser Thr Gln Arg Leu Leu Val Pro Leu
    3905                3910                3915

His Thr Asp Thr Glu Ile Arg Leu Gly Lys Ile Trp Met Glu Val
    3920                3925                3930

Leu Lys Trp Asp Ser Val Ser Ala Leu Asp Asp Phe Phe Glu Ser
    3935                3940                3945

Gly Gly Asn Ser Leu Met Ala Val Ala Met Val Asn Lys Ile Asn
    3950                3955                3960

Ala Ala Phe Asn Ile Arg Phe Pro Leu Gln Ile Leu Phe Gln Ser
    3965                3970                3975

Pro Asn Ile Ala Glu Leu Ala Lys Trp Ile Glu Gln Thr Asp Ser
    3980                3985                3990

Lys Thr Ile Ser Arg Leu Ile Leu Leu Asn Gln Ala Ser Lys Asp
    3995                4000                4005

Pro Ile Tyr Cys Trp Pro Gly Leu Gly Gly Tyr Pro Met Ser Leu
    4010                4015                4020

Arg Leu Leu Ala Asn Lys Val Val Pro Asp Arg Ala Phe Tyr Gly
    4025                4030                4035

Ile Gln Ala Tyr Gly Ile Asn Glu Ser Glu Ile Pro Phe Ser Ser
    4040                4045                4050

Ile Gln Arg Met Ala Glu Glu Asp Ile Lys Glu Ile Lys Lys Ile
    4055                4060                4065

Gln Pro Glu Gly Pro Tyr Ile Leu Trp Gly Tyr Ser Phe Gly Ala
    4070                4075                4080

Arg Val Ala Phe Glu Val Ala Tyr Gln Leu Glu Gln Ala Gly Glu
    4085                4090                4095

Glu Val Asn Ala Leu Asn Leu Leu Ala Pro Gly Ser Pro His Leu
    4100                4105                4110

Asp Met Lys Gln Ala Glu Tyr Met Asp Lys Gly Ala Glu Phe Thr
    4115                4120                4125

Asn Pro Ala Phe Val Lys Ile Leu Phe Ser Val Phe Ser Arg Ser
    4130                4135                4140

Ile Asn Ser Pro Met Val Lys Thr Cys Leu Glu Gln Val Asn Ser
    4145                4150                4155

Glu Thr Thr Phe Ile Asn Phe Ile Cys Ser Arg Phe Lys Asn Leu
    4160                4165                4170

Glu Pro Ser Leu Val Lys Arg Ile Val Arg Ile Val Thr Leu Thr

4175                4180                4185

Tyr Asp Phe Lys Tyr Ser Ile Asp Glu Leu Tyr His Arg His Leu
    4190                4195                4200

Lys Ala Pro Ile Thr Ile Phe Lys Ala Asn Arg Asp Asn Asp Ser
    4205                4210                4215

Phe Ile Glu Glu Ser Asp Val Ile Ser Ser Met Ser Pro Lys Ile
    4220                4225                4230

Ile Glu Leu Ile Ser Asp His Tyr Gln Leu Leu Glu Ser Glu Gly
    4235                4240                4245

Val Ala Glu Ile Glu Lys Ile Ile
    4250                4255

<210>  30
<211>  2194
<212>  PRT
<213>  Artificial Sequence

<220>
<223>  NRPSase of a fusion peptide consisting of Phenylalanine and
       Indigoidine

<400>  30

Met Leu Ala Asn Gln Ala Asn Leu Ile Asp Asn Lys Arg Glu Leu Glu
1               5                   10                  15

Gln His Ala Leu Val Pro Tyr Ala Gln Gly Lys Ser Ile His Gln Leu
            20                  25                  30

Phe Glu Glu Gln Ala Glu Ala Phe Pro Asp Arg Val Ala Ile Val Phe
        35                  40                  45

Glu Asn Arg Arg Leu Ser Tyr Gln Glu Leu Asn Arg Lys Ala Asn Gln
    50                  55                  60

Leu Ala Arg Ala Leu Leu Glu Lys Gly Val Gln Thr Asp Ser Ile Val
65                  70                  75                  80

Gly Val Met Met Glu Lys Ser Ile Glu Asn Val Ile Ala Ile Leu Ala
                85                  90                  95

Val Leu Lys Ala Gly Gly Ala Tyr Val Pro Ile Asp Ile Glu Tyr Pro
            100                 105                 110

Arg Asp Arg Ile Gln Tyr Ile Leu Gln Asp Ser Gln Thr Lys Ile Val

115                 120                 125

Leu Thr Gln Lys Ser Val Ser Gln Leu Val His Asp Val Gly Tyr Ser
    130                 135                 140

Gly Glu Val Val Val Leu Asp Glu Glu Gln Leu Asp Ala Arg Glu Thr
145                 150                 155                 160

Ala Asn Leu His Gln Pro Ser Lys Pro Thr Asp Leu Ala Tyr Val Ile
                165                 170                 175

Tyr Thr Ser Gly Thr Thr Gly Lys Pro Lys Gly Thr Met Leu Glu His
            180                 185                 190

Lys Gly Ile Ala Asn Leu Gln Ser Phe Phe Gln Asn Ser Phe Gly Val
        195                 200                 205

Thr Glu Gln Asp Arg Ile Gly Leu Phe Ala Ser Met Ser Phe Asp Ala
    210                 215                 220

Ser Val Trp Glu Met Phe Met Ala Leu Leu Ser Gly Ala Ser Leu Tyr
225                 230                 235                 240

Ile Leu Ser Lys Gln Thr Ile His Asp Phe Ala Ala Phe Glu His Tyr
                245                 250                 255

Leu Ser Glu Asn Glu Leu Thr Ile Ile Thr Leu Pro Pro Thr Tyr Leu
            260                 265                 270

Thr His Leu Thr Pro Glu Arg Ile Thr Ser Leu Arg Ile Met Ile Thr
        275                 280                 285

Ala Gly Ser Ala Ser Ser Ala Pro Leu Val Asn Lys Trp Lys Asp Lys
    290                 295                 300

Leu Arg Tyr Ile Asn Ala Tyr Gly Pro Thr Glu Thr Ser Ile Cys Ala
305                 310                 315                 320

Thr Ile Trp Glu Ala Pro Ser Asn Gln Leu Ser Val Gln Ser Val Pro
                325                 330                 335

Ile Gly Lys Pro Ile Gln Asn Thr His Ile Tyr Ile Val Asn Glu Asp
            340                 345                 350

Leu Gln Leu Leu Pro Thr Gly Ser Glu Gly Glu Leu Cys Ile Gly Gly
        355                 360                 365

Val Gly Leu Ala Arg Gly Tyr Trp Asn Arg Pro Asp Leu Thr Ala Glu
    370                 375                 380

Lys Phe Val Asp Asn Pro Phe Val Pro Gly Glu Lys Met Tyr Arg Thr
385                 390                 395                 400

Gly Asp Leu Ala Lys Trp Leu Thr Asp Gly Thr Ile Glu Phe Leu Gly
                405                 410                 415

Arg Ile Asp His Gln Val Lys Ile Arg Gly His Arg Ile Glu Leu Gly
            420                 425                 430

Glu Ile Glu Ser Val Leu Leu Ala His Glu His Ile Thr Glu Ala Val
        435                 440                 445

Val Ile Ala Arg Glu Asp Gln His Ala Gly Gln Tyr Leu Cys Ala Tyr
    450                 455                 460

Tyr Ile Ser Gln Gln Glu Ala Thr Pro Ala Gln Leu Arg Asp Tyr Ala
465                 470                 475                 480

Ala Gln Lys Leu Pro Ala Tyr Met Leu Pro Ser Tyr Phe Val Lys Leu
                485                 490                 495

Asp Lys Met Pro Leu Thr Pro Asn Asp Lys Ile Asp Arg Lys Ala Leu
            500                 505                 510

Pro Glu Pro Asp Leu Thr Ala Asn Gln Ser Gln Ala Ala Tyr His Pro
        515                 520                 525

Pro Arg Thr Glu Thr Glu Ser Ile Leu Val Ser Ile Trp Gln Asn Val
    530                 535                 540

Leu Gly Ile Glu Lys Ile Gly Ile Arg Asp Asn Phe Tyr Ser Leu Gly
545                 550                 555                 560

Gly Asp Ser Ile Gln Ala Ile Gln Val Val Ala Arg Leu His Ser Tyr
                565                 570                 575

Gln Leu Lys Leu Glu Thr Lys Asp Leu Leu Asn Tyr Pro Thr Ile Glu
            580                 585                 590

Gln Val Ala Glu Leu Ala Arg Phe Leu Ser Arg Ser Glu Lys Thr Glu
        595                 600                 605

Tyr Thr Ala Ile Gln Pro Val Ala Ala Gln Glu Phe Tyr Pro Val Ser
    610                 615                 620

Ser Ala Gln Lys Arg Met Tyr Ile Leu Gln Gln Phe Glu Gly Asn Gly
625                 630                 635                 640

Ile Ser Tyr Asn Ile Ser Gly Ala Ile Leu Leu Glu Gly Lys Leu Asp
                645                 650                 655

Tyr Ala Arg Phe Ala Ser Ala Val Gln Gln Leu Ala Glu Arg His Glu
            660                 665                 670

Ala Leu Arg Thr Ser Phe His Arg Ile Asp Gly Glu Pro Val Gln Lys
        675                 680                 685

Val His Glu Glu Val Glu Val Pro Leu Phe Met Leu Glu Ala Pro Glu
    690                 695                 700

Asp Gln Ala Glu Lys Ile Met Arg Glu Phe Val Arg Pro Phe Asp Leu
705                 710                 715                 720

Gly Val Ala Pro Leu Met Arg Thr Gly Leu Leu Lys Leu Gly Lys Asp
                725                 730                 735

Arg His Leu Phe Leu Leu Asp Met His His Ile Ile Ser Asp Gly Val
            740                 745                 750

Ser Ser Gln Ile Leu Leu Arg Glu Phe Ala Glu Leu Tyr Gln Gly Ala
        755                 760                 765

Asp Leu Gln Pro Leu Ser Leu Gln Tyr Lys Asp Phe Ala Ala Trp Gln
    770                 775                 780

Asn Glu Leu Phe Gln Thr Glu Ala Tyr Lys Lys Gln Glu Gln His Trp
785                 790                 795                 800

Leu Asn Thr Phe Ala Asp Glu Ile Pro Leu Leu Asn Leu Pro Thr Asp
                805                 810                 815

Tyr Pro Arg Pro Ser Val Gln Ser Phe Ala Gly Asp Leu Val Leu Phe
            820                 825                 830

Ala Ala Gly Lys Glu Leu Leu Glu Arg Leu Gln Gln Val Ala Ser Glu
        835                 840                 845

Thr Gly Thr Thr Leu Tyr Met Ile Leu Leu Ala Ala Tyr Asn Val Leu
    850                 855                 860

Leu Ser Lys Tyr Thr Gly Gln Glu Asp Ile Ile Val Gly Thr Pro Val
865                 870                 875                 880

Ala Gly Arg Ser His Ala Asp Val Glu Asn Ile Met Gly Ile Phe Val
                885                 890                 895

Asn Thr Leu Ala Leu Arg Asn Gln Pro Ala Ser Ser Lys Thr Met Leu
            900                 905                 910

Glu Asn Asn Ile Thr Gln Cys Asp Ser Ile Asn Asp Val Tyr Leu Lys
        915                 920                 925

Glu Glu Ala Ile Thr Leu Met Asp Met Leu Glu Ser Gln Leu Lys His
    930                 935                 940

Gln Ala Asp Gly Tyr Val Val Ile Asp Gln Glu Glu Ser Leu Ser Tyr
945                 950                 955                 960

Ala Asp Phe Tyr Leu Arg Val Lys Glu Ile Gly Tyr Cys Leu Ser Glu
                965                 970                 975

Ile Ser Ser Lys Asn Ser Val Gly Ile Gly Leu Phe Cys Asp Pro Ser
            980                 985                 990

Ile Asp Leu Ile Cys Gly Ala Trp  Gly Ile Leu Ser Ala  Asp Lys Ala
        995                 1000                 1005

Tyr Leu  Pro Leu Ser Pro Asp  Tyr Pro Thr Glu Arg  Leu Lys Tyr
    1010                 1015                 1020

Met Ile  Glu Asp Ser Gly Ile  Asp Val Ile Phe Thr  Gln Ser His
    1025                 1030                 1035

Leu Lys  Ala Gln Leu Gln Asp  Ile Ala Pro Lys Ser  Val Leu Ile
    1040                 1045                 1050

Met Thr  Pro Glu Asp Val Ala  Leu Thr Ile Lys Thr  Arg Thr Ile
    1055                 1060                 1065

Glu Asp  Ile Leu Gly Thr Val  Gln Val Pro Lys Pro  Thr Ser Leu
    1070                 1075                 1080

Ala Tyr  Ile Ile Tyr Thr Ser  Gly Ser Thr Gly Lys  Pro Lys Gly
    1085                 1090                 1095

Val Met  Ile Glu His His Ser  Ile Val Asn Gln Met  Arg Phe Leu
    1100                 1105                 1110

Ala Lys  Ala Phe Lys Leu Gly  Cys His Ser Arg Ile  Leu Gln Lys

1115                1120                1125

Thr Pro Met Ser Phe Asp Ala Ala Gln Trp Glu Ile Leu Ala Pro
    1130                1135                1140

Ala Ile Gly Gly Gln Val Ile Met Gly Pro Leu Gly Cys Tyr Arg
    1145                1150                1155

Asp Pro Asp Ala Ile Ile Lys Thr Ile Leu Gln His Gln Val Thr
    1160                1165                1170

Thr Leu Gln Cys Val Pro Thr Leu Leu Gln Ala Leu Leu Asp Asn
    1175                1180                1185

Pro Asn Phe Leu Asp Cys Leu Ser Leu Thr Gln Val Phe Ser Gly
    1190                1195                1200

Gly Glu Ala Leu Thr Thr Lys Leu Ala Thr Gln Phe Leu Asn Ser
    1205                1210                1215

Phe Thr His Cys Glu Leu Ile Asn Leu Tyr Gly Pro Thr Glu Cys
    1220                1225                1230

Thr Ile Asn Ser Ser Phe Phe Arg Val Thr Asn Glu Thr Leu Pro
    1235                1240                1245

Asn Tyr Gln Thr Ser Ile Ser Ile Gly Ala Pro Val Asp Asn Thr
    1250                1255                1260

Glu Tyr Tyr Val Leu Asp Asp Asp Arg Leu Pro Val Ala Val Gly
    1265                1270                1275

Glu Ile Gly Glu Leu Tyr Ile Ser Gly Ala Gln Leu Ala Arg Gly
    1280                1285                1290

Tyr Leu His Lys Pro Glu Met Thr Lys Asp Lys Phe Ile Cys Asn
    1295                1300                1305

His Leu Val Ser Gly Thr Gln His Gln Trp Leu Tyr Arg Thr Gly
    1310                1315                1320

Asp Leu Val Thr Arg Gly Ala Asp Gly Asn Thr Tyr Phe Val Gly
    1325                1330                1335

Arg Val Asp Ser Gln Val Lys Leu Arg Gly Tyr Arg Ile Glu Leu
    1340                1345                1350

Asp Glu Ile Arg His Ala Ile Glu Glu His Ser Trp Ile Lys Thr
    1355                1360                1365

Ala Ala Met Leu Ile Lys Lys Asp Ala Arg Thr Gly Phe Gln Asn
    1370                1375                1380

Leu Ile Ala Cys Val Glu Leu Asp Glu Lys Glu Ala Ala Leu Met
    1385                1390                1395

Asp Gln Gly Asn Ser Ser Ser His His Lys Ser Lys Ala Asp Lys
    1400                1405                1410

Leu Gln Val Lys Ala Gln Leu Ser Asn Ser Gly Cys Arg Ser Glu
    1415                1420                1425

Glu Leu Cys Glu Asn Arg Pro Thr Phe Leu Leu Pro Tyr Gln Glu
    1430                1435                1440

Gly Glu Ile Lys Gln Arg Glu Tyr Ala Phe Gly Arg Lys Thr Tyr
    1445                1450                1455

Arg Tyr Phe Glu Gly Thr Glu Ile Thr Val Glu Lys Leu Lys Lys
    1460                1465                1470

Leu Leu Thr Ala Thr Gln Ser Asn Glu Ile Ser Ser Leu Pro Leu
    1475                1480                1485

Ser His Leu Thr Leu Asn Asp Phe Gly Tyr Ala Leu Arg Tyr Phe
    1490                1495                1500

Gly Gln Phe Thr Ser His Gln Arg Leu Leu Pro Lys Tyr Ala Tyr
    1505                1510                1515

Ala Ser Pro Gly Ala Leu Tyr Ala Thr Gln Met Tyr Phe Glu Leu
    1520                1525                1530

His Asn Val Leu Gly Leu Asp Ala Gly Ile Tyr Tyr Tyr His Pro
    1535                1540                1545

Val Thr His Lys Leu Ile Lys Ile Ser Thr Leu Ser Arg Arg Gln
    1550                1555                1560

Met Pro Thr Ile Lys Val His Phe Ile Gly Lys His Glu Ala Ile
    1565                1570                1575

Glu Pro Val Tyr Lys Asn Asn Ile Gln Glu Val Leu Glu Met Glu
    1580                1585                1590

Ala Gly His Met Met Gly Leu Phe Asp Asp Val Leu Pro Glu Ile
    1595                1600                1605

Gly Leu Ser Ile Gly Lys Ser Glu Tyr Gln Asp Glu Cys Pro Asp
    1610                1615                1620

Trp Tyr Asp Gly Asp Ile Gln Asp Tyr Tyr Leu Gly Ala Phe Glu
    1625                1630                1635

Ile Cys Ser Tyr Glu His Gly Leu Pro Pro Phe Glu Thr Asp Ile
    1640                1645                1650

Tyr Leu Gln Thr His Ala His Lys Ile Pro Glu Met Pro Cys Gly
    1655                1660                1665

Leu Tyr His Phe Ser Asn Gly Glu Phe Val Arg Ile Ser Asp Asp
    1670                1675                1680

Ile Val Arg Lys Lys Asp Val Ile Ala Ile Asn Gln Gln Val Tyr
    1685                1690                1695

Asp Arg Ser Ser Phe Gly Val Ser Ile Ile Pro Arg Cys Val Pro
    1700                1705                1710

Glu Trp His Tyr Tyr Ile Thr Leu Gly Arg Arg Leu His Ala Leu
    1715                1720                1725

Gln Ser Asn Pro Leu Tyr Ile Gly Leu Met Ser Ser Gly Tyr Ser
    1730                1735                1740

Ser Lys Ser Asn Asn Asp Leu Pro Ser Ala Lys Arg Met Arg Ser
    1745                1750                1755

Ile Leu Asn Ala Leu Asp Arg Pro Met Ala Ala Phe Tyr Phe Cys
    1760                1765                1770

Ile Gly Gly Gly Ile Ser Gln Ala Gln Tyr Met Cys Glu Gly Met
    1775                1780                1785

Lys Glu Asp Val Val His Met Lys Gly Pro Val Glu Ile Ile Lys
    1790                1795                1800

Asp Asp Leu Gln Gln Gln Leu Pro Gln Tyr Met Ile Pro Asn Lys
    1805                1810                1815

Val Leu Val Phe Asp Lys Leu Pro Leu Thr Ala Asn Gly Lys Val
    1820                1825                1830

Asp Tyr Gln Ser Leu Ser Glu Ser Lys Ala Val Glu Asn Val Ser
    1835                1840                1845

Thr Gln Arg Leu Leu Val Pro Leu His Thr Asp Thr Glu Ile Arg
    1850                1855                1860

Leu Gly Lys Ile Trp Met Glu Val Leu Lys Trp Asp Ser Val Ser
    1865                1870                1875

Ala Leu Asp Asp Phe Phe Glu Ser Gly Gly Asn Ser Leu Met Ala
    1880                1885                1890

Val Ala Met Val Asn Lys Ile Asn Ala Ala Phe Asn Ile Arg Phe
    1895                1900                1905

Pro Leu Gln Ile Leu Phe Gln Ser Pro Asn Ile Ala Glu Leu Ala
    1910                1915                1920

Lys Trp Ile Glu Gln Thr Asp Ser Lys Thr Ile Ser Arg Leu Ile
    1925                1930                1935

Leu Leu Asn Gln Ala Ser Lys Asp Pro Ile Tyr Cys Trp Pro Gly
    1940                1945                1950

Leu Gly Gly Tyr Pro Met Ser Leu Arg Leu Leu Ala Asn Lys Val
    1955                1960                1965

Val Pro Asp Arg Ala Phe Tyr Gly Ile Gln Ala Tyr Gly Ile Asn
    1970                1975                1980

Glu Ser Glu Ile Pro Phe Ser Ser Ile Gln Arg Met Ala Glu Glu
    1985                1990                1995

Asp Ile Lys Glu Ile Lys Lys Ile Gln Pro Glu Gly Pro Tyr Ile
    2000                2005                2010

Leu Trp Gly Tyr Ser Phe Gly Ala Arg Val Ala Phe Glu Val Ala
    2015                2020                2025

Tyr Gln Leu Glu Gln Ala Gly Glu Glu Val Asn Ala Leu Asn Leu
    2030                2035                2040

Leu Ala Pro Gly Ser Pro His Leu Asp Met Lys Gln Ala Glu Tyr
    2045                2050                2055

Met Asp Lys Gly Ala Glu Phe Thr Asn Pro Ala Phe Val Lys Ile

2060                2065                2070

Leu Phe  Ser Val Phe Ser Arg  Ser Ile Asn Ser Pro  Met Val Lys
    2075                2080                2085

Thr Cys  Leu Glu Gln Val Asn  Ser Glu Thr Thr Phe  Ile Asn Phe
    2090                2095                2100

Ile Cys  Ser Arg Phe Lys Asn  Leu Glu Pro Ser Leu  Val Lys Arg
    2105                2110                2115

Ile Val  Arg Ile Val Thr Leu  Thr Tyr Asp Phe Lys  Tyr Ser Ile
    2120                2125                2130

Asp Glu  Leu Tyr His Arg His  Leu Lys Ala Pro Ile  Thr Ile Phe
    2135                2140                2145

Lys Ala  Asn Arg Asp Asn Asp  Ser Phe Ile Glu Glu  Ser Asp Val
    2150                2155                2160

Ile Ser  Ser Met Ser Pro Lys  Ile Ile Glu Leu Ile  Ser Asp His
    2165                2170                2175

Tyr Gln  Leu Leu Glu Ser Glu  Gly Val Ala Glu Ile  Glu Lys Ile
    2180                2185                2190

Ile

<210>  31
<211>  4744
<212>  PRT
<213>  Artificial Sequence

<220>
<223>  NRPSase synthesizing a Indigoidine-tagged Tripeptide consisting
       of Phenylalanine, Ornithine and Leucine

<400>  31

Met Leu Ala Asn Gln Ala Asn Leu Ile Asp Asn Lys Arg Glu Leu Glu
1               5                   10                  15

Gln His Ala Leu Val Pro Tyr Ala Gln Gly Lys Ser Ile His Gln Leu
            20                  25                  30

Phe Glu Glu Gln Ala Glu Ala Phe Pro Asp Arg Val Ala Ile Val Phe
        35                  40                  45

Glu Asn Arg Arg Leu Ser Tyr Gln Glu Leu Asn Arg Lys Ala Asn Gln

50                  55                  60

Leu Ala Arg Ala Leu Leu Glu Lys Gly Val Gln Thr Asp Ser Ile Val
65                  70                  75                  80

Gly Val Met Met Glu Lys Ser Ile Glu Asn Val Ile Ala Ile Leu Ala
                85                  90                  95

Val Leu Lys Ala Gly Gly Ala Tyr Val Pro Ile Asp Ile Glu Tyr Pro
            100                 105                 110

Arg Asp Arg Ile Gln Tyr Ile Leu Gln Asp Ser Gln Thr Lys Ile Val
        115                 120                 125

Leu Thr Gln Lys Ser Val Ser Gln Leu Val His Asp Val Gly Tyr Ser
    130                 135                 140

Gly Glu Val Val Val Leu Asp Glu Glu Gln Leu Asp Ala Arg Glu Thr
145                 150                 155                 160

Ala Asn Leu His Gln Pro Ser Lys Pro Thr Asp Leu Ala Tyr Val Ile
                165                 170                 175

Tyr Thr Ser Gly Thr Thr Gly Lys Pro Lys Gly Thr Met Leu Glu His
            180                 185                 190

Lys Gly Ile Ala Asn Leu Gln Ser Phe Phe Gln Asn Ser Phe Gly Val
        195                 200                 205

Thr Glu Gln Asp Arg Ile Gly Leu Phe Ala Ser Met Ser Phe Asp Ala
    210                 215                 220

Ser Val Trp Glu Met Phe Met Ala Leu Leu Ser Gly Ala Ser Leu Tyr
225                 230                 235                 240

Ile Leu Ser Lys Gln Thr Ile His Asp Phe Ala Ala Phe Glu His Tyr
                245                 250                 255

Leu Ser Glu Asn Glu Leu Thr Ile Ile Thr Leu Pro Pro Thr Tyr Leu
            260                 265                 270

Thr His Leu Thr Pro Glu Arg Ile Thr Ser Leu Arg Ile Met Ile Thr
        275                 280                 285

Ala Gly Ser Ala Ser Ser Ala Pro Leu Val Asn Lys Trp Lys Asp Lys
    290                 295                 300

Leu Arg Tyr Ile Asn Ala Tyr Gly Pro Thr Glu Thr Ser Ile Cys Ala
305                 310                 315                 320

Thr Ile Trp Glu Ala Pro Ser Asn Gln Leu Ser Val Gln Ser Val Pro
                325                 330                 335

Ile Gly Lys Pro Ile Gln Asn Thr His Ile Tyr Ile Val Asn Glu Asp
            340                 345                 350

Leu Gln Leu Leu Pro Thr Gly Ser Glu Gly Glu Leu Cys Ile Gly Gly
        355                 360                 365

Val Gly Leu Ala Arg Gly Tyr Trp Asn Arg Pro Asp Leu Thr Ala Glu
    370                 375                 380

Lys Phe Val Asp Asn Pro Phe Val Pro Gly Glu Lys Met Tyr Arg Thr
385                 390                 395                 400

Gly Asp Leu Ala Lys Trp Leu Thr Asp Gly Thr Ile Glu Phe Leu Gly
                405                 410                 415

Arg Ile Asp His Gln Val Lys Ile Arg Gly His Arg Ile Glu Leu Gly
            420                 425                 430

Glu Ile Glu Ser Val Leu Leu Ala His Glu His Ile Thr Glu Ala Val
        435                 440                 445

Val Ile Ala Arg Glu Asp Gln His Ala Gly Gln Tyr Leu Cys Ala Tyr
    450                 455                 460

Tyr Ile Ser Gln Gln Glu Ala Thr Pro Ala Gln Leu Arg Asp Tyr Ala
465                 470                 475                 480

Ala Gln Lys Leu Pro Ala Tyr Met Leu Pro Ser Tyr Phe Val Lys Leu
                485                 490                 495

Asp Lys Met Pro Leu Thr Pro Asn Asp Lys Ile Asp Arg Lys Ala Leu
            500                 505                 510

Pro Glu Pro Asp Leu Thr Ala Asn Gln Ser Gln Ala Ala Tyr His Pro
        515                 520                 525

Pro Arg Thr Glu Thr Glu Ser Ile Leu Val Ser Ile Trp Gln Asn Val
    530                 535                 540

Leu Gly Ile Glu Lys Ile Gly Ile Arg Asp Asn Phe Tyr Ser Leu Gly
545                 550                 555                 560

Gly Asp Ser Ile Gln Ala Ile Gln Val Val Ala Arg Leu His Ser Tyr
                565                 570                 575

Gln Leu Lys Leu Glu Thr Lys Asp Leu Leu Asn Tyr Pro Thr Ile Glu
            580                 585                 590

Gln Val Ala Leu Phe Val Lys Ser Thr Thr Arg Lys Ser Asp Gln Gly
        595                 600                 605

Ile Ile Ala Gly Asn Val Pro Leu Thr Pro Ile Gln Lys Trp Phe Phe
    610                 615                 620

Gly Lys Asn Phe Thr Asn Thr Gly His Trp Asn Gln Ser Ser Val Leu
625                 630                 635                 640

Tyr Arg Pro Glu Gly Phe Asp Pro Lys Val Ile Gln Ser Val Met Asp
                645                 650                 655

Lys Ile Ile Glu His His Asp Ala Leu Arg Met Val Tyr Gln His Glu
            660                 665                 670

Asn Gly Asn Val Val Gln His Asn Arg Gly Leu Gly Gly Gln Leu Tyr
        675                 680                 685

Asp Phe Phe Ser Tyr Asn Leu Thr Ala Gln Pro Asp Val Gln Gln Ala
    690                 695                 700

Ile Glu Ala Glu Thr Gln Arg Leu His Ser Ser Met Asn Leu Gln Glu
705                 710                 715                 720

Gly Pro Leu Val Lys Val Ala Leu Phe Gln Thr Leu His Gly Asp His
                725                 730                 735

Leu Phe Leu Ala Ile His His Leu Val Val Asp Gly Ile Ser Trp Arg
            740                 745                 750

Ile Leu Phe Glu Asp Leu Ala Thr Gly Tyr Ala Gln Ala Leu Ala Gly
        755                 760                 765

Gln Ala Ile Ser Leu Pro Glu Lys Thr Asp Ser Phe Gln Ser Trp Ser
    770                 775                 780

Gln Trp Leu Gln Glu Tyr Ala Asn Glu Ala Asp Leu Leu Ser Glu Ile
785                 790                 795                 800

Pro Tyr Trp Glu Ser Leu Glu Ser Gln Ala Lys Asn Val Ser Leu Pro
                805                 810                 815

Lys Asp Tyr Glu Val Thr Asp Cys Lys Gln Lys Ser Val Arg Asn Met
            820                 825                 830

Arg Ile Arg Leu His Pro Glu Glu Thr Glu Gln Leu Leu Lys His Ala
        835                 840                 845

Asn Gln Ala Tyr Gln Thr Glu Ile Asn Asp Leu Leu Leu Ala Ala Leu
    850                 855                 860

Gly Leu Ala Phe Ala Glu Trp Ser Lys Leu Ala Gln Ile Val Ile His
865                 870                 875                 880

Leu Glu Gly His Gly Arg Glu Asp Ile Ile Glu Gln Ala Asn Val Ala
                885                 890                 895

Arg Thr Val Gly Trp Phe Thr Ser Gln Tyr Pro Val Leu Leu Asp Leu
            900                 905                 910

Lys Gln Thr Ala Pro Leu Ser Asp Tyr Ile Lys Leu Thr Lys Glu Asn
        915                 920                 925

Met Arg Lys Ile Pro Arg Lys Gly Ile Gly Tyr Asp Ile Leu Lys His
    930                 935                 940

Val Thr Leu Pro Glu Asn Arg Gly Ser Leu Ser Phe Arg Val Gln Pro
945                 950                 955                 960

Glu Val Thr Phe Asn Tyr Leu Gly Gln Phe Asp Ala Asp Met Arg Thr
                965                 970                 975

Glu Leu Phe Thr Arg Ser Pro Tyr Ser Gly Gly Asn Thr Leu Gly Ala
            980                 985                 990

Asp Gly Lys Asn Asn Leu Ser Pro Glu Ser Glu Val Tyr Thr Ala Leu
        995                 1000                1005

Asn Ile Thr Gly Leu Ile Glu Gly Gly Glu Leu Val Leu Thr Phe
    1010                1015                1020

Ser Tyr Ser Ser Glu Gln Tyr Arg Glu Glu Ser Ile Gln Gln Leu
    1025                1030                1035

Ser Gln Ser Tyr Gln Lys His Leu Leu Ala Ile Ile Ala His Cys
    1040                1045                1050

Thr Glu Lys Lys Glu Val Glu Arg Thr Ala His Ile Ala Glu Ser

1055                1060                1065

Ala Phe Glu Gln Phe Glu Thr Ile Gln Pro Val Glu Pro Ala Ala
    1070                1075                1080

Phe Tyr Pro Val Ser Phe Ala Gln Lys Arg Met Tyr Ile Leu His
    1085                1090                1095

Gln Phe Glu Gly Ser Gly Ile Ser Tyr Asn Val Pro Ser Val Leu
    1100                1105                1110

Val Leu Glu Gly Lys Leu Asp Tyr Asp Arg Phe Ala Ala Ala Ile
    1115                1120                1125

Gln Ser Leu Val Lys Arg His Glu Ser Leu Arg Thr Ser Phe His
    1130                1135                1140

Ser Val Asn Gly Glu Pro Leu Gln Arg Val His Pro Asp Val Glu
    1145                1150                1155

Leu Pro Val Arg Leu Leu Glu Ala Thr Glu Asp Gln Ser Glu Ser
    1160                1165                1170

Leu Ile Gln Glu Leu Ile Gln Pro Phe Asp Leu Glu Ile Ala Pro
    1175                1180                1185

Leu Phe Arg Val Asn Leu Ile Lys Leu Gly Ala Glu Arg His Leu
    1190                1195                1200

Phe Phe Met Asp Met His His Ile Ile Ser Asp Gly Val Ser Leu
    1205                1210                1215

Ala Val Ile Val Glu Glu Ile Ala Ser Leu Tyr Ala Gly Lys Gln
    1220                1225                1230

Leu Ser Asp Leu Arg Ile Gln Tyr Lys Asp Phe Ala Val Trp Gln
    1235                1240                1245

Thr Lys Leu Ala Gln Ser Asp Arg Phe Gln Lys Gln Glu Asp Phe
    1250                1255                1260

Trp Thr Arg Thr Phe Ala Gly Glu Ile Pro Leu Leu Asn Leu Pro
    1265                1270                1275

His Asp Tyr Pro Arg Pro Ser Val Gln Ser Phe Asp Gly Asp Thr
    1280                1285                1290

Val Ala Leu Gly Thr Gly His His Leu Leu Glu Gln Leu Arg Lys
    1295                1300                1305

Leu Ala Ala Glu Thr Gly Thr Thr Leu Phe Met Val Leu Leu Ala
    1310                1315                1320

Ala Tyr His Val Leu Leu Ser Lys Tyr Ala Gly Gln Glu Glu Ile
    1325                1330                1335

Val Val Gly Thr Pro Ile Ala Gly Arg Ser His Ala Asp Val Glu
    1340                1345                1350

Arg Ile Val Gly Met Phe Val Asn Thr Leu Ala Leu Lys Asn Thr
    1355                1360                1365

Ala Ala Gly Ser Leu Ser Phe Arg Ala Phe Leu Glu Asp Val Lys
    1370                1375                1380

Gln Asn Ala Leu His Ala Phe Glu His Gln Asp Tyr Pro Phe Glu
    1385                1390                1395

His Leu Val Glu Lys Leu Gln Val Arg Arg Asp Leu Ser Arg Asn
    1400                1405                1410

Pro Leu Phe Asp Thr Met Phe Ser Leu Gly Leu Ala Glu Ser Ala
    1415                1420                1425

Glu Gly Glu Val Ala Asp Leu Lys Val Ser Pro Tyr Pro Val Asn
    1430                1435                1440

Gly His Ile Ala Lys Phe Asp Leu Ser Leu Asp Ala Met Glu Lys
    1445                1450                1455

Gln Asp Gly Leu Leu Val Gln Phe Ser Tyr Cys Thr Lys Leu Phe
    1460                1465                1470

Ala Lys Glu Thr Val Asp Arg Leu Ala Ala His Tyr Val Gln Leu
    1475                1480                1485

Leu Gln Thr Ile Thr Ala Asp Pro Asp Ile Glu Leu Ala Arg Ile
    1490                1495                1500

Ser Val Leu Ser Lys Ala Glu Thr Glu His Met Leu His Ser Phe
    1505                1510                1515

Leu Ala Thr Lys Thr Ala Tyr Pro Thr Asp Lys Thr Phe Gln Lys
    1520                1525                1530

Leu Phe Glu Glu Gln Val Glu Lys Thr Pro Asn Glu Ile Ala Val
    1535                1540                1545

Leu Phe Gly Asn Glu Gln Leu Thr Tyr Gln Glu Leu Asn Ala Lys
    1550                1555                1560

Ala Asn Gln Leu Ala Arg Val Leu Arg Arg Lys Gly Val Lys Pro
    1565                1570                1575

Glu Ser Thr Val Gly Ile Leu Val Asp Arg Ser Leu Tyr Met Val
    1580                1585                1590

Ile Gly Met Leu Ala Val Leu Lys Ala Gly Gly Thr Phe Val Pro
    1595                1600                1605

Ile Asp Pro Asp Tyr Pro Leu Glu Arg Gln Ala Phe Met Leu Glu
    1610                1615                1620

Asp Ser Glu Ala Lys Leu Leu Leu Thr Leu Gln Lys Met Asn Ser
    1625                1630                1635

Gln Val Ala Phe Pro Tyr Glu Thr Phe Tyr Leu Asp Thr Glu Thr
    1640                1645                1650

Val Asp Gln Glu Glu Thr Gly Asn Leu Glu His Val Ala Gln Pro
    1655                1660                1665

Glu Asn Val Ala Tyr Ile Ile Tyr Thr Ser Gly Thr Thr Gly Lys
    1670                1675                1680

Pro Lys Gly Val Val Ile Glu His Arg Ser Tyr Ala Asn Val Ala
    1685                1690                1695

Phe Ala Trp Lys Asp Glu Tyr His Leu Asp Ser Phe Pro Val Arg
    1700                1705                1710

Leu Leu Gln Met Ala Ser Phe Ala Phe Asp Val Ser Thr Gly Asp
    1715                1720                1725

Phe Ala Arg Ala Leu Leu Thr Gly Gly Gln Leu Val Ile Cys Pro
    1730                1735                1740

Asn Gly Val Lys Met Asp Pro Ala Ser Leu Tyr Glu Thr Ile Arg
    1745                1750                1755

Arg His Glu Ile Thr Ile Phe Glu Ala Thr Pro Ala Leu Ile Met
    1760                1765                1770

Pro Leu Met His Tyr Val Tyr Glu Asn Glu Leu Asp Met Ser Gln
    1775                1780                1785

Met Lys Leu Leu Ile Leu Gly Ala Asp Ser Cys Pro Ala Glu Asp
    1790                1795                1800

Phe Lys Thr Leu Leu Ala Arg Phe Gly Gln Lys Met Arg Ile Ile
    1805                1810                1815

Asn Ser Tyr Gly Val Thr Glu Ala Cys Ile Asp Thr Ser Tyr Tyr
    1820                1825                1830

Glu Glu Thr Asp Val Thr Ala Ile Arg Ser Gly Thr Val Pro Ile
    1835                1840                1845

Gly Lys Pro Leu Pro Asn Met Thr Met Tyr Val Val Asp Ala His
    1850                1855                1860

Leu Asn Leu Gln Pro Val Gly Val Val Gly Glu Leu Cys Ile Gly
    1865                1870                1875

Gly Ala Gly Val Ala Arg Gly Tyr Leu Asn Arg Pro Glu Leu Thr
    1880                1885                1890

Glu Glu Lys Phe Val Pro Asn Pro Phe Ala Pro Gly Glu Arg Leu
    1895                1900                1905

Tyr Arg Thr Gly Asp Leu Ala Lys Trp Arg Ala Asp Gly Asn Val
    1910                1915                1920

Glu Phe Leu Gly Arg Asn Asp His Gln Val Lys Ile Arg Gly Val
    1925                1930                1935

Arg Ile Glu Leu Gly Glu Ile Glu Thr Gln Leu Arg Lys Leu Asp
    1940                1945                1950

Gly Ile Thr Glu Ala Val Val Val Ala Arg Glu Asp Arg Gly Gln
    1955                1960                1965

Glu Lys Glu Leu Cys Ala Tyr Val Val Ala Asp His Lys Leu Asp
    1970                1975                1980

Thr Ala Glu Leu Arg Ala Asn Leu Leu Lys Glu Leu Pro Gln Ala
    1985                1990                1995

Met Ile Pro Ala Tyr Phe Val Thr Leu Asp Ala Leu Pro Leu Thr

2000                2005                2010

Ala Asn Gly Lys Val Asp Arg Arg Ser Leu Pro Ala Pro Asp Val
    2015                2020                2025

Thr Met Leu Arg Thr Thr Glu Tyr Val Ala Pro Arg Ser Val Trp
    2030                2035                2040

Glu Ala Arg Leu Ala Gln Val Trp Glu Gln Val Leu Asn Val Pro
    2045                2050                2055

Gln Val Gly Ala Leu Asp Asp Phe Phe Ala Leu Gly Gly His Ser
    2060                2065                2070

Leu Arg Ala Met Arg Val Leu Ser Ser Met His Asn Glu Tyr Gln
    2075                2080                2085

Val Asp Ile Pro Leu Arg Ile Leu Phe Glu Lys Pro Thr Ile Gln
    2090                2095                2100

Glu Leu Ala Ala Phe Ile Glu Glu Thr Ala Lys Gly Asn Val Phe
    2105                2110                2115

Ser Ile Glu Pro Val Gln Lys Gln Ala Tyr Tyr Pro Val Ser Ser
    2120                2125                2130

Ala Gln Lys Arg Met Tyr Ile Leu Asp Gln Phe Glu Gly Val Gly
    2135                2140                2145

Ile Ser Tyr Asn Met Pro Ser Thr Met Leu Ile Glu Gly Lys Leu
    2150                2155                2160

Glu Arg Thr Arg Val Glu Ala Ala Phe Gln Arg Leu Ile Ala Arg
    2165                2170                2175

His Glu Ser Leu Arg Thr Ser Phe Ala Val Val Asn Gly Glu Pro
    2180                2185                2190

Val Gln Asn Ile His Glu Asp Val Pro Phe Ala Leu Ala Tyr Ser
    2195                2200                2205

Glu Val Thr Glu Gln Glu Ala Arg Glu Leu Val Ser Ser Leu Val
    2210                2215                2220

Gln Pro Phe Asp Leu Glu Val Ala Pro Leu Ile Arg Val Ser Leu
    2225                2230                2235

Leu Lys Ile Gly Glu Asp Arg Tyr Val Leu Phe Thr Asp Met His
    2240                2245                2250

His Ser Ile Ser Asp Gly Val Ser Ser Gly Ile Leu Leu Ala Glu
    2255                2260                2265

Trp Val Gln Leu Tyr Gln Gly Asp Val Leu Pro Glu Leu Arg Ile
    2270                2275                2280

Gln Tyr Lys Asp Phe Ala Val Trp Gln Gln Glu Phe Ser Gln Ser
    2285                2290                2295

Ala Ala Phe His Lys Gln Glu Ala Tyr Trp Leu Gln Thr Phe Ala
    2300                2305                2310

Asp Asp Ile Pro Val Leu Asn Leu Pro Thr Asp Phe Thr Arg Pro
    2315                2320                2325

Ser Thr Gln Ser Phe Ala Gly Asp Gln Cys Thr Ile Gly Ala Gly
    2330                2335                2340

Lys Ala Leu Thr Glu Gly Leu His Gln Leu Ala Gln Ala Thr Gly
    2345                2350                2355

Thr Thr Leu Tyr Met Val Leu Leu Ala Ala Tyr Asn Val Leu Leu
    2360                2365                2370

Ala Lys Tyr Ala Gly Gln Glu Asp Ile Ile Val Gly Thr Pro Ile
    2375                2380                2385

Thr Gly Arg Ser His Ala Asp Leu Glu Pro Ile Val Gly Met Phe
    2390                2395                2400

Val Asn Thr Leu Ala Met Arg Asn Lys Pro Gln Arg Glu Lys Thr
    2405                2410                2415

Phe Ser Glu Phe Leu Gln Glu Val Lys Gln Asn Ala Leu Asp Ala
    2420                2425                2430

Tyr Gly His Gln Asp Tyr Pro Phe Glu Glu Leu Val Glu Lys Leu
    2435                2440                2445

Ala Ile Ala Arg Asp Leu Ser Arg Asn Pro Leu Phe Asp Thr Val
    2450                2455                2460

Phe Thr Phe Gln Asn Ser Thr Glu Glu Val Met Thr Leu Pro Glu
    2465                2470                2475

Cys Thr Leu Ala Pro Phe Met Thr Asp Glu Thr Gly Gln His Ala
    2480                2485                2490

Lys Phe Asp Leu Thr Phe Ser Ala Thr Glu Glu Arg Glu Glu Met
    2495                2500                2505

Thr Ile Gly Val Glu Tyr Ser Thr Ser Leu Phe Thr Arg Glu Thr
    2510                2515                2520

Met Glu Arg Phe Ser Arg His Phe Leu Thr Ile Ala Ala Ser Ile
    2525                2530                2535

Val Gln Asn Pro His Ile Arg Leu Gly Glu Ile Asp Met Leu Leu
    2540                2545                2550

Pro Glu Glu Lys Gln Gln Ile Leu Ala Gly Phe Asn Asp Thr Ala
    2555                2560                2565

Val Ser Tyr Ala Leu Asp Lys Thr Leu His Gln Leu Phe Glu Glu
    2570                2575                2580

Gln Val Asp Lys Thr Pro Asp Gln Ala Ala Leu Leu Phe Ser Glu
    2585                2590                2595

Gln Ser Leu Thr Tyr Ser Glu Leu Asn Glu Arg Ala Asn Arg Leu
    2600                2605                2610

Ala Arg Val Leu Arg Ala Lys Gly Val Gly Pro Asp Arg Leu Val
    2615                2620                2625

Ala Ile Met Ala Glu Arg Ser Pro Glu Met Val Ile Gly Ile Leu
    2630                2635                2640

Gly Ile Leu Lys Ala Gly Gly Ala Tyr Val Pro Val Asp Pro Gly
    2645                2650                2655

Tyr Pro Gln Glu Arg Ile Gln Tyr Leu Leu Glu Asp Ser Asn Ala
    2660                2665                2670

Ala Leu Leu Leu Ser Gln Ala His Leu Leu Pro Leu Leu Ala Gln
    2675                2680                2685

Val Ser Ser Glu Leu Pro Glu Cys Leu Asp Leu Asn Ala Glu Leu
    2690                2695                2700

Asp Ala Gly Leu Ser Gly Ser Asn Leu Pro Ala Val Asn Gln Pro
    2705                2710                2715

Thr Asp Leu Ala Tyr Val Ile Tyr Thr Ser Gly Thr Thr Gly Lys
    2720                2725                2730

Pro Lys Gly Val Met Ile Pro His Gln Gly Ile Val Asn Cys Leu
    2735                2740                2745

Gln Trp Arg Arg Asp Glu Tyr Gly Phe Gly Pro Ser Asp Lys Ala
    2750                2755                2760

Leu Gln Val Phe Ser Phe Ala Phe Asp Gly Phe Val Ala Ser Leu
    2765                2770                2775

Phe Ala Pro Leu Leu Gly Gly Ala Thr Cys Val Leu Pro Gln Glu
    2780                2785                2790

Ala Ala Ala Lys Asp Pro Val Ala Leu Lys Lys Leu Met Ala Ala
    2795                2800                2805

Thr Glu Val Thr His Tyr Tyr Gly Val Pro Ser Leu Phe Gln Ala
    2810                2815                2820

Ile Leu Asp Cys Ser Thr Thr Thr Asp Phe Asn Gln Leu Arg Cys
    2825                2830                2835

Val Thr Leu Gly Gly Glu Lys Leu Pro Val Gln Leu Val Gln Lys
    2840                2845                2850

Thr Lys Glu Lys His Pro Ala Ile Glu Ile Asn Asn Glu Tyr Gly
    2855                2860                2865

Pro Thr Glu Asn Ser Val Val Thr Thr Ile Ser Arg Ser Ile Glu
    2870                2875                2880

Ala Gly Gln Ala Ile Thr Ile Gly Arg Pro Leu Ala Asn Val Gln
    2885                2890                2895

Val Tyr Ile Val Asp Glu Gln His His Leu Gln Pro Ile Gly Val
    2900                2905                2910

Val Gly Glu Leu Cys Ile Gly Gly Ala Gly Leu Ala Arg Gly Tyr
    2915                2920                2925

Leu Asn Lys Pro Glu Leu Thr Ala Glu Lys Phe Val Ala Asn Pro
    2930                2935                2940

Phe Arg Pro Gly Glu Arg Met Tyr Lys Thr Gly Asp Leu Val Lys

2945                2950                2955

Trp Arg Thr Asp Gly Thr Ile Glu Tyr Ile Gly Arg Ala Asp Glu
    2960                2965                2970

Gln Val Lys Val Arg Gly Tyr Arg Ile Glu Ile Gly Glu Ile Glu
    2975                2980                2985

Ser Ala Val Leu Ala Tyr Gln Gly Ile Asp Gln Ala Val Val Val
    2990                2995                3000

Ala Arg Asp Asp Asp Ala Thr Ala Gly Ser Tyr Leu Cys Ala Tyr
    3005                3010                3015

Phe Val Ala Ala Thr Ala Val Ser Val Ser Gly Leu Arg Ser His
    3020                3025                3030

Leu Ala Lys Glu Leu Pro Ala Tyr Met Ile Pro Ser Tyr Phe Val
    3035                3040                3045

Glu Leu Asp Gln Leu Pro Leu Ser Ala Asn Gly Lys Val Asp Arg
    3050                3055                3060

Lys Ala Leu Pro Lys Pro Gln Gln Ser Asp Ala Thr Thr Arg Glu
    3065                3070                3075

Tyr Val Ala Pro Arg Asn Ala Thr Glu Gln Gln Leu Ala Ala Ile
    3080                3085                3090

Trp Gln Glu Val Leu Gly Val Glu Pro Ile Gly Ile Thr Asp Gln
    3095                3100                3105

Phe Phe Glu Leu Gly Gly His Ser Leu Lys Ala Thr Leu Leu Ile
    3110                3115                3120

Ala Lys Val Tyr Glu Tyr Met Gln Ile Glu Leu Pro Leu Asn Leu
    3125                3130                3135

Ile Phe Gln Tyr Pro Thr Ile Glu Lys Val Ala Asp Phe Ile Thr
    3140                3145                3150

Ser Glu Lys Thr Glu Tyr Thr Ala Ile Gln Pro Val Ala Ala Gln
    3155                3160                3165

Glu Phe Tyr Pro Val Ser Ser Ala Gln Lys Arg Met Tyr Ile Leu
    3170                3175                3180

Gln Gln Phe Glu Gly Asn Gly Ile Ser Tyr Asn Ile Ser Gly Ala
    3185                3190                3195

Ile Leu Leu Glu Gly Lys Leu Asp Tyr Ala Arg Phe Ala Ser Ala
    3200                3205                3210

Val Gln Gln Leu Ala Glu Arg His Glu Ala Leu Arg Thr Ser Phe
    3215                3220                3225

His Arg Ile Asp Gly Glu Pro Val Gln Lys Val His Glu Glu Val
    3230                3235                3240

Glu Val Pro Leu Phe Met Leu Glu Ala Pro Glu Asp Gln Ala Glu
    3245                3250                3255

Lys Ile Met Arg Glu Phe Val Arg Pro Phe Asp Leu Gly Val Ala
    3260                3265                3270

Pro Leu Met Arg Thr Gly Leu Leu Lys Leu Gly Lys Asp Arg His
    3275                3280                3285

Leu Phe Leu Leu Asp Met His His Ile Ile Ser Asp Gly Val Ser
    3290                3295                3300

Ser Gln Ile Leu Leu Arg Glu Phe Ala Glu Leu Tyr Gln Gly Ala
    3305                3310                3315

Asp Leu Gln Pro Leu Ser Leu Gln Tyr Lys Asp Phe Ala Ala Trp
    3320                3325                3330

Gln Asn Glu Leu Phe Gln Thr Glu Ala Tyr Lys Lys Gln Glu Gln
    3335                3340                3345

His Trp Leu Asn Thr Phe Ala Asp Glu Ile Pro Leu Leu Asn Leu
    3350                3355                3360

Pro Thr Asp Tyr Pro Arg Pro Ser Val Gln Ser Phe Ala Gly Asp
    3365                3370                3375

Leu Val Leu Phe Ala Ala Gly Lys Glu Leu Leu Glu Arg Leu Gln
    3380                3385                3390

Gln Val Ala Ser Glu Thr Gly Thr Thr Leu Tyr Met Ile Leu Leu
    3395                3400                3405

Ala Ala Tyr Asn Val Leu Leu Ser Lys Tyr Thr Gly Gln Glu Asp
    3410                3415                3420

Ile Ile Val Gly Thr Pro Val Ala Gly Arg Ser His Ala Asp Val
    3425                3430                3435

Glu Asn Ile Met Gly Ile Phe Val Asn Thr Leu Ala Leu Arg Asn
    3440                3445                3450

Gln Pro Ala Ser Ser Lys Thr Met Leu Glu Asn Asn Ile Thr Gln
    3455                3460                3465

Cys Asp Ser Ile Asn Asp Val Tyr Leu Lys Glu Glu Ala Ile Thr
    3470                3475                3480

Leu Met Asp Met Leu Glu Ser Gln Leu Lys His Gln Ala Asp Gly
    3485                3490                3495

Tyr Val Val Ile Asp Gln Glu Glu Ser Leu Ser Tyr Ala Asp Phe
    3500                3505                3510

Tyr Leu Arg Val Lys Glu Ile Gly Tyr Cys Leu Ser Glu Ile Ser
    3515                3520                3525

Ser Lys Asn Ser Val Gly Ile Gly Leu Phe Cys Asp Pro Ser Ile
    3530                3535                3540

Asp Leu Ile Cys Gly Ala Trp Gly Ile Leu Ser Ala Asp Lys Ala
    3545                3550                3555

Tyr Leu Pro Leu Ser Pro Asp Tyr Pro Thr Glu Arg Leu Lys Tyr
    3560                3565                3570

Met Ile Glu Asp Ser Gly Ile Asp Val Ile Phe Thr Gln Ser His
    3575                3580                3585

Leu Lys Ala Gln Leu Gln Asp Ile Ala Pro Lys Ser Val Leu Ile
    3590                3595                3600

Met Thr Pro Glu Asp Val Ala Leu Thr Ile Lys Thr Arg Thr Ile
    3605                3610                3615

Glu Asp Ile Leu Gly Thr Val Gln Val Pro Lys Pro Thr Ser Leu
    3620                3625                3630

Ala Tyr Ile Ile Tyr Thr Ser Gly Ser Thr Gly Lys Pro Lys Gly
    3635                3640                3645

Val Met Ile Glu His His Ser Ile Val Asn Gln Met Arg Phe Leu
    3650                3655                3660

Ala Lys Ala Phe Lys Leu Gly Cys His Ser Arg Ile Leu Gln Lys
    3665                3670                3675

Thr Pro Met Ser Phe Asp Ala Ala Gln Trp Glu Ile Leu Ala Pro
    3680                3685                3690

Ala Ile Gly Gly Gln Val Ile Met Gly Pro Leu Gly Cys Tyr Arg
    3695                3700                3705

Asp Pro Asp Ala Ile Ile Lys Thr Ile Leu Gln His Gln Val Thr
    3710                3715                3720

Thr Leu Gln Cys Val Pro Thr Leu Leu Gln Ala Leu Leu Asp Asn
    3725                3730                3735

Pro Asn Phe Leu Asp Cys Leu Ser Leu Thr Gln Val Phe Ser Gly
    3740                3745                3750

Gly Glu Ala Leu Thr Thr Lys Leu Ala Thr Gln Phe Leu Asn Ser
    3755                3760                3765

Phe Thr His Cys Glu Leu Ile Asn Leu Tyr Gly Pro Thr Glu Cys
    3770                3775                3780

Thr Ile Asn Ser Ser Phe Phe Arg Val Thr Asn Glu Thr Leu Pro
    3785                3790                3795

Asn Tyr Gln Thr Ser Ile Ser Ile Gly Ala Pro Val Asp Asn Thr
    3800                3805                3810

Glu Tyr Tyr Val Leu Asp Asp Asp Arg Leu Pro Val Ala Val Gly
    3815                3820                3825

Glu Ile Gly Glu Leu Tyr Ile Ser Gly Ala Gln Leu Ala Arg Gly
    3830                3835                3840

Tyr Leu His Lys Pro Glu Met Thr Lys Asp Lys Phe Ile Cys Asn
    3845                3850                3855

His Leu Val Ser Gly Thr Gln His Gln Trp Leu Tyr Arg Thr Gly
    3860                3865                3870

Asp Leu Val Thr Arg Gly Ala Asp Gly Asn Thr Tyr Phe Val Gly
    3875                3880                3885

Arg Val Asp Ser Gln Val Lys Leu Arg Gly Tyr Arg Ile Glu Leu

3890                3895                3900

Asp Glu Ile Arg His Ala Ile Glu Glu His Ser Trp Ile Lys Thr
    3905                3910                3915

Ala Ala Met Leu Ile Lys Lys Asp Ala Arg Thr Gly Phe Gln Asn
    3920                3925                3930

Leu Ile Ala Cys Val Glu Leu Asp Glu Lys Glu Ala Ala Leu Met
    3935                3940                3945

Asp Gln Gly Asn Ser Ser Ser His His Lys Ser Lys Ala Asp Lys
    3950                3955                3960

Leu Gln Val Lys Ala Gln Leu Ser Asn Ser Gly Cys Arg Ser Glu
    3965                3970                3975

Glu Leu Cys Glu Asn Arg Pro Thr Phe Leu Leu Pro Tyr Gln Glu
    3980                3985                3990

Gly Glu Ile Lys Gln Arg Glu Tyr Ala Phe Gly Arg Lys Thr Tyr
    3995                4000                4005

Arg Tyr Phe Glu Gly Thr Glu Ile Thr Val Glu Lys Leu Lys Lys
    4010                4015                4020

Leu Leu Thr Ala Thr Gln Ser Asn Glu Ile Ser Ser Leu Pro Leu
    4025                4030                4035

Ser His Leu Thr Leu Asn Asp Phe Gly Tyr Ala Leu Arg Tyr Phe
    4040                4045                4050

Gly Gln Phe Thr Ser His Gln Arg Leu Leu Pro Lys Tyr Ala Tyr
    4055                4060                4065

Ala Ser Pro Gly Ala Leu Tyr Ala Thr Gln Met Tyr Phe Glu Leu
    4070                4075                4080

His Asn Val Leu Gly Leu Asp Ala Gly Ile Tyr Tyr Tyr His Pro
    4085                4090                4095

Val Thr His Lys Leu Ile Lys Ile Ser Thr Leu Ser Arg Arg Gln
    4100                4105                4110

Met Pro Thr Ile Lys Val His Phe Ile Gly Lys His Glu Ala Ile
    4115                4120                4125

Glu Pro Val Tyr Lys Asn Asn Ile Gln Glu Val Leu Glu Met Glu
    4130                4135                4140

Ala Gly His Met Met Gly Leu Phe Asp Asp Val Leu Pro Glu Ile
    4145                4150                4155

Gly Leu Ser Ile Gly Lys Ser Glu Tyr Gln Asp Glu Cys Pro Asp
    4160                4165                4170

Trp Tyr Asp Gly Asp Ile Gln Asp Tyr Tyr Leu Gly Ala Phe Glu
    4175                4180                4185

Ile Cys Ser Tyr Glu His Gly Leu Pro Pro Phe Glu Thr Asp Ile
    4190                4195                4200

Tyr Leu Gln Thr His Ala His Lys Ile Pro Glu Met Pro Cys Gly
    4205                4210                4215

Leu Tyr His Phe Ser Asn Gly Glu Phe Val Arg Ile Ser Asp Asp
    4220                4225                4230

Ile Val Arg Lys Lys Asp Val Ile Ala Ile Asn Gln Gln Val Tyr
    4235                4240                4245

Asp Arg Ser Ser Phe Gly Val Ser Ile Ile Pro Arg Cys Val Pro
    4250                4255                4260

Glu Trp His Tyr Tyr Ile Thr Leu Gly Arg Arg Leu His Ala Leu
    4265                4270                4275

Gln Ser Asn Pro Leu Tyr Ile Gly Leu Met Ser Ser Gly Tyr Ser
    4280                4285                4290

Ser Lys Ser Asn Asn Asp Leu Pro Ser Ala Lys Arg Met Arg Ser
    4295                4300                4305

Ile Leu Asn Ala Leu Asp Arg Pro Met Ala Ala Phe Tyr Phe Cys
    4310                4315                4320

Ile Gly Gly Gly Ile Ser Gln Ala Gln Tyr Met Cys Glu Gly Met
    4325                4330                4335

Lys Glu Asp Val Val His Met Lys Gly Pro Val Glu Ile Ile Lys
    4340                4345                4350

Asp Asp Leu Gln Gln Gln Leu Pro Gln Tyr Met Ile Pro Asn Lys
    4355                4360                4365

Val Leu Val Phe Asp Lys Leu Pro Leu Thr Ala Asn Gly Lys Val
    4370                4375                4380

Asp Tyr Gln Ser Leu Ser Glu Ser Lys Ala Val Glu Asn Val Ser
    4385                4390                4395

Thr Gln Arg Leu Leu Val Pro Leu His Thr Asp Thr Glu Ile Arg
    4400                4405                4410

Leu Gly Lys Ile Trp Met Glu Val Leu Lys Trp Asp Ser Val Ser
    4415                4420                4425

Ala Leu Asp Asp Phe Phe Glu Ser Gly Gly Asn Ser Leu Met Ala
    4430                4435                4440

Val Ala Met Val Asn Lys Ile Asn Ala Ala Phe Asn Ile Arg Phe
    4445                4450                4455

Pro Leu Gln Ile Leu Phe Gln Ser Pro Asn Ile Ala Glu Leu Ala
    4460                4465                4470

Lys Trp Ile Glu Gln Thr Asp Ser Lys Thr Ile Ser Arg Leu Ile
    4475                4480                4485

Leu Leu Asn Gln Ala Ser Lys Asp Pro Ile Tyr Cys Trp Pro Gly
    4490                4495                4500

Leu Gly Gly Tyr Pro Met Ser Leu Arg Leu Leu Ala Asn Lys Val
    4505                4510                4515

Val Pro Asp Arg Ala Phe Tyr Gly Ile Gln Ala Tyr Gly Ile Asn
    4520                4525                4530

Glu Ser Glu Ile Pro Phe Ser Ser Ile Gln Arg Met Ala Glu Glu
    4535                4540                4545

Asp Ile Lys Glu Ile Lys Lys Ile Gln Pro Glu Gly Pro Tyr Ile
    4550                4555                4560

Leu Trp Gly Tyr Ser Phe Gly Ala Arg Val Ala Phe Glu Val Ala
    4565                4570                4575

Tyr Gln Leu Glu Gln Ala Gly Glu Glu Val Asn Ala Leu Asn Leu
    4580                4585                4590

Leu Ala Pro Gly Ser Pro His Leu Asp Met Lys Gln Ala Glu Tyr
    4595                4600                4605

Met Asp Lys Gly Ala Glu Phe Thr Asn Pro Ala Phe Val Lys Ile
    4610                4615                4620

Leu Phe Ser Val Phe Ser Arg Ser Ile Asn Ser Pro Met Val Lys
    4625                4630                4635

Thr Cys Leu Glu Gln Val Asn Ser Glu Thr Thr Phe Ile Asn Phe
    4640                4645                4650

Ile Cys Ser Arg Phe Lys Asn Leu Glu Pro Ser Leu Val Lys Arg
    4655                4660                4665

Ile Val Arg Ile Val Thr Leu Thr Tyr Asp Phe Lys Tyr Ser Ile
    4670                4675                4680

Asp Glu Leu Tyr His Arg His Leu Lys Ala Pro Ile Thr Ile Phe
    4685                4690                4695

Lys Ala Asn Arg Asp Asn Asp Ser Phe Ile Glu Glu Ser Asp Val
    4700                4705                4710

Ile Ser Ser Met Ser Pro Lys Ile Ile Glu Leu Ile Ser Asp His
    4715                4720                4725

Tyr Gln Leu Leu Glu Ser Glu Gly Val Ala Glu Ile Glu Lys Ile
    4730                4735                4740

Ile

<210>  32
<211>  5777
<212>  PRT
<213>  Artificial Sequence

<220>
<223>  NRPSase synthesizing a Valine-Indigoidine-tagged Tripeptide
       consisting of Phenylalanine, Ornithine and Leucine. Valine is
       here used as spacer.

<400>  32

Met Leu Ala Asn Gln Ala Asn Leu Ile Asp Asn Lys Arg Glu Leu Glu
1               5                   10                  15

Gln His Ala Leu Val Pro Tyr Ala Gln Gly Lys Ser Ile His Gln Leu
            20                  25                  30

Phe Glu Glu Gln Ala Glu Ala Phe Pro Asp Arg Val Ala Ile Val Phe

35                  40                  45

Glu Asn Arg Arg Leu Ser Tyr Gln Glu Leu Asn Arg Lys Ala Asn Gln
    50                  55                  60

Leu Ala Arg Ala Leu Leu Glu Lys Gly Val Gln Thr Asp Ser Ile Val
65                  70                  75                  80

Gly Val Met Met Glu Lys Ser Ile Glu Asn Val Ile Ala Ile Leu Ala
                85                  90                  95

Val Leu Lys Ala Gly Gly Ala Tyr Val Pro Ile Asp Ile Glu Tyr Pro
            100                 105                 110

Arg Asp Arg Ile Gln Tyr Ile Leu Gln Asp Ser Gln Thr Lys Ile Val
        115                 120                 125

Leu Thr Gln Lys Ser Val Ser Gln Leu Val His Asp Val Gly Tyr Ser
    130                 135                 140

Gly Glu Val Val Val Leu Asp Glu Glu Gln Leu Asp Ala Arg Glu Thr
145                 150                 155                 160

Ala Asn Leu His Gln Pro Ser Lys Pro Thr Asp Leu Ala Tyr Val Ile
                165                 170                 175

Tyr Thr Ser Gly Thr Thr Gly Lys Pro Lys Gly Thr Met Leu Glu His
            180                 185                 190

Lys Gly Ile Ala Asn Leu Gln Ser Phe Phe Gln Asn Ser Phe Gly Val
        195                 200                 205

Thr Glu Gln Asp Arg Ile Gly Leu Phe Ala Ser Met Ser Phe Asp Ala
    210                 215                 220

Ser Val Trp Glu Met Phe Met Ala Leu Leu Ser Gly Ala Ser Leu Tyr
225                 230                 235                 240

Ile Leu Ser Lys Gln Thr Ile His Asp Phe Ala Ala Phe Glu His Tyr
                245                 250                 255

Leu Ser Glu Asn Glu Leu Thr Ile Ile Thr Leu Pro Pro Thr Tyr Leu
            260                 265                 270

Thr His Leu Thr Pro Glu Arg Ile Thr Ser Leu Arg Ile Met Ile Thr
        275                 280                 285

Ala Gly Ser Ala Ser Ser Ala Pro Leu Val Asn Lys Trp Lys Asp Lys
    290                 295                 300

Leu Arg Tyr Ile Asn Ala Tyr Gly Pro Thr Glu Thr Ser Ile Cys Ala
305                 310                 315                 320

Thr Ile Trp Glu Ala Pro Ser Asn Gln Leu Ser Val Gln Ser Val Pro
                325                 330                 335

Ile Gly Lys Pro Ile Gln Asn Thr His Ile Tyr Ile Val Asn Glu Asp
            340                 345                 350

Leu Gln Leu Leu Pro Thr Gly Ser Glu Gly Glu Leu Cys Ile Gly Gly
        355                 360                 365

Val Gly Leu Ala Arg Gly Tyr Trp Asn Arg Pro Asp Leu Thr Ala Glu
    370                 375                 380

Lys Phe Val Asp Asn Pro Phe Val Pro Gly Glu Lys Met Tyr Arg Thr
385                 390                 395                 400

Gly Asp Leu Ala Lys Trp Leu Thr Asp Gly Thr Ile Glu Phe Leu Gly
                405                 410                 415

Arg Ile Asp His Gln Val Lys Ile Arg Gly His Arg Ile Glu Leu Gly
            420                 425                 430

Glu Ile Glu Ser Val Leu Leu Ala His Glu His Ile Thr Glu Ala Val
        435                 440                 445

Val Ile Ala Arg Glu Asp Gln His Ala Gly Gln Tyr Leu Cys Ala Tyr
    450                 455                 460

Tyr Ile Ser Gln Gln Glu Ala Thr Pro Ala Gln Leu Arg Asp Tyr Ala
465                 470                 475                 480

Ala Gln Lys Leu Pro Ala Tyr Met Leu Pro Ser Tyr Phe Val Lys Leu
                485                 490                 495

Asp Lys Met Pro Leu Thr Pro Asn Asp Lys Ile Asp Arg Lys Ala Leu
            500                 505                 510

Pro Glu Pro Asp Leu Thr Ala Asn Gln Ser Gln Ala Ala Tyr His Pro
        515                 520                 525

Pro Arg Thr Glu Thr Glu Ser Ile Leu Val Ser Ile Trp Gln Asn Val
    530                 535                 540

Leu Gly Ile Glu Lys Ile Gly Ile Arg Asp Asn Phe Tyr Ser Leu Gly
545                 550                 555                 560

Gly Asp Ser Ile Gln Ala Ile Gln Val Val Ala Arg Leu His Ser Tyr
                565                 570                 575

Gln Leu Lys Leu Glu Thr Lys Asp Leu Leu Asn Tyr Pro Thr Ile Glu
            580                 585                 590

Gln Val Ala Leu Phe Val Lys Ser Thr Thr Arg Lys Ser Asp Gln Gly
        595                 600                 605

Ile Ile Ala Gly Asn Val Pro Leu Thr Pro Ile Gln Lys Trp Phe Phe
    610                 615                 620

Gly Lys Asn Phe Thr Asn Thr Gly His Trp Asn Gln Ser Ser Val Leu
625                 630                 635                 640

Tyr Arg Pro Glu Gly Phe Asp Pro Lys Val Ile Gln Ser Val Met Asp
                645                 650                 655

Lys Ile Ile Glu His His Asp Ala Leu Arg Met Val Tyr Gln His Glu
            660                 665                 670

Asn Gly Asn Val Val Gln His Asn Arg Gly Leu Gly Gly Gln Leu Tyr
        675                 680                 685

Asp Phe Phe Ser Tyr Asn Leu Thr Ala Gln Pro Asp Val Gln Gln Ala
    690                 695                 700

Ile Glu Ala Glu Thr Gln Arg Leu His Ser Ser Met Asn Leu Gln Glu
705                 710                 715                 720

Gly Pro Leu Val Lys Val Ala Leu Phe Gln Thr Leu His Gly Asp His
                725                 730                 735

Leu Phe Leu Ala Ile His His Leu Val Val Asp Gly Ile Ser Trp Arg
            740                 745                 750

Ile Leu Phe Glu Asp Leu Ala Thr Gly Tyr Ala Gln Ala Leu Ala Gly
        755                 760                 765

Gln Ala Ile Ser Leu Pro Glu Lys Thr Asp Ser Phe Gln Ser Trp Ser
    770                 775                 780

Gln Trp Leu Gln Glu Tyr Ala Asn Glu Ala Asp Leu Leu Ser Glu Ile
785                 790                 795                 800

Pro Tyr Trp Glu Ser Leu Glu Ser Gln Ala Lys Asn Val Ser Leu Pro
                805                 810                 815

Lys Asp Tyr Glu Val Thr Asp Cys Lys Gln Lys Ser Val Arg Asn Met
            820                 825                 830

Arg Ile Arg Leu His Pro Glu Glu Thr Glu Gln Leu Leu Lys His Ala
        835                 840                 845

Asn Gln Ala Tyr Gln Thr Glu Ile Asn Asp Leu Leu Leu Ala Ala Leu
    850                 855                 860

Gly Leu Ala Phe Ala Glu Trp Ser Lys Leu Ala Gln Ile Val Ile His
865                 870                 875                 880

Leu Glu Gly His Gly Arg Glu Asp Ile Ile Glu Gln Ala Asn Val Ala
                885                 890                 895

Arg Thr Val Gly Trp Phe Thr Ser Gln Tyr Pro Val Leu Leu Asp Leu
            900                 905                 910

Lys Gln Thr Ala Pro Leu Ser Asp Tyr Ile Lys Leu Thr Lys Glu Asn
        915                 920                 925

Met Arg Lys Ile Pro Arg Lys Gly Ile Gly Tyr Asp Ile Leu Lys His
    930                 935                 940

Val Thr Leu Pro Glu Asn Arg Gly Ser Leu Ser Phe Arg Val Gln Pro
945                 950                 955                 960

Glu Val Thr Phe Asn Tyr Leu Gly Gln Phe Asp Ala Asp Met Arg Thr
                965                 970                 975

Glu Leu Phe Thr Arg Ser Pro Tyr Ser Gly Gly Asn Thr Leu Gly Ala
            980                 985                 990

Asp Gly Lys Asn Asn Leu Ser Pro  Glu Ser Glu Val Tyr  Thr Ala Leu
        995                 1000                1005

Asn Ile  Thr Gly Leu Ile Glu  Gly Gly Glu Leu Val  Leu Thr Phe
    1010                1015                1020

Ser Tyr  Ser Ser Glu Gln Tyr  Arg Glu Glu Ser Ile  Gln Gln Leu
    1025                1030                1035

Ser Gln  Ser Tyr Gln Lys His  Leu Leu Ala Ile Ile  Ala His Cys

1040                1045                1050

Thr Glu Lys Lys Glu Val Glu Arg Thr Ala His Ile Ala Glu Ser
    1055                1060                1065

Ala Phe Glu Gln Phe Glu Thr Ile Gln Pro Val Glu Pro Ala Ala
    1070                1075                1080

Phe Tyr Pro Val Ser Phe Ala Gln Lys Arg Met Tyr Ile Leu His
    1085                1090                1095

Gln Phe Glu Gly Ser Gly Ile Ser Tyr Asn Val Pro Ser Val Leu
    1100                1105                1110

Val Leu Glu Gly Lys Leu Asp Tyr Asp Arg Phe Ala Ala Ala Ile
    1115                1120                1125

Gln Ser Leu Val Lys Arg His Glu Ser Leu Arg Thr Ser Phe His
    1130                1135                1140

Ser Val Asn Gly Glu Pro Leu Gln Arg Val His Pro Asp Val Glu
    1145                1150                1155

Leu Pro Val Arg Leu Leu Glu Ala Thr Glu Asp Gln Ser Glu Ser
    1160                1165                1170

Leu Ile Gln Glu Leu Ile Gln Pro Phe Asp Leu Glu Ile Ala Pro
    1175                1180                1185

Leu Phe Arg Val Asn Leu Ile Lys Leu Gly Ala Glu Arg His Leu
    1190                1195                1200

Phe Phe Met Asp Met His His Ile Ile Ser Asp Gly Val Ser Leu
    1205                1210                1215

Ala Val Ile Val Glu Glu Ile Ala Ser Leu Tyr Ala Gly Lys Gln
    1220                1225                1230

Leu Ser Asp Leu Arg Ile Gln Tyr Lys Asp Phe Ala Val Trp Gln
    1235                1240                1245

Thr Lys Leu Ala Gln Ser Asp Arg Phe Gln Lys Gln Glu Asp Phe
    1250                1255                1260

Trp Thr Arg Thr Phe Ala Gly Glu Ile Pro Leu Leu Asn Leu Pro
    1265                1270                1275

His Asp Tyr Pro Arg Pro Ser Val Gln Ser Phe Asp Gly Asp Thr
    1280                1285                1290

Val Ala Leu Gly Thr Gly His His Leu Leu Glu Gln Leu Arg Lys
    1295                1300                1305

Leu Ala Ala Glu Thr Gly Thr Thr Leu Phe Met Val Leu Leu Ala
    1310                1315                1320

Ala Tyr His Val Leu Leu Ser Lys Tyr Ala Gly Gln Glu Glu Ile
    1325                1330                1335

Val Val Gly Thr Pro Ile Ala Gly Arg Ser His Ala Asp Val Glu
    1340                1345                1350

Arg Ile Val Gly Met Phe Val Asn Thr Leu Ala Leu Lys Asn Thr
    1355                1360                1365

Ala Ala Gly Ser Leu Ser Phe Arg Ala Phe Leu Glu Asp Val Lys
    1370                1375                1380

Gln Asn Ala Leu His Ala Phe Glu His Gln Asp Tyr Pro Phe Glu
    1385                1390                1395

His Leu Val Glu Lys Leu Gln Val Arg Arg Asp Leu Ser Arg Asn
    1400                1405                1410

Pro Leu Phe Asp Thr Met Phe Ser Leu Gly Leu Ala Glu Ser Ala
    1415                1420                1425

Glu Gly Glu Val Ala Asp Leu Lys Val Ser Pro Tyr Pro Val Asn
    1430                1435                1440

Gly His Ile Ala Lys Phe Asp Leu Ser Leu Asp Ala Met Glu Lys
    1445                1450                1455

Gln Asp Gly Leu Leu Val Gln Phe Ser Tyr Cys Thr Lys Leu Phe
    1460                1465                1470

Ala Lys Glu Thr Val Asp Arg Leu Ala Ala His Tyr Val Gln Leu
    1475                1480                1485

Leu Gln Thr Ile Thr Ala Asp Pro Asp Ile Glu Leu Ala Arg Ile
    1490                1495                1500

Ser Val Leu Ser Lys Ala Glu Thr Glu His Met Leu His Ser Phe
    1505                1510                1515

Leu Ala Thr Lys Thr Ala Tyr Pro Thr Asp Lys Thr Phe Gln Lys
    1520                1525                1530

Leu Phe Glu Glu Gln Val Glu Lys Thr Pro Asn Glu Ile Ala Val
    1535                1540                1545

Leu Phe Gly Asn Glu Gln Leu Thr Tyr Gln Glu Leu Asn Ala Lys
    1550                1555                1560

Ala Asn Gln Leu Ala Arg Val Leu Arg Arg Lys Gly Val Lys Pro
    1565                1570                1575

Glu Ser Thr Val Gly Ile Leu Val Asp Arg Ser Leu Tyr Met Val
    1580                1585                1590

Ile Gly Met Leu Ala Val Leu Lys Ala Gly Gly Thr Phe Val Pro
    1595                1600                1605

Ile Asp Pro Asp Tyr Pro Leu Glu Arg Gln Ala Phe Met Leu Glu
    1610                1615                1620

Asp Ser Glu Ala Lys Leu Leu Leu Thr Leu Gln Lys Met Asn Ser
    1625                1630                1635

Gln Val Ala Phe Pro Tyr Glu Thr Phe Tyr Leu Asp Thr Glu Thr
    1640                1645                1650

Val Asp Gln Glu Glu Thr Gly Asn Leu Glu His Val Ala Gln Pro
    1655                1660                1665

Glu Asn Val Ala Tyr Ile Ile Tyr Thr Ser Gly Thr Thr Gly Lys
    1670                1675                1680

Pro Lys Gly Val Val Ile Glu His Arg Ser Tyr Ala Asn Val Ala
    1685                1690                1695

Phe Ala Trp Lys Asp Glu Tyr His Leu Asp Ser Phe Pro Val Arg
    1700                1705                1710

Leu Leu Gln Met Ala Ser Phe Ala Phe Asp Val Ser Thr Gly Asp
    1715                1720                1725

Phe Ala Arg Ala Leu Leu Thr Gly Gly Gln Leu Val Ile Cys Pro
    1730                1735                1740

Asn Gly Val Lys Met Asp Pro Ala Ser Leu Tyr Glu Thr Ile Arg
    1745                1750                1755

Arg His Glu Ile Thr Ile Phe Glu Ala Thr Pro Ala Leu Ile Met
    1760                1765                1770

Pro Leu Met His Tyr Val Tyr Glu Asn Glu Leu Asp Met Ser Gln
    1775                1780                1785

Met Lys Leu Leu Ile Leu Gly Ala Asp Ser Cys Pro Ala Glu Asp
    1790                1795                1800

Phe Lys Thr Leu Leu Ala Arg Phe Gly Gln Lys Met Arg Ile Ile
    1805                1810                1815

Asn Ser Tyr Gly Val Thr Glu Ala Cys Ile Asp Thr Ser Tyr Tyr
    1820                1825                1830

Glu Glu Thr Asp Val Thr Ala Ile Arg Ser Gly Thr Val Pro Ile
    1835                1840                1845

Gly Lys Pro Leu Pro Asn Met Thr Met Tyr Val Val Asp Ala His
    1850                1855                1860

Leu Asn Leu Gln Pro Val Gly Val Val Gly Glu Leu Cys Ile Gly
    1865                1870                1875

Gly Ala Gly Val Ala Arg Gly Tyr Leu Asn Arg Pro Glu Leu Thr
    1880                1885                1890

Glu Glu Lys Phe Val Pro Asn Pro Phe Ala Pro Gly Glu Arg Leu
    1895                1900                1905

Tyr Arg Thr Gly Asp Leu Ala Lys Trp Arg Ala Asp Gly Asn Val
    1910                1915                1920

Glu Phe Leu Gly Arg Asn Asp His Gln Val Lys Ile Arg Gly Val
    1925                1930                1935

Arg Ile Glu Leu Gly Glu Ile Glu Thr Gln Leu Arg Lys Leu Asp
    1940                1945                1950

Gly Ile Thr Glu Ala Val Val Val Ala Arg Glu Asp Arg Gly Gln
    1955                1960                1965

Glu Lys Glu Leu Cys Ala Tyr Val Val Ala Asp His Lys Leu Asp
    1970                1975                1980

Thr Ala Glu Leu Arg Ala Asn Leu Leu Lys Glu Leu Pro Gln Ala

1985                1990                1995

Met Ile Pro Ala Tyr Phe Val Thr Leu Asp Ala Leu Pro Leu Thr
    2000                2005                2010

Ala Asn Gly Lys Val Asp Arg Arg Ser Leu Pro Ala Pro Asp Val
    2015                2020                2025

Thr Met Leu Arg Thr Thr Glu Tyr Val Ala Pro Arg Ser Val Trp
    2030                2035                2040

Glu Ala Arg Leu Ala Gln Val Trp Glu Gln Val Leu Asn Val Pro
    2045                2050                2055

Gln Val Gly Ala Leu Asp Asp Phe Phe Ala Leu Gly Gly His Ser
    2060                2065                2070

Leu Arg Ala Met Arg Val Leu Ser Ser Met His Asn Glu Tyr Gln
    2075                2080                2085

Val Asp Ile Pro Leu Arg Ile Leu Phe Glu Lys Pro Thr Ile Gln
    2090                2095                2100

Glu Leu Ala Ala Phe Ile Glu Glu Thr Ala Lys Gly Asn Val Phe
    2105                2110                2115

Ser Ile Glu Pro Val Gln Lys Gln Ala Tyr Tyr Pro Val Ser Ser
    2120                2125                2130

Ala Gln Lys Arg Met Tyr Ile Leu Asp Gln Phe Glu Gly Val Gly
    2135                2140                2145

Ile Ser Tyr Asn Met Pro Ser Thr Met Leu Ile Glu Gly Lys Leu
    2150                2155                2160

Glu Arg Thr Arg Val Glu Ala Ala Phe Gln Arg Leu Ile Ala Arg
    2165                2170                2175

His Glu Ser Leu Arg Thr Ser Phe Ala Val Val Asn Gly Glu Pro
    2180                2185                2190

Val Gln Asn Ile His Glu Asp Val Pro Phe Ala Leu Ala Tyr Ser
    2195                2200                2205

Glu Val Thr Glu Gln Glu Ala Arg Glu Leu Val Ser Ser Leu Val
    2210                2215                2220

Gln Pro Phe Asp Leu Glu Val Ala Pro Leu Ile Arg Val Ser Leu
    2225                2230                2235

Leu Lys Ile Gly Glu Asp Arg Tyr Val Leu Phe Thr Asp Met His
    2240                2245                2250

His Ser Ile Ser Asp Gly Val Ser Ser Gly Ile Leu Leu Ala Glu
    2255                2260                2265

Trp Val Gln Leu Tyr Gln Gly Asp Val Leu Pro Glu Leu Arg Ile
    2270                2275                2280

Gln Tyr Lys Asp Phe Ala Val Trp Gln Gln Glu Phe Ser Gln Ser
    2285                2290                2295

Ala Ala Phe His Lys Gln Glu Ala Tyr Trp Leu Gln Thr Phe Ala
    2300                2305                2310

Asp Asp Ile Pro Val Leu Asn Leu Pro Thr Asp Phe Thr Arg Pro
    2315                2320                2325

Ser Thr Gln Ser Phe Ala Gly Asp Gln Cys Thr Ile Gly Ala Gly
    2330                2335                2340

Lys Ala Leu Thr Glu Gly Leu His Gln Leu Ala Gln Ala Thr Gly
    2345                2350                2355

Thr Thr Leu Tyr Met Val Leu Leu Ala Ala Tyr Asn Val Leu Leu
    2360                2365                2370

Ala Lys Tyr Ala Gly Gln Glu Asp Ile Ile Val Gly Thr Pro Ile
    2375                2380                2385

Thr Gly Arg Ser His Ala Asp Leu Glu Pro Ile Val Gly Met Phe
    2390                2395                2400

Val Asn Thr Leu Ala Met Arg Asn Lys Pro Gln Arg Glu Lys Thr
    2405                2410                2415

Phe Ser Glu Phe Leu Gln Glu Val Lys Gln Asn Ala Leu Asp Ala
    2420                2425                2430

Tyr Gly His Gln Asp Tyr Pro Phe Glu Glu Leu Val Glu Lys Leu
    2435                2440                2445

Ala Ile Ala Arg Asp Leu Ser Arg Asn Pro Leu Phe Asp Thr Val
    2450                2455                2460

Phe Thr Phe Gln Asn Ser Thr Glu Glu Val Met Thr Leu Pro Glu
    2465                2470                2475

Cys Thr Leu Ala Pro Phe Met Thr Asp Glu Thr Gly Gln His Ala
    2480                2485                2490

Lys Phe Asp Leu Thr Phe Ser Ala Thr Glu Glu Arg Glu Glu Met
    2495                2500                2505

Thr Ile Gly Val Glu Tyr Ser Thr Ser Leu Phe Thr Arg Glu Thr
    2510                2515                2520

Met Glu Arg Phe Ser Arg His Phe Leu Thr Ile Ala Ala Ser Ile
    2525                2530                2535

Val Gln Asn Pro His Ile Arg Leu Gly Glu Ile Asp Met Leu Leu
    2540                2545                2550

Pro Glu Glu Lys Gln Gln Ile Leu Ala Gly Phe Asn Asp Thr Ala
    2555                2560                2565

Val Ser Tyr Ala Leu Asp Lys Thr Leu His Gln Leu Phe Glu Glu
    2570                2575                2580

Gln Val Asp Lys Thr Pro Asp Gln Ala Ala Leu Leu Phe Ser Glu
    2585                2590                2595

Gln Ser Leu Thr Tyr Ser Glu Leu Asn Glu Arg Ala Asn Arg Leu
    2600                2605                2610

Ala Arg Val Leu Arg Ala Lys Gly Val Gly Pro Asp Arg Leu Val
    2615                2620                2625

Ala Ile Met Ala Glu Arg Ser Pro Glu Met Val Ile Gly Ile Leu
    2630                2635                2640

Gly Ile Leu Lys Ala Gly Gly Ala Tyr Val Pro Val Asp Pro Gly
    2645                2650                2655

Tyr Pro Gln Glu Arg Ile Gln Tyr Leu Leu Glu Asp Ser Asn Ala
    2660                2665                2670

Ala Leu Leu Leu Ser Gln Ala His Leu Leu Pro Leu Leu Ala Gln
    2675                2680                2685

Val Ser Ser Glu Leu Pro Glu Cys Leu Asp Leu Asn Ala Glu Leu
    2690                2695                2700

Asp Ala Gly Leu Ser Gly Ser Asn Leu Pro Ala Val Asn Gln Pro
    2705                2710                2715

Thr Asp Leu Ala Tyr Val Ile Tyr Thr Ser Gly Thr Thr Gly Lys
    2720                2725                2730

Pro Lys Gly Val Met Ile Pro His Gln Gly Ile Val Asn Cys Leu
    2735                2740                2745

Gln Trp Arg Arg Asp Glu Tyr Gly Phe Gly Pro Ser Asp Lys Ala
    2750                2755                2760

Leu Gln Val Phe Ser Phe Ala Phe Asp Gly Phe Val Ala Ser Leu
    2765                2770                2775

Phe Ala Pro Leu Leu Gly Gly Ala Thr Cys Val Leu Pro Gln Glu
    2780                2785                2790

Ala Ala Ala Lys Asp Pro Val Ala Leu Lys Lys Leu Met Ala Ala
    2795                2800                2805

Thr Glu Val Thr His Tyr Tyr Gly Val Pro Ser Leu Phe Gln Ala
    2810                2815                2820

Ile Leu Asp Cys Ser Thr Thr Thr Asp Phe Asn Gln Leu Arg Cys
    2825                2830                2835

Val Thr Leu Gly Gly Glu Lys Leu Pro Val Gln Leu Val Gln Lys
    2840                2845                2850

Thr Lys Glu Lys His Pro Ala Ile Glu Ile Asn Asn Glu Tyr Gly
    2855                2860                2865

Pro Thr Glu Asn Ser Val Val Thr Thr Ile Ser Arg Ser Ile Glu
    2870                2875                2880

Ala Gly Gln Ala Ile Thr Ile Gly Arg Pro Leu Ala Asn Val Gln
    2885                2890                2895

Val Tyr Ile Val Asp Glu Gln His His Leu Gln Pro Ile Gly Val
    2900                2905                2910

Val Gly Glu Leu Cys Ile Gly Gly Ala Gly Leu Ala Arg Gly Tyr
    2915                2920                2925

Leu Asn Lys Pro Glu Leu Thr Ala Glu Lys Phe Val Ala Asn Pro

2930                2935                2940

Phe Arg Pro Gly Glu Arg Met Tyr Lys Thr Gly Asp Leu Val Lys
    2945                2950                2955

Trp Arg Thr Asp Gly Thr Ile Glu Tyr Ile Gly Arg Ala Asp Glu
    2960                2965                2970

Gln Val Lys Val Arg Gly Tyr Arg Ile Glu Ile Gly Glu Ile Glu
    2975                2980                2985

Ser Ala Val Leu Ala Tyr Gln Gly Ile Asp Gln Ala Val Val Val
    2990                2995                3000

Ala Arg Asp Asp Asp Ala Thr Ala Gly Ser Tyr Leu Cys Ala Tyr
    3005                3010                3015

Phe Val Ala Ala Thr Ala Val Ser Val Ser Gly Leu Arg Ser His
    3020                3025                3030

Leu Ala Lys Glu Leu Pro Ala Tyr Met Ile Pro Ser Tyr Phe Val
    3035                3040                3045

Glu Leu Asp Gln Leu Pro Leu Ser Ala Asn Gly Lys Val Asp Arg
    3050                3055                3060

Lys Ala Leu Pro Lys Pro Gln Gln Ser Asp Ala Thr Thr Arg Glu
    3065                3070                3075

Tyr Val Ala Pro Arg Asn Ala Thr Glu Gln Gln Leu Ala Ala Ile
    3080                3085                3090

Trp Gln Glu Val Leu Gly Val Glu Pro Ile Gly Ile Thr Asp Gln
    3095                3100                3105

Phe Phe Glu Leu Gly Gly His Ser Leu Lys Ala Thr Leu Leu Ile
    3110                3115                3120

Ala Lys Val Tyr Glu Tyr Met Gln Ile Glu Leu Pro Leu Asn Leu
    3125                3130                3135

Ile Phe Gln Tyr Pro Thr Ile Glu Lys Val Ala Asp Phe Ile Thr
    3140                3145                3150

Thr Ser Gly Lys Glu Thr Tyr Val Pro Ile Glu Pro Ala Pro Leu
    3155                3160                3165

Gln Glu Tyr Tyr Pro Val Ser Ser Ala Gln Lys Arg Met Tyr Val
    3170                3175                3180

Leu Arg Gln Phe Ala Asp Thr Gly Thr Val Tyr Asn Met Pro Ser
    3185                3190                3195

Ala Leu Tyr Ile Glu Gly Asp Leu Asp Arg Lys Arg Phe Glu Ala
    3200                3205                3210

Ala Ile His Gly Leu Val Glu Arg His Glu Ser Leu Arg Thr Ser
    3215                3220                3225

Phe His Thr Val Asn Gly Glu Pro Val Gln Arg Val His Glu His
    3230                3235                3240

Val Glu Leu Asn Val Gln Tyr Ala Glu Val Thr Glu Ala Gln Val
    3245                3250                3255

Glu Pro Thr Val Glu Ser Phe Val Gln Ala Phe Asp Leu Thr Lys
    3260                3265                3270

Ala Pro Leu Leu Arg Val Gly Leu Phe Lys Leu Ala Ala Lys Arg
    3275                3280                3285

His Leu Phe Leu Leu Asp Met His His Ile Ile Ser Asp Gly Val
    3290                3295                3300

Ser Ala Gly Ile Ile Met Glu Glu Phe Ser Lys Leu Tyr Arg Gly
    3305                3310                3315

Glu Glu Leu Pro Ala Leu Ser Val His Tyr Lys Asp Phe Ala Val
    3320                3325                3330

Trp Gln Ser Glu Leu Phe Gln Ser Asp Val Tyr Thr Glu His Glu
    3335                3340                3345

Asn Tyr Trp Leu Asn Ala Phe Ser Gly Asp Ile Pro Val Leu Asn
    3350                3355                3360

Leu Pro Ala Asp Phe Ser Arg Pro Leu Thr Gln Ser Phe Glu Gly
    3365                3370                3375

Asp Cys Val Ser Phe Gln Ala Asp Lys Ala Leu Leu Asp Asp Leu
    3380                3385                3390

His Lys Leu Ala Gln Glu Ser Gln Ser Thr Leu Phe Met Val Leu
    3395                3400                3405

Leu Ala Ala Tyr Asn Val Leu Leu Ala Lys Tyr Ser Gly Gln Glu
    3410                3415                3420

Asp Ile Val Val Gly Thr Pro Ile Ala Gly Arg Ser His Ala Asp
    3425                3430                3435

Ile Glu Asn Val Leu Gly Met Phe Val Asn Thr Leu Ala Leu Arg
    3440                3445                3450

Asn Tyr Pro Val Glu Thr Lys His Phe Gln Ala Phe Leu Glu Glu
    3455                3460                3465

Val Lys Gln Asn Thr Leu Gln Ala Tyr Ala His Gln Asp Tyr Pro
    3470                3475                3480

Phe Glu Ala Leu Val Glu Lys Leu Asp Ile Gln Arg Asp Leu Ser
    3485                3490                3495

Arg Asn Pro Leu Phe Asp Thr Met Phe Ile Leu Gln Asn Leu Asp
    3500                3505                3510

Gln Lys Ala Tyr Glu Leu Asp Gly Leu Lys Leu Glu Ala Tyr Pro
    3515                3520                3525

Ala Gln Ala Gly Asn Ala Lys Phe Asp Leu Thr Leu Glu Ala His
    3530                3535                3540

Glu Asp Glu Thr Gly Ile His Phe Ala Leu Val Tyr Ser Thr Lys
    3545                3550                3555

Leu Phe Gln Arg Glu Ser Ile Glu Arg Met Ala Gly His Phe Leu
    3560                3565                3570

Gln Val Leu Arg Gln Val Val Ala Asp Gln Ala Thr Ala Leu Arg
    3575                3580                3585

Glu Ile Ser Leu Leu Ser Glu Glu Glu Arg Arg Ile Val Thr Val
    3590                3595                3600

Asp Phe Asn Asn Thr Phe Ala Tyr Pro Arg Asp Leu Thr Ile Gln
    3605                3610                3615

Glu Leu Phe Glu Gln Gln Ala Ala Lys Thr Pro Glu His Ala Ala
    3620                3625                3630

Val Val Met Asp Gly Gln Met Leu Thr Tyr Arg Glu Leu Asn Glu
    3635                3640                3645

Lys Ala Asn Gln Leu Ala His Val Leu Arg Gln Asn Gly Val Gly
    3650                3655                3660

Lys Glu Ser Ile Val Gly Leu Leu Ala Asp Arg Ser Leu Glu Met
    3665                3670                3675

Ile Thr Gly Ile Met Gly Ile Leu Lys Ala Gly Gly Ala Tyr Leu
    3680                3685                3690

Gly Leu Asp Pro Glu His Pro Ser Glu Arg Leu Ala Tyr Met Leu
    3695                3700                3705

Glu Asp Gly Gly Val Lys Val Val Leu Val Gln Lys His Leu Leu
    3710                3715                3720

Pro Leu Val Gly Glu Gly Leu Met Pro Ile Val Leu Glu Glu Glu
    3725                3730                3735

Ser Leu Arg Pro Glu Asp Cys Gly Asn Pro Ala Ile Val Asn Gly
    3740                3745                3750

Ala Ser Asp Leu Ala Tyr Val Met Tyr Thr Ser Gly Ser Thr Gly
    3755                3760                3765

Lys Pro Lys Gly Val Met Val Glu His Arg Asn Val Thr Arg Leu
    3770                3775                3780

Val Met His Thr Asn Tyr Val Gln Val Arg Glu Ser Asp Arg Met
    3785                3790                3795

Ile Gln Thr Gly Ala Ile Gly Phe Asp Ala Met Thr Phe Glu Ile
    3800                3805                3810

Phe Gly Ala Leu Leu His Gly Ala Ser Leu Tyr Leu Val Ser Lys
    3815                3820                3825

Asp Val Leu Leu Asp Ala Glu Lys Leu Gly Asp Phe Leu Arg Thr
    3830                3835                3840

Asn Gln Ile Thr Thr Met Trp Leu Thr Ser Pro Leu Phe Asn Gln
    3845                3850                3855

Leu Ser Gln Asp Asn Pro Ala Met Phe Asp Ser Leu Arg Ala Leu
    3860                3865                3870

Ile Val Gly Gly Glu Ala Leu Ser Pro Lys His Ile Asn Arg Val

3875                 3880                3885

Lys Ser Ala Leu Pro Asp Leu Glu Ile Trp Asn Gly Tyr Gly Pro
    3890                3895                3900

Thr Glu Asn Thr Thr Phe Ser Thr Cys Tyr Leu Ile Glu Gln His
    3905                3910                3915

Phe Glu Glu Gln Ile Pro Ile Gly Lys Pro Ile Ala Asn Ser Thr
    3920                3925                3930

Ala Tyr Ile Val Asp Gly Asn Asn Gln Pro Gln Pro Ile Gly Val
    3935                3940                3945

Pro Gly Glu Leu Cys Val Gly Gly Asp Gly Val Ala Arg Gly Tyr
    3950                3955                3960

Val Asn Lys Pro Glu Leu Thr Ala Glu Lys Phe Val Pro Asn Pro
    3965                3970                3975

Phe Ala Pro Gly Glu Thr Met Tyr Arg Thr Gly Asp Leu Ala Arg
    3980                3985                3990

Trp Leu Pro Asp Gly Thr Ile Glu Tyr Leu Gly Arg Ile Asp Gln
    3995                4000                4005

Gln Val Lys Ile Arg Gly Tyr Arg Ile Glu Leu Gly Glu Ile Glu
    4010                4015                4020

Thr Val Leu Ser Gln Gln Ala Gln Val Lys Glu Ala Val Val Ala
    4025                4030                4035

Val Ile Glu Glu Ala Asn Gly Gln Lys Ala Leu Cys Ala Tyr Phe
    4040                4045                4050

Val Pro Glu Gln Ala Val Asp Ala Ala Glu Leu Arg Glu Ala Met
    4055                4060                4065

Ser Lys Gln Leu Pro Gly Tyr Met Val Pro Ala Tyr Tyr Val Gln
    4070                4075                4080

Met Glu Lys Leu Pro Leu Thr Ala Asn Gly Lys Val Asp Arg Arg
    4085                4090                4095

Ala Leu Pro Gln Pro Ser Gly Glu Arg Thr Thr Gly Ser Ala Phe
    4100                4105                4110

Val Ala Ala Gln Asn Asp Thr Glu Ala Lys Leu Gln Gln Ile Trp
    4115                4120                4125

Gln Glu Val Leu Gly Ile Pro Ala Ile Gly Ile His Asp Asn Phe
    4130                4135                4140

Phe Glu Ile Gly Gly His Ser Leu Lys Ala Met Asn Val Ile Thr
    4145                4150                4155

Gln Val His Lys Thr Phe Gln Val Glu Leu Pro Leu Lys Ala Leu
    4160                4165                4170

Phe Ala Thr Pro Thr Ile His Glu Leu Ala Ala His Ile Ser Glu
    4175                4180                4185

Lys Thr Glu Tyr Thr Ala Ile Gln Pro Val Ala Ala Gln Glu Phe
    4190                4195                4200

Tyr Pro Val Ser Ser Ala Gln Lys Arg Met Tyr Ile Leu Gln Gln
    4205                4210                4215

Phe Glu Gly Asn Gly Ile Ser Tyr Asn Ile Ser Gly Ala Ile Leu
    4220                4225                4230

Leu Glu Gly Lys Leu Asp Tyr Ala Arg Phe Ala Ser Ala Val Gln
    4235                4240                4245

Gln Leu Ala Glu Arg His Glu Ala Leu Arg Thr Ser Phe His Arg
    4250                4255                4260

Ile Asp Gly Glu Pro Val Gln Lys Val His Glu Glu Val Glu Val
    4265                4270                4275

Pro Leu Phe Met Leu Glu Ala Pro Glu Asp Gln Ala Glu Lys Ile
    4280                4285                4290

Met Arg Glu Phe Val Arg Pro Phe Asp Leu Gly Val Ala Pro Leu
    4295                4300                4305

Met Arg Thr Gly Leu Leu Lys Leu Gly Lys Asp Arg His Leu Phe
    4310                4315                4320

Leu Leu Asp Met His His Ile Ile Ser Asp Gly Val Ser Ser Gln
    4325                4330                4335

Ile Leu Leu Arg Glu Phe Ala Glu Leu Tyr Gln Gly Ala Asp Leu
    4340                4345                4350

Gln Pro Leu Ser Leu Gln Tyr Lys Asp Phe Ala Ala Trp Gln Asn
    4355                4360                4365

Glu Leu Phe Gln Thr Glu Ala Tyr Lys Lys Gln Glu Gln His Trp
    4370                4375                4380

Leu Asn Thr Phe Ala Asp Glu Ile Pro Leu Leu Asn Leu Pro Thr
    4385                4390                4395

Asp Tyr Pro Arg Pro Ser Val Gln Ser Phe Ala Gly Asp Leu Val
    4400                4405                4410

Leu Phe Ala Ala Gly Lys Glu Leu Leu Glu Arg Leu Gln Gln Val
    4415                4420                4425

Ala Ser Glu Thr Gly Thr Thr Leu Tyr Met Ile Leu Leu Ala Ala
    4430                4435                4440

Tyr Asn Val Leu Leu Ser Lys Tyr Thr Gly Gln Glu Asp Ile Ile
    4445                4450                4455

Val Gly Thr Pro Val Ala Gly Arg Ser His Ala Asp Val Glu Asn
    4460                4465                4470

Ile Met Gly Ile Phe Val Asn Thr Leu Ala Leu Arg Asn Gln Pro
    4475                4480                4485

Ala Ser Ser Lys Thr Met Leu Glu Asn Asn Ile Thr Gln Cys Asp
    4490                4495                4500

Ser Ile Asn Asp Val Tyr Leu Lys Glu Glu Ala Ile Thr Leu Met
    4505                4510                4515

Asp Met Leu Glu Ser Gln Leu Lys His Gln Ala Asp Gly Tyr Val
    4520                4525                4530

Val Ile Asp Gln Glu Glu Ser Leu Ser Tyr Ala Asp Phe Tyr Leu
    4535                4540                4545

Arg Val Lys Glu Ile Gly Tyr Cys Leu Ser Glu Ile Ser Ser Lys
    4550                4555                4560

Asn Ser Val Gly Ile Gly Leu Phe Cys Asp Pro Ser Ile Asp Leu
    4565                4570                4575

Ile Cys Gly Ala Trp Gly Ile Leu Ser Ala Asp Lys Ala Tyr Leu
    4580                4585                4590

Pro Leu Ser Pro Asp Tyr Pro Thr Glu Arg Leu Lys Tyr Met Ile
    4595                4600                4605

Glu Asp Ser Gly Ile Asp Val Ile Phe Thr Gln Ser His Leu Lys
    4610                4615                4620

Ala Gln Leu Gln Asp Ile Ala Pro Lys Ser Val Leu Ile Met Thr
    4625                4630                4635

Pro Glu Asp Val Ala Leu Thr Ile Lys Thr Arg Thr Ile Glu Asp
    4640                4645                4650

Ile Leu Gly Thr Val Gln Val Pro Lys Pro Thr Ser Leu Ala Tyr
    4655                4660                4665

Ile Ile Tyr Thr Ser Gly Ser Thr Gly Lys Pro Lys Gly Val Met
    4670                4675                4680

Ile Glu His His Ser Ile Val Asn Gln Met Arg Phe Leu Ala Lys
    4685                4690                4695

Ala Phe Lys Leu Gly Cys His Ser Arg Ile Leu Gln Lys Thr Pro
    4700                4705                4710

Met Ser Phe Asp Ala Ala Gln Trp Glu Ile Leu Ala Pro Ala Ile
    4715                4720                4725

Gly Gly Gln Val Ile Met Gly Pro Leu Gly Cys Tyr Arg Asp Pro
    4730                4735                4740

Asp Ala Ile Ile Lys Thr Ile Leu Gln His Gln Val Thr Thr Leu
    4745                4750                4755

Gln Cys Val Pro Thr Leu Leu Gln Ala Leu Leu Asp Asn Pro Asn
    4760                4765                4770

Phe Leu Asp Cys Leu Ser Leu Thr Gln Val Phe Ser Gly Gly Glu
    4775                4780                4785

Ala Leu Thr Thr Lys Leu Ala Thr Gln Phe Leu Asn Ser Phe Thr
    4790                4795                4800

His Cys Glu Leu Ile Asn Leu Tyr Gly Pro Thr Glu Cys Thr Ile
    4805                4810                4815

Asn Ser Ser Phe Phe Arg Val Thr Asn Glu Thr Leu Pro Asn Tyr

4820                4825                4830

Gln Thr Ser Ile Ser Ile Gly Ala Pro Val Asp Asn Thr Glu Tyr
    4835                4840                4845

Tyr Val Leu Asp Asp Asp Arg Leu Pro Val Ala Val Gly Glu Ile
    4850                4855                4860

Gly Glu Leu Tyr Ile Ser Gly Ala Gln Leu Ala Arg Gly Tyr Leu
    4865                4870                4875

His Lys Pro Glu Met Thr Lys Asp Lys Phe Ile Cys Asn His Leu
    4880                4885                4890

Val Ser Gly Thr Gln His Gln Trp Leu Tyr Arg Thr Gly Asp Leu
    4895                4900                4905

Val Thr Arg Gly Ala Asp Gly Asn Thr Tyr Phe Val Gly Arg Val
    4910                4915                4920

Asp Ser Gln Val Lys Leu Arg Gly Tyr Arg Ile Glu Leu Asp Glu
    4925                4930                4935

Ile Arg His Ala Ile Glu Glu His Ser Trp Ile Lys Thr Ala Ala
    4940                4945                4950

Met Leu Ile Lys Lys Asp Ala Arg Thr Gly Phe Gln Asn Leu Ile
    4955                4960                4965

Ala Cys Val Glu Leu Asp Glu Lys Glu Ala Ala Leu Met Asp Gln
    4970                4975                4980

Gly Asn Ser Ser Ser His His Lys Ser Lys Ala Asp Lys Leu Gln
    4985                4990                4995

Val Lys Ala Gln Leu Ser Asn Ser Gly Cys Arg Ser Glu Glu Leu
    5000                5005                5010

Cys Glu Asn Arg Pro Thr Phe Leu Leu Pro Tyr Gln Glu Gly Glu
    5015                5020                5025

Ile Lys Gln Arg Glu Tyr Ala Phe Gly Arg Lys Thr Tyr Arg Tyr
    5030                5035                5040

Phe Glu Gly Thr Glu Ile Thr Val Glu Lys Leu Lys Lys Leu Leu
    5045                5050                5055

Thr Ala Thr Gln Ser Asn Glu Ile Ser Ser Leu Pro Leu Ser His
    5060                5065                5070

Leu Thr Leu Asn Asp Phe Gly Tyr Ala Leu Arg Tyr Phe Gly Gln
    5075                5080                5085

Phe Thr Ser His Gln Arg Leu Leu Pro Lys Tyr Ala Tyr Ala Ser
    5090                5095                5100

Pro Gly Ala Leu Tyr Ala Thr Gln Met Tyr Phe Glu Leu His Asn
    5105                5110                5115

Val Leu Gly Leu Asp Ala Gly Ile Tyr Tyr Tyr His Pro Val Thr
    5120                5125                5130

His Lys Leu Ile Lys Ile Ser Thr Leu Ser Arg Arg Gln Met Pro
    5135                5140                5145

Thr Ile Lys Val His Phe Ile Gly Lys His Glu Ala Ile Glu Pro
    5150                5155                5160

Val Tyr Lys Asn Asn Ile Gln Glu Val Leu Glu Met Glu Ala Gly
    5165                5170                5175

His Met Met Gly Leu Phe Asp Asp Val Leu Pro Glu Ile Gly Leu
    5180                5185                5190

Ser Ile Gly Lys Ser Glu Tyr Gln Asp Glu Cys Pro Asp Trp Tyr
    5195                5200                5205

Asp Gly Asp Ile Gln Asp Tyr Tyr Leu Gly Ala Phe Glu Ile Cys
    5210                5215                5220

Ser Tyr Glu His Gly Leu Pro Pro Phe Glu Thr Asp Ile Tyr Leu
    5225                5230                5235

Gln Thr His Ala His Lys Ile Pro Glu Met Pro Cys Gly Leu Tyr
    5240                5245                5250

His Phe Ser Asn Gly Glu Phe Val Arg Ile Ser Asp Asp Ile Val
    5255                5260                5265

Arg Lys Lys Asp Val Ile Ala Ile Asn Gln Gln Val Tyr Asp Arg
    5270                5275                5280

Ser Ser Phe Gly Val Ser Ile Ile Pro Arg Cys Val Pro Glu Trp
    5285                5290                5295

His Tyr Tyr Ile Thr Leu Gly Arg Arg Leu His Ala Leu Gln Ser
    5300                5305                5310

Asn Pro Leu Tyr Ile Gly Leu Met Ser Ser Gly Tyr Ser Ser Lys
    5315                5320                5325

Ser Asn Asn Asp Leu Pro Ser Ala Lys Arg Met Arg Ser Ile Leu
    5330                5335                5340

Asn Ala Leu Asp Arg Pro Met Ala Ala Phe Tyr Phe Cys Ile Gly
    5345                5350                5355

Gly Gly Ile Ser Gln Ala Gln Tyr Met Cys Glu Gly Met Lys Glu
    5360                5365                5370

Asp Val Val His Met Lys Gly Pro Val Glu Ile Ile Lys Asp Asp
    5375                5380                5385

Leu Gln Gln Gln Leu Pro Gln Tyr Met Ile Pro Asn Lys Val Leu
    5390                5395                5400

Val Phe Asp Lys Leu Pro Leu Thr Ala Asn Gly Lys Val Asp Tyr
    5405                5410                5415

Gln Ser Leu Ser Glu Ser Lys Ala Val Glu Asn Val Ser Thr Gln
    5420                5425                5430

Arg Leu Leu Val Pro Leu His Thr Asp Thr Glu Ile Arg Leu Gly
    5435                5440                5445

Lys Ile Trp Met Glu Val Leu Lys Trp Asp Ser Val Ser Ala Leu
    5450                5455                5460

Asp Asp Phe Phe Glu Ser Gly Gly Asn Ser Leu Met Ala Val Ala
    5465                5470                5475

Met Val Asn Lys Ile Asn Ala Ala Phe Asn Ile Arg Phe Pro Leu
    5480                5485                5490

Gln Ile Leu Phe Gln Ser Pro Asn Ile Ala Glu Leu Ala Lys Trp
    5495                5500                5505

Ile Glu Gln Thr Asp Ser Lys Thr Ile Ser Arg Leu Ile Leu Leu
    5510                5515                5520

Asn Gln Ala Ser Lys Asp Pro Ile Tyr Cys Trp Pro Gly Leu Gly
    5525                5530                5535

Gly Tyr Pro Met Ser Leu Arg Leu Leu Ala Asn Lys Val Val Pro
    5540                5545                5550

Asp Arg Ala Phe Tyr Gly Ile Gln Ala Tyr Gly Ile Asn Glu Ser
    5555                5560                5565

Glu Ile Pro Phe Ser Ser Ile Gln Arg Met Ala Glu Glu Asp Ile
    5570                5575                5580

Lys Glu Ile Lys Lys Ile Gln Pro Glu Gly Pro Tyr Ile Leu Trp
    5585                5590                5595

Gly Tyr Ser Phe Gly Ala Arg Val Ala Phe Glu Val Ala Tyr Gln
    5600                5605                5610

Leu Glu Gln Ala Gly Glu Glu Val Asn Ala Leu Asn Leu Leu Ala
    5615                5620                5625

Pro Gly Ser Pro His Leu Asp Met Lys Gln Ala Glu Tyr Met Asp
    5630                5635                5640

Lys Gly Ala Glu Phe Thr Asn Pro Ala Phe Val Lys Ile Leu Phe
    5645                5650                5655

Ser Val Phe Ser Arg Ser Ile Asn Ser Pro Met Val Lys Thr Cys
    5660                5665                5670

Leu Glu Gln Val Asn Ser Glu Thr Thr Phe Ile Asn Phe Ile Cys
    5675                5680                5685

Ser Arg Phe Lys Asn Leu Glu Pro Ser Leu Val Lys Arg Ile Val
    5690                5695                5700

Arg Ile Val Thr Leu Thr Tyr Asp Phe Lys Tyr Ser Ile Asp Glu
    5705                5710                5715

Leu Tyr His Arg His Leu Lys Ala Pro Ile Thr Ile Phe Lys Ala
    5720                5725                5730

Asn Arg Asp Asn Asp Ser Phe Ile Glu Glu Ser Asp Val Ile Ser
    5735                5740                5745

Ser Met Ser Pro Lys Ile Ile Glu Leu Ile Ser Asp His Tyr Gln
    5750                5755                5760

Leu Leu Glu Ser Glu Gly Val Ala Glu Ile Glu Lys Ile Ile

5765                5770                5775

<210>  33
<211>  3251
<212>  PRT
<213>  Artificial Sequence

<220>
<223>  NRPSase synthesizing a Indigoidine-tagged Dipeptide consisting of
       Proline and Leucine

<400>  33

Met Asp Cys Val Ala Asn Asn Ser Gly Val Glu Leu Cys Gln Ile Pro
1               5                   10                  15

Leu Leu Thr Glu Ala Glu Thr Ser Gln Leu Leu Ala Lys Arg Thr Glu
            20                  25                  30

Thr Ala Ala Asp Tyr Pro Ala Ala Thr Met His Glu Leu Phe Ser Arg
        35                  40                  45

Gln Ala Glu Lys Thr Pro Glu Gln Val Ala Val Val Phe Ala Asp Gln
    50                  55                  60

His Leu Thr Tyr Arg Glu Leu Asp Glu Lys Ser Asn Gln Leu Ala Arg
65                  70                  75                  80

Phe Leu Arg Lys Lys Gly Ile Gly Thr Gly Ser Leu Val Gly Thr Leu
                85                  90                  95

Leu Asp Arg Ser Leu Asp Met Ile Val Gly Ile Leu Gly Val Leu Lys
            100                 105                 110

Ala Gly Gly Ala Phe Val Pro Ile Asp Pro Glu Leu Pro Ala Glu Arg
        115                 120                 125

Ile Ala Tyr Met Leu Thr His Ser Arg Val Pro Leu Val Val Thr Gln
    130                 135                 140

Asn His Leu Arg Ala Lys Val Thr Thr Pro Thr Glu Thr Ile Asp Ile
145                 150                 155                 160

Asn Thr Ala Val Ile Gly Glu Glu Ser Arg Ala Pro Ile Glu Ser Leu
                165                 170                 175

Asn Gln Pro His Asp Leu Phe Tyr Ile Ile Tyr Thr Ser Gly Thr Thr
            180                 185                 190

Gly Gln Pro Lys Gly Val Met Leu Glu His Arg Asn Met Ala Asn Leu

195                 200                 205

Met His Phe Thr Phe Asp Gln Thr Asn Ile Ala Phe His Glu Lys Val
    210                 215                 220

Leu Gln Tyr Thr Thr Cys Ser Phe Asp Val Cys Tyr Gln Glu Ile Phe
225                 230                 235                 240

Ser Thr Leu Leu Ser Gly Gly Gln Leu Tyr Leu Ile Thr Asn Glu Leu
                245                 250                 255

Arg Arg His Val Glu Lys Leu Phe Ala Phe Ile Gln Glu Lys Gln Ile
            260                 265                 270

Ser Ile Leu Ser Leu Pro Val Ser Phe Leu Lys Phe Ile Phe Asn Glu
        275                 280                 285

Gln Asp Tyr Ala Gln Ser Phe Pro Arg Cys Val Lys His Ile Ile Thr
    290                 295                 300

Ala Gly Glu Gln Leu Val Val Thr His Glu Leu Gln Lys Tyr Leu Arg
305                 310                 315                 320

Gln His Arg Val Phe Leu His Asn His Tyr Gly Pro Ser Glu Thr His
                325                 330                 335

Val Val Thr Thr Cys Thr Met Asp Pro Gly Gln Ala Ile Pro Glu Leu
            340                 345                 350

Pro Pro Ile Gly Lys Pro Ile Ser Asn Thr Gly Ile Tyr Ile Leu Asp
        355                 360                 365

Glu Gly Leu Gln Leu Lys Pro Glu Gly Ile Val Gly Glu Leu Tyr Ile
    370                 375                 380

Ser Gly Ala Asn Val Gly Arg Gly Tyr Leu His Gln Pro Glu Leu Thr
385                 390                 395                 400

Ala Glu Lys Phe Leu Asp Asn Pro Tyr Gln Pro Gly Glu Arg Met Tyr
                405                 410                 415

Arg Thr Gly Asp Leu Ala Leu Trp Leu Pro Asp Gly Gln Leu Glu Phe
            420                 425                 430

Leu Gly Arg Ile Asp His Gln Val Lys Ile Arg Gly His Arg Ile Glu
        435                 440                 445

Leu Gly Glu Ile Glu Ser Arg Leu Leu Asn His Pro Ala Ile Lys Glu
    450                 455                 460

Ala Val Val Ile Asp Arg Ala Asp Glu Thr Gly Gly Lys Phe Leu Cys
465                 470                 475                 480

Ala Tyr Val Val Leu Gln Lys Ala Leu Ser Asp Glu Glu Met Arg Ala
                485                 490                 495

Tyr Leu Ala Gln Ala Leu Pro Glu Tyr Met Ile Pro Ser Phe Phe Val
            500                 505                 510

Thr Leu Glu Arg Ile Pro Val Thr Pro Asn Gly Lys Thr Asp Arg Arg
        515                 520                 525

Ala Leu Pro Lys Pro Glu Gly Ser Ala Lys Thr Lys Ala Asp Tyr Val
    530                 535                 540

Ala Pro Thr Thr Glu Leu Glu Gln Lys Leu Val Ala Ile Trp Glu Gln
545                 550                 555                 560

Ile Leu Gly Val Ser Pro Ile Gly Ile Gln Asp His Phe Phe Thr Leu
                565                 570                 575

Gly Gly His Ser Leu Lys Ala Ile Gln Leu Ile Ser Arg Ile Gln Lys
            580                 585                 590

Glu Cys Gln Ala Asp Val Pro Leu Arg Val Leu Phe Glu Gln Pro Thr
        595                 600                 605

Ile Gln Ala Leu Ala Ala Tyr Val Glu Gly Gly Glu Glu Gly Asn Val
    610                 615                 620

Phe Ser Ile Glu Pro Val Gln Lys Gln Ala Tyr Tyr Pro Val Ser Ser
625                 630                 635                 640

Ala Gln Lys Arg Met Tyr Ile Leu Asp Gln Phe Glu Gly Val Gly Ile
                645                 650                 655

Ser Tyr Asn Met Pro Ser Thr Met Leu Ile Glu Gly Lys Leu Glu Arg
            660                 665                 670

Thr Arg Val Glu Ala Ala Phe Gln Arg Leu Ile Ala Arg His Glu Ser
        675                 680                 685

Leu Arg Thr Ser Phe Ala Val Val Asn Gly Glu Pro Val Gln Asn Ile
    690                 695                 700

His Glu Asp Val Pro Phe Ala Leu Ala Tyr Ser Glu Val Thr Glu Gln
705                 710                 715                 720

Glu Ala Arg Glu Leu Val Ser Ser Leu Val Gln Pro Phe Asp Leu Glu
                725                 730                 735

Val Ala Pro Leu Ile Arg Val Ser Leu Leu Lys Ile Gly Glu Asp Arg
            740                 745                 750

Tyr Val Leu Phe Thr Asp Met His His Ser Ile Ser Asp Gly Val Ser
        755                 760                 765

Ser Gly Ile Leu Leu Ala Glu Trp Val Gln Leu Tyr Gln Gly Asp Val
    770                 775                 780

Leu Pro Glu Leu Arg Ile Gln Tyr Lys Asp Phe Ala Val Trp Gln Gln
785                 790                 795                 800

Glu Phe Ser Gln Ser Ala Ala Phe His Lys Gln Glu Ala Tyr Trp Leu
                805                 810                 815

Gln Thr Phe Ala Asp Asp Ile Pro Val Leu Asn Leu Pro Thr Asp Phe
            820                 825                 830

Thr Arg Pro Ser Thr Gln Ser Phe Ala Gly Asp Gln Cys Thr Ile Gly
        835                 840                 845

Ala Gly Lys Ala Leu Thr Glu Gly Leu His Gln Leu Ala Gln Ala Thr
    850                 855                 860

Gly Thr Thr Leu Tyr Met Val Leu Leu Ala Ala Tyr Asn Val Leu Leu
865                 870                 875                 880

Ala Lys Tyr Ala Gly Gln Glu Asp Ile Ile Val Gly Thr Pro Ile Thr
                885                 890                 895

Gly Arg Ser His Ala Asp Leu Glu Pro Ile Val Gly Met Phe Val Asn
            900                 905                 910

Thr Leu Ala Met Arg Asn Lys Pro Gln Arg Glu Lys Thr Phe Ser Glu
        915                 920                 925

Phe Leu Gln Glu Val Lys Gln Asn Ala Leu Asp Ala Tyr Gly His Gln
    930                 935                 940

Asp Tyr Pro Phe Glu Glu Leu Val Glu Lys Leu Ala Ile Ala Arg Asp
945                 950                 955                 960

Leu Ser Arg Asn Pro Leu Phe Asp Thr Val Phe Thr Phe Gln Asn Ser
                965                 970                 975

Thr Glu Glu Val Met Thr Leu Pro Glu Cys Thr Leu Ala Pro Phe Met
            980                 985                 990

Thr Asp Glu Thr Gly Gln His Ala Lys Phe Asp Leu Thr Phe Ser Ala
        995                 1000                1005

Thr Glu Glu Arg Glu Glu Met Thr Ile Gly Val Glu Tyr Ser Thr
    1010                1015                1020

Ser Leu Phe Thr Arg Glu Thr Met Glu Arg Phe Ser Arg His Phe
    1025                1030                1035

Leu Thr Ile Ala Ala Ser Ile Val Gln Asn Pro His Ile Arg Leu
    1040                1045                1050

Gly Glu Ile Asp Met Leu Leu Pro Glu Glu Lys Gln Gln Ile Leu
    1055                1060                1065

Ala Gly Phe Asn Asp Thr Ala Val Ser Tyr Ala Leu Asp Lys Thr
    1070                1075                1080

Leu His Gln Leu Phe Glu Glu Gln Val Asp Lys Thr Pro Asp Gln
    1085                1090                1095

Ala Ala Leu Leu Phe Ser Glu Gln Ser Leu Thr Tyr Ser Glu Leu
    1100                1105                1110

Asn Glu Arg Ala Asn Arg Leu Ala Arg Val Leu Arg Ala Lys Gly
    1115                1120                1125

Val Gly Pro Asp Arg Leu Val Ala Ile Met Ala Glu Arg Ser Pro
    1130                1135                1140

Glu Met Val Ile Gly Ile Leu Gly Ile Leu Lys Ala Gly Gly Ala
    1145                1150                1155

Tyr Val Pro Val Asp Pro Gly Tyr Pro Gln Glu Arg Ile Gln Tyr
    1160                1165                1170

Leu Leu Glu Asp Ser Asn Ala Ala Leu Leu Leu Ser Gln Ala His
    1175                1180                1185

Leu Leu Pro Leu Leu Ala Gln Val Ser Ser Glu Leu Pro Glu Cys

1190                1195                1200

Leu Asp Leu Asn Ala Glu Leu Asp Ala Gly Leu Ser Gly Ser Asn
    1205                1210                1215

Leu Pro Ala Val Asn Gln Pro Thr Asp Leu Ala Tyr Val Ile Tyr
    1220                1225                1230

Thr Ser Gly Thr Thr Gly Lys Pro Lys Gly Val Met Ile Pro His
    1235                1240                1245

Gln Gly Ile Val Asn Cys Leu Gln Trp Arg Arg Asp Glu Tyr Gly
    1250                1255                1260

Phe Gly Pro Ser Asp Lys Ala Leu Gln Val Phe Ser Phe Ala Phe
    1265                1270                1275

Asp Gly Phe Val Ala Ser Leu Phe Ala Pro Leu Leu Gly Gly Ala
    1280                1285                1290

Thr Cys Val Leu Pro Gln Glu Ala Ala Ala Lys Asp Pro Val Ala
    1295                1300                1305

Leu Lys Lys Leu Met Ala Ala Thr Glu Val Thr His Tyr Tyr Gly
    1310                1315                1320

Val Pro Ser Leu Phe Gln Ala Ile Leu Asp Cys Ser Thr Thr Thr
    1325                1330                1335

Asp Phe Asn Gln Leu Arg Cys Val Thr Leu Gly Gly Glu Lys Leu
    1340                1345                1350

Pro Val Gln Leu Val Gln Lys Thr Lys Glu Lys His Pro Ala Ile
    1355                1360                1365

Glu Ile Asn Asn Glu Tyr Gly Pro Thr Glu Asn Ser Val Val Thr
    1370                1375                1380

Thr Ile Ser Arg Ser Ile Glu Ala Gly Gln Ala Ile Thr Ile Gly
    1385                1390                1395

Arg Pro Leu Ala Asn Val Gln Val Tyr Ile Val Asp Glu Gln His
    1400                1405                1410

His Leu Gln Pro Ile Gly Val Val Gly Glu Leu Cys Ile Gly Gly
    1415                1420                1425

Ala Gly Leu Ala Arg Gly Tyr Leu Asn Lys Pro Glu Leu Thr Ala
    1430                1435                1440

Glu Lys Phe Val Ala Asn Pro Phe Arg Pro Gly Glu Arg Met Tyr
    1445                1450                1455

Lys Thr Gly Asp Leu Val Lys Trp Arg Thr Asp Gly Thr Ile Glu
    1460                1465                1470

Tyr Ile Gly Arg Ala Asp Glu Gln Val Lys Val Arg Gly Tyr Arg
    1475                1480                1485

Ile Glu Ile Gly Glu Ile Glu Ser Ala Val Leu Ala Tyr Gln Gly
    1490                1495                1500

Ile Asp Gln Ala Val Val Val Ala Arg Asp Asp Asp Ala Thr Ala
    1505                1510                1515

Gly Ser Tyr Leu Cys Ala Tyr Phe Val Ala Ala Thr Ala Val Ser
    1520                1525                1530

Val Ser Gly Leu Arg Ser His Leu Ala Lys Glu Leu Pro Ala Tyr
    1535                1540                1545

Met Ile Pro Ser Tyr Phe Val Glu Leu Asp Gln Leu Pro Leu Ser
    1550                1555                1560

Ala Asn Gly Lys Val Asp Arg Lys Ala Leu Pro Lys Pro Gln Gln
    1565                1570                1575

Ser Asp Ala Thr Thr Arg Glu Tyr Val Ala Pro Arg Asn Ala Thr
    1580                1585                1590

Glu Gln Gln Leu Ala Ala Ile Trp Gln Glu Val Leu Gly Val Glu
    1595                1600                1605

Pro Ile Gly Ile Thr Asp Gln Phe Phe Glu Leu Gly Gly His Ser
    1610                1615                1620

Leu Lys Ala Thr Leu Leu Ile Ala Lys Val Tyr Glu Tyr Met Gln
    1625                1630                1635

Ile Glu Leu Pro Leu Asn Leu Ile Phe Gln Tyr Pro Thr Ile Glu
    1640                1645                1650

Lys Val Ala Asp Phe Ile Thr Ser Glu Lys Thr Glu Tyr Thr Ala
    1655                1660                1665

Ile Gln Pro Val Ala Ala Gln Glu Phe Tyr Pro Val Ser Ser Ala
    1670                1675                1680

Gln Lys Arg Met Tyr Ile Leu Gln Gln Phe Glu Gly Asn Gly Ile
    1685                1690                1695

Ser Tyr Asn Ile Ser Gly Ala Ile Leu Leu Glu Gly Lys Leu Asp
    1700                1705                1710

Tyr Ala Arg Phe Ala Ser Ala Val Gln Gln Leu Ala Glu Arg His
    1715                1720                1725

Glu Ala Leu Arg Thr Ser Phe His Arg Ile Asp Gly Glu Pro Val
    1730                1735                1740

Gln Lys Val His Glu Glu Val Glu Val Pro Leu Phe Met Leu Glu
    1745                1750                1755

Ala Pro Glu Asp Gln Ala Glu Lys Ile Met Arg Glu Phe Val Arg
    1760                1765                1770

Pro Phe Asp Leu Gly Val Ala Pro Leu Met Arg Thr Gly Leu Leu
    1775                1780                1785

Lys Leu Gly Lys Asp Arg His Leu Phe Leu Leu Asp Met His His
    1790                1795                1800

Ile Ile Ser Asp Gly Val Ser Ser Gln Ile Leu Leu Arg Glu Phe
    1805                1810                1815

Ala Glu Leu Tyr Gln Gly Ala Asp Leu Gln Pro Leu Ser Leu Gln
    1820                1825                1830

Tyr Lys Asp Phe Ala Ala Trp Gln Asn Glu Leu Phe Gln Thr Glu
    1835                1840                1845

Ala Tyr Lys Lys Gln Glu Gln His Trp Leu Asn Thr Phe Ala Asp
    1850                1855                1860

Glu Ile Pro Leu Leu Asn Leu Pro Thr Asp Tyr Pro Arg Pro Ser
    1865                1870                1875

Val Gln Ser Phe Ala Gly Asp Leu Val Leu Phe Ala Ala Gly Lys
    1880                1885                1890

Glu Leu Leu Glu Arg Leu Gln Gln Val Ala Ser Glu Thr Gly Thr
    1895                1900                1905

Thr Leu Tyr Met Ile Leu Leu Ala Ala Tyr Asn Val Leu Leu Ser
    1910                1915                1920

Lys Tyr Thr Gly Gln Glu Asp Ile Ile Val Gly Thr Pro Val Ala
    1925                1930                1935

Gly Arg Ser His Ala Asp Val Glu Asn Ile Met Gly Ile Phe Val
    1940                1945                1950

Asn Thr Leu Ala Leu Arg Asn Gln Pro Ala Ser Ser Lys Thr Met
    1955                1960                1965

Leu Glu Asn Asn Ile Thr Gln Cys Asp Ser Ile Asn Asp Val Tyr
    1970                1975                1980

Leu Lys Glu Glu Ala Ile Thr Leu Met Asp Met Leu Glu Ser Gln
    1985                1990                1995

Leu Lys His Gln Ala Asp Gly Tyr Val Val Ile Asp Gln Glu Glu
    2000                2005                2010

Ser Leu Ser Tyr Ala Asp Phe Tyr Leu Arg Val Lys Glu Ile Gly
    2015                2020                2025

Tyr Cys Leu Ser Glu Ile Ser Ser Lys Asn Ser Val Gly Ile Gly
    2030                2035                2040

Leu Phe Cys Asp Pro Ser Ile Asp Leu Ile Cys Gly Ala Trp Gly
    2045                2050                2055

Ile Leu Ser Ala Asp Lys Ala Tyr Leu Pro Leu Ser Pro Asp Tyr
    2060                2065                2070

Pro Thr Glu Arg Leu Lys Tyr Met Ile Glu Asp Ser Gly Ile Asp
    2075                2080                2085

Val Ile Phe Thr Gln Ser His Leu Lys Ala Gln Leu Gln Asp Ile
    2090                2095                2100

Ala Pro Lys Ser Val Leu Ile Met Thr Pro Glu Asp Val Ala Leu
    2105                2110                2115

Thr Ile Lys Thr Arg Thr Ile Glu Asp Ile Leu Gly Thr Val Gln
    2120                2125                2130

Val Pro Lys Pro Thr Ser Leu Ala Tyr Ile Ile Tyr Thr Ser Gly

2135                2140                2145

Ser Thr Gly Lys Pro Lys Gly Val Met Ile Glu His His Ser Ile
    2150                2155                2160

Val Asn Gln Met Arg Phe Leu Ala Lys Ala Phe Lys Leu Gly Cys
    2165                2170                2175

His Ser Arg Ile Leu Gln Lys Thr Pro Met Ser Phe Asp Ala Ala
    2180                2185                2190

Gln Trp Glu Ile Leu Ala Pro Ala Ile Gly Gly Gln Val Ile Met
    2195                2200                2205

Gly Pro Leu Gly Cys Tyr Arg Asp Pro Asp Ala Ile Ile Lys Thr
    2210                2215                2220

Ile Leu Gln His Gln Val Thr Thr Leu Gln Cys Val Pro Thr Leu
    2225                2230                2235

Leu Gln Ala Leu Leu Asp Asn Pro Asn Phe Leu Asp Cys Leu Ser
    2240                2245                2250

Leu Thr Gln Val Phe Ser Gly Gly Glu Ala Leu Thr Thr Lys Leu
    2255                2260                2265

Ala Thr Gln Phe Leu Asn Ser Phe Thr His Cys Glu Leu Ile Asn
    2270                2275                2280

Leu Tyr Gly Pro Thr Glu Cys Thr Ile Asn Ser Ser Phe Phe Arg
    2285                2290                2295

Val Thr Asn Glu Thr Leu Pro Asn Tyr Gln Thr Ser Ile Ser Ile
    2300                2305                2310

Gly Ala Pro Val Asp Asn Thr Glu Tyr Tyr Val Leu Asp Asp Asp
    2315                2320                2325

Arg Leu Pro Val Ala Val Gly Glu Ile Gly Glu Leu Tyr Ile Ser
    2330                2335                2340

Gly Ala Gln Leu Ala Arg Gly Tyr Leu His Lys Pro Glu Met Thr
    2345                2350                2355

Lys Asp Lys Phe Ile Cys Asn His Leu Val Ser Gly Thr Gln His
    2360                2365                2370

Gln Trp Leu Tyr Arg Thr Gly Asp Leu Val Thr Arg Gly Ala Asp
    2375                2380                2385

Gly Asn Thr Tyr Phe Val Gly Arg Val Asp Ser Gln Val Lys Leu
    2390                2395                2400

Arg Gly Tyr Arg Ile Glu Leu Asp Glu Ile Arg His Ala Ile Glu
    2405                2410                2415

Glu His Ser Trp Ile Lys Thr Ala Ala Met Leu Ile Lys Lys Asp
    2420                2425                2430

Ala Arg Thr Gly Phe Gln Asn Leu Ile Ala Cys Val Glu Leu Asp
    2435                2440                2445

Glu Lys Glu Ala Ala Leu Met Asp Gln Gly Asn Ser Ser Ser His
    2450                2455                2460

His Lys Ser Lys Ala Asp Lys Leu Gln Val Lys Ala Gln Leu Ser
    2465                2470                2475

Asn Ser Gly Cys Arg Ser Glu Glu Leu Cys Glu Asn Arg Pro Thr
    2480                2485                2490

Phe Leu Leu Pro Tyr Gln Glu Gly Glu Ile Lys Gln Arg Glu Tyr
    2495                2500                2505

Ala Phe Gly Arg Lys Thr Tyr Arg Tyr Phe Glu Gly Thr Glu Ile
    2510                2515                2520

Thr Val Glu Lys Leu Lys Lys Leu Leu Thr Ala Thr Gln Ser Asn
    2525                2530                2535

Glu Ile Ser Ser Leu Pro Leu Ser His Leu Thr Leu Asn Asp Phe
    2540                2545                2550

Gly Tyr Ala Leu Arg Tyr Phe Gly Gln Phe Thr Ser His Gln Arg
    2555                2560                2565

Leu Leu Pro Lys Tyr Ala Tyr Ala Ser Pro Gly Ala Leu Tyr Ala
    2570                2575                2580

Thr Gln Met Tyr Phe Glu Leu His Asn Val Leu Gly Leu Asp Ala
    2585                2590                2595

Gly Ile Tyr Tyr Tyr His Pro Val Thr His Lys Leu Ile Lys Ile
    2600                2605                2610

Ser Thr Leu Ser Arg Arg Gln Met Pro Thr Ile Lys Val His Phe
    2615                2620                2625

Ile Gly Lys His Glu Ala Ile Glu Pro Val Tyr Lys Asn Asn Ile
    2630                2635                2640

Gln Glu Val Leu Glu Met Glu Ala Gly His Met Met Gly Leu Phe
    2645                2650                2655

Asp Asp Val Leu Pro Glu Ile Gly Leu Ser Ile Gly Lys Ser Glu
    2660                2665                2670

Tyr Gln Asp Glu Cys Pro Asp Trp Tyr Asp Gly Asp Ile Gln Asp
    2675                2680                2685

Tyr Tyr Leu Gly Ala Phe Glu Ile Cys Ser Tyr Glu His Gly Leu
    2690                2695                2700

Pro Pro Phe Glu Thr Asp Ile Tyr Leu Gln Thr His Ala His Lys
    2705                2710                2715

Ile Pro Glu Met Pro Cys Gly Leu Tyr His Phe Ser Asn Gly Glu
    2720                2725                2730

Phe Val Arg Ile Ser Asp Asp Ile Val Arg Lys Lys Asp Val Ile
    2735                2740                2745

Ala Ile Asn Gln Gln Val Tyr Asp Arg Ser Ser Phe Gly Val Ser
    2750                2755                2760

Ile Ile Pro Arg Cys Val Pro Glu Trp His Tyr Tyr Ile Thr Leu
    2765                2770                2775

Gly Arg Arg Leu His Ala Leu Gln Ser Asn Pro Leu Tyr Ile Gly
    2780                2785                2790

Leu Met Ser Ser Gly Tyr Ser Ser Lys Ser Asn Asn Asp Leu Pro
    2795                2800                2805

Ser Ala Lys Arg Met Arg Ser Ile Leu Asn Ala Leu Asp Arg Pro
    2810                2815                2820

Met Ala Ala Phe Tyr Phe Cys Ile Gly Gly Gly Ile Ser Gln Ala
    2825                2830                2835

Gln Tyr Met Cys Glu Gly Met Lys Glu Asp Val Val His Met Lys
    2840                2845                2850

Gly Pro Val Glu Ile Ile Lys Asp Asp Leu Gln Gln Gln Leu Pro
    2855                2860                2865

Gln Tyr Met Ile Pro Asn Lys Val Leu Val Phe Asp Lys Leu Pro
    2870                2875                2880

Leu Thr Ala Asn Gly Lys Val Asp Tyr Gln Ser Leu Ser Glu Ser
    2885                2890                2895

Lys Ala Val Glu Asn Val Ser Thr Gln Arg Leu Leu Val Pro Leu
    2900                2905                2910

His Thr Asp Thr Glu Ile Arg Leu Gly Lys Ile Trp Met Glu Val
    2915                2920                2925

Leu Lys Trp Asp Ser Val Ser Ala Leu Asp Asp Phe Phe Glu Ser
    2930                2935                2940

Gly Gly Asn Ser Leu Met Ala Val Ala Met Val Asn Lys Ile Asn
    2945                2950                2955

Ala Ala Phe Asn Ile Arg Phe Pro Leu Gln Ile Leu Phe Gln Ser
    2960                2965                2970

Pro Asn Ile Ala Glu Leu Ala Lys Trp Ile Glu Gln Thr Asp Ser
    2975                2980                2985

Lys Thr Ile Ser Arg Leu Ile Leu Leu Asn Gln Ala Ser Lys Asp
    2990                2995                3000

Pro Ile Tyr Cys Trp Pro Gly Leu Gly Gly Tyr Pro Met Ser Leu
    3005                3010                3015

Arg Leu Leu Ala Asn Lys Val Val Pro Asp Arg Ala Phe Tyr Gly
    3020                3025                3030

Ile Gln Ala Tyr Gly Ile Asn Glu Ser Glu Ile Pro Phe Ser Ser
    3035                3040                3045

Ile Gln Arg Met Ala Glu Glu Asp Ile Lys Glu Ile Lys Lys Ile
    3050                3055                3060

Gln Pro Glu Gly Pro Tyr Ile Leu Trp Gly Tyr Ser Phe Gly Ala
    3065                3070                3075

Arg Val Ala Phe Glu Val Ala Tyr Gln Leu Glu Gln Ala Gly Glu

3080                3085                3090

Glu Val  Asn Ala Leu Asn Leu  Leu Ala Pro Gly Ser  Pro His Leu
    3095                3100                3105

Asp Met  Lys Gln Ala Glu Tyr  Met Asp Lys Gly Ala  Glu Phe Thr
    3110                3115                3120

Asn Pro  Ala Phe Val Lys Ile  Leu Phe Ser Val Phe  Ser Arg Ser
    3125                3130                3135

Ile Asn  Ser Pro Met Val Lys  Thr Cys Leu Glu Gln  Val Asn Ser
    3140                3145                3150

Glu Thr  Thr Phe Ile Asn Phe  Ile Cys Ser Arg Phe  Lys Asn Leu
    3155                3160                3165

Glu Pro  Ser Leu Val Lys Arg  Ile Val Arg Ile Val  Thr Leu Thr
    3170                3175                3180

Tyr Asp  Phe Lys Tyr Ser Ile  Asp Glu Leu Tyr His  Arg His Leu
    3185                3190                3195

Lys Ala  Pro Ile Thr Ile Phe  Lys Ala Asn Arg Asp  Asn Asp Ser
    3200                3205                3210

Phe Ile  Glu Glu Ser Asp Val  Ile Ser Ser Met Ser  Pro Lys Ile
    3215                3220                3225

Ile Glu  Leu Ile Ser Asp His  Tyr Gln Leu Leu Glu  Ser Glu Gly
    3230                3235                3240

Val Ala  Glu Ile Glu Lys Ile  Ile
    3245                3250

<210>  34
<211>  4284
<212>  PRT
<213>  Artificial Sequence

<220>
<223>  NRPS synthesizing a Valine-Indigoidine-tagged Dipeptide
       consisting of Proline and Leucine. Valine is here used as spacer.

<400>  34

Met Asp Cys Val Ala Asn Asn Ser Gly Val Glu Leu Cys Gln Ile Pro
1               5                   10                  15

Leu Leu Thr Glu Ala Glu Thr Ser Gln Leu Leu Ala Lys Arg Thr Glu

20                  25                  30

Thr Ala Ala Asp Tyr Pro Ala Ala Thr Met His Glu Leu Phe Ser Arg
        35                  40                  45

Gln Ala Glu Lys Thr Pro Glu Gln Val Ala Val Val Phe Ala Asp Gln
    50                  55                  60

His Leu Thr Tyr Arg Glu Leu Asp Glu Lys Ser Asn Gln Leu Ala Arg
65                  70                  75                  80

Phe Leu Arg Lys Lys Gly Ile Gly Thr Gly Ser Leu Val Gly Thr Leu
                85                  90                  95

Leu Asp Arg Ser Leu Asp Met Ile Val Gly Ile Leu Gly Val Leu Lys
            100                 105                 110

Ala Gly Gly Ala Phe Val Pro Ile Asp Pro Glu Leu Pro Ala Glu Arg
        115                 120                 125

Ile Ala Tyr Met Leu Thr His Ser Arg Val Pro Leu Val Val Thr Gln
    130                 135                 140

Asn His Leu Arg Ala Lys Val Thr Thr Pro Thr Glu Thr Ile Asp Ile
145                 150                 155                 160

Asn Thr Ala Val Ile Gly Glu Glu Ser Arg Ala Pro Ile Glu Ser Leu
                165                 170                 175

Asn Gln Pro His Asp Leu Phe Tyr Ile Ile Tyr Thr Ser Gly Thr Thr
            180                 185                 190

Gly Gln Pro Lys Gly Val Met Leu Glu His Arg Asn Met Ala Asn Leu
        195                 200                 205

Met His Phe Thr Phe Asp Gln Thr Asn Ile Ala Phe His Glu Lys Val
    210                 215                 220

Leu Gln Tyr Thr Thr Cys Ser Phe Asp Val Cys Tyr Gln Glu Ile Phe
225                 230                 235                 240

Ser Thr Leu Leu Ser Gly Gly Gln Leu Tyr Leu Ile Thr Asn Glu Leu
                245                 250                 255

Arg Arg His Val Glu Lys Leu Phe Ala Phe Ile Gln Glu Lys Gln Ile
            260                 265                 270

Ser Ile Leu Ser Leu Pro Val Ser Phe Leu Lys Phe Ile Phe Asn Glu
        275                 280                 285

Gln Asp Tyr Ala Gln Ser Phe Pro Arg Cys Val Lys His Ile Ile Thr
    290                 295                 300

Ala Gly Glu Gln Leu Val Val Thr His Glu Leu Gln Lys Tyr Leu Arg
305                 310                 315                 320

Gln His Arg Val Phe Leu His Asn His Tyr Gly Pro Ser Glu Thr His
                325                 330                 335

Val Val Thr Thr Cys Thr Met Asp Pro Gly Gln Ala Ile Pro Glu Leu
            340                 345                 350

Pro Pro Ile Gly Lys Pro Ile Ser Asn Thr Gly Ile Tyr Ile Leu Asp
        355                 360                 365

Glu Gly Leu Gln Leu Lys Pro Glu Gly Ile Val Gly Glu Leu Tyr Ile
    370                 375                 380

Ser Gly Ala Asn Val Gly Arg Gly Tyr Leu His Gln Pro Glu Leu Thr
385                 390                 395                 400

Ala Glu Lys Phe Leu Asp Asn Pro Tyr Gln Pro Gly Glu Arg Met Tyr
                405                 410                 415

Arg Thr Gly Asp Leu Ala Leu Trp Leu Pro Asp Gly Gln Leu Glu Phe
            420                 425                 430

Leu Gly Arg Ile Asp His Gln Val Lys Ile Arg Gly His Arg Ile Glu
        435                 440                 445

Leu Gly Glu Ile Glu Ser Arg Leu Leu Asn His Pro Ala Ile Lys Glu
    450                 455                 460

Ala Val Val Ile Asp Arg Ala Asp Glu Thr Gly Gly Lys Phe Leu Cys
465                 470                 475                 480

Ala Tyr Val Val Leu Gln Lys Ala Leu Ser Asp Glu Glu Met Arg Ala
                485                 490                 495

Tyr Leu Ala Gln Ala Leu Pro Glu Tyr Met Ile Pro Ser Phe Phe Val
            500                 505                 510

Thr Leu Glu Arg Ile Pro Val Thr Pro Asn Gly Lys Thr Asp Arg Arg
        515                 520                 525

Ala Leu Pro Lys Pro Glu Gly Ser Ala Lys Thr Lys Ala Asp Tyr Val
    530                 535                 540

Ala Pro Thr Thr Glu Leu Glu Gln Lys Leu Val Ala Ile Trp Glu Gln
545                 550                 555                 560

Ile Leu Gly Val Ser Pro Ile Gly Ile Gln Asp His Phe Phe Thr Leu
                565                 570                 575

Gly Gly His Ser Leu Lys Ala Ile Gln Leu Ile Ser Arg Ile Gln Lys
            580                 585                 590

Glu Cys Gln Ala Asp Val Pro Leu Arg Val Leu Phe Glu Gln Pro Thr
        595                 600                 605

Ile Gln Ala Leu Ala Ala Tyr Val Glu Gly Gly Glu Glu Gly Asn Val
    610                 615                 620

Phe Ser Ile Glu Pro Val Gln Lys Gln Ala Tyr Tyr Pro Val Ser Ser
625                 630                 635                 640

Ala Gln Lys Arg Met Tyr Ile Leu Asp Gln Phe Glu Gly Val Gly Ile
                645                 650                 655

Ser Tyr Asn Met Pro Ser Thr Met Leu Ile Glu Gly Lys Leu Glu Arg
            660                 665                 670

Thr Arg Val Glu Ala Ala Phe Gln Arg Leu Ile Ala Arg His Glu Ser
        675                 680                 685

Leu Arg Thr Ser Phe Ala Val Val Asn Gly Glu Pro Val Gln Asn Ile
    690                 695                 700

His Glu Asp Val Pro Phe Ala Leu Ala Tyr Ser Glu Val Thr Glu Gln
705                 710                 715                 720

Glu Ala Arg Glu Leu Val Ser Ser Leu Val Gln Pro Phe Asp Leu Glu
                725                 730                 735

Val Ala Pro Leu Ile Arg Val Ser Leu Leu Lys Ile Gly Glu Asp Arg
            740                 745                 750

Tyr Val Leu Phe Thr Asp Met His His Ser Ile Ser Asp Gly Val Ser
        755                 760                 765

Ser Gly Ile Leu Leu Ala Glu Trp Val Gln Leu Tyr Gln Gly Asp Val
    770                 775                 780

Leu Pro Glu Leu Arg Ile Gln Tyr Lys Asp Phe Ala Val Trp Gln Gln
785                 790                 795                 800

Glu Phe Ser Gln Ser Ala Ala Phe His Lys Gln Glu Ala Tyr Trp Leu
                805                 810                 815

Gln Thr Phe Ala Asp Asp Ile Pro Val Leu Asn Leu Pro Thr Asp Phe
            820                 825                 830

Thr Arg Pro Ser Thr Gln Ser Phe Ala Gly Asp Gln Cys Thr Ile Gly
        835                 840                 845

Ala Gly Lys Ala Leu Thr Glu Gly Leu His Gln Leu Ala Gln Ala Thr
    850                 855                 860

Gly Thr Thr Leu Tyr Met Val Leu Leu Ala Ala Tyr Asn Val Leu Leu
865                 870                 875                 880

Ala Lys Tyr Ala Gly Gln Glu Asp Ile Ile Val Gly Thr Pro Ile Thr
                885                 890                 895

Gly Arg Ser His Ala Asp Leu Glu Pro Ile Val Gly Met Phe Val Asn
            900                 905                 910

Thr Leu Ala Met Arg Asn Lys Pro Gln Arg Glu Lys Thr Phe Ser Glu
        915                 920                 925

Phe Leu Gln Glu Val Lys Gln Asn Ala Leu Asp Ala Tyr Gly His Gln
    930                 935                 940

Asp Tyr Pro Phe Glu Glu Leu Val Glu Lys Leu Ala Ile Ala Arg Asp
945                 950                 955                 960

Leu Ser Arg Asn Pro Leu Phe Asp Thr Val Phe Thr Phe Gln Asn Ser
                965                 970                 975

Thr Glu Glu Val Met Thr Leu Pro Glu Cys Thr Leu Ala Pro Phe Met
            980                 985                 990

Thr Asp Glu Thr Gly Gln His Ala  Lys Phe Asp Leu Thr  Phe Ser Ala
        995                 1000                 1005

Thr Glu  Glu Arg Glu Glu Met  Thr Ile Gly Val Glu  Tyr Ser Thr
    1010                 1015                 1020

Ser Leu  Phe Thr Arg Glu Thr  Met Glu Arg Phe Ser  Arg His Phe

1025                1030                1035

Leu Thr  Ile Ala Ala Ser Ile  Val Gln Asn Pro His  Ile Arg Leu
    1040                1045                1050

Gly Glu  Ile Asp Met Leu Leu  Pro Glu Glu Lys Gln  Gln Ile Leu
    1055                1060                1065

Ala Gly  Phe Asn Asp Thr Ala  Val Ser Tyr Ala Leu  Asp Lys Thr
    1070                1075                1080

Leu His  Gln Leu Phe Glu Glu  Gln Val Asp Lys Thr  Pro Asp Gln
    1085                1090                1095

Ala Ala  Leu Leu Phe Ser Glu  Gln Ser Leu Thr Tyr  Ser Glu Leu
    1100                1105                1110

Asn Glu  Arg Ala Asn Arg Leu  Ala Arg Val Leu Arg  Ala Lys Gly
    1115                1120                1125

Val Gly  Pro Asp Arg Leu Val  Ala Ile Met Ala Glu  Arg Ser Pro
    1130                1135                1140

Glu Met  Val Ile Gly Ile Leu  Gly Ile Leu Lys Ala  Gly Gly Ala
    1145                1150                1155

Tyr Val  Pro Val Asp Pro Gly  Tyr Pro Gln Glu Arg  Ile Gln Tyr
    1160                1165                1170

Leu Leu  Glu Asp Ser Asn Ala  Ala Leu Leu Leu Ser  Gln Ala His
    1175                1180                1185

Leu Leu  Pro Leu Leu Ala Gln  Val Ser Ser Glu Leu  Pro Glu Cys
    1190                1195                1200

Leu Asp  Leu Asn Ala Glu Leu  Asp Ala Gly Leu Ser  Gly Ser Asn
    1205                1210                1215

Leu Pro  Ala Val Asn Gln Pro  Thr Asp Leu Ala Tyr  Val Ile Tyr
    1220                1225                1230

Thr Ser  Gly Thr Thr Gly Lys  Pro Lys Gly Val Met  Ile Pro His
    1235                1240                1245

Gln Gly  Ile Val Asn Cys Leu  Gln Trp Arg Arg Asp  Glu Tyr Gly
    1250                1255                1260

Phe Gly Pro Ser Asp Lys Ala Leu Gln Val Phe Ser Phe Ala Phe
    1265                1270                1275

Asp Gly Phe Val Ala Ser Leu Phe Ala Pro Leu Leu Gly Gly Ala
    1280                1285                1290

Thr Cys Val Leu Pro Gln Glu Ala Ala Ala Lys Asp Pro Val Ala
    1295                1300                1305

Leu Lys Lys Leu Met Ala Ala Thr Glu Val Thr His Tyr Tyr Gly
    1310                1315                1320

Val Pro Ser Leu Phe Gln Ala Ile Leu Asp Cys Ser Thr Thr Thr
    1325                1330                1335

Asp Phe Asn Gln Leu Arg Cys Val Thr Leu Gly Gly Glu Lys Leu
    1340                1345                1350

Pro Val Gln Leu Val Gln Lys Thr Lys Glu Lys His Pro Ala Ile
    1355                1360                1365

Glu Ile Asn Asn Glu Tyr Gly Pro Thr Glu Asn Ser Val Val Thr
    1370                1375                1380

Thr Ile Ser Arg Ser Ile Glu Ala Gly Gln Ala Ile Thr Ile Gly
    1385                1390                1395

Arg Pro Leu Ala Asn Val Gln Val Tyr Ile Val Asp Glu Gln His
    1400                1405                1410

His Leu Gln Pro Ile Gly Val Val Gly Glu Leu Cys Ile Gly Gly
    1415                1420                1425

Ala Gly Leu Ala Arg Gly Tyr Leu Asn Lys Pro Glu Leu Thr Ala
    1430                1435                1440

Glu Lys Phe Val Ala Asn Pro Phe Arg Pro Gly Glu Arg Met Tyr
    1445                1450                1455

Lys Thr Gly Asp Leu Val Lys Trp Arg Thr Asp Gly Thr Ile Glu
    1460                1465                1470

Tyr Ile Gly Arg Ala Asp Glu Gln Val Lys Val Arg Gly Tyr Arg
    1475                1480                1485

Ile Glu Ile Gly Glu Ile Glu Ser Ala Val Leu Ala Tyr Gln Gly
    1490                1495                1500

Ile Asp Gln Ala Val Val Val Ala Arg Asp Asp Asp Ala Thr Ala
    1505                1510                1515

Gly Ser Tyr Leu Cys Ala Tyr Phe Val Ala Ala Thr Ala Val Ser
    1520                1525                1530

Val Ser Gly Leu Arg Ser His Leu Ala Lys Glu Leu Pro Ala Tyr
    1535                1540                1545

Met Ile Pro Ser Tyr Phe Val Glu Leu Asp Gln Leu Pro Leu Ser
    1550                1555                1560

Ala Asn Gly Lys Val Asp Arg Lys Ala Leu Pro Lys Pro Gln Gln
    1565                1570                1575

Ser Asp Ala Thr Thr Arg Glu Tyr Val Ala Pro Arg Asn Ala Thr
    1580                1585                1590

Glu Gln Gln Leu Ala Ala Ile Trp Gln Glu Val Leu Gly Val Glu
    1595                1600                1605

Pro Ile Gly Ile Thr Asp Gln Phe Phe Glu Leu Gly Gly His Ser
    1610                1615                1620

Leu Lys Ala Thr Leu Leu Ile Ala Lys Val Tyr Glu Tyr Met Gln
    1625                1630                1635

Ile Glu Leu Pro Leu Asn Leu Ile Phe Gln Tyr Pro Thr Ile Glu
    1640                1645                1650

Lys Val Ala Asp Phe Ile Thr Thr Ser Gly Lys Glu Thr Tyr Val
    1655                1660                1665

Pro Ile Glu Pro Ala Pro Leu Gln Glu Tyr Tyr Pro Val Ser Ser
    1670                1675                1680

Ala Gln Lys Arg Met Tyr Val Leu Arg Gln Phe Ala Asp Thr Gly
    1685                1690                1695

Thr Val Tyr Asn Met Pro Ser Ala Leu Tyr Ile Glu Gly Asp Leu
    1700                1705                1710

Asp Arg Lys Arg Phe Glu Ala Ala Ile His Gly Leu Val Glu Arg
    1715                1720                1725

His Glu Ser Leu Arg Thr Ser Phe His Thr Val Asn Gly Glu Pro
    1730                1735                1740

Val Gln Arg Val His Glu His Val Glu Leu Asn Val Gln Tyr Ala
    1745                1750                1755

Glu Val Thr Glu Ala Gln Val Glu Pro Thr Val Glu Ser Phe Val
    1760                1765                1770

Gln Ala Phe Asp Leu Thr Lys Ala Pro Leu Leu Arg Val Gly Leu
    1775                1780                1785

Phe Lys Leu Ala Ala Lys Arg His Leu Phe Leu Leu Asp Met His
    1790                1795                1800

His Ile Ile Ser Asp Gly Val Ser Ala Gly Ile Ile Met Glu Glu
    1805                1810                1815

Phe Ser Lys Leu Tyr Arg Gly Glu Glu Leu Pro Ala Leu Ser Val
    1820                1825                1830

His Tyr Lys Asp Phe Ala Val Trp Gln Ser Glu Leu Phe Gln Ser
    1835                1840                1845

Asp Val Tyr Thr Glu His Glu Asn Tyr Trp Leu Asn Ala Phe Ser
    1850                1855                1860

Gly Asp Ile Pro Val Leu Asn Leu Pro Ala Asp Phe Ser Arg Pro
    1865                1870                1875

Leu Thr Gln Ser Phe Glu Gly Asp Cys Val Ser Phe Gln Ala Asp
    1880                1885                1890

Lys Ala Leu Leu Asp Asp Leu His Lys Leu Ala Gln Glu Ser Gln
    1895                1900                1905

Ser Thr Leu Phe Met Val Leu Leu Ala Ala Tyr Asn Val Leu Leu
    1910                1915                1920

Ala Lys Tyr Ser Gly Gln Glu Asp Ile Val Val Gly Thr Pro Ile
    1925                1930                1935

Ala Gly Arg Ser His Ala Asp Ile Glu Asn Val Leu Gly Met Phe
    1940                1945                1950

Val Asn Thr Leu Ala Leu Arg Asn Tyr Pro Val Glu Thr Lys His
    1955                1960                1965

Phe Gln Ala Phe Leu Glu Glu Val Lys Gln Asn Thr Leu Gln Ala

1970                1975                1980

Tyr Ala His Gln Asp Tyr Pro Phe Glu Ala Leu Val Glu Lys Leu
    1985                1990                1995

Asp Ile Gln Arg Asp Leu Ser Arg Asn Pro Leu Phe Asp Thr Met
    2000                2005                2010

Phe Ile Leu Gln Asn Leu Asp Gln Lys Ala Tyr Glu Leu Asp Gly
    2015                2020                2025

Leu Lys Leu Glu Ala Tyr Pro Ala Gln Ala Gly Asn Ala Lys Phe
    2030                2035                2040

Asp Leu Thr Leu Glu Ala His Glu Asp Glu Thr Gly Ile His Phe
    2045                2050                2055

Ala Leu Val Tyr Ser Thr Lys Leu Phe Gln Arg Glu Ser Ile Glu
    2060                2065                2070

Arg Met Ala Gly His Phe Leu Gln Val Leu Arg Gln Val Val Ala
    2075                2080                2085

Asp Gln Ala Thr Ala Leu Arg Glu Ile Ser Leu Leu Ser Glu Glu
    2090                2095                2100

Glu Arg Arg Ile Val Thr Val Asp Phe Asn Asn Thr Phe Ala Tyr
    2105                2110                2115

Pro Arg Asp Leu Thr Ile Gln Glu Leu Phe Glu Gln Gln Ala Ala
    2120                2125                2130

Lys Thr Pro Glu His Ala Ala Val Val Met Asp Gly Gln Met Leu
    2135                2140                2145

Thr Tyr Arg Glu Leu Asn Glu Lys Ala Asn Gln Leu Ala His Val
    2150                2155                2160

Leu Arg Gln Asn Gly Val Gly Lys Glu Ser Ile Val Gly Leu Leu
    2165                2170                2175

Ala Asp Arg Ser Leu Glu Met Ile Thr Gly Ile Met Gly Ile Leu
    2180                2185                2190

Lys Ala Gly Gly Ala Tyr Leu Gly Leu Asp Pro Glu His Pro Ser
    2195                2200                2205

Glu Arg Leu Ala Tyr Met Leu Glu Asp Gly Gly Val Lys Val Val
    2210                2215                2220

Leu Val Gln Lys His Leu Leu Pro Leu Val Gly Glu Gly Leu Met
    2225                2230                2235

Pro Ile Val Leu Glu Glu Glu Ser Leu Arg Pro Glu Asp Cys Gly
    2240                2245                2250

Asn Pro Ala Ile Val Asn Gly Ala Ser Asp Leu Ala Tyr Val Met
    2255                2260                2265

Tyr Thr Ser Gly Ser Thr Gly Lys Pro Lys Gly Val Met Val Glu
    2270                2275                2280

His Arg Asn Val Thr Arg Leu Val Met His Thr Asn Tyr Val Gln
    2285                2290                2295

Val Arg Glu Ser Asp Arg Met Ile Gln Thr Gly Ala Ile Gly Phe
    2300                2305                2310

Asp Ala Met Thr Phe Glu Ile Phe Gly Ala Leu Leu His Gly Ala
    2315                2320                2325

Ser Leu Tyr Leu Val Ser Lys Asp Val Leu Leu Asp Ala Glu Lys
    2330                2335                2340

Leu Gly Asp Phe Leu Arg Thr Asn Gln Ile Thr Thr Met Trp Leu
    2345                2350                2355

Thr Ser Pro Leu Phe Asn Gln Leu Ser Gln Asp Asn Pro Ala Met
    2360                2365                2370

Phe Asp Ser Leu Arg Ala Leu Ile Val Gly Gly Glu Ala Leu Ser
    2375                2380                2385

Pro Lys His Ile Asn Arg Val Lys Ser Ala Leu Pro Asp Leu Glu
    2390                2395                2400

Ile Trp Asn Gly Tyr Gly Pro Thr Glu Asn Thr Thr Phe Ser Thr
    2405                2410                2415

Cys Tyr Leu Ile Glu Gln His Phe Glu Glu Gln Ile Pro Ile Gly
    2420                2425                2430

Lys Pro Ile Ala Asn Ser Thr Ala Tyr Ile Val Asp Gly Asn Asn
    2435                2440                2445

Gln Pro Gln Pro Ile Gly Val Pro Gly Glu Leu Cys Val Gly Gly
    2450                2455                2460

Asp Gly Val Ala Arg Gly Tyr Val Asn Lys Pro Glu Leu Thr Ala
    2465                2470                2475

Glu Lys Phe Val Pro Asn Pro Phe Ala Pro Gly Glu Thr Met Tyr
    2480                2485                2490

Arg Thr Gly Asp Leu Ala Arg Trp Leu Pro Asp Gly Thr Ile Glu
    2495                2500                2505

Tyr Leu Gly Arg Ile Asp Gln Gln Val Lys Ile Arg Gly Tyr Arg
    2510                2515                2520

Ile Glu Leu Gly Glu Ile Glu Thr Val Leu Ser Gln Gln Ala Gln
    2525                2530                2535

Val Lys Glu Ala Val Val Ala Val Ile Glu Glu Ala Asn Gly Gln
    2540                2545                2550

Lys Ala Leu Cys Ala Tyr Phe Val Pro Glu Gln Ala Val Asp Ala
    2555                2560                2565

Ala Glu Leu Arg Glu Ala Met Ser Lys Gln Leu Pro Gly Tyr Met
    2570                2575                2580

Val Pro Ala Tyr Tyr Val Gln Met Glu Lys Leu Pro Leu Thr Ala
    2585                2590                2595

Asn Gly Lys Val Asp Arg Arg Ala Leu Pro Gln Pro Ser Gly Glu
    2600                2605                2610

Arg Thr Thr Gly Ser Ala Phe Val Ala Ala Gln Asn Asp Thr Glu
    2615                2620                2625

Ala Lys Leu Gln Gln Ile Trp Gln Glu Val Leu Gly Ile Pro Ala
    2630                2635                2640

Ile Gly Ile His Asp Asn Phe Phe Glu Ile Gly Gly His Ser Leu
    2645                2650                2655

Lys Ala Met Asn Val Ile Thr Gln Val His Lys Thr Phe Gln Val
    2660                2665                2670

Glu Leu Pro Leu Lys Ala Leu Phe Ala Thr Pro Thr Ile His Glu
    2675                2680                2685

Leu Ala Ala His Ile Ser Glu Lys Thr Glu Tyr Thr Ala Ile Gln
    2690                2695                2700

Pro Val Ala Ala Gln Glu Phe Tyr Pro Val Ser Ser Ala Gln Lys
    2705                2710                2715

Arg Met Tyr Ile Leu Gln Gln Phe Glu Gly Asn Gly Ile Ser Tyr
    2720                2725                2730

Asn Ile Ser Gly Ala Ile Leu Leu Glu Gly Lys Leu Asp Tyr Ala
    2735                2740                2745

Arg Phe Ala Ser Ala Val Gln Gln Leu Ala Glu Arg His Glu Ala
    2750                2755                2760

Leu Arg Thr Ser Phe His Arg Ile Asp Gly Glu Pro Val Gln Lys
    2765                2770                2775

Val His Glu Glu Val Glu Val Pro Leu Phe Met Leu Glu Ala Pro
    2780                2785                2790

Glu Asp Gln Ala Glu Lys Ile Met Arg Glu Phe Val Arg Pro Phe
    2795                2800                2805

Asp Leu Gly Val Ala Pro Leu Met Arg Thr Gly Leu Leu Lys Leu
    2810                2815                2820

Gly Lys Asp Arg His Leu Phe Leu Leu Asp Met His His Ile Ile
    2825                2830                2835

Ser Asp Gly Val Ser Ser Gln Ile Leu Leu Arg Glu Phe Ala Glu
    2840                2845                2850

Leu Tyr Gln Gly Ala Asp Leu Gln Pro Leu Ser Leu Gln Tyr Lys
    2855                2860                2865

Asp Phe Ala Ala Trp Gln Asn Glu Leu Phe Gln Thr Glu Ala Tyr
    2870                2875                2880

Lys Lys Gln Glu Gln His Trp Leu Asn Thr Phe Ala Asp Glu Ile
    2885                2890                2895

Pro Leu Leu Asn Leu Pro Thr Asp Tyr Pro Arg Pro Ser Val Gln
    2900                2905                2910

Ser Phe Ala Gly Asp Leu Val Leu Phe Ala Ala Gly Lys Glu Leu

2915                2920                2925

Leu Glu Arg Leu Gln Gln Val Ala Ser Glu Thr Gly Thr Thr Leu
    2930                2935                2940

Tyr Met Ile Leu Leu Ala Ala Tyr Asn Val Leu Leu Ser Lys Tyr
    2945                2950                2955

Thr Gly Gln Glu Asp Ile Ile Val Gly Thr Pro Val Ala Gly Arg
    2960                2965                2970

Ser His Ala Asp Val Glu Asn Ile Met Gly Ile Phe Val Asn Thr
    2975                2980                2985

Leu Ala Leu Arg Asn Gln Pro Ala Ser Ser Lys Thr Met Leu Glu
    2990                2995                3000

Asn Asn Ile Thr Gln Cys Asp Ser Ile Asn Asp Val Tyr Leu Lys
    3005                3010                3015

Glu Glu Ala Ile Thr Leu Met Asp Met Leu Glu Ser Gln Leu Lys
    3020                3025                3030

His Gln Ala Asp Gly Tyr Val Val Ile Asp Gln Glu Glu Ser Leu
    3035                3040                3045

Ser Tyr Ala Asp Phe Tyr Leu Arg Val Lys Glu Ile Gly Tyr Cys
    3050                3055                3060

Leu Ser Glu Ile Ser Ser Lys Asn Ser Val Gly Ile Gly Leu Phe
    3065                3070                3075

Cys Asp Pro Ser Ile Asp Leu Ile Cys Gly Ala Trp Gly Ile Leu
    3080                3085                3090

Ser Ala Asp Lys Ala Tyr Leu Pro Leu Ser Pro Asp Tyr Pro Thr
    3095                3100                3105

Glu Arg Leu Lys Tyr Met Ile Glu Asp Ser Gly Ile Asp Val Ile
    3110                3115                3120

Phe Thr Gln Ser His Leu Lys Ala Gln Leu Gln Asp Ile Ala Pro
    3125                3130                3135

Lys Ser Val Leu Ile Met Thr Pro Glu Asp Val Ala Leu Thr Ile
    3140                3145                3150

Lys Thr Arg Thr Ile Glu Asp Ile Leu Gly Thr Val Gln Val Pro
    3155                3160                3165

Lys Pro Thr Ser Leu Ala Tyr Ile Ile Tyr Thr Ser Gly Ser Thr
    3170                3175                3180

Gly Lys Pro Lys Gly Val Met Ile Glu His His Ser Ile Val Asn
    3185                3190                3195

Gln Met Arg Phe Leu Ala Lys Ala Phe Lys Leu Gly Cys His Ser
    3200                3205                3210

Arg Ile Leu Gln Lys Thr Pro Met Ser Phe Asp Ala Ala Gln Trp
    3215                3220                3225

Glu Ile Leu Ala Pro Ala Ile Gly Gly Gln Val Ile Met Gly Pro
    3230                3235                3240

Leu Gly Cys Tyr Arg Asp Pro Asp Ala Ile Ile Lys Thr Ile Leu
    3245                3250                3255

Gln His Gln Val Thr Thr Leu Gln Cys Val Pro Thr Leu Leu Gln
    3260                3265                3270

Ala Leu Leu Asp Asn Pro Asn Phe Leu Asp Cys Leu Ser Leu Thr
    3275                3280                3285

Gln Val Phe Ser Gly Gly Glu Ala Leu Thr Thr Lys Leu Ala Thr
    3290                3295                3300

Gln Phe Leu Asn Ser Phe Thr His Cys Glu Leu Ile Asn Leu Tyr
    3305                3310                3315

Gly Pro Thr Glu Cys Thr Ile Asn Ser Ser Phe Phe Arg Val Thr
    3320                3325                3330

Asn Glu Thr Leu Pro Asn Tyr Gln Thr Ser Ile Ser Ile Gly Ala
    3335                3340                3345

Pro Val Asp Asn Thr Glu Tyr Tyr Val Leu Asp Asp Asp Arg Leu
    3350                3355                3360

Pro Val Ala Val Gly Glu Ile Gly Glu Leu Tyr Ile Ser Gly Ala
    3365                3370                3375

Gln Leu Ala Arg Gly Tyr Leu His Lys Pro Glu Met Thr Lys Asp
    3380                3385                3390

Lys Phe Ile Cys Asn His Leu Val Ser Gly Thr Gln His Gln Trp
    3395                3400                3405

Leu Tyr Arg Thr Gly Asp Leu Val Thr Arg Gly Ala Asp Gly Asn
    3410                3415                3420

Thr Tyr Phe Val Gly Arg Val Asp Ser Gln Val Lys Leu Arg Gly
    3425                3430                3435

Tyr Arg Ile Glu Leu Asp Glu Ile Arg His Ala Ile Glu Glu His
    3440                3445                3450

Ser Trp Ile Lys Thr Ala Ala Met Leu Ile Lys Lys Asp Ala Arg
    3455                3460                3465

Thr Gly Phe Gln Asn Leu Ile Ala Cys Val Glu Leu Asp Glu Lys
    3470                3475                3480

Glu Ala Ala Leu Met Asp Gln Gly Asn Ser Ser Ser His His Lys
    3485                3490                3495

Ser Lys Ala Asp Lys Leu Gln Val Lys Ala Gln Leu Ser Asn Ser
    3500                3505                3510

Gly Cys Arg Ser Glu Glu Leu Cys Glu Asn Arg Pro Thr Phe Leu
    3515                3520                3525

Leu Pro Tyr Gln Glu Gly Glu Ile Lys Gln Arg Glu Tyr Ala Phe
    3530                3535                3540

Gly Arg Lys Thr Tyr Arg Tyr Phe Glu Gly Thr Glu Ile Thr Val
    3545                3550                3555

Glu Lys Leu Lys Lys Leu Leu Thr Ala Thr Gln Ser Asn Glu Ile
    3560                3565                3570

Ser Ser Leu Pro Leu Ser His Leu Thr Leu Asn Asp Phe Gly Tyr
    3575                3580                3585

Ala Leu Arg Tyr Phe Gly Gln Phe Thr Ser His Gln Arg Leu Leu
    3590                3595                3600

Pro Lys Tyr Ala Tyr Ala Ser Pro Gly Ala Leu Tyr Ala Thr Gln
    3605                3610                3615

Met Tyr Phe Glu Leu His Asn Val Leu Gly Leu Asp Ala Gly Ile
    3620                3625                3630

Tyr Tyr Tyr His Pro Val Thr His Lys Leu Ile Lys Ile Ser Thr
    3635                3640                3645

Leu Ser Arg Arg Gln Met Pro Thr Ile Lys Val His Phe Ile Gly
    3650                3655                3660

Lys His Glu Ala Ile Glu Pro Val Tyr Lys Asn Asn Ile Gln Glu
    3665                3670                3675

Val Leu Glu Met Glu Ala Gly His Met Met Gly Leu Phe Asp Asp
    3680                3685                3690

Val Leu Pro Glu Ile Gly Leu Ser Ile Gly Lys Ser Glu Tyr Gln
    3695                3700                3705

Asp Glu Cys Pro Asp Trp Tyr Asp Gly Asp Ile Gln Asp Tyr Tyr
    3710                3715                3720

Leu Gly Ala Phe Glu Ile Cys Ser Tyr Glu His Gly Leu Pro Pro
    3725                3730                3735

Phe Glu Thr Asp Ile Tyr Leu Gln Thr His Ala His Lys Ile Pro
    3740                3745                3750

Glu Met Pro Cys Gly Leu Tyr His Phe Ser Asn Gly Glu Phe Val
    3755                3760                3765

Arg Ile Ser Asp Asp Ile Val Arg Lys Lys Asp Val Ile Ala Ile
    3770                3775                3780

Asn Gln Gln Val Tyr Asp Arg Ser Ser Phe Gly Val Ser Ile Ile
    3785                3790                3795

Pro Arg Cys Val Pro Glu Trp His Tyr Tyr Ile Thr Leu Gly Arg
    3800                3805                3810

Arg Leu His Ala Leu Gln Ser Asn Pro Leu Tyr Ile Gly Leu Met
    3815                3820                3825

Ser Ser Gly Tyr Ser Ser Lys Ser Asn Asn Asp Leu Pro Ser Ala
    3830                3835                3840

Lys Arg Met Arg Ser Ile Leu Asn Ala Leu Asp Arg Pro Met Ala
    3845                3850                3855

Ala Phe Tyr Phe Cys Ile Gly Gly Gly Ile Ser Gln Ala Gln Tyr

3860                3865                3870

Met Cys Glu Gly Met Lys Glu Asp Val Val His Met Lys Gly Pro
    3875                3880                3885

Val Glu Ile Ile Lys Asp Asp Leu Gln Gln Gln Leu Pro Gln Tyr
    3890                3895                3900

Met Ile Pro Asn Lys Val Leu Val Phe Asp Lys Leu Pro Leu Thr
    3905                3910                3915

Ala Asn Gly Lys Val Asp Tyr Gln Ser Leu Ser Glu Ser Lys Ala
    3920                3925                3930

Val Glu Asn Val Ser Thr Gln Arg Leu Leu Val Pro Leu His Thr
    3935                3940                3945

Asp Thr Glu Ile Arg Leu Gly Lys Ile Trp Met Glu Val Leu Lys
    3950                3955                3960

Trp Asp Ser Val Ser Ala Leu Asp Asp Phe Phe Glu Ser Gly Gly
    3965                3970                3975

Asn Ser Leu Met Ala Val Ala Met Val Asn Lys Ile Asn Ala Ala
    3980                3985                3990

Phe Asn Ile Arg Phe Pro Leu Gln Ile Leu Phe Gln Ser Pro Asn
    3995                4000                4005

Ile Ala Glu Leu Ala Lys Trp Ile Glu Gln Thr Asp Ser Lys Thr
    4010                4015                4020

Ile Ser Arg Leu Ile Leu Leu Asn Gln Ala Ser Lys Asp Pro Ile
    4025                4030                4035

Tyr Cys Trp Pro Gly Leu Gly Gly Tyr Pro Met Ser Leu Arg Leu
    4040                4045                4050

Leu Ala Asn Lys Val Val Pro Asp Arg Ala Phe Tyr Gly Ile Gln
    4055                4060                4065

Ala Tyr Gly Ile Asn Glu Ser Glu Ile Pro Phe Ser Ser Ile Gln
    4070                4075                4080

Arg Met Ala Glu Glu Asp Ile Lys Glu Ile Lys Lys Ile Gln Pro
    4085                4090                4095

Glu Gly Pro Tyr Ile Leu Trp Gly Tyr Ser Phe Gly Ala Arg Val
    4100                4105                4110

Ala Phe Glu Val Ala Tyr Gln Leu Glu Gln Ala Gly Glu Glu Val
    4115                4120                4125

Asn Ala Leu Asn Leu Leu Ala Pro Gly Ser Pro His Leu Asp Met
    4130                4135                4140

Lys Gln Ala Glu Tyr Met Asp Lys Gly Ala Glu Phe Thr Asn Pro
    4145                4150                4155

Ala Phe Val Lys Ile Leu Phe Ser Val Phe Ser Arg Ser Ile Asn
    4160                4165                4170

Ser Pro Met Val Lys Thr Cys Leu Glu Gln Val Asn Ser Glu Thr
    4175                4180                4185

Thr Phe Ile Asn Phe Ile Cys Ser Arg Phe Lys Asn Leu Glu Pro
    4190                4195                4200

Ser Leu Val Lys Arg Ile Val Arg Ile Val Thr Leu Thr Tyr Asp
    4205                4210                4215

Phe Lys Tyr Ser Ile Asp Glu Leu Tyr His Arg His Leu Lys Ala
    4220                4225                4230

Pro Ile Thr Ile Phe Lys Ala Asn Arg Asp Asn Asp Ser Phe Ile
    4235                4240                4245

Glu Glu Ser Asp Val Ile Ser Ser Met Ser Pro Lys Ile Ile Glu
    4250                4255                4260

Leu Ile Ser Asp His Tyr Gln Leu Leu Glu Ser Glu Gly Val Ala
    4265                4270                4275

Glu Ile Glu Lys Ile Ile
    4280

<210>  35
<211>  2168
<212>  PRT
<213>  Artificial Sequence

<220>
<223>  NRPS being a synthetase of a fusion peptide consisting of Valine
       and Indigoidine. Due to its sterical advantages, Valine may be
       used as a spacer between the indigoidine pigment and the NRPS
       oligopeptide of interest to be tagged with the pigment.

<400>  35

Met Tyr Pro Arg Asp Leu Thr Ile Gln Glu Leu Phe Glu Gln Gln Ala
1               5                   10                  15

Ala Lys Thr Pro Glu His Ala Ala Val Val Met Asp Gly Gln Met Leu
            20                  25                  30

Thr Tyr Arg Glu Leu Asn Glu Lys Ala Asn Gln Leu Ala His Val Leu
        35                  40                  45

Arg Gln Asn Gly Val Gly Lys Glu Ser Ile Val Gly Leu Leu Ala Asp
    50                  55                  60

Arg Ser Leu Glu Met Ile Thr Gly Ile Met Gly Ile Leu Lys Ala Gly
65                  70                  75                  80

Gly Ala Tyr Leu Gly Leu Asp Pro Glu His Pro Ser Glu Arg Leu Ala
                85                  90                  95

Tyr Met Leu Glu Asp Gly Gly Val Lys Val Val Leu Val Gln Lys His
            100                 105                 110

Leu Leu Pro Leu Val Gly Glu Gly Leu Met Pro Ile Val Leu Glu Glu
        115                 120                 125

Glu Ser Leu Arg Pro Glu Asp Cys Gly Asn Pro Ala Ile Val Asn Gly
    130                 135                 140

Ala Ser Asp Leu Ala Tyr Val Met Tyr Thr Ser Gly Ser Thr Gly Lys
145                 150                 155                 160

Pro Lys Gly Val Met Val Glu His Arg Asn Val Thr Arg Leu Val Met
                165                 170                 175

His Thr Asn Tyr Val Gln Val Arg Glu Ser Asp Arg Met Ile Gln Thr
            180                 185                 190

Gly Ala Ile Gly Phe Asp Ala Met Thr Phe Glu Ile Phe Gly Ala Leu
        195                 200                 205

Leu His Gly Ala Ser Leu Tyr Leu Val Ser Lys Asp Val Leu Leu Asp
    210                 215                 220

Ala Glu Lys Leu Gly Asp Phe Leu Arg Thr Asn Gln Ile Thr Thr Met
225                 230                 235                 240

Trp Leu Thr Ser Pro Leu Phe Asn Gln Leu Ser Gln Asp Asn Pro Ala

245                 250                 255

Met Phe Asp Ser Leu Arg Ala Leu Ile Val Gly Gly Glu Ala Leu Ser
        260                 265                 270

Pro Lys His Ile Asn Arg Val Lys Ser Ala Leu Pro Asp Leu Glu Ile
    275                 280                 285

Trp Asn Gly Tyr Gly Pro Thr Glu Asn Thr Thr Phe Ser Thr Cys Tyr
    290                 295                 300

Leu Ile Glu Gln His Phe Glu Glu Gln Ile Pro Ile Gly Lys Pro Ile
305                 310                 315                 320

Ala Asn Ser Thr Ala Tyr Ile Val Asp Gly Asn Asn Gln Pro Gln Pro
                325                 330                 335

Ile Gly Val Pro Gly Glu Leu Cys Val Gly Gly Asp Gly Val Ala Arg
            340                 345                 350

Gly Tyr Val Asn Lys Pro Glu Leu Thr Ala Glu Lys Phe Val Pro Asn
        355                 360                 365

Pro Phe Ala Pro Gly Glu Thr Met Tyr Arg Thr Gly Asp Leu Ala Arg
    370                 375                 380

Trp Leu Pro Asp Gly Thr Ile Glu Tyr Leu Gly Arg Ile Asp Gln Gln
385                 390                 395                 400

Val Lys Ile Arg Gly Tyr Arg Ile Glu Leu Gly Glu Ile Glu Thr Val
                405                 410                 415

Leu Ser Gln Gln Ala Gln Val Lys Glu Ala Val Val Ala Val Ile Glu
            420                 425                 430

Glu Ala Asn Gly Gln Lys Ala Leu Cys Ala Tyr Phe Val Pro Glu Gln
        435                 440                 445

Ala Val Asp Ala Ala Glu Leu Arg Glu Ala Met Ser Lys Gln Leu Pro
    450                 455                 460

Gly Tyr Met Val Pro Ala Tyr Tyr Val Gln Met Glu Lys Leu Pro Leu
465                 470                 475                 480

Thr Ala Asn Gly Lys Val Asp Arg Arg Ala Leu Pro Gln Pro Ser Gly
                485                 490                 495

Glu Arg Thr Thr Gly Ser Ala Phe Val Ala Ala Gln Asn Asp Thr Glu
        500                 505                 510

Ala Lys Leu Gln Gln Ile Trp Gln Glu Val Leu Gly Ile Pro Ala Ile
    515                 520                 525

Gly Ile His Asp Asn Phe Phe Glu Ile Gly Gly His Ser Leu Lys Ala
530                 535                 540

Met Asn Val Ile Thr Gln Val His Lys Thr Phe Gln Val Glu Leu Pro
545                 550                 555                 560

Leu Lys Ala Leu Phe Ala Thr Pro Thr Ile His Glu Leu Ala Ala His
                565                 570                 575

Ile Ser Glu Lys Thr Glu Tyr Thr Ala Ile Gln Pro Val Ala Ala Gln
            580                 585                 590

Glu Phe Tyr Pro Val Ser Ser Ala Gln Lys Arg Met Tyr Ile Leu Gln
        595                 600                 605

Gln Phe Glu Gly Asn Gly Ile Ser Tyr Asn Ile Ser Gly Ala Ile Leu
    610                 615                 620

Leu Glu Gly Lys Leu Asp Tyr Ala Arg Phe Ala Ser Ala Val Gln Gln
625                 630                 635                 640

Leu Ala Glu Arg His Glu Ala Leu Arg Thr Ser Phe His Arg Ile Asp
                645                 650                 655

Gly Glu Pro Val Gln Lys Val His Glu Glu Val Glu Val Pro Leu Phe
            660                 665                 670

Met Leu Glu Ala Pro Glu Asp Gln Ala Glu Lys Ile Met Arg Glu Phe
        675                 680                 685

Val Arg Pro Phe Asp Leu Gly Val Ala Pro Leu Met Arg Thr Gly Leu
    690                 695                 700

Leu Lys Leu Gly Lys Asp Arg His Leu Phe Leu Leu Asp Met His His
705                 710                 715                 720

Ile Ile Ser Asp Gly Val Ser Ser Gln Ile Leu Leu Arg Glu Phe Ala
                725                 730                 735

Glu Leu Tyr Gln Gly Ala Asp Leu Gln Pro Leu Ser Leu Gln Tyr Lys
            740                 745                 750

Asp Phe Ala Ala Trp Gln Asn Glu Leu Phe Gln Thr Glu Ala Tyr Lys
        755                 760                 765

Lys Gln Glu Gln His Trp Leu Asn Thr Phe Ala Asp Glu Ile Pro Leu
    770                 775                 780

Leu Asn Leu Pro Thr Asp Tyr Pro Arg Pro Ser Val Gln Ser Phe Ala
785                 790                 795                 800

Gly Asp Leu Val Leu Phe Ala Ala Gly Lys Glu Leu Leu Glu Arg Leu
                805                 810                 815

Gln Gln Val Ala Ser Glu Thr Gly Thr Thr Leu Tyr Met Ile Leu Leu
            820                 825                 830

Ala Ala Tyr Asn Val Leu Leu Ser Lys Tyr Thr Gly Gln Glu Asp Ile
        835                 840                 845

Ile Val Gly Thr Pro Val Ala Gly Arg Ser His Ala Asp Val Glu Asn
    850                 855                 860

Ile Met Gly Ile Phe Val Asn Thr Leu Ala Leu Arg Asn Gln Pro Ala
865                 870                 875                 880

Ser Ser Lys Thr Met Leu Glu Asn Asn Ile Thr Gln Cys Asp Ser Ile
                885                 890                 895

Asn Asp Val Tyr Leu Lys Glu Glu Ala Ile Thr Leu Met Asp Met Leu
            900                 905                 910

Glu Ser Gln Leu Lys His Gln Ala Asp Gly Tyr Val Val Ile Asp Gln
        915                 920                 925

Glu Glu Ser Leu Ser Tyr Ala Asp Phe Tyr Leu Arg Val Lys Glu Ile
    930                 935                 940

Gly Tyr Cys Leu Ser Glu Ile Ser Ser Lys Ser Ser Val Gly Ile Gly
945                 950                 955                 960

Leu Phe Cys Asp Pro Ser Ile Asp Leu Ile Cys Gly Ala Trp Gly Ile
                965                 970                 975

Leu Ser Ala Asp Lys Ala Tyr Leu Pro Leu Ser Pro Asp Tyr Pro Thr
            980                 985                 990

Glu Arg Leu Lys Tyr Met Ile Glu  Asp Ser Gly Ile Asp  Val Ile Phe
        995                 1000                1005

Thr Gln Ser His Leu Lys Ala Gln Leu Gln Asp Ile Ala Pro Lys
    1010                1015                1020

Ser Val Leu Ile Met Thr Pro Glu Asp Val Ala Leu Thr Ile Lys
    1025                1030                1035

Thr Arg Thr Ile Glu Asp Ile Leu Gly Thr Val Gln Val Pro Lys
    1040                1045                1050

Pro Thr Ser Leu Ala Tyr Ile Ile Tyr Thr Ser Gly Ser Thr Gly
    1055                1060                1065

Lys Pro Lys Gly Val Met Ile Glu His His Ser Ile Val Asn Gln
    1070                1075                1080

Met Arg Phe Leu Ala Lys Ala Phe Lys Leu Gly Cys His Ser Arg
    1085                1090                1095

Ile Leu Gln Lys Thr Pro Met Ser Phe Asp Ala Ala Gln Trp Glu
    1100                1105                1110

Ile Leu Ala Pro Ala Ile Gly Gly Gln Val Ile Met Gly Pro Leu
    1115                1120                1125

Gly Cys Tyr Arg Asp Pro Asp Ala Ile Ile Lys Thr Ile Leu Gln
    1130                1135                1140

His Gln Val Thr Thr Leu Gln Cys Val Pro Thr Leu Leu Gln Ala
    1145                1150                1155

Leu Leu Asp Asn Pro Asn Phe Leu Asp Cys Leu Ser Leu Thr Gln
    1160                1165                1170

Val Phe Ser Gly Gly Glu Ala Leu Thr Thr Lys Leu Ala Thr Gln
    1175                1180                1185

Phe Leu Asn Ser Phe Thr His Cys Glu Leu Ile Asn Leu Tyr Gly
    1190                1195                1200

Pro Thr Glu Cys Thr Ile Asn Ser Ser Phe Phe Arg Val Thr Asn
    1205                1210                1215

Glu Thr Leu Pro Asn Tyr Gln Thr Ser Ile Ser Ile Gly Ala Pro
    1220                1225                1230

Val Asp Asn Thr Glu Tyr Tyr Val Leu Asp Asp Asp Arg Leu Pro

1235                1240                1245

Val Ala Val Gly Glu Ile Gly Glu Leu Tyr Ile Ser Gly Ala Gln
    1250                1255                1260

Leu Ala Arg Gly Tyr Leu His Lys Pro Glu Met Thr Lys Asp Lys
    1265                1270                1275

Phe Ile Cys Asn His Leu Val Ser Gly Thr Gln His Gln Trp Leu
    1280                1285                1290

Tyr Arg Thr Gly Asp Leu Val Thr Arg Gly Ala Asp Gly Asn Thr
    1295                1300                1305

Tyr Phe Val Gly Arg Val Asp Ser Gln Val Lys Leu Arg Gly Tyr
    1310                1315                1320

Arg Ile Glu Leu Asp Glu Ile Arg His Ala Ile Glu Glu His Ser
    1325                1330                1335

Trp Ile Lys Thr Ala Ala Met Leu Ile Lys Lys Asp Ala Arg Thr
    1340                1345                1350

Gly Phe Gln Asn Leu Ile Ala Cys Val Glu Leu Asp Glu Lys Glu
    1355                1360                1365

Ala Ala Leu Met Asp Gln Gly Asn Ser Ser Ser His His Lys Ser
    1370                1375                1380

Lys Ala Asp Lys Leu Gln Val Lys Ala Gln Leu Ser Asn Ser Gly
    1385                1390                1395

Cys Arg Ser Glu Glu Leu Cys Glu Asn Arg Pro Thr Phe Leu Leu
    1400                1405                1410

Pro Tyr Gln Glu Gly Glu Ile Lys Gln Arg Glu Tyr Ala Phe Gly
    1415                1420                1425

Arg Lys Thr Tyr Arg Tyr Phe Glu Gly Thr Glu Ile Thr Val Glu
    1430                1435                1440

Lys Leu Lys Lys Leu Leu Thr Ala Thr Gln Ser Asn Glu Ile Ser
    1445                1450                1455

Ser Leu Pro Leu Ser His Leu Thr Leu Asn Asp Phe Gly Tyr Ala
    1460                1465                1470

Leu Arg Tyr Phe Gly Gln Phe Thr Ser His Gln Arg Leu Leu Pro
    1475                1480                1485

Lys Tyr Ala Tyr Ala Ser Pro Gly Ala Leu Tyr Ala Thr Gln Met
    1490                1495                1500

Tyr Phe Glu Leu His Asn Val Leu Gly Leu Asp Ala Gly Ile Tyr
    1505                1510                1515

Tyr Tyr His Pro Val Thr His Lys Leu Ile Lys Ile Ser Thr Leu
    1520                1525                1530

Ser Arg Arg Gln Met Pro Thr Ile Lys Val His Phe Ile Gly Lys
    1535                1540                1545

His Glu Ala Ile Glu Pro Val Tyr Lys Asn Asn Ile Gln Glu Val
    1550                1555                1560

Leu Glu Met Glu Ala Gly His Met Met Gly Leu Phe Asp Asp Val
    1565                1570                1575

Leu Pro Glu Ile Gly Leu Ser Ile Gly Lys Ser Glu Tyr Gln Asp
    1580                1585                1590

Glu Cys Pro Asp Trp Tyr Asp Gly Asp Ile Gln Asp Tyr Tyr Leu
    1595                1600                1605

Gly Ala Phe Glu Ile Cys Ser Tyr Glu His Gly Leu Pro Pro Phe
    1610                1615                1620

Glu Thr Asp Ile Tyr Leu Gln Thr His Ala His Lys Ile Pro Glu
    1625                1630                1635

Met Pro Cys Gly Leu Tyr His Phe Ser Asn Gly Glu Phe Val Arg
    1640                1645                1650

Ile Ser Asp Asp Ile Val Arg Lys Lys Asp Val Ile Ala Ile Asn
    1655                1660                1665

Gln Gln Val Tyr Asp Arg Ser Ser Phe Gly Val Ser Ile Ile Pro
    1670                1675                1680

Arg Cys Val Pro Glu Trp His Tyr Tyr Ile Thr Leu Gly Arg Arg
    1685                1690                1695

Leu His Ala Leu Gln Ser Asn Pro Leu Tyr Ile Gly Leu Met Ser
    1700                1705                1710

Ser Gly Tyr Ser Ser Lys Ser Asn Asn Asp Leu Pro Ser Ala Lys
    1715                1720                1725

Arg Met Arg Ser Ile Leu Asn Ala Leu Asp Arg Pro Met Ala Ala
    1730                1735                1740

Phe Tyr Phe Cys Ile Gly Gly Gly Ile Ser Gln Ala Gln Tyr Met
    1745                1750                1755

Cys Glu Gly Met Lys Glu Asp Val Val His Met Lys Gly Pro Val
    1760                1765                1770

Glu Ile Ile Lys Asp Asp Leu Gln Gln Gln Leu Pro Gln Tyr Met
    1775                1780                1785

Ile Pro Asn Lys Val Leu Val Phe Asp Lys Leu Pro Leu Thr Ala
    1790                1795                1800

Asn Gly Lys Val Asp Tyr Gln Ser Leu Ser Glu Ser Lys Ala Val
    1805                1810                1815

Glu Asn Val Ser Thr Gln Arg Leu Leu Val Pro Leu His Thr Asp
    1820                1825                1830

Thr Glu Ile Arg Leu Gly Lys Ile Trp Met Glu Val Leu Lys Trp
    1835                1840                1845

Asp Ser Val Ser Ala Leu Asp Asp Phe Phe Glu Ser Gly Gly Asn
    1850                1855                1860

Ser Leu Met Ala Val Ala Met Val Asn Lys Ile Asn Ala Ala Phe
    1865                1870                1875

Asn Ile Arg Phe Pro Leu Gln Ile Leu Phe Gln Ser Pro Asn Ile
    1880                1885                1890

Ala Glu Leu Ala Lys Trp Ile Glu Gln Thr Asp Ser Lys Thr Ile
    1895                1900                1905

Ser Arg Leu Ile Leu Leu Asn Gln Ala Ser Lys Asp Pro Ile Tyr
    1910                1915                1920

Cys Trp Pro Gly Leu Gly Gly Tyr Pro Met Ser Leu Arg Leu Leu
    1925                1930                1935

Ala Asn Lys Val Val Pro Asp Arg Ala Phe Tyr Gly Ile Gln Ala
    1940                1945                1950

Tyr Gly Ile Asn Glu Ser Glu Ile Pro Phe Ser Ser Ile Gln Arg
    1955                1960                1965

Met Ala Glu Glu Asp Ile Lys Glu Ile Lys Lys Ile Gln Pro Glu
    1970                1975                1980

Gly Pro Tyr Ile Leu Trp Gly Tyr Ser Phe Gly Ala Arg Val Ala
    1985                1990                1995

Phe Glu Val Ala Tyr Gln Leu Glu Gln Ala Gly Glu Glu Val Asn
    2000                2005                2010

Ala Leu Asn Leu Leu Ala Pro Gly Ser Pro His Leu Asp Met Lys
    2015                2020                2025

Gln Ala Glu Tyr Met Asp Lys Gly Ala Glu Phe Thr Asn Pro Ala
    2030                2035                2040

Phe Val Lys Ile Leu Phe Ser Val Phe Ser Arg Ser Ile Asn Ser
    2045                2050                2055

Pro Met Val Lys Thr Cys Leu Glu Gln Val Asn Ser Glu Thr Thr
    2060                2065                2070

Phe Ile Asn Phe Ile Cys Ser Arg Phe Lys Asn Leu Glu Pro Ser
    2075                2080                2085

Leu Val Lys Arg Ile Val Arg Ile Val Thr Leu Thr Tyr Asp Phe
    2090                2095                2100

Lys Tyr Ser Ile Asp Glu Leu Tyr His Arg His Leu Lys Ala Pro
    2105                2110                2115

Ile Thr Ile Phe Lys Ala Asn Arg Asp Asn Asp Ser Phe Ile Glu
    2120                2125                2130

Glu Ser Asp Val Ile Ser Ser Met Ser Pro Lys Ile Ile Glu Leu
    2135                2140                2145

Ile Ser Asp His Tyr Gln Leu Leu Glu Ser Glu Gly Val Ala Glu
    2150                2155                2160

Ile Glu Lys Ile Ile
    2165

<210>  36

<211>  3202
<212>  PRT
<213>  Artificial Sequence

<220>
<223>  NRPSase synthesizing a Indigoidine-tagged Dipeptide consisting of
       two Valine-monomers.

<400>  36

Met Tyr Pro Arg Asp Leu Thr Ile Gln Glu Leu Phe Glu Gln Gln Ala
1               5                   10                  15

Ala Lys Thr Pro Glu His Ala Ala Val Val Met Asp Gly Gln Met Leu
            20                  25                  30

Thr Tyr Arg Glu Leu Asn Glu Lys Ala Asn Gln Leu Ala His Val Leu
        35                  40                  45

Arg Gln Asn Gly Val Gly Lys Glu Ser Ile Val Gly Leu Leu Ala Asp
    50                  55                  60

Arg Ser Leu Glu Met Ile Thr Gly Ile Met Gly Ile Leu Lys Ala Gly
65                  70                  75                  80

Gly Ala Tyr Leu Gly Leu Asp Pro Glu His Pro Ser Glu Arg Leu Ala
                85                  90                  95

Tyr Met Leu Glu Asp Gly Gly Val Lys Val Val Leu Val Gln Lys His
            100                 105                 110

Leu Leu Pro Leu Val Gly Glu Gly Leu Met Pro Ile Val Leu Glu Glu
        115                 120                 125

Glu Ser Leu Arg Pro Glu Asp Cys Gly Asn Pro Ala Ile Val Asn Gly
    130                 135                 140

Ala Ser Asp Leu Ala Tyr Val Met Tyr Thr Ser Gly Ser Thr Gly Lys
145                 150                 155                 160

Pro Lys Gly Val Met Val Glu His Arg Asn Val Thr Arg Leu Val Met
                165                 170                 175

His Thr Asn Tyr Val Gln Val Arg Glu Ser Asp Arg Met Ile Gln Thr
            180                 185                 190

Gly Ala Ile Gly Phe Asp Ala Met Thr Phe Glu Ile Phe Gly Ala Leu
        195                 200                 205

Leu His Gly Ala Ser Leu Tyr Leu Val Ser Lys Asp Val Leu Leu Asp

210                 215                 220

Ala Glu Lys Leu Gly Asp Phe Leu Arg Thr Asn Gln Ile Thr Thr Met
225                 230                 235                 240

Trp Leu Thr Ser Pro Leu Phe Asn Gln Leu Ser Gln Asp Asn Pro Ala
                245                 250                 255

Met Phe Asp Ser Leu Arg Ala Leu Ile Val Gly Gly Glu Ala Leu Ser
            260                 265                 270

Pro Lys His Ile Asn Arg Val Lys Ser Ala Leu Pro Asp Leu Glu Ile
        275                 280                 285

Trp Asn Gly Tyr Gly Pro Thr Glu Asn Thr Thr Phe Ser Thr Cys Tyr
    290                 295                 300

Leu Ile Glu Gln His Phe Glu Glu Gln Ile Pro Ile Gly Lys Pro Ile
305                 310                 315                 320

Ala Asn Ser Thr Ala Tyr Ile Val Asp Gly Asn Asn Gln Pro Gln Pro
                325                 330                 335

Ile Gly Val Pro Gly Glu Leu Cys Val Gly Gly Asp Gly Val Ala Arg
            340                 345                 350

Gly Tyr Val Asn Lys Pro Glu Leu Thr Ala Glu Lys Phe Val Pro Asn
        355                 360                 365

Pro Phe Ala Pro Gly Glu Thr Met Tyr Arg Thr Gly Asp Leu Ala Arg
    370                 375                 380

Trp Leu Pro Asp Gly Thr Ile Glu Tyr Leu Gly Arg Ile Asp Gln Gln
385                 390                 395                 400

Val Lys Ile Arg Gly Tyr Arg Ile Glu Leu Gly Glu Ile Glu Thr Val
                405                 410                 415

Leu Ser Gln Gln Ala Gln Val Lys Glu Ala Val Val Ala Val Ile Glu
            420                 425                 430

Glu Ala Asn Gly Gln Lys Ala Leu Cys Ala Tyr Phe Val Pro Glu Gln
        435                 440                 445

Ala Val Asp Ala Ala Glu Leu Arg Glu Ala Met Ser Lys Gln Leu Pro
    450                 455                 460

Gly Tyr Met Val Pro Ala Tyr Tyr Val Gln Met Glu Lys Leu Pro Leu
465                 470                 475                 480

Thr Ala Asn Gly Lys Val Asp Arg Arg Ala Leu Pro Gln Pro Ser Gly
                485                 490                 495

Glu Arg Thr Thr Gly Ser Ala Phe Val Ala Ala Gln Asn Asp Thr Glu
            500                 505                 510

Ala Lys Leu Gln Gln Ile Trp Gln Glu Val Leu Gly Ile Pro Ala Ile
        515                 520                 525

Gly Ile His Asp Asn Phe Phe Glu Ile Gly Gly His Ser Leu Lys Ala
    530                 535                 540

Met Asn Val Ile Thr Gln Val His Lys Thr Phe Gln Val Glu Leu Pro
545                 550                 555                 560

Leu Lys Ala Leu Phe Ala Thr Pro Thr Ile His Glu Leu Ala Ala His
                565                 570                 575

Ile Ala Thr Ser Gly Lys Glu Thr Tyr Val Pro Ile Glu Pro Ala Pro
            580                 585                 590

Leu Gln Glu Tyr Tyr Pro Val Ser Ser Ala Gln Lys Arg Met Tyr Val
        595                 600                 605

Leu Arg Gln Phe Ala Asp Thr Gly Thr Val Tyr Asn Met Pro Ser Ala
    610                 615                 620

Leu Tyr Ile Glu Gly Asp Leu Asp Arg Lys Arg Phe Glu Ala Ala Ile
625                 630                 635                 640

His Gly Leu Val Glu Arg His Glu Ser Leu Arg Thr Ser Phe His Thr
                645                 650                 655

Val Asn Gly Glu Pro Val Gln Arg Val His Glu His Val Glu Leu Asn
            660                 665                 670

Val Gln Tyr Ala Glu Val Thr Glu Ala Gln Val Glu Pro Thr Val Glu
        675                 680                 685

Ser Phe Val Gln Ala Phe Asp Leu Thr Lys Ala Pro Leu Leu Arg Val
    690                 695                 700

Gly Leu Phe Lys Leu Ala Ala Lys Arg His Leu Phe Leu Leu Asp Met
705                 710                 715                 720

His His Ile Ile Ser Asp Gly Val Ser Ala Gly Ile Ile Met Glu Glu
                725                 730                 735

Phe Ser Lys Leu Tyr Arg Gly Glu Glu Leu Pro Ala Leu Ser Val His
            740                 745                 750

Tyr Lys Asp Phe Ala Val Trp Gln Ser Glu Leu Phe Gln Ser Asp Val
        755                 760                 765

Tyr Thr Glu His Glu Asn Tyr Trp Leu Asn Ala Phe Ser Gly Asp Ile
    770                 775                 780

Pro Val Leu Asn Leu Pro Ala Asp Phe Ser Arg Pro Leu Thr Gln Ser
785                 790                 795                 800

Phe Glu Gly Asp Cys Val Ser Phe Gln Ala Asp Lys Ala Leu Leu Asp
                805                 810                 815

Asp Leu His Lys Leu Ala Gln Glu Ser Gln Ser Thr Leu Phe Met Val
            820                 825                 830

Leu Leu Ala Ala Tyr Asn Val Leu Leu Ala Lys Tyr Ser Gly Gln Glu
        835                 840                 845

Asp Ile Val Val Gly Thr Pro Ile Ala Gly Arg Ser His Ala Asp Ile
    850                 855                 860

Glu Asn Val Leu Gly Met Phe Val Asn Thr Leu Ala Leu Arg Asn Tyr
865                 870                 875                 880

Pro Val Glu Thr Lys His Phe Gln Ala Phe Leu Glu Glu Val Lys Gln
                885                 890                 895

Asn Thr Leu Gln Ala Tyr Ala His Gln Asp Tyr Pro Phe Glu Ala Leu
            900                 905                 910

Val Glu Lys Leu Asp Ile Gln Arg Asp Leu Ser Arg Asn Pro Leu Phe
        915                 920                 925

Asp Thr Met Phe Ile Leu Gln Asn Leu Asp Gln Lys Ala Tyr Glu Leu
    930                 935                 940

Asp Gly Leu Lys Leu Glu Ala Tyr Pro Ala Gln Ala Gly Asn Ala Lys
945                 950                 955                 960

Phe Asp Leu Thr Leu Glu Ala His Glu Asp Glu Thr Gly Ile His Phe
                965                 970                 975

Ala Leu Val Tyr Ser Thr Lys Leu Phe Gln Arg Glu Ser Ile Glu Arg
            980                 985                 990

Met Ala Gly His Phe Leu Gln Val Leu Arg Gln Val Val Ala Asp Gln
        995                 1000                1005

Ala Thr Ala Leu Arg Glu Ile Ser Leu Leu Ser Glu Glu Glu Arg
    1010                1015                1020

Arg Ile Val Thr Val Asp Phe Asn Asn Thr Phe Ala Tyr Pro Arg
    1025                1030                1035

Asp Leu Thr Ile Gln Glu Leu Phe Glu Gln Gln Ala Ala Lys Thr
    1040                1045                1050

Pro Glu His Ala Ala Val Val Met Asp Gly Gln Met Leu Thr Tyr
    1055                1060                1065

Arg Glu Leu Asn Glu Lys Ala Asn Gln Leu Ala His Val Leu Arg
    1070                1075                1080

Gln Asn Gly Val Gly Lys Glu Ser Ile Val Gly Leu Leu Ala Asp
    1085                1090                1095

Arg Ser Leu Glu Met Ile Thr Gly Ile Met Gly Ile Leu Lys Ala
    1100                1105                1110

Gly Gly Ala Tyr Leu Gly Leu Asp Pro Glu His Pro Ser Glu Arg
    1115                1120                1125

Leu Ala Tyr Met Leu Glu Asp Gly Gly Val Lys Val Val Leu Val
    1130                1135                1140

Gln Lys His Leu Leu Pro Leu Val Gly Glu Gly Leu Met Pro Ile
    1145                1150                1155

Val Leu Glu Glu Glu Ser Leu Arg Pro Glu Asp Cys Gly Asn Pro
    1160                1165                1170

Ala Ile Val Asn Gly Ala Ser Asp Leu Ala Tyr Val Met Tyr Thr
    1175                1180                1185

Ser Gly Ser Thr Gly Lys Pro Lys Gly Val Met Val Glu His Arg
    1190                1195                1200

Asn Val Thr Arg Leu Val Met His Thr Asn Tyr Val Gln Val Arg

1205                 1210                 1215

Glu Ser Asp Arg Met Ile Gln Thr Gly Ala Ile Gly Phe Asp Ala
    1220                 1225                 1230

Met Thr Phe Glu Ile Phe Gly Ala Leu Leu His Gly Ala Ser Leu
    1235                 1240                 1245

Tyr Leu Val Ser Lys Asp Val Leu Leu Asp Ala Glu Lys Leu Gly
    1250                 1255                 1260

Asp Phe Leu Arg Thr Asn Gln Ile Thr Thr Met Trp Leu Thr Ser
    1265                 1270                 1275

Pro Leu Phe Asn Gln Leu Ser Gln Asp Asn Pro Ala Met Phe Asp
    1280                 1285                 1290

Ser Leu Arg Ala Leu Ile Val Gly Gly Glu Ala Leu Ser Pro Lys
    1295                 1300                 1305

His Ile Asn Arg Val Lys Ser Ala Leu Pro Asp Leu Glu Ile Trp
    1310                 1315                 1320

Asn Gly Tyr Gly Pro Thr Glu Asn Thr Thr Phe Ser Thr Cys Tyr
    1325                 1330                 1335

Leu Ile Glu Gln His Phe Glu Glu Gln Ile Pro Ile Gly Lys Pro
    1340                 1345                 1350

Ile Ala Asn Ser Thr Ala Tyr Ile Val Asp Gly Asn Asn Gln Pro
    1355                 1360                 1365

Gln Pro Ile Gly Val Pro Gly Glu Leu Cys Val Gly Gly Asp Gly
    1370                 1375                 1380

Val Ala Arg Gly Tyr Val Asn Lys Pro Glu Leu Thr Ala Glu Lys
    1385                 1390                 1395

Phe Val Pro Asn Pro Phe Ala Pro Gly Glu Thr Met Tyr Arg Thr
    1400                 1405                 1410

Gly Asp Leu Ala Arg Trp Leu Pro Asp Gly Thr Ile Glu Tyr Leu
    1415                 1420                 1425

Gly Arg Ile Asp Gln Gln Val Lys Ile Arg Gly Tyr Arg Ile Glu
    1430                 1435                 1440

Leu Gly Glu Ile Glu Thr Val Leu Ser Gln Gln Ala Gln Val Lys
    1445                1450                1455

Glu Ala Val Val Ala Val Ile Glu Glu Ala Asn Gly Gln Lys Ala
    1460                1465                1470

Leu Cys Ala Tyr Phe Val Pro Glu Gln Ala Val Asp Ala Ala Glu
    1475                1480                1485

Leu Arg Glu Ala Met Ser Lys Gln Leu Pro Gly Tyr Met Val Pro
    1490                1495                1500

Ala Tyr Tyr Val Gln Met Glu Lys Leu Pro Leu Thr Ala Asn Gly
    1505                1510                1515

Lys Val Asp Arg Arg Ala Leu Pro Gln Pro Ser Gly Glu Arg Thr
    1520                1525                1530

Thr Gly Ser Ala Phe Val Ala Ala Gln Asn Asp Thr Glu Ala Lys
    1535                1540                1545

Leu Gln Gln Ile Trp Gln Glu Val Leu Gly Ile Pro Ala Ile Gly
    1550                1555                1560

Ile His Asp Asn Phe Phe Glu Ile Gly Gly His Ser Leu Lys Ala
    1565                1570                1575

Met Asn Val Ile Thr Gln Val His Lys Thr Phe Gln Val Glu Leu
    1580                1585                1590

Pro Leu Lys Ala Leu Phe Ala Thr Pro Thr Ile His Glu Leu Ala
    1595                1600                1605

Ala His Ile Ser Glu Lys Thr Glu Tyr Thr Ala Ile Gln Pro Val
    1610                1615                1620

Ala Ala Gln Glu Phe Tyr Pro Val Ser Ser Ala Gln Lys Arg Met
    1625                1630                1635

Tyr Ile Leu Gln Gln Phe Glu Gly Asn Gly Ile Ser Tyr Asn Ile
    1640                1645                1650

Ser Gly Ala Ile Leu Leu Glu Gly Lys Leu Asp Tyr Ala Arg Phe
    1655                1660                1665

Ala Ser Ala Val Gln Gln Leu Ala Glu Arg His Glu Ala Leu Arg
    1670                1675                1680

Thr Ser Phe His Arg Ile Asp Gly Glu Pro Val Gln Lys Val His
    1685                1690                1695

Glu Glu Val Glu Val Pro Leu Phe Met Leu Glu Ala Pro Glu Asp
    1700                1705                1710

Gln Ala Glu Lys Ile Met Arg Glu Phe Val Arg Pro Phe Asp Leu
    1715                1720                1725

Gly Val Ala Pro Leu Met Arg Thr Gly Leu Leu Lys Leu Gly Lys
    1730                1735                1740

Asp Arg His Leu Phe Leu Leu Asp Met His His Ile Ile Ser Asp
    1745                1750                1755

Gly Val Ser Ser Gln Ile Leu Leu Arg Glu Phe Ala Glu Leu Tyr
    1760                1765                1770

Gln Gly Ala Asp Leu Gln Pro Leu Ser Leu Gln Tyr Lys Asp Phe
    1775                1780                1785

Ala Ala Trp Gln Asn Glu Leu Phe Gln Thr Glu Ala Tyr Lys Lys
    1790                1795                1800

Gln Glu Gln His Trp Leu Asn Thr Phe Ala Asp Glu Ile Pro Leu
    1805                1810                1815

Leu Asn Leu Pro Thr Asp Tyr Pro Arg Pro Ser Val Gln Ser Phe
    1820                1825                1830

Ala Gly Asp Leu Val Leu Phe Ala Ala Gly Lys Glu Leu Leu Glu
    1835                1840                1845

Arg Leu Gln Gln Val Ala Ser Glu Thr Gly Thr Thr Leu Tyr Met
    1850                1855                1860

Ile Leu Leu Ala Ala Tyr Asn Val Leu Leu Ser Lys Tyr Thr Gly
    1865                1870                1875

Gln Glu Asp Ile Ile Val Gly Thr Pro Val Ala Gly Arg Ser His
    1880                1885                1890

Ala Asp Val Glu Asn Ile Met Gly Ile Phe Val Asn Thr Leu Ala
    1895                1900                1905

Leu Arg Asn Gln Pro Ala Ser Ser Lys Thr Met Leu Glu Asn Asn
    1910                1915                1920

Ile Thr Gln Cys Asp Ser Ile Asn Asp Val Tyr Leu Lys Glu Glu
    1925                1930                1935

Ala Ile Thr Leu Met Asp Met Leu Glu Ser Gln Leu Lys His Gln
    1940                1945                1950

Ala Asp Gly Tyr Val Val Ile Asp Gln Glu Glu Ser Leu Ser Tyr
    1955                1960                1965

Ala Asp Phe Tyr Leu Arg Val Lys Glu Ile Gly Tyr Cys Leu Ser
    1970                1975                1980

Glu Ile Ser Ser Lys Asn Ser Val Gly Ile Gly Leu Phe Cys Asp
    1985                1990                1995

Pro Ser Ile Asp Leu Ile Cys Gly Ala Trp Gly Ile Leu Ser Ala
    2000                2005                2010

Asp Lys Ala Tyr Leu Pro Leu Ser Pro Asp Tyr Pro Thr Glu Arg
    2015                2020                2025

Leu Lys Tyr Met Ile Glu Asp Ser Gly Ile Asp Val Ile Phe Thr
    2030                2035                2040

Gln Ser His Leu Lys Ala Gln Leu Gln Asp Ile Ala Pro Lys Ser
    2045                2050                2055

Val Leu Ile Met Thr Pro Glu Asp Val Ala Leu Thr Ile Lys Thr
    2060                2065                2070

Arg Thr Ile Glu Asp Ile Leu Gly Thr Val Gln Val Pro Lys Pro
    2075                2080                2085

Thr Ser Leu Ala Tyr Ile Ile Tyr Thr Ser Gly Ser Thr Gly Lys
    2090                2095                2100

Pro Lys Gly Val Met Ile Glu His His Ser Ile Val Asn Gln Met
    2105                2110                2115

Arg Phe Leu Ala Lys Ala Phe Lys Leu Gly Cys His Ser Arg Ile
    2120                2125                2130

Leu Gln Lys Thr Pro Met Ser Phe Asp Ala Ala Gln Trp Glu Ile
    2135                2140                2145

Leu Ala Pro Ala Ile Gly Gly Gln Val Ile Met Gly Pro Leu Gly

2150                2155                2160

Cys Tyr Arg Asp Pro Asp Ala Ile Ile Lys Thr Ile Leu Gln His
    2165                2170                2175

Gln Val Thr Thr Leu Gln Cys Val Pro Thr Leu Leu Gln Ala Leu
    2180                2185                2190

Leu Asp Asn Pro Asn Phe Leu Asp Cys Leu Ser Leu Thr Gln Val
    2195                2200                2205

Phe Ser Gly Gly Glu Ala Leu Thr Thr Lys Leu Ala Thr Gln Phe
    2210                2215                2220

Leu Asn Ser Phe Thr His Cys Glu Leu Ile Asn Leu Tyr Gly Pro
    2225                2230                2235

Thr Glu Cys Thr Ile Asn Ser Ser Phe Phe Arg Val Thr Asn Glu
    2240                2245                2250

Thr Leu Pro Asn Tyr Gln Thr Ser Ile Ser Ile Gly Ala Pro Val
    2255                2260                2265

Asp Asn Thr Glu Tyr Tyr Val Leu Asp Asp Asp Arg Leu Pro Val
    2270                2275                2280

Ala Val Gly Glu Ile Gly Glu Leu Tyr Ile Ser Gly Ala Gln Leu
    2285                2290                2295

Ala Arg Gly Tyr Leu His Lys Pro Glu Met Thr Lys Asp Lys Phe
    2300                2305                2310

Ile Cys Asn His Leu Val Ser Gly Thr Gln His Gln Trp Leu Tyr
    2315                2320                2325

Arg Thr Gly Asp Leu Val Thr Arg Gly Ala Asp Gly Asn Thr Tyr
    2330                2335                2340

Phe Val Gly Arg Val Asp Ser Gln Val Lys Leu Arg Gly Tyr Arg
    2345                2350                2355

Ile Glu Leu Asp Glu Ile Arg His Ala Ile Glu Glu His Ser Trp
    2360                2365                2370

Ile Lys Thr Ala Ala Met Leu Ile Lys Lys Asp Ala Arg Thr Gly
    2375                2380                2385

Phe Gln Asn Leu Ile Ala Cys Val Glu Leu Asp Glu Lys Glu Ala
    2390                2395                2400

Ala Leu Met Asp Gln Gly Asn Ser Ser Ser His His Lys Ser Lys
    2405                2410                2415

Ala Asp Lys Leu Gln Val Lys Ala Gln Leu Ser Asn Ser Gly Cys
    2420                2425                2430

Arg Ser Glu Glu Leu Cys Glu Asn Arg Pro Thr Phe Leu Leu Pro
    2435                2440                2445

Tyr Gln Glu Gly Glu Ile Lys Gln Arg Glu Tyr Ala Phe Gly Arg
    2450                2455                2460

Lys Thr Tyr Arg Tyr Phe Glu Gly Thr Glu Ile Thr Val Glu Lys
    2465                2470                2475

Leu Lys Lys Leu Leu Thr Ala Thr Gln Ser Asn Glu Ile Ser Ser
    2480                2485                2490

Leu Pro Leu Ser His Leu Thr Leu Asn Asp Phe Gly Tyr Ala Leu
    2495                2500                2505

Arg Tyr Phe Gly Gln Phe Thr Ser His Gln Arg Leu Leu Pro Lys
    2510                2515                2520

Tyr Ala Tyr Ala Ser Pro Gly Ala Leu Tyr Ala Thr Gln Met Tyr
    2525                2530                2535

Phe Glu Leu His Asn Val Leu Gly Leu Asp Ala Gly Ile Tyr Tyr
    2540                2545                2550

Tyr His Pro Val Thr His Lys Leu Ile Lys Ile Ser Thr Leu Ser
    2555                2560                2565

Arg Arg Gln Met Pro Thr Ile Lys Val His Phe Ile Gly Lys His
    2570                2575                2580

Glu Ala Ile Glu Pro Val Tyr Lys Asn Asn Ile Gln Glu Val Leu
    2585                2590                2595

Glu Met Glu Ala Gly His Met Met Gly Leu Phe Asp Asp Val Leu
    2600                2605                2610

Pro Glu Ile Gly Leu Ser Ile Gly Lys Ser Glu Tyr Gln Asp Glu
    2615                2620                2625

Cys Pro Asp Trp Tyr Asp Gly Asp Ile Gln Asp Tyr Tyr Leu Gly
    2630                2635                2640

Ala Phe Glu Ile Cys Ser Tyr Glu His Gly Leu Pro Pro Phe Glu
    2645                2650                2655

Thr Asp Ile Tyr Leu Gln Thr His Ala His Lys Ile Pro Glu Met
    2660                2665                2670

Pro Cys Gly Leu Tyr His Phe Ser Asn Gly Glu Phe Val Arg Ile
    2675                2680                2685

Ser Asp Asp Ile Val Arg Lys Lys Asp Val Ile Ala Ile Asn Gln
    2690                2695                2700

Gln Val Tyr Asp Arg Ser Ser Phe Gly Val Ser Ile Ile Pro Arg
    2705                2710                2715

Cys Val Pro Glu Trp His Tyr Tyr Ile Thr Leu Gly Arg Arg Leu
    2720                2725                2730

His Ala Leu Gln Ser Asn Pro Leu Tyr Ile Gly Leu Met Ser Ser
    2735                2740                2745

Gly Tyr Ser Ser Lys Ser Asn Asn Asp Leu Pro Ser Ala Lys Arg
    2750                2755                2760

Met Arg Ser Ile Leu Asn Ala Leu Asp Arg Pro Met Ala Ala Phe
    2765                2770                2775

Tyr Phe Cys Ile Gly Gly Gly Ile Ser Gln Ala Gln Tyr Met Cys
    2780                2785                2790

Glu Gly Met Lys Glu Asp Val Val His Met Lys Gly Pro Val Glu
    2795                2800                2805

Ile Ile Lys Asp Asp Leu Gln Gln Gln Leu Pro Gln Tyr Met Ile
    2810                2815                2820

Pro Asn Lys Val Leu Val Phe Asp Lys Leu Pro Leu Thr Ala Asn
    2825                2830                2835

Gly Lys Val Asp Tyr Gln Ser Leu Ser Glu Ser Lys Ala Val Glu
    2840                2845                2850

Asn Val Ser Thr Gln Arg Leu Leu Val Pro Leu His Thr Asp Thr
    2855                2860                2865

Glu Ile Arg Leu Gly Lys Ile Trp Met Glu Val Leu Lys Trp Asp
    2870                2875                2880

Ser Val Ser Ala Leu Asp Asp Phe Phe Glu Ser Gly Gly Asn Ser
    2885                2890                2895

Leu Met Ala Val Ala Met Val Asn Lys Ile Asn Ala Ala Phe Asn
    2900                2905                2910

Ile Arg Phe Pro Leu Gln Ile Leu Phe Gln Ser Pro Asn Ile Ala
    2915                2920                2925

Glu Leu Ala Lys Trp Ile Glu Gln Thr Asp Ser Lys Thr Ile Ser
    2930                2935                2940

Arg Leu Ile Leu Leu Asn Gln Ala Ser Lys Asp Pro Ile Tyr Cys
    2945                2950                2955

Trp Pro Gly Leu Gly Gly Tyr Pro Met Ser Leu Arg Leu Leu Ala
    2960                2965                2970

Asn Lys Val Val Pro Asp Arg Ala Phe Tyr Gly Ile Gln Ala Tyr
    2975                2980                2985

Gly Ile Asn Glu Ser Glu Ile Pro Phe Ser Ser Ile Gln Arg Met
    2990                2995                3000

Ala Glu Glu Asp Ile Lys Glu Ile Lys Lys Ile Gln Pro Glu Gly
    3005                3010                3015

Pro Tyr Ile Leu Trp Gly Tyr Ser Phe Gly Ala Arg Val Ala Phe
    3020                3025                3030

Glu Val Ala Tyr Gln Leu Glu Gln Ala Gly Glu Glu Val Asn Ala
    3035                3040                3045

Leu Asn Leu Leu Ala Pro Gly Ser Pro His Leu Asp Met Lys Gln
    3050                3055                3060

Ala Glu Tyr Met Asp Lys Gly Ala Glu Phe Thr Asn Pro Ala Phe
    3065                3070                3075

Val Lys Ile Leu Phe Ser Val Phe Ser Arg Ser Ile Asn Ser Pro
    3080                3085                3090

Met Val Lys Thr Cys Leu Glu Gln Val Asn Ser Glu Thr Thr Phe

3095                    3100                    3105

Ile Asn  Phe Ile Cys Ser Arg  Phe Lys Asn Leu Glu  Pro Ser Leu
    3110                    3115                    3120

Val Lys  Arg Ile Val Arg Ile  Val Thr Leu Thr Tyr  Asp Phe Lys
    3125                    3130                    3135

Tyr Ser  Ile Asp Glu Leu Tyr  His Arg His Leu Lys  Ala Pro Ile
    3140                    3145                    3150

Thr Ile  Phe Lys Ala Asn Arg  Asp Asn Asp Ser Phe  Ile Glu Glu
    3155                    3160                    3165

Ser Asp  Val Ile Ser Ser Met  Ser Pro Lys Ile Ile  Glu Leu Ile
    3170                    3175                    3180

Ser Asp  His Tyr Gln Leu Leu  Glu Ser Glu Gly Val  Ala Glu Ile
    3185                    3190                    3195

Glu Lys  Ile Ile
    3200

<210>  37
<211>  1591
<212>  PRT
<213>  Artificial Sequence

<220>
<223>  minimal construct C(of TycC2)-Ind

<400>  37

Ser Glu Lys Thr Glu Tyr Thr Ala Ile Gln Pro Val Ala Ala Gln Glu
1               5                   10                  15

Phe Tyr Pro Val Ser Ser Ala Gln Lys Arg Met Tyr Ile Leu Gln Gln
            20                  25                  30

Phe Glu Gly Asn Gly Ile Ser Tyr Asn Ile Ser Gly Ala Ile Leu Leu
        35                  40                  45

Glu Gly Lys Leu Asp Tyr Ala Arg Phe Ala Ser Ala Val Gln Gln Leu
    50                  55                  60

Ala Glu Arg His Glu Ala Leu Arg Thr Ser Phe His Arg Ile Asp Gly
65                  70                  75                  80

Glu Pro Val Gln Lys Val His Glu Glu Val Glu Val Pro Leu Phe Met
                85                  90                  95

Leu Glu Ala Pro Glu Asp Gln Ala Glu Lys Ile Met Arg Glu Phe Val
        100                 105                 110

Arg Pro Phe Asp Leu Gly Val Ala Pro Leu Met Arg Thr Gly Leu Leu
    115                 120                 125

Lys Leu Gly Lys Asp Arg His Leu Phe Leu Leu Asp Met His His Ile
    130                 135                 140

Ile Ser Asp Gly Val Ser Ser Gln Ile Leu Leu Arg Glu Phe Ala Glu
145                 150                 155                 160

Leu Tyr Gln Gly Ala Asp Leu Gln Pro Leu Ser Leu Gln Tyr Lys Asp
                165                 170                 175

Phe Ala Ala Trp Gln Asn Glu Leu Phe Gln Thr Glu Ala Tyr Lys Lys
        180                 185                 190

Gln Glu Gln His Trp Leu Asn Thr Phe Ala Asp Glu Ile Pro Leu Leu
    195                 200                 205

Asn Leu Pro Thr Asp Tyr Pro Arg Pro Ser Val Gln Ser Phe Ala Gly
    210                 215                 220

Asp Leu Val Leu Phe Ala Ala Gly Lys Glu Leu Leu Glu Arg Leu Gln
225                 230                 235                 240

Gln Val Ala Ser Glu Thr Gly Thr Thr Leu Tyr Met Ile Leu Leu Ala
                245                 250                 255

Ala Tyr Asn Val Leu Leu Ser Lys Tyr Thr Gly Gln Glu Asp Ile Ile
        260                 265                 270

Val Gly Thr Pro Val Ala Gly Arg Ser His Ala Asp Val Glu Asn Ile
    275                 280                 285

Met Gly Ile Phe Val Asn Thr Leu Ala Leu Arg Asn Gln Pro Ala Ser
    290                 295                 300

Ser Lys Thr Met Leu Glu Asn Asn Ile Thr Gln Cys Asp Ser Ile Asn
305                 310                 315                 320

Asp Val Tyr Leu Lys Glu Glu Ala Ile Thr Leu Met Asp Met Leu Glu
                325                 330                 335

Ser Gln Leu Lys His Gln Ala Asp Gly Tyr Val Val Ile Asp Gln Glu

            340                 345                 350

Glu Ser Leu Ser Tyr Ala Asp Phe Tyr Leu Arg Val Lys Glu Ile Gly
        355                 360                 365

Tyr Cys Leu Ser Glu Ile Ser Ser Lys Ser Ser Val Gly Ile Gly Leu
    370                 375                 380

Phe Cys Asp Pro Ser Ile Asp Leu Ile Cys Gly Ala Trp Gly Ile Leu
385                 390                 395                 400

Ser Ala Asp Lys Ala Tyr Leu Pro Leu Ser Pro Asp Tyr Pro Thr Glu
                405                 410                 415

Arg Leu Lys Tyr Met Ile Glu Asp Ser Gly Ile Asp Val Ile Phe Thr
            420                 425                 430

Gln Ser His Leu Lys Ala Gln Leu Gln Asp Ile Ala Pro Lys Ser Val
        435                 440                 445

Leu Ile Met Thr Pro Glu Asp Val Ala Leu Thr Ile Lys Thr Arg Thr
    450                 455                 460

Ile Glu Asp Ile Leu Gly Thr Val Gln Val Pro Lys Pro Thr Ser Leu
465                 470                 475                 480

Ala Tyr Ile Ile Tyr Thr Ser Gly Ser Thr Gly Lys Pro Lys Gly Val
                485                 490                 495

Met Ile Glu His His Ser Ile Val Asn Gln Met Arg Phe Leu Ala Lys
            500                 505                 510

Ala Phe Lys Leu Gly Cys His Ser Arg Ile Leu Gln Lys Thr Pro Met
        515                 520                 525

Ser Phe Asp Ala Ala Gln Trp Glu Ile Leu Ala Pro Ala Ile Gly Gly
    530                 535                 540

Gln Val Ile Met Gly Pro Leu Gly Cys Tyr Arg Asp Pro Asp Ala Ile
545                 550                 555                 560

Ile Lys Thr Ile Leu Gln His Gln Val Thr Thr Leu Gln Cys Val Pro
                565                 570                 575

Thr Leu Leu Gln Ala Leu Leu Asp Asn Pro Asn Phe Leu Asp Cys Leu
            580                 585                 590

Ser Leu Thr Gln Val Phe Ser Gly Gly Glu Ala Leu Thr Thr Lys Leu
        595                 600                 605

Ala Thr Gln Phe Leu Asn Ser Phe Thr His Cys Glu Leu Ile Asn Leu
    610                 615                 620

Tyr Gly Pro Thr Glu Cys Thr Ile Asn Ser Ser Phe Phe Arg Val Thr
625                 630                 635                 640

Asn Glu Thr Leu Pro Asn Tyr Gln Thr Ser Ile Ser Ile Gly Ala Pro
                645                 650                 655

Val Asp Asn Thr Glu Tyr Tyr Val Leu Asp Asp Asp Arg Leu Pro Val
            660                 665                 670

Ala Val Gly Glu Ile Gly Glu Leu Tyr Ile Ser Gly Ala Gln Leu Ala
        675                 680                 685

Arg Gly Tyr Leu His Lys Pro Glu Met Thr Lys Asp Lys Phe Ile Cys
    690                 695                 700

Asn His Leu Val Ser Gly Thr Gln His Gln Trp Leu Tyr Arg Thr Gly
705                 710                 715                 720

Asp Leu Val Thr Arg Gly Ala Asp Gly Asn Thr Tyr Phe Val Gly Arg
                725                 730                 735

Val Asp Ser Gln Val Lys Leu Arg Gly Tyr Arg Ile Glu Leu Asp Glu
            740                 745                 750

Ile Arg His Ala Ile Glu Glu His Ser Trp Ile Lys Thr Ala Ala Met
        755                 760                 765

Leu Ile Lys Lys Asp Ala Arg Thr Gly Phe Gln Asn Leu Ile Ala Cys
    770                 775                 780

Val Glu Leu Asp Glu Lys Glu Ala Ala Leu Met Asp Gln Gly Asn Ser
785                 790                 795                 800

Ser Ser His His Lys Ser Lys Ala Asp Lys Leu Gln Val Lys Ala Gln
                805                 810                 815

Leu Ser Asn Ser Gly Cys Arg Ser Glu Glu Leu Cys Glu Asn Arg Pro
            820                 825                 830

Thr Phe Leu Leu Pro Tyr Gln Glu Gly Glu Ile Lys Gln Arg Glu Tyr
        835                 840                 845

Ala Phe Gly Arg Lys Thr Tyr Arg Tyr Phe Glu Gly Thr Glu Ile Thr
    850                 855                 860

Val Glu Lys Leu Lys Lys Leu Leu Thr Ala Thr Gln Ser Asn Glu Ile
865                 870                 875                 880

Ser Ser Leu Pro Leu Ser His Leu Thr Leu Asn Asp Phe Gly Tyr Ala
                885                 890                 895

Leu Arg Tyr Phe Gly Gln Phe Thr Ser His Gln Arg Leu Leu Pro Lys
            900                 905                 910

Tyr Ala Tyr Ala Ser Pro Gly Ala Leu Tyr Ala Thr Gln Met Tyr Phe
        915                 920                 925

Glu Leu His Asn Val Leu Gly Leu Asp Ala Gly Ile Tyr Tyr Tyr His
    930                 935                 940

Pro Val Thr His Lys Leu Ile Lys Ile Ser Thr Leu Ser Arg Arg Gln
945                 950                 955                 960

Met Pro Thr Ile Lys Val His Phe Ile Gly Lys His Glu Ala Ile Glu
                965                 970                 975

Pro Val Tyr Lys Asn Asn Ile Gln Glu Val Leu Glu Met Glu Ala Gly
            980                 985                 990

His Met Met Gly Leu Phe Asp Asp  Val Leu Pro Glu Ile  Gly Leu Ser
        995                 1000                1005

Ile Gly  Lys Ser Glu Tyr Gln  Asp Glu Cys Pro Asp  Trp Tyr Asp
    1010                1015                1020

Gly Asp  Ile Gln Asp Tyr Tyr  Leu Gly Ala Phe Glu  Ile Cys Ser
    1025                1030                1035

Tyr Glu  His Gly Leu Pro Pro  Phe Glu Thr Asp Ile  Tyr Leu Gln
    1040                1045                1050

Thr His  Ala His Lys Ile Pro  Glu Met Pro Cys Gly  Leu Tyr His
    1055                1060                1065

Phe Ser  Asn Gly Glu Phe Val  Arg Ile Ser Asp Asp  Ile Val Arg
    1070                1075                1080

Lys Lys  Asp Val Ile Ala Ile  Asn Gln Gln Val Tyr  Asp Arg Ser
    1085                1090                1095

Ser Phe Gly Val Ser Ile Ile Pro Arg Cys Val Pro Glu Trp His
    1100                1105                1110

Tyr Tyr Ile Thr Leu Gly Arg Arg Leu His Ala Leu Gln Ser Asn
    1115                1120                1125

Pro Leu Tyr Ile Gly Leu Met Ser Ser Gly Tyr Ser Ser Lys Ser
    1130                1135                1140

Asn Asn Asp Leu Pro Ser Ala Lys Arg Met Arg Ser Ile Leu Asn
    1145                1150                1155

Ala Leu Asp Arg Pro Met Ala Ala Phe Tyr Phe Cys Ile Gly Gly
    1160                1165                1170

Gly Ile Ser Gln Ala Gln Tyr Met Cys Glu Gly Met Lys Glu Asp
    1175                1180                1185

Val Val His Met Lys Gly Pro Val Glu Ile Ile Lys Asp Asp Leu
    1190                1195                1200

Gln Gln Gln Leu Pro Gln Tyr Met Ile Pro Asn Lys Val Leu Val
    1205                1210                1215

Phe Asp Lys Leu Pro Leu Thr Ala Asn Gly Lys Val Asp Tyr Gln
    1220                1225                1230

Ser Leu Ser Glu Ser Lys Ala Val Glu Asn Val Ser Thr Gln Arg
    1235                1240                1245

Leu Leu Val Pro Leu His Thr Asp Thr Glu Ile Arg Leu Gly Lys
    1250                1255                1260

Ile Trp Met Glu Val Leu Lys Trp Asp Ser Val Ser Ala Leu Asp
    1265                1270                1275

Asp Phe Phe Glu Ser Gly Gly Asn Ser Leu Met Ala Val Ala Met
    1280                1285                1290

Val Asn Lys Ile Asn Ala Ala Phe Asn Ile Arg Phe Pro Leu Gln
    1295                1300                1305

Ile Leu Phe Gln Ser Pro Asn Ile Ala Glu Leu Ala Lys Trp Ile
    1310                1315                1320

Glu Gln Thr Asp Ser Lys Thr Ile Ser Arg Leu Ile Leu Leu Asn

1325                1330                1335

Gln Ala Ser Lys Asp Pro Ile Tyr Cys Trp Pro Gly Leu Gly Gly
    1340                1345                1350

Tyr Pro Met Ser Leu Arg Leu Leu Ala Asn Lys Val Val Pro Asp
    1355                1360                1365

Arg Ala Phe Tyr Gly Ile Gln Ala Tyr Gly Ile Asn Glu Ser Glu
    1370                1375                1380

Ile Pro Phe Ser Ser Ile Gln Arg Met Ala Glu Glu Asp Ile Lys
    1385                1390                1395

Glu Ile Lys Lys Ile Gln Pro Glu Gly Pro Tyr Ile Leu Trp Gly
    1400                1405                1410

Tyr Ser Phe Gly Ala Arg Val Ala Phe Glu Val Ala Tyr Gln Leu
    1415                1420                1425

Glu Gln Ala Gly Glu Glu Val Asn Ala Leu Asn Leu Leu Ala Pro
    1430                1435                1440

Gly Ser Pro His Leu Asp Met Lys Gln Ala Glu Tyr Met Asp Lys
    1445                1450                1455

Gly Ala Glu Phe Thr Asn Pro Ala Phe Val Lys Ile Leu Phe Ser
    1460                1465                1470

Val Phe Ser Arg Ser Ile Asn Ser Pro Met Val Lys Thr Cys Leu
    1475                1480                1485

Glu Gln Val Asn Ser Glu Thr Thr Phe Ile Asn Phe Ile Cys Ser
    1490                1495                1500

Arg Phe Lys Asn Leu Glu Pro Ser Leu Val Lys Arg Ile Val Arg
    1505                1510                1515

Ile Val Thr Leu Thr Tyr Asp Phe Lys Tyr Ser Ile Asp Glu Leu
    1520                1525                1530

Tyr His Arg His Leu Lys Ala Pro Ile Thr Ile Phe Lys Ala Asn
    1535                1540                1545

Arg Asp Asn Asp Ser Phe Ile Glu Glu Ser Asp Val Ile Ser Ser
    1550                1555                1560

Met Ser Pro Lys Ile Ile Glu Leu Ile Ser Asp His Tyr Gln Leu
    1565                1570                1575

Leu Glu Ser Glu Gly Val Ala Glu Ile Glu Lys Ile Ile
    1580                1585                1590

## Description

The present invention relates to a polypeptide or polypeptide complex, comprising at least one non-ribosomal peptide synthesis (NRPS) amino acid module functionally connected to at least one pigment module. The present invention further relates to a labeled oligopeptide comprising a non-naturally attached NRPS pigment or/and polyketide pigment, to a polynucleotide encoding a fusion polypeptide, a vector, preferably an expression vector, comprising the polynucleotide of the present invention and to a host cell comprising the polypeptide or polypeptide complex and/or the polynucleotide, and/or the vector according to the present invention. Moreover, the present invention relates to in vitro and in vivo method of producing a labeled oligopeptide, as well as to methods of optimizing the same.

Non-ribosomal peptides (NRPs) are secondary metabolites produced by microorganisms, e.g. bacteria and fungi. Unlike ribosomal protein biosynthesis, non-ribosomal protein synthesis (NRPS) does not require mRNA to direct the sequence of monomers incorporated into the growing peptide chain. In NRPS, this sequence is controlled by the sequence of amino acid modules within the enzyme, the non-ribosomal peptide synthetase (NRPSase), wherein each module is specific for one amino acid, respectively.

Every NRPS module contains a Thiolation-domain (T-domain), also called PCP-domain (Peptidyl-carrier-protein-domain). In the synthesis of non-ribosomal peptides, growing peptide chains are handed from one module to the next one. A new amino acid is first adenylated by the A-domain and then bound to the T-domain via a thioester bond. The C-domain catalyzes the condensation of an existing peptide chain - which is bound to the T-domain of the previous module - and the amino acid of the next module. The T-domain itself does not exhibit any substrate specificity but is just a carrier domain to keep the peptide attached to the NRPS module complex. The core of every T-domain is a conserved 4'-phosphopanthetheinylated (4'-PPT) serine. The 4'-PPT residue is added by a 4'-Phosphopanthetheinyl-transferase (PPTase), which brings the NRPS apo-enzyme to its active holo-form.

NRPSases have been known to have a modular architecture, with single modules being specific for a specific amino acid as described above. The single modules can be connected covalently to form large multi-module-proteins. However, single modules can also associate via protein-protein interactions, mostly via so called communication domains. Both, in covalently connected modules as well as in non-covalently connected modules, it was shown that single modules can generally be exchanged for modules having different specificities, and still the NRPSase is functional. Also, it was found to be generally possible to exchange domains comprised in a module for another domain conferring the same functionality, emphasizing the modular architecture of NRPSases on several levels. These approaches have been used to create new NRPSases synthesizing novel peptides (Mootz et al (2000), PNAS 97(11):5848-53; Nguyen et al. (2006), PNAS 103(46):17462-7; Stachelhaus et al. (1998), J Biol Chem 273(35):22773-81; Finking & Marahiel (2004), Annu Rev Mibrobiol 58:453-88).

Indigoidine is an insoluble blue pigment probably formed by oxidation of two cyclic glutamines. There exist several enzymes catalyzing indigoidine synthesis, all of which are NRPSases and consist of only one single module with an A-Ox- (adenylation and oxidation), T- and a TE-domain.

Previous publications showed that exchanging the T-domain of the indigoidine synthetase bpsA from S. lavendulae with other T-domains results in a loss of function, i.e. the indigoidine synthetase loses its ability to produce the blue pigment (Owen et al. (2012), Environ Microbiol 14: 1198-1209). In the same study a method is described, in which the T-domain of the E. coli entF gene was inserted into bpsA and afterwards modified using a random mutagenesis strategy, until blue colonies were obtained. Studies of Marahiel and Doekel (Doekel & Marahiel (2000, Chem Biol 7(6):373-84) showed that it is possible to exchange the A-domains of NRPS modules to yield modified nonribosomal peptide products for some module combinations.

However, peptide production via NRPSases has been hampered by the fact that the peptides have to be purified from the reaction mixture in order to be able to evaluate the amount and the quality of the product. Thus, NRPS at present is amenable to high-throughput methods only to a very limited extent. Accordingly, there is a need in the art for improved methods of allowing detection of peptides produced by NRPS. This Problem is solved by the embodiments of the present invention described herein.

Accordingly, the present invention relates to a polypeptide or polypeptide complex, comprising at least one non-ribosomal peptide synthesis (NRPS) amino acid module functionally connected to at least one pigment module.

The term "oligopeptide", as used herein, relates to a chemical compound comprising at least one peptide bond. Preferably, the oligopeptide of the present invention comprises at least one alpha-amino acid involved in a peptide bond. Preferably, the oligopeptide is a non-ribosomal peptide synthesis (NRPS) oligopeptide, i.e. an oligopeptide synthesized by an NRPSase as described herein. Preferably, the oligopeptide comprises 2 to 25 amino acid units, more preferably 3 to 20 amino acid units, most preferably 5 to 15 amino acid units. It is understood by the skilled person that in NRPS, amino acids as well as derivatives thereof, including short peptides, and other chemical compounds may be added to the growing oligopeptide chain; moreover, said compounds integrated may subsequently be modified chemically, e.g. by oxidation, reduction, or isomerization, e.g. epimerization. Accordingly, the term amino acid unit, as used herein, relates to a subpart of the oligopeptide, which is incorporated in a condensation step of NRPS as described elsewhere in this specification. Preferably, the oligopeptide is a peptide, a natural NRPS oligopeptide or a derivative thereof; also preferably, the oligopeptide is a non-natural NRPS peptide generated by module-shuffling as described elsewhere herein, or a derivative thereof.

The term "non-ribosomal peptide synthesis" or "NRPS" is known in the art and relates to the formation of at least one peptide bond in the absence of polynucleotides, preferably mRNA, catalyzed by a polypeptide or polypeptide complex as defined herein below. Preferably, NRPS is the enzymatically catalyzed condensation of at least two amino acid units. More preferably, NRPS is the synthesis of a peptide by one of a group of specific enzymes known as non-ribosomal peptide synthetases (NRPSases).

As used herein, the term "NRPS amino acid module" relates to a subpart of a NRPSase catalyzing at least activation of an amino acid and condensation thereof to the growing oligopeptide chain. Preferably, the NRPS amino acid module comprises at least one condensation domain, at least one adenylation domain, and at least one thiolation domain. Examples for NRPS amino acid modules and domains comprised therein are well known in the art. Preferably, the NRPS amino acid module is specific for activation of a specific amino acid. NRPS amino acid modules have been covered extensively by databases, e.g. "A database of NonRibosomal Peptide Synthetases" at the New Delhi National Institute of Immunology" by M.Z. Ansari, R.S. Gokhale, and D. Mohanty (http://linux1.nii.res.in/∼zeeshan/webpages/home.html), "NORINE": Caboche et al. (2008), Nucleic Acids Res. 36 (Database issue): D326-31 (http://bioinfo.lifl.fr/norine/), and "ClusterMine360": Conwayet al. (2013), Nucleic Acids Res. 41 (Database issue): D402-7, (http://www.clustermine360.ca/). Preferably, the NRPS amino acid module is an NRPS amino acid module as described herein. As it was detailed herein above, NRPSases are structured modularly, both in comprising modules typically catalzing the addition of one amino acid unit, and in that each module itself comprises specific domains. Thus, preferably, the term NRPS amino acid module also includes to non-natural modules, preferably including synthetic modules. Also preferably, the NRPS amino acid module of the present invention may comprise one or more non-natural domain(s), more preferably one or more synthetic domains(s).

The term "pigment", as used herein, relates to a chemical compound having at least one absorption maximum at a wavelength of visible light. Preferably, the at least one absorption maximum is at a wavelength between 380 nm and 750 nm, more preferably between 400 nm and 650 nm. Methods of determining absorption maxima of chemical compounds are known in the art. Preferably, the pigment is synthesized as a pro-pigment, requiring further, spontaneous or catalyzed, modification, including, preferably, oxidation, cyclisation, or aggregation. Also preferably, the pigment is synthesized as an active pigment, i.e., preferably, as a pigment having said absorption maximum. Preferably, the pigment is a pigment generated by a polyketide synthase, i.e. a polyketide pigment. More preferably, the pigment is a pigment generated by a non-ribosomal peptide synthetase, i.e. the pigment is an NRPS pigment.

The term "NRPS pigment", as used herein, relates to a chemical compound generated at least in part by NRPS having at least one absorption maximum at a wavelength of visible light. Preferably, the NRPS pigment comprises at least two amino acid units. Preferably, the at least one absorption maximum is at a wavelength between 380 nm and 750 nm, more preferably between 400 nm and 650 nm. Methods of determining absorption maxima of chemical compounds are known in the art. Preferably, the NRPS pigment is synthesized as a pro-pigment in the NRPS process, requiring further, spontaneous or catalyzed, modification, including, preferably, oxidation, cyclisation, or aggregation. Also preferably, the NRPS pigment is synthesized as an active pigment, i.e., preferably, as a pigment having said absorption maximum, in the NRPS. Preferably, the NRPS pigment is actinomycin (Schauwecker et al. (1998), J Bacteriol. 180(9): 2468-74). More preferably, the NRPS pigment is indigoidine (IUPAC Name: (5E)-3-amino-5-(5-amino-2,6-dioxopyridin-3-ylidene)pyridine-2,6-dione, CAS Registry Number: 2435-59-8). Preferably, the term NRPS pigment relates to the complete pigment molecule as it is produced by a naturally occurring NRPS pigment module.

The term "pigment module" relates to a polypeptide or polypeptide complex catalyzing the synthesis of a pigment according to the present invention. Preferably, the pigment module is a polyketide synthase module. More preferably, the pigment module is an NRPS pigment module.

The term "NRPS pigment module" relates to a polypeptide or polypeptide complex contributing to the synthesis of an NRPS pigment. Preferably, the NRPS pigment module is a polypeptide or polypeptide complex catalyzing the synthesis of an NRPS pigment. More preferably, the NRPS pigment module is a polypeptide catalyzing indigoidine synthesis, e.g. an indigoidine synthetase as shown in Table 1, more preferably the NRPS pigment module is the indigoidine synthetase encoded by the indC gene of Photorhabdus luminescens (gene: SEQ ID NO: 1, protein: SEQ ID NO: 25). Preferably, the term NRPS pigment module also includes to non-natural modules, preferably including synthetic modules. Also preferably, the NRPS pigment module of the present invention may comprise one or more non-natural domain(s), more preferably one or more synthetic domains(s).

**Table 1: Indigoidine Synthetases**

| **name** | **synonym and description** | **NCBI Acc No** | **source organism** | **reference** |
|---|---|---|---|---|
| indC (SEQ ID NO:25) | hypothetical protein plu2186 | NP_929446.1 | Photorhabdus luminescens subsp. laumondii TTO1 | Duchaud, E. et. al., Nat. Biotechnol. 21 (11), 1307-1313 (2003), The genome sequence of the entomopathogenic bacterium Photorhabdus luminescens |
| | | GI:37526102 | | |
| | | | | Brachmann AO et. al. (2012) Triggering the production of the cryptic blue pigment indigoidine from Photorhabdus luminescens. J Biotechnol 157: 96-99. |
| | indigoidine synthase | WP_017892269.1 | Serratia sp. S4 | |
| | | GI:516503831 | | |
| | indigoidine synthase | WP_017237530.1 | Streptomyces sp. SS | |
| | | GI:515806777 | | |
| indC | putative indigoidine synthase | YP_004349727.1 | Burkholderia gladioli BSR3 | Seo,Y.S. et. al., Complete Genome Sequence of Burkholderia gladioli BSR3, J. Bacteriol. 193 (12), 3149 (2011) |
| | | GI:330820865 | | |
| indC | putative indigoidine synthase sa8 | ACK77757.1 | Streptomyces aureofaciens | Novakova,R. et. al., Identification and characterization of an indigoidine-like gene for a blue pigment biosynthesis in Streptomyces aureofaciens CCM 3239, Folia Microbiol. (Praha) 55 (2), 119-125 (2010) |
| | | GI:218511496 | | |
| bpsA | Blue-pigment synthetase | BAE93896.1 | Streptomyces lavendulae subsp. | Takahashi, H. et.al., Cloning and Characterization of a Streptomyces Single Module Type Non-ribosomal Peptide Synthetase Catalyzing a Blue Pigment Synthesis, J. Biol. Chem. 282 (12), 9073-9081 (2007) |
| | | GI:94467513 | lavendulae | |
| | Blue-pigment synthetase igiD, indC, bpsA,sa8 | YP_007934704.1 | Streptomyces fulvissimus DSM | Myronovskyi, M., et. al. Complete genome sequence of Streptomyces fulvissimus, Submitted (03-APR-2013) Helmholtz Institute for Pharmaceutical Research Saarland, Helmholtz Center for Infectious Research, University Campus, Building C 2.3, Saarbrucken, Saarland 66123, Germany |
| | | GI:488613368 | 40593 | |
| | blue-pigment synthetase | WP_007269003.1 | Streptomyces sp. C | |
| | | GI:494479526 | | |
| indC | Putative indigoidine synthase | WP_003963722.1 | Streptomyces clavuligerus | |
| | | GI:490061478 | | |
| indC | blue-pigment synthetase | WP_003952690.1 | Streptomyces clavuligerus | |
| | | GI:490050342 | | |
| | indigoidine synthase | WP_016941560.1 | Dickeya zeae | |
| | | GI:515508306 | | |
| | indigoidine synthase | WP_019435944.1 | Streptomyces sp. AA0539 | |
| | | GI:518265736 | | |
| | indigoidine synthase | WP_019844163.1 | Dickeya zeae | |
| | | GI:518682470 | | |
| indC | indigoidine synthase | AFV27434.1 | Streptomyces chromofuscus (ATCC 49982) | Yu,D.et. al. An indigoidine biosynthetic gene cluster from Streptomyces chromofuscus ATCC 49982 contains an unusual IndB homologue, J. Ind. Microbiol. Biotechnol. 40 (1), 159-168 (2013) |
| | | GI:409183839 | | |
| | indigoidine synthase | YP_003885171.1 | Dickeya dadantii 3937 | Glasner, J.D. et. al., Genome Sequence of the Plant-Pathogenic Bacterium Dickeya dadantii 3937, J. Bacteriol. 193 (8), 2076-2077 (2011) |
| | | GI:307133155 | | |
| indC | indigoidine synthase | CAB87990.1 | Erwinia chrysanthemi | Reverchon,S. et. al., Characterization of indigoidine biosynthetic genes in Erwinia chrysanthemi and role of this blue pigment in pathogenicity, J. Bacteriol. 184 (3), 654-665 (2002) |
| | | GI:7576265 | | |
| | Putative indigoidine synthase | YP_007526476.1 | Streptomyces davawensis JCM 4913 | Jankowitsch, F., et. al., Genome Sequence of the Bacterium Streptomyces davawensis JCM 4913 and Heterologous Production of the Unique Antibiotic Roseoflavin, J. Bacteriol. 194 (24), 6818-6827 (2012) |
| | | GI:471327446 | | |
| | indigoidine synthase | WP_010472001.1 | Streptomyces somaliensis | |
| | | GI:498157845 | | |
| | indigoidine synthase | WP_018512405.1 | Streptomyces sp. ScaeMP-e10 | |
| | | GI:517336913 | | |
| | Blue-pigment synthetase, igiD, indC, bpsA, sa8 | YP_007931314.1 | Streptomyces fulvissimus DSM 40593 Myronovskyi, M. et. al., Complete genome sequence of Streptomyces fulvissimus, Submitted (03-APR-2013) Helmholtz Institute for Pharmaceutical Research Saarland, Helmholtz Center for Infectious Research, University Campus, Building C 2.3, Saarbrucken, Saarland 66123, Germany | |
| | | GI:488609978 | | |
| | indigoidine synthase | WP_018894040.1 | Streptomyces sp. CNY228 | |
| | | GI:517723832 | | |
| | Blue-pigment synthetase | YP_007748943.1 | Streptomyces albus J1074 | Rabyk, M. et. al., Complete genome analysis and transcriptional profile of Streptomyces albus J1074, |
| | | GI:478692133 | | |
| | | | | Submitted (25-FEB-2013) Department of Genetics and Biotechnology, Ivan Franko National University of Lviv, Hrushevskyy str., 4, Lviv 79005, Ukraine |
| | indigoidine synthase | WP_018488639.1 | Streptomyces sp. CcaIMP-8W | |
| | | GI:517299821 | | |
| | indigoidine synthase | WP_018471088.1 | Streptomyces sp. LaPpAH-202 | |
| | | GI:517282270 | | |
| | blue-pigment synthetase | WP_003946752.1 | Streptomyces albus | |
| | | GI:490044390 | | |
| igiD | | AAD54007.1 | Vogesella indigofera | van de Loo, F.J., et. al., Structural and regulatory genes controlling indigoidine production in Vogesella indigofera: involvement of a peptide synthetase homolog, Submitted (01-SEP-1998) Plant Industry, CSIRO, G.P.O. Box 1600, Canberra, ACT 2601, Australia |
| | | GI:5852326 | | |
| | blue pigment synthetase | AFT64148.1 | alpha proteobacterium U95 | Penesyan, A. et. al.,Assessing the effectiveness of functional genetic screens for the identification of bioactive metabolites, Mar Drugs 11 (1), 40-49 (2013) |
| | | GI:407188354 | | |
| | indigoidine synthase, Arad_12458 | YP_002546883.1 | Agrobacterium radiobacter K84 | Setubal, J. et. al., Genome sequencing of three Agrobacterium biovars illustrates the role of gene flow among plasmids and chromosomes in the evolution of pathogenic and symbiotic alpha proteobacteria, J. Bacteriol. (2009) |
| | | GI:222102293 | | |
| indC | Putative indigoidine synthase, partial | WP_010110104.1 | Verminephrobacter aporrectodeae | |
| | | GI:497795920 | | |
| | indigoidine synthase | YP_001624984.1 | Renibacterium salmoninarum ATCC 33209 | Wiens, G.D., et. al., Genome sequence of the fish pathogen Renibacterium salmoninarum suggests reductive evolution away from an environmental Arthrobacter ancestor, J. Bacteriol. 190 (21), 6970-6982 (2008) |
| | | GI:163840579 | | |
| indC | indigoidine synthase, partial | CAD27331.1 | Erwinia chrysanthemi (Pectobacterium chrysanthemi = Dickeya chrysanthemi) | Reverchon, S., vfm genes of Erwinia chrysanthemi modulate the synthesis of multiple virulence factors, Submitted (05-MAR-2002) Reverchon S., Biochimie, Institut des Sciences Appliquees de Lyon, 11 avenue Jean Capelle, Villeurbanne 69621, FRANCE |
| | | GI:19571812 | | |
| | indigoidine synthetase , NRPS2, bpsA/indC homolog | | Streptomyces laurentii ATCC 31255 | Tala Mubadda Suidan, Mining the cryptic non ribosomal peptide systems of Streptomyces laurentii, BSc. thesis, Georgia Institute of Technology, Dec. 2010; Wendy L. K. et. al., Thiostrepton Biosynthesis: Prototype for a New Family of Bacteriocins, J. Am. Chem. Soc., 2009, 131 (12), pp 4327-4334 |
| | | | | |
| igiD | indigoidine synthetase | RBY4I-2890 | Roseobacter Phaeobacter sp. Strain Y4I | Cude, W. N., Mooney, J., Tavanaei, A. a, Hadden, M. K., Frank, A. M., Gulvik, C. a, ... Buchan, A. (2012). Production of the antimicrobial secondary metabolite indigoidine contributes to competitive surface colonization by the marine roseobacter Phaeobacter sp. strain Y4I. Applied and environmental microbiology, 78(14), 4771-80 |

The term "polypeptide", as used herein, relates to a macromolecule comprising at least the modules and/or domains as defined herein. Preferably, the polypeptide comprises a contiguous chain of peptide bonds forming the backbone of the polypeptide. More preferably, the polypeptide comprises a contiguous chain of alpha-amino acids interconnected by peptide bonds. Even more preferably, the polypeptide is synthesized by in vitro protein biosynthesis; most preferably, the polypeptide is synthesized by in vivo protein biosynthesis. In the context of the present invention, the term polypeptide, preferably, relates to a polypeptide of between 50 and 30000 amino acids in length comprising at least one domain of an NRPS amino acid module and/or at least one domain of an NRPS pigment module. The polypeptide may comprise further amino acids which may serve as a tag for purification or detection, as a linker, or as a communication domain. In a preferred embodiment of the polypeptide of the present invention, said polypeptide further comprises a detectable tag. The term "detectable tag" refers to a stretch of amino acids which are added to or introduced into the polypeptide of the invention. Preferably, the tag shall be added C- or N- terminally to the polypeptide. The said stretch of amino acids shall allow for detection of the polypeptide by an antibody which specifically recognizes the tag or it shall allow for forming a functional conformation, such as a chelator or it shall allow for visualization by fluorescent tags. Preferred tags are the Myctag, FLAG-tag, 6-His-tag, HA-tag, GST-tag or GFP-tag. These tags are all well known in the art. The term polypeptide also includes chemically modified polypeptides, e.g., containing modified amino acids or being biotinylated or coupled to fluorophores, such as fluorescein, or Cy 3, being conformationally restricted, e.g. by disulfide bridging or by stapling (Walensky 2004, Science 305(5689): 1466-1470), or being linked to cell penetration polypeptides or protein transduction domains (Snyder 2004, Pharm Res 21(3): 389-393). Such modifications may improve the biological properties of the polypeptide, e.g., complex formation, binding, stability, or may be used as detection labels.

As used herein, the term polypeptide, preferably, also relates to a variant of a polypeptide, including variants characterized by one or more amino acid exchanges or deletions or additions of amino acids. The term, preferably, also includes fragments of the polypeptides specifically mentioned, provided that said polypeptide variants still have an activity as detailed herein.

Preferably, the NRPS amino acid module or modules and the pigment module or modules are comprised in a fusion polypeptide catalyzing the synthesis of a pigment, more preferably an NRPS pigment, covalently connected to the amino acid activated by the NRPS amino acid module. More preferably, at least two NRPS amino acid modules and a pigment module are comprised in a fusion polypeptide catalyzing the synthesis of a pigment covalently connected to the oligopeptide synthesized by the NRPS amino acid modules.

Accordingly, the term "polypeptide complex" relates to a complex comprising at least two polypeptides of the present invention. In such case, the polypeptides comprised in the polypeptide complex may be referred to as subunits of the complex. Preferably, polypeptides of the polypeptide complex are connected by a chemical linkage. It is envisaged by the present invention that the chemical bond between the subunits is an ester bond, a disulfide bond, or any other suitable covalent chemical bond known to the skilled artisan. More preferably, the subunits are connected via non-covalent bonds with a dissociation constant so low that the subunits will only dissociate to a negligible extent. Preferably, the dissociation constant for said non-covalent bond is less than 10⁻⁵ mol/l (as it is the case with the Strep-Tag : Strep-Tactin binding), less than 10⁻⁶ mol/l (as it is the case in the Strep-TagII : Strep-Tactin binding), less than 10⁻⁸ mol/l, less than 10⁻¹⁰ mol/l, or less than 10⁻¹² mol/l (as it is the case for the Streptavidin : Biotin binding). Methods of determining dissociation constants are well known to the skilled artisan and include, e.g., spectroscopic titration methods, surface plasmon resonance measurements, equilibrium dialysis and the like. In a preferred embodiment, the polypeptide consists of the components as described herein. Most preferably, at least one of the polypeptides of the polypeptide complex comprises a communication domain of an NRPS amino acid domain known in the art.

Preferably, the polypeptide or polypeptide complex of the present invention comprises a fusion polypeptide comprising at least a NRPS amino acid module condensation domain and a NRPS pigment module adenylation domain. Preferably, the polypeptide or polypeptide complex of present invention is a polypeptide or polypeptide complex comprising the C domain of the at least one NRPS amino acid module and the at least one NRPS pigment module as a fusion polypeptide. It is understood by the one skilled in the art that in such case, preferably, the polypeptide or polypeptide complex comprises the adenylation domain and the thiolation domain as further components of a fusion polypeptide; more preferably, the adenylation domain and the thiolation domain are comprised as a subunit or as subunits of a polypeptide complex. More preferably, the polypeptide or polypeptide complex is a polypeptide or polypeptide complex comprising the at least one NRPS amino acid module and the at least one NRPS pigment module as a fusion polypeptide; preferably, the NRPS amino acid module comprises at least a condensation domain, an adenylation domain, and a thiolation domain as detailed herein above. Preferably, the polypeptide or polypeptide complex comprises at least one NRPS amino acid module. More preferably, the polypeptide or polypeptide complex comprises at least two NRPS amino acid modules. Most preferably, the polypeptide or polypeptide complex comprises the number of NRPS amino acid module corresponding to the number of amino acid units comprised in the oligopeptide to be synthesized. Since a given NRPS amino acid module is specific for a specific amino acid unit, it will be appreciated that the selection of the specific NRPS amino acid module or NRPS amino acid modules and their sequence in the polypeptide or polypeptide complex determines the sequence of amino acid units in the oligopeptide produced. The NRPS pigment module can, in principle, be located within the polypeptide or polypeptide complex at any location deemed appropriate. Accordingly, the NRPS pigment may be included at any position within the oligopeptide. Preferably, the NRPS pigment module is functionally connected to the NRPS module mediating the last elongation step. Accordingly, preferably, the NRPS pigment is the last unit synthesized onto the oligopeptide. Preferably, the NRPS amino acid module preceding the pigment module is specific for a small, neutral amino acid; more preferably, the NRPS amino acid module preceding the pigment module is specific for glycine, alanine, or valine.

Preferably, the polypeptide or polypeptide complex of the present invention comprises NRPS amino acid modules in an arrangement as it is found in nature. Accordingly, preferably, the polypeptide or polypeptide complex catalyzes the synthesis of a labeled natural NRPS oligopeptide. More preferably, the polypeptide or polypeptide complex comprises NRPS amino acid modules in an arrangement not found in nature. Accordingly, more preferably, polypeptide or polypeptide complex catalyzes the synthesis of a labeled non-natural NRPS oligopeptide.

The term "functionally connected" is understood by the skilled person. Preferably, the term relates to two polypeptides of the present invention being connected in a way that enables transfer of a growing oligopeptide chain from one polypeptide of a polypeptide complex to the other, thus permitting condensation of at least one further amino acid unit onto the oligopeptide after transfer. Preferably, functional connection is mediated by protein-protein interaction, more preferably by protein-protein interaction between a communication domain and a polypeptide or between two communication domains present on two different polypeptides. More preferably, functional connection is mediated by covalent interconnection of two polypeptides. Most preferably, functional connection is achieved by combining two polypeptides as a fusion polypeptide by methods well known to the skilled person and as described herein.

Advantageously, it was surprisingly found in the work underlying the present invention that an NRPS pigment module can be functionally connected to a non-ribosomal peptide synthetase, resulting in the production of labeled NRPS peptides. As will be appreciated, these tools are especially useful in, e.g., tracking or determining the amount of NRPS oligopeptides. Moreover, the tools are useful in the optimization of in vitro or in vivo NRPS peptide production, since they allow for convenient identification of producer cells and for convenient determination of the amount of NRPS oligopeptide produced, e.g. semiquantitatively by thin layer chromatography (TLC) or quantitatively by photometric measurement of culture coloring as described herein in the Examples.

The definitions made above apply mutatis mutandis to the following. Additional definitions and explanations made further below also apply for all embodiments described in this specification mutatis mutandis.

The present invention further relates to a labeled oligopeptide comprising a non-naturally attached NRPS pigment or/and polyketide pigment.

As used herein, the term "labeled oligopeptide" relates to an oligopeptide as described herein comprising covalently attached at least one NRPS pigment or/and at least one polyketide pigment. Preferably, the NRPS pigment is a non-naturally attached NRPS pigment, i.e., preferably, an NRPS pigment not known to be covalently attached to said oligopeptide in nature. Also preferably, the polyketide pigment is a non-naturally attached polyketide pigment, i.e., preferably, a polyketide pigment not known to be covalently attached to said oligopeptide in nature. More preferably, the labeled oligopeptide is an indigoidine-labeled oligopeptide. i.e. the NRPS pigment comprised in the labeled oligopeptide is indigoidine.

Moreover, the present invention relates to a polynucleotide encoding a fusion polypeptide according to the present invention.

The term "polynucleotide", as used in accordance with the present invention, relates to a polynucleotide comprising a nucleic acid sequence which encodes a fusion polypeptide as described herein above having the activity of catalyzing the condensation of an amino acid unit activated by an NRPS amino acid module adenylation domain with a second amino acid unit activated by an NRPS pigment module adenylation domain. Suitable assays for measuring the activities mentioned before are described in the accompanying Examples or in Owen et al. (2011), The Biochemical journal, 436(3): 709-17; Owen et al. (2012), Environmental microbiology, 14(5): 1198-209; Mülleret al. (2012), Metabolic engineering, 14(4): 325-35; Takahashi et al. (2007), JBC 282(12): 9073-9081; Myerset al. (2013), BMC Biophysics, 6(1): 4).

The polynucleotide, preferably, comprises the nucleic acid sequence shown in SEQ ID NO: 11, 13-19, 23, 24, or, more preferably, SEQ ID NO:26 encoding fusion polypeptides of SEQ ID NOs: 27, 28-34, 35, 36, and 37, respectively. It is to be understood that a fusion polypeptide encoded by one of the aforementioned polynucleotides may be also encoded due to the degenerated genetic code by other polynucleotides as well.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still encode a fusion polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 ' sodium chloride/sodium citrate (= SSC) at approximately 45°C, followed by one or more wash steps in 0.2 ' SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 ' SSC (pH 7.2). If organic solvent is present in the abovementioned buffer, for example 50% formamide, the temperature under standard conditions is approximately 42°C. The hybridization conditions for DNA:DNA hybrids are preferably for example 0.1 ' SSC and 20°C to 45°C, preferably between 30°C and 45°C. The hybridization conditions for DNA:RNA hybrids are preferably, for example, 0.1 ' SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the fusion polypeptides of the present invention. Conserved domains of the fusion polypeptide of the present invention may be identified by a sequence comparison of the nucleic acid sequence of the polynucleotide or the amino acid sequence of the fusion polypeptide of the present invention with sequences of other non-ribosomal peptide synthetases or non-ribosomal pigment synthetases. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 70%, at least 75%, at least 80%, at least 85%, at least 90%, at least 95%, at least 98% or at least 99% identical to the nucleic acid sequences detailed herein. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences of the fusion polypeptides of the present invention. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the aforementioned nucleic acid sequences, preferably, is also encompassed as a polynucleotide of the present invention. The fragment shall encode a fusion polypeptide which still has the activity as specified above. Accordingly, the fusion polypeptide may comprise or consist of the domains of the polypeptide of the present invention conferring the said biological activity. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences.

The polynucleotides of the present invention either essentially consist of the aforementioned nucleic acid sequences or comprise the aforementioned nucleic acid sequences. Thus, they may contain further nucleic acid sequences as well. The fusion proteins encoded may comprise as additional part other enzymes, polypeptides for monitoring expression (e.g., green, yellow, blue or red fluorescent proteins, alkaline phosphatase and the like) or so called "tags" which may serve as a detectable marker or as an auxiliary measure for purification purposes as detailed elsewhere herein.

The polynucleotide of the present invention shall be provided, preferably, either as an isolated polynucleotide (i.e. isolated from its natural context) or in genetically modified form. The polynucleotide, preferably, is DNA, including cDNA, or RNA. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides.

The present invention also relates to a vector comprising the polynucleotide according the present invention.

The term "vector", preferably, encompasses phage, plasmid, viral or retroviral vectors as well artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination. The vector encompassing the polynucleotide of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerenes. Alternatively, a plasmid vector may be introduced by heat shock or electroporation techniques. Should the vector be a virus, it may be packaged in vitro using an appropriate packaging cell line prior to application to host cells.

More preferably, in the vector of the invention the polynucleotide is operatively linked to expression control sequences allowing expression in prokaryotic or eukaryotic cells or isolated fractions thereof. Expression of said polynucleotide comprises transcription of the polynucleotide, preferably into a translatable mRNA. Regulatory elements ensuring expression in prokaryotic or eukaryotic cells are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in prokaryotic host cells comprise, e.g., the lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used in an expression vector encompassed by the present invention. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (InVitrogene) or pSPORT1 (GIBCO BRL). Preferably, said vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

The present invention further relates to a host cell comprising the polypeptide or polypeptide complex and/or the polynucleotide and/or the vector according to the present invention.

The term "host cell", as used herein, relates to any bacterial, archeal, or eukaryotic cell. Preferably, the cell is a eukaryotic cell, more preferably an insect or mammalian cell; more preferably, the eukaryotic cell is a fungal cell, most preferably a yeast cell, e.g. a cell of Saccharomyces cerevisiae. More preferably, the cell is bacterial cell, even more preferably an Escherichia cell (e.g. E. coli) or a Bacillus cell (e.g. B. subtilis) (Zhang et al. (2011), Nat Prod Rep 28: 125-151). Preferably, the host cell further comprises a broad-specificity phosphopantetheinyl transferase (PPTase), more preferably an Sfp-type PPTase.

The following methods of the present invention may, preferably, comprise further steps in addition to those explicitly mentioned. E.g., in all methods, a further step may be detecting or determining the amount of labeled oligopeptide. Further examples of preferred additional steps are described for the respective methods. Moreover, one or more of the steps of the methods of the present invention may be performed by automated equipment.

The present invention also relates to an in vitro method of producing a labeled oligopeptide, comprising:
a) incubating a polypeptide or polypeptide complex according to present invention with appropriate amino acid substrates,
b) thereby producing a labeled oligopeptide.

The present invention further relates to a method for optimizing in vitro production of a labeled oligopeptide by improving incubation conditions, comprising
a) incubating the polypeptide or polypeptide complex according the present invention under modified conditions suspected to improve production of said labeled oligopeptide,
b) comparing the amount of labeled oligopeptide produced to the amount produced by said polypeptide or polypeptide complex under unmodified conditions, and, thereby
c) optimizing in vitro production of a labeled oligopeptide.

Moreover, the present invention relates to a method for optimizing in vitro production of a labeled oligopeptide, comprising
a) incubating a variant of a polypeptide or polypeptide complex according to the present invention under conditions suitable for production of said labeled oligopeptide,
b) comparing the amount of labeled oligopeptide produced to the amount produced by the unmodified polypeptide or polypeptide complex, and, thereby
c) optimizing in vitro production of a labeled oligopeptide.

Further steps preferably included in the in vitro methods of the present invention may relate, e.g., to including further compounds in the incubation step, or to purifying the labeled oligopeptide. It is understood that the definitions below apply to variants of the polypeptides or polypeptide complexes mutatis mutandis.

The term "incubating the polypeptide or polypeptide complex", as used in the context of the in vitro methods of the present invention, is understood by the skilled person. Preferably, the term relates to bringing a polypeptide or polypeptide complex according to present invention in physical contact with amino acids and thereby, e.g. allowing the polypeptide or polypeptide complex and the amino acids to interact. As will be appreciated, incubating, preferably, relates to mixing the polypeptide or polypeptide complex of the present invention with at least the other components as defined herein in an appropriate solvent under appropriate conditions, e.g. temperature. Appropriate solvents, preferably, are water-based buffers known in the art. As will also be appreciated, the incubation mixture may, preferably, comprise further ingredients, e.g. an energy source, e.g. ATP, or ions required by the polypeptide or polypeptide complex of the invention, e.g. magnesium ions. Preferably, the composition of the incubation solution is one of the compositions known in the art, (e.g. in Doekel & Marahiel (2000), Chem. Biol. 7(6): 373-84; Stein et al. (2006), Chembiochem 7(11): 1807-14; Owen et al. (2011), Biochem. J. 436(3): 709-17). Preferably, the term relates to maintaining the polypeptide or polypeptide complex under conditions allowing production of labeled oligopeptide by said polypeptide or polypeptide complex. Appropriate conditions, including, without limitation, buffers, ion concentrations, temperature, and the like, depend on the polypeptide or polypeptide complex selected and are known in the art. The summary of the conditions used in incubating a host cell is known as "incubation conditions". Thus, "modified incubation conditions", as used herein, relates to incubation conditions wherein at least one parameter has been modified as compared to the incubation conditions used before optimization. Accordingly, the term "improving incubation conditions" relates to modifying one more incubation parameters for a polypeptide or polypeptide complex such that the production of labeled oligopeptide is improved.

The term "amino acid substrate" relates to a chemical compound corresponding to the amino acid unit activated by a NRPS amino acid module or pigment module included in the polypeptide or polypeptide complex used. Accordingly, appropriate amino acid substrates are the compounds corresponding to the amino acid units activated by the NRPS amino acid modules or pigment modules included in the polypeptide or polypeptide complex used in the method.

The term "optimizing" as used herein, relates to improving the yield of labeled oligopeptide obtainable by a process. Preferably, the term relates to improving the yield of full-length oligopeptide.

"Comparing," as used herein, relates to a comparison of corresponding parameters or values, e.g., an absolute amount is compared to an absolute reference amount; a concentration compared to a reference concentration; an intensity signal obtained is compared to the same type of intensity signal. The comparison referred to in the methods of the present invention may be carried out manually or computer assisted. For a computer assisted comparison, the value of the determined amount or ratio may be compared to values corresponding to suitable references which are stored in a database by a computer program. The computer program may further evaluate the result of the comparison by means of an expert system. Accordingly, the result of the comparison referred to herein may be automatically provided in a suitable output format.

The term "conditions suspected to improve production", as used herein, preferably relates to any conditions not known not to improve production. As will be appreciated by the skilled person, factors affecting in vitro production of oligopeptides are difficult to predict. Accordingly, in principle, any modification of incubation conditions is suspected to improve production, unless it is known that this is not the case. More preferably, incubation conditions are suspected to improve production if said improvement appears to be a reasonable conclusion in view of what is known on the production process, or if an improvement was found by applying said conditions in a similar case.

Moreover, the present invention relates to an in vivo method of producing a labeled oligopeptide, comprising:
a) incubating a host cell comprising the polypeptide or polypeptide complex and/or the expression vector according to the present invention,
b) thereby producing a labeled oligopeptide.

Also, the present invention relates to a method for optimizing in vivo production of a labeled oligopeptide by improving culture conditions, comprising
a) incubating a host cell comprising the polypeptide or polypeptide complex and/or the expression vector according to the present invention under modified conditions suspected to improve production of said labeled oligopeptide,
b) comparing the amount of labeled oligopeptide produced to the amount produced by said host cell under unmodified conditions, and, thereby
c) optimizing in vivo production of a labeled oligopeptide.

Further, the present invention relates to a method for optimizing in vivo production of a labeled oligopeptide, comprising
a) incubating a host cell comprising a polypeptide or polypeptide complex variant and/or an expression vector comprising a polynucleotide variant according to the present invention under conditions suitable for production of said labeled oligopeptide,
b) comparing the amount of labeled oligopeptide produced to the amount produced by a host cell comprising an unmodified polypeptide or polypeptide complex and/or an unmodified expression vector according the present invention, and, thereby
c) optimizing in vivo production of a labeled oligopeptide.

The term "incubating a host cell", as used in the context of the in vivo methods of the present invention, is understood by the skilled person. Preferably, the term relates to maintaining the host cell comprising the polypeptide or polypeptide complex and/or the expression vector under conditions allowing proliferation of said host cell and/or production of labeled oligopeptide by said host cell. Appropriate conditions, including, without limitation, medium, temperature, oxygen and/or carbon dioxide tension, and the like, depend on the host cell selected and are known in the art. The summary of the conditions used in incubating a host cell is known as "culture conditions". Thus, "modified culture conditions", as used herein, relates to culture conditions wherein at least one parameter has been modified as compared to the culture conditions used before optimization. Accordingly, the term "improving culture conditions" relates to modifying one more incubation parameters for a host cell such that the production of labeled oligopeptide is improved.

The term "conditions suspected to improve production", as used herein, preferably relates to any conditions not known not to improve production. As will be appreciated by the skilled person, factors affecting in vivo production of oligopeptides are difficult to predict. Accordingly, in principle, any modification of culture conditions is suspected to improve production, unless it is known that this is not the case. More preferably, culture conditions are suspected to improve production if it said improvement appears a reasonable conclusion in view what is known on the production process, or if an improvement was found by applying said conditions in a similar case.

The present invention further relates to a labeled oligopeptide obtainable by one of the methods of producing a labeled oligopeptide according to the present invention.

The present invention also relates to a kit for in vivo synthesis of a labeled oligopeptide comprising an expression vector according to of the present invention and an expression vector encoding at least one further NRPS amino acid module.

Moreover, the present invention relates to a kit for in vitro synthesis of a labeled oligopeptide comprising a polypeptide or polypeptide complex according to the present invention and at least one further NRPS amino acid module.

The term "kit", as used herein, refers to a collection of the aforementioned means, preferably, provided separately or within a single container. The container, also preferably, comprises instructions for carrying out the method of the present invention. The components of the kit are provided, preferably, in a "ready-to-use" manner, e.g., concentrations are adjusted accordingly, etc.

Summarizing the findings of the present invention, the following embodiments are preferred:
Embodiment 1: A polypeptide or polypeptide complex, comprising at least one non-ribosomal peptide synthesis (NRPS) amino acid module functionally connected to at least one pigment module.
Embodiment 2: The polypeptide or polypeptide complex of embodiment 1, wherein the at least one NRPS amino acid module and the at least one pigment module are comprised in a fusion polypeptide catalyzing the synthesis of a pigment covalently connected to the amino acid activated by the NRPS amino acid module.
Embodiment 3: The polypeptide or polypeptide complex of embodiment 1 or 2, comprising the C domain of the at least one NRPS amino acid module and the at least one pigment module as a fusion polypeptide.
Embodiment 4: The polypeptide or polypeptide complex of any one of embodiments 1 to 3, comprising the at least one NRPS amino acid module and the at least one pigment module as a fusion polypeptide.
Embodiment 5: The polypeptide or polypeptide complex of any one of embodiments 1 to 4, wherein the polypeptide or polypeptide complex comprises at least two NRPS amino acid modules.
Embodiment 6: The polypeptide or polypeptide complex of embodiment 5, wherein the pigment module is functionally connected to the NRPS module mediating the last elongation step.
Embodiment 7: The polypeptide or polypeptide complex of any one of embodiments 1 to 6, wherein the pigment module is an NRPS pigment module.
Embodiment 8: The polypeptide or polypeptide complex of any one of embodiments 1 to 7, wherein the pigment module is an indigoidine synthetase.
Embodiment 9: The polypeptide or polypeptide complex of any one of embodiments 1 to 8, wherein the pigment module is an indigoidine synthetase encoded by one of the genes specified in Table 1, preferably the indC gene of Photorhabdus luminescens, more preferably the indC gene of Photorhabdus luminescens laumondii TT01.
Embodiment 10: The polypeptide or polypeptide complex of any one of embodiments 1 to 9, wherein the NRPS amino acid module or the NRPS amino acid modules is/are selected from the NRPS amino acid modules encoded by any one of SEQ ID NO: 11 to 24.
Embodiment 11: A labeled oligopeptide comprising a non-naturally attached NRPS pigment or/and polyketide pigment.
Embodiment 12: The labeled oligopeptide of embodiment 10, wherein the pigment is indigoidine.
Embodiment 13: A polynucleotide encoding a fusion polypeptide according to any one of embodiments 3 to 10.
Embodiment 14: A vector comprising the polynucleotide according to embodiment 13.
Embodiment 15: The vector of embodiment 14, wherein the vector is an expression vector.
Embodiment 16: A host cell comprising the polypeptide or polypeptide complex according to any one of embodiments 1 to 10 and/or the polynucleotide according to embodiment 13, and/or the vector according to embodiment 14 or 15.
Embodiment 17: An in vitro method of producing a labeled oligopeptide, comprising:
   a) incubating a polypeptide or polypeptide complex according to any one of embodiments 1 to 10 with appropriate amino acid substrates,
   b) thereby producing a labeled oligopeptide.
Embodiment 18: An in vivo method of producing a labeled oligopeptide, comprising:
   a) incubating a host cell comprising the polypeptide or polypeptide complex according to any one of embodiments 1 to 10 and/or the expression vector according to embodiment 15,
   b) thereby producing a labeled oligopeptide.
Embodiment 19: A labeled oligopeptide obtainable by the method according to embodiment 17 or by the method according to embodiment 18.
Embodiment 20: A method for optimizing in vivo production of a labeled oligopeptide by improving culture conditions, comprising
   a) incubating a host cell comprising the polypeptide or polypeptide complex according to any one of embodiments 1 to 10 and/or the expression vector according to embodiment 15 under modified conditions suspected to improve production of said labeled oligopeptide,
   b) comparing the amount of labeled oligopeptide produced to the amount produced by said host cell under unmodified conditions, and, thereby
   c) optimizing in vivo production of a labeled oligopeptide.
Embodiment 21: A method for optimizing in vitro production of a labeled oligopeptide by improving incubation conditions, comprising
   a) incubating the polypeptide or polypeptide complex according to any one of embodiments 1 to 10 under modified conditions suspected to improve production of said labeled oligopeptide,
   b) comparing the amount of labeled oligopeptide produced to the amount produced by said polypeptide or polypeptide complex under unmodified conditions, and, thereby
   c) optimizing in vitro production of a labeled oligopeptide.
Embodiment 22: A kit for in vivo synthesis of a labeled oligopeptide comprising an expression vector according to embodiment 15 and an expression vector encoding at least one further NRPS amino acid module.
Embodiment 23: A kit for in vitro synthesis of a labeled oligopeptide comprising a polypeptide or polypeptide complex according to any one of embodiments 1 to 10 and at least one further NRPS amino acid module.
Embodiment 24: A method for optimizing in vivo production of a labeled oligopeptide, comprising
   a) incubating a host cell comprising a polypeptide or polypeptide complex variant and/or an expression vector comprising a polynucleotide variant according to the present invention under conditions suitable for production of said labeled oligopeptide,
   b) comparing the amount of labeled oligopeptide produced to the amount produced by a host cell comprising an unmodified polypeptide or polypeptide complex according to any one of embodiments 1 to 10 and/or an unmodified expression vector according to embodiment 15, and, thereby
   c) optimizing in vivo production of a labeled oligopeptide.
Embodiment 25: A method for optimizing in vitro production of a labeled oligopeptide, comprising
   a) incubating a variant of a polypeptide or polypeptide complex according to any one of embodiments 1 to 10 under conditions suitable for production of said labeled oligopeptide,
   b) comparing the amount of labeled oligopeptide produced to the amount produced by the unmodified polypeptide or polypeptide complex, and, thereby
   c) optimizing in vitro production of a labeled oligopeptide.

All references cited in this specification are herewith incorporated by reference with respect to their entire disclosure content and the disclosure content specifically mentioned in this specification.

### Figure Legends

Figure 1: Constructs encoding fusion NRPSs consisting of one module from the Tyrocidine-cluster (incorporating the indicated amino acids), a linker C-domain and the Indigoidine-module. NRPS expression was driven from an IPTG-inducible promoter.
Figure 2: Restriction digest (EcoRI) of three different NRPSs that are putative synthetases for respective Indigoidine-fusion peptides.
Figure 3: SDS-PAGE of three different NRPSs that are putative synthetases for respective Indigoidine-fusion peptides. Annotation: pPW03 = Phe-Ind, pPW04 = Asn-Ind, pPW05 = Val-Ind. Arrows indicate the position the expected bands indicating expression of the different synthetic NRPSs.
Figure 4: Comparison of Valine-Indigoidine fusion NRP and Indigoidine-control by Thin Layer Chromatography (TLC). 3 different biological replicates of the fusion peptide and technical replicates of the indigoidine control are shown, alongside with the DiMethylSulfoxid (DMSO)-control .Note: All samples were solved in DMSO during the purification prior to the TLC.
Figure 5: TLC of three technical replicates of Orn-Val-Ind with two technical Indigoidine controls. Orn-Val-Ind shows a slower migration behavior compared to Indigoidine control on silica-gel with Dichloromethane as mobile phase.

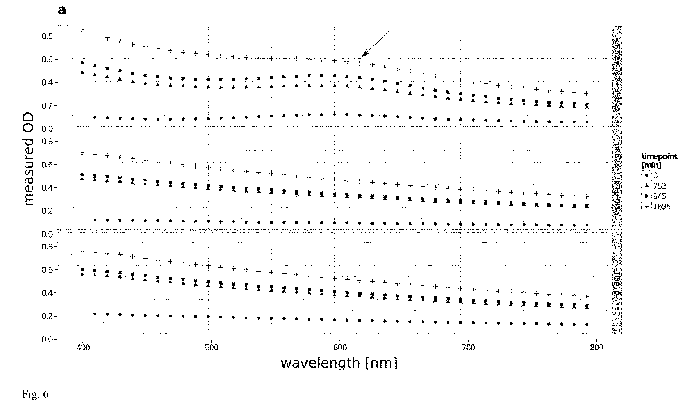

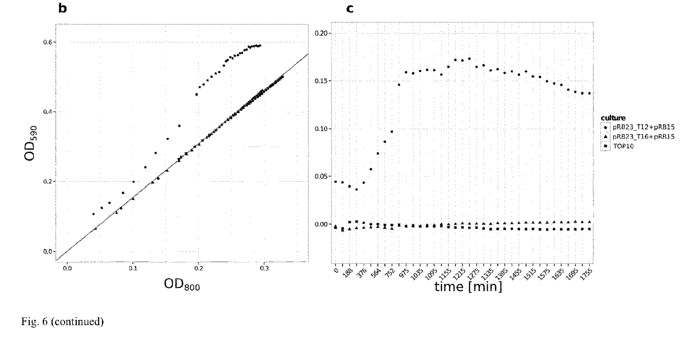

Figure 6: Quantitative indigoidine assay by Optical Density measurements. For quantitative assessment of indigoidine production OD values at two wavelengths are considered: the sensitive OD value at 590 nm is superposed by the absorption by cell matter as well as indigoidine accumulating in the culture media. For this reason a second robust OD value is taken at 800 nm which is only affected by absorption from cell matter. a) Absorption spectra for a positive indigoidine producing strain of TOP10 carrying the indigoidine synthetase indC_T12 (pRB23_T12) and the PPTase sfp (pRB15) as well as for two different types of negative controls: TOP10 without additional plasmids and with an unfunctional indigoidine synthetase indC_T16 (pRB23_T16) and sfp. The arrow marks a peak between 550 nm and 650 nm for the indigoidine producing stain. b) When the sensitive OD at 590 is analyzed with respect to the robust OD at 800 nm. For non indigoidine producing control strains TOP10 and TOP10 with pRB23_T16 and with pRB15 these values depend proportionally on each other.

Thereby a specific delta value can be derived which is used for the calculation of the indigoidine produced. c) The derived concentration of indigoidine in the inspected culture volume over time is shown. Both negative results show no indigoidine production whereas TOP10 with pRB23_T12 and pRB15 show a increase in indigoidine within the first 16.5 h after incubation leading to a steady state. All plots are derived from measurements taken under similar conditions (37 °C, Luria Broth media, 200 µl culture volume).

The following Examples shall merely illustrate the invention. They shall not be construed, whatsoever, to limit the scope of the invention.

### Example 1: Generation of NRPS peptide - indigoidine fusions

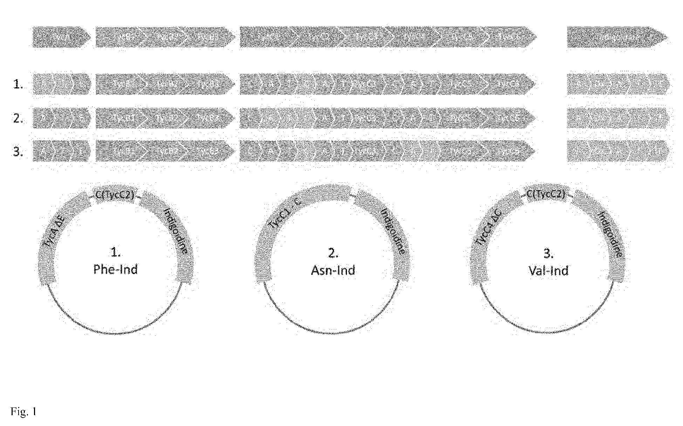

In order to create fusion-NRPSs consisting of a module derived from the Tyrocidine-cluster and the IndC-module that is responsible for the formation of Indigoidine, we assembled the three constructs as depicted in Fig. 1.

Some days after Gibson Assembly of the constructs and Transformation of E. coli BAP1-cells (BAP1 cells express the Sfp, a PPTase with broad substrate specificity necessary for NRPS activation) with the NRPS-expressing plasmids, we could detect blue colonies on our plates for all three different synthetic NRPS expression constructs (Phe-Ind, Asn-Ind and Val-Ind); Occurance of blue colonies directly on plates without any IPTG-induction is due to the leakiness of the IPTG-inducible promoter used for driving NRPS expression). Successful assemblies of NRPS-expressing plasmids present in the blue colonies were further confirmed by restriction digest and sequencing.

### A) Validation of the genotype

All constructs were digested with EcoRI, which should lead to fragments of the following expected sizes:
- Phe-Ind: 5858 and 3183
- Asn-Ind: 5858 and 3582
- Val-Ind: 5858 and 3105

Restriction digest was carried out with 1µl enzyme (EcoRI from New England Biolabs) and 200ng to 1000ng DNA per reaction, reaction volume was 20µl with 2µl 10x CutSmart buffer from New England Biolabs. Samples were mixed with 4µl 6x loading dye and electrophoresis was conducted in 0.8% agarose gel, with 10µl Ethidiumbromide, at 100V for 40 minutes. As standard, 1kb+ gene ruler from Fermentas was used.

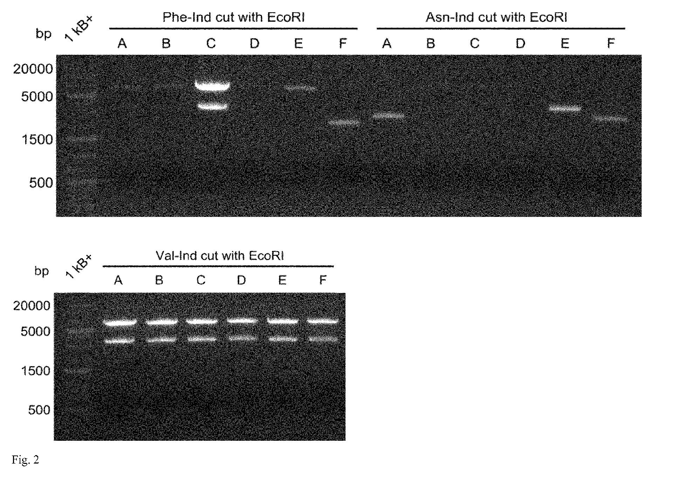

As one can clearly see in figure 2, clones A, B, C and D for Phe-Ind, samples B, C and D for Asn-Ind and all samples for Val-Ind show the expected restriction pattern.

### B) Validation of Expression

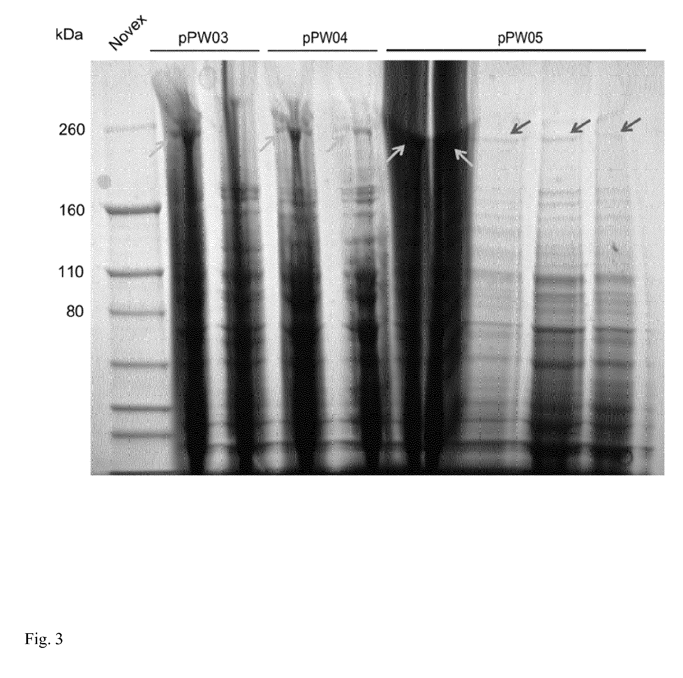

Blue colonies were subsequently inoculated in LB media and NRPS-expression was induced using 1 mM IPTG. Expression of the full-length, fusion NRPS-Protein was confirmed by SDS-PAGE followed by coomassie staining. Figure 3 shows the coomassie staining for the fusion-NRPSs that are capable of synthesizing the Indigoidine-labelled NRPs. According to calculated protein mass, the expected full-length-NRPS bands would appear at (IndC alone has a size of 145kDa):
- Phe-Ind-NRPS: 247kDa
- Asn-Ind-NRPS: 261kDa
- Val-Ind-NRPS: 242kDa

As the gel in figure 3 shows, the obtained bands match well with what has been expected. As pPWO5 containing clones showed both, the clearest band at the expected size on the SDS page indicating successful expression of the Val-IndC NRPS as well as a dark-blue coloring of the corresponding liquid culture, we analyzed these clones further.

### C) Characterization of the synthetic Val-Ind NRPS encoded on pPW05

As mentioned beforehand, liquid cultures containing BAP1 cells expressing the Val-Ind fusion NRPS turned blue after induction with 1mM IPTG. On plates colonies turned blue after several days without induction, due to leaky expression of the IPTG inducible promoter.

In order to show that NRPS expression and NRP production can be performed in different E. coli strains, we transformed the pPW05 plasmid in TOP10 cells and co-expressed the Sfp plasmid in trans from a separate construct.

Finally, we wanted to show that Indigoidine labeled NRPs can easily be characterized by thin-layer chromatography in order to show functioning of the corresponding, synthetic NRPS.

Therefore, the Valine-Indigoidine NRP was purified from BAP1 cells. 1ml of induced, blue culture was therefore spun down at full speed (14,000rpm) for 20 minutes. The blue pellet was washed in 1ml of methanol and centrifuged once more for 5 minutes at 14,000rpm. Methanol was discarded and the blue pellet dissolved in 200-400µl DMSO (Yu et al. (2013), J Ind Microbiol Biotechnol 40: 159-168).

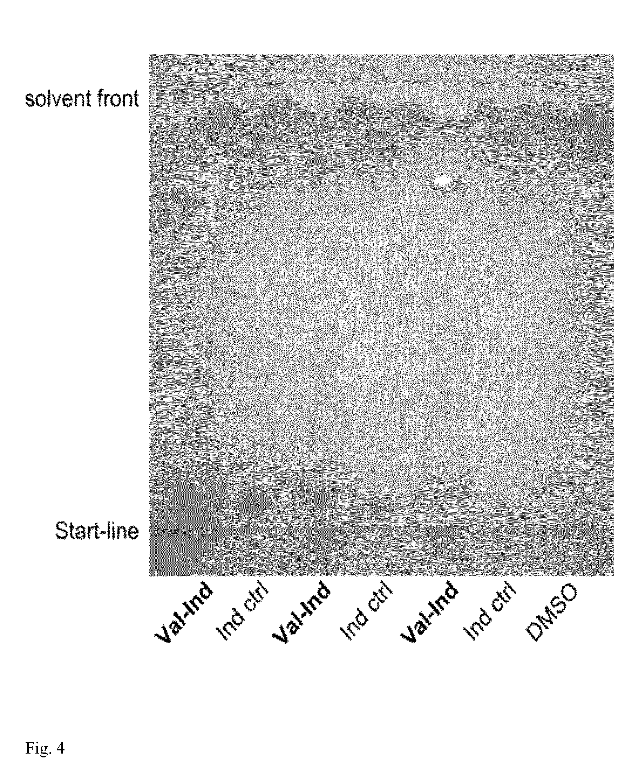

Valine-Indigoidine has a bigger mass than indigoidine alone, the Valine-Indigoidine fusion NRP is expected to migrate slower on the TLC. Using three different biological replicates of the produced NRP and technical replicates of an Indigoidine control (Figure 4), we could show that the produced NRP indeed shows a slower migration behavior.

### D) Confirmation with an Orn-Val-Ind oligopeptide

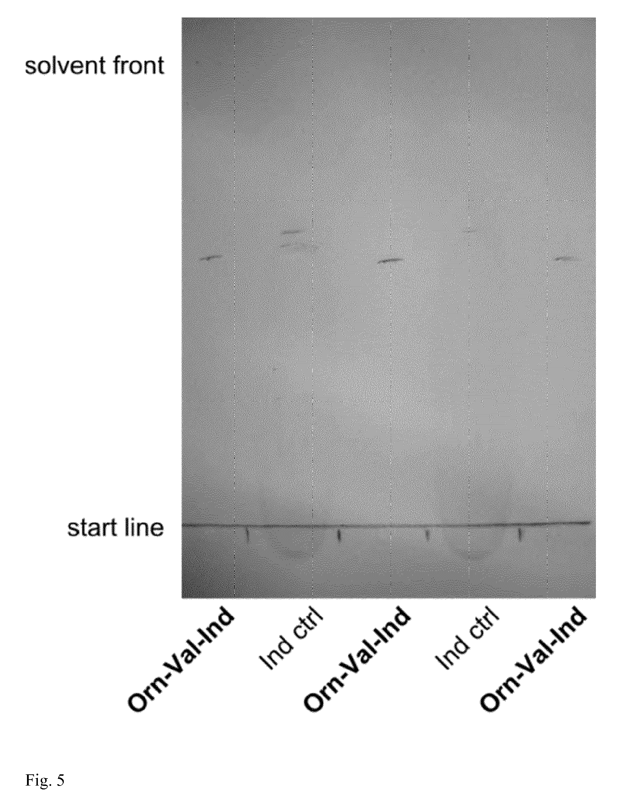

With the aforementioned protocols for purification and TLC, we were able to confirm the successful expression of an Orn-Val-Ind fusion peptide, which shows slower migration on the TLC compared to Indigoidine control (Figure 5).

### Example 2: Quantitative Indigoidine Assay

### OD Measurement

We prepared a pre-culture in 96 well plates with 100 µl of LB-media with the respective antibiotics (chloramphenicol and kanamycin) and picked colonies from every positive plate from a cotransformation experiment.where an indC-expression construct was transformed alongside with a PPTase-expression construct. We incubated the pre-cultures for 24 hours at 37 °C and inoculated the measurement plate with 20 ul of the pre-culture and 180µl of LB-medium. We measured the absorbance from 400 nm to 800 nm in intervals of 10 nm for each well every 30 minutes for 30 hours at 30 °C in a Tecan infinite M200 plate reader. For the measurement, we used Greiner 96 well flat black plates with a clear lid.

### Data Analysis

Indigoidine has a maximum absorption at a wavelength about 590 nm. Since usually the cell density is measured at 600 nm we had to find another method to be able to track both the optical density of the liquid culture and the contribution of indigoidine to the absorption at 590 nm.

For the analysis we basically used the OD values at two wavelengths: The OD590 for the absorption of indigoidine and liquid culture and the OD800 as a robust wavelength to measure the cell density without the influence of the indigoidine absorption spectrum. Assuming that in a normal liquid culture without indigoidine OD590=δ*OD800 (Myers et al. (2013), Bmc Biophysics 6: 4), we used a negative control (TOP10 cotransformed with a PPTase and an unfunctional indigoidine synthetase variant) to determine δ. If we now take the OD590 of our indigoidine producing liquid cultures and substract δ*OD800 we get the absorption of indigoidine without the background of the liquid culture. We are now able to quantitatively observe the indigoidine production of a liquid culture over time as well as the indigoidine production in relation to the cell growth when comparing the OD590 of indigoidine with the OD800 of the cells.

**Table 2: DNA sequences used in the Examples:**

| **Sequence name** | **Brief Description** | **SEQ ID NO** | **encoded polypeptide SED ID NO** |
|---|---|---|---|
| indC | native indigoidine synthetase indC from Photorhabdus luminescens laumondii TT01 | 1 | |
| indC-ccdB | engineered and functional indC from P. luminescens where we replaced the native T-domain with a ccdB gene which is toxic to normal E. coli cells. We used this variant to easily exchange T-domains avoiding any negative clones (cloning background). | 2 | |
| indC-T2 | engineered and functional indC from P. luminescens where we replaced the native T-domain with the T-domain of the bpsA indigoidine synthetase from Streptomyces lavendulae lavendulae ATCC11924 | 3 | |
| indC-T8 | engineered and functional indC from P. luminescens where we replaced the native T-domain with the T-domain of the plu2642 protein from P. luminescens | 4 | |
| indC-T6 | engineered and functional indC from P. luminescens where we replaced the native T-domain with the T-domain of the delH4 protein from Delftia acidovorans SPH-1 | 5 | |
| indC-T10 | engineered and functional indC from P. luminescens where we replaced the native T-domain with a synthetic T-domain of our own design (variant 1) | 6 | |
| indC-T12 | engineered and functional indC from P. luminescens where we replaced the native T-domain with a synthetic T-domain of our own design (variant 3) | 7 | |
| indC-T13 | engineered and functional indC from P. luminescens where we replaced the native T-domain with a synthetic T-domain of our own design (variant 4) | 8 | |
| indC-T14 | engineered and functional indC from P. luminescens where we replaced the native T-domain with a synthetic T-domain of our own design (variant 5) | 9 | |
| plu2642 | gene of unknown function from P. luminescens laumondii TT01; Pfam prediction suggests a single module NRPS with glutamine specificity and and an amino acid sequence being similar to other Indigoidine-synthetase sequences | 10 | |
| Asn-Ind | NRPSase being a synthetase of a fusion peptide consisting of Asparagine and Indigoidine | 11 | 27 |
| ccdB-Ind | Construct that enables easy cloning of NRPS modules in front of Indigoidine module through the exchange of ccdB. | 12 | |
| Orn-Val-Ind | NRPSase synthesizing a Indigoidine-tagged Dipeptide consisting of Ornithine and Valine | 13 | 28 |
| Orn-Val-Val-Ind | NRPSase synthesizing a Indigoidine-tagged Tripeptide consisting of Ornithine and two Valines | 14 | 29 |
| Phe-Ind | NRPSase being a putative synthetase of a fusion peptide consisting of Phenylalanine and Indigoidine | 15 | 30 |
| Phe-Orn-Leu-Ind | NRPSase synthesizing a Indigoidine-tagged Tripeptide consisting of Phenylalanine, Ornithine and Leucine | 16 | 31 |
| Phe-Orn-Leu-Val-Ind | NRPSase synthesizing a Valine-Indigoidine-tagged Tripeptide consisting of Phenylalanine, Ornithine and Leucine. Valine is here used as spacer. | 17 | 32 |
| Pro-Leu-Ind | NRPSase synthesizing a Indigoidine-tagged Dipeptide consisting of Proline and Leucine | 18 | 33 |
| Pro-Leu-Val-Ind | NRPSase synthesizing a Valine-Indigoidine-tagged Dipeptide consisting of Proline and Leucine. Valine is here used as spacer. | 19 | 34 |
| TycA | Entire gene from the Tyrocidine-cluster. TycA is one module consisting of an A-, T- and E-domain | 20 | |
| TycB | Entire gene from the Tyrocidine-cluster. TycB consists of 3 modules. | 21 | |
| TycC | Entire gene from the Tyrocidine-cluster. TycC consists of 6 modules, in the final module of TycC6, Tyrocidine is cyclized | 22 | |
| Val-Ind | NRPSase being a synthetase of a fusion peptide consisting of Valine and Indigoidine. Due to its sterical advantages, Valine may be used as a spacer for other tags. | 23 | 35 |
| Val-Val-Ind | NRPSase synthesizing a Indigoidine-tagged Dipeptide consisting of two Valine-monomers. | 24 | 36 |
| C(TycC2)-Ind | Minimal construct -requires addition of at least one A and T domain | 26 | 37 |

## Claims

1. A polypeptide or polypeptide complex, comprising at least one non-ribosomal peptide synthesis (NRPS) amino acid module functionally connected to at least one pigment module.

2. The polypeptide or polypeptide complex of claim 1 or 2, wherein the pigment module is an NRPS pigment module.

3. The polypeptide or polypeptide complex of claim 2, comprising the C domain of the at least one NRPS amino acid module and the at least one NRPS pigment module as a fusion polypeptide.

4. The polypeptide or polypeptide complex of any one of claims 1 to 3, wherein the pigment module is an indigoidine synthetase.

5. A labeled oligopeptide comprising a non-naturally attached NRPS pigment or/and polyketide pigment.

6. The labeled oligopeptide of claim 6, wherein the pigment is indigoidine.

7. A polynucleotide encoding a fusion polypeptide according to claim 3.

8. A vector, preferably an expression vector, comprising the polynucleotide according to claim 7,

9. A host cell comprising the polypeptide or polypeptide complex according to any one of claims 1 to 4 and/or the polynucleotide according to claim 7, and/or the vector according to claim 8.

10. An in vitro method of producing a labeled oligopeptide, comprising:
a) incubating a polypeptide or polypeptide complex according to any one of claims 1 to 3 with appropriate amino acid substrates,
b) thereby producing a labeled oligopeptide.

11. An in vivo method of producing a labeled oligopeptide, comprising:
a) incubating a host cell comprising the polypeptide or polypeptide complex according to any one of claims 1 to 3 and/or the expression vector according to claim 8,
b) thereby producing a labeled oligopeptide.

12. A labeled oligopeptide obtainable by the method according to claim 10 or by the method according to claim 11.

13. A kit (i) for in vivo synthesis of a labeled oligopeptide comprising an expression vector according to claim 8 and an expression vector encoding at least one further NRPS amino acid module, or (ii) for in vitro synthesis of a labeled oligopeptide comprising a polypeptide or polypeptide complex according to any one of claims 1 to 3 and at least one further NRPS amino acid module.

14. A method for optimizing in vivo production of a labeled oligopeptide, comprising
a) incubating a host cell comprising a variant of a polypeptide or polypeptide complex according to any one of claims 1 to 3 and/or an expression vector comprising a variant of a polynucleotide according to claim 7 under conditions suitable for production of said labeled oligopeptide,
b) comparing the amount of labeled oligopeptide produced to the amount produced by a host cell comprising an unmodified polypeptide or polypeptide complex according to any one of claims 1 to 3 and/or an unmodified expression vector according to claim 8, and, thereby
c) optimizing in vivo production of a labeled oligopeptide.

15. A method for optimizing in vitro production of a labeled oligopeptide, comprising
a) incubating a variant of a polypeptide or polypeptide complex according to any one of claims 1 to 3 under conditions suitable for production of said labeled oligopeptide,
b) comparing the amount of labeled oligopeptide produced to the amount produced by the unmodified polypeptide or polypeptide complex, and, thereby
c) optimizing in vitro production of a labeled oligopeptide.
